# EUROPÄISCHE PATENTANMELDUNG

(11) **EP 1 739 078 A1**
(43) Veröffentlichungstag der Anmeldung: **03.01.2007**
(21) Anmeldenummer: 05011620.1
(22) Anmeldetag: 30.05.2005
(51) Int. Cl.: C07C 275/40, C07C 275/32, C07C 275/30, A61K 31/17

(54) **C5a-Rezeptor-Antagonisten**

(71) Anmelder: Jerini AG, 10115 Berlin (DE)
(72) Erfinder: Schnatbaum, Karsten., D-10245 Berlin (DE); Scharn, Dirk., D-13507 Berlin (DE); Locardi, Elsa., D-10551 Berlin (DE); Polakowski, Thomas., D-10439 Berlin (DE); Richter, Uwe., D-12489 Berlin (DE); Hummel, Gerd., D-13357 Berlin (DE); Reineke, Ulrich, D-10249 Berlin (DE)
(74) Vertreter: Bohmann, Armin K.

(57) **Zusammenfassung**

Die vorliegende Erfindung betrifft eine Verbindung, bevorzugtererweise ein C5a-Rezeptorantagonist, mit der folgenden Struktur (IV): 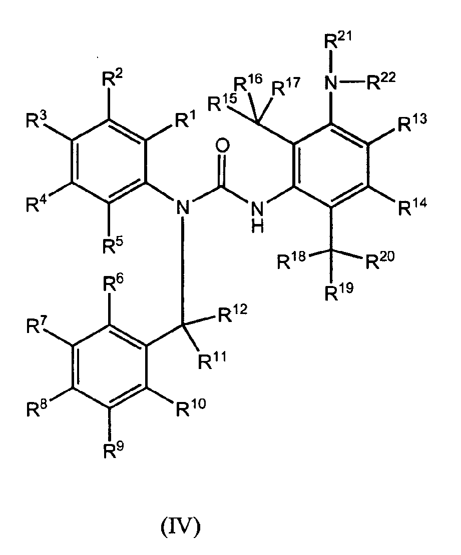 wobei R1, R2, R3, R4, R5, R6, R7, R8, R9, R10, R11, R12, R13, R14, R15, R16, R17, R18, R19, R20, R21 und R22 jeweils einzeln und unabhängig voneinander ausgewählt ist aus der Gruppe, die H, Alkyl, substituiertes Alkyl, Alkenyl, substituiertes Alkenyl, Alkynyl, substituiertes Alkynyl, Cycloalkyl, substituiertes Cycloalkyl, Heterocyclyl, substituiertes Heterocyclyl, Aryl, substituiertes Aryl, Heteroaryl, substituiertes Heteroaryl, Arylalkyl, substituiertes Arylalkyl, Heteroarylalkyl, substituiertes Heteroarylalkyl, Alkoxyl, substituiertes Alkokyl, Aryloxy, substituiertes Aryloxy, Arylalkyloxy, substituiertes Arylalkyloxy, Acyloxy, substituiertes Acyloxy, Halogen, Hydroxyl, Nitro, Cyano, Acyl, substituiertes Acyl, Mercapto, Alkylthio, substituiertes Alkylthio, Amino, substituiertes Amino, Alkylamino, substituiertes Alkylamino, Dialkylamino, substituiertes Dialkylamino, cyclisches Amino, substituiertes cyclisches Amino, Carbamoyl (-CONH₂), substituiertes Carbamoyl, Carboxyl, Carbamat, Alkoxycarbonyl, substituiertes Alkoxycarbonyl, Acylamino, substituiertes Acylamino, Sulfamoyl (-SO₂NH₂), substituiertes Sulfamoyl, Haloalkyl, Haloalkyloxy, -C(O)H, Trialkylsilyl und Azido umfaßt.

## Beschreibung

Die vorliegende Erfindung betrifft Antagonisten des C5a-Rezeptors sowie Verwendungen derselben.

Neben dem adaptiven Immunsystem gibt es ein stammesgeschichtlich deutlich älteres System zur Abwehr von Infektionen. Dieses System wird Complementsystem genannt und besteht aus mehr als 30 löslichen und membrangebundenen Proteinen. Das Complementsystem kann zusammen mit der adaptiven Immunantwort oder eigenständig aktiv werden, um z.B. pathogene Bakterien zu töten. Eine überschiessende Aktivierung bzw. nicht hinreichende Regulierung des Complementsystems wird mit einer großen Zahl von entzündlichen Erkrankungen in Verbindung gebracht wie z.B. septischer Schock, Reperfusionsschaden, rheumatoide Arthritis, Transplantatabstossung, akutes Lungenversagen (adult respiratory distress syndrome, ARDS), systemischer Lupus erythematodes (SLE) und Glomerulonephritis. Übersichten über die Beziehungen zwischen dem Complementsystem und Erkrankungen sind in großer Zahl publiziert worden (z.B.: Kirschfink 1997 Immunopharmacology 38: 51; Markides 1998 Pharmacological Reviews 50: 59, Walport 2001 The New England Journal of Medicine 344: 1140, Walport 2001 The New England Journal of Medicine 344: 1058).

Die Aktivierung des Complementsystems kann über drei unterschiedliche Wege stattfinden. Diese werden klassischer, alternativer und Mannose-binding Lectin (MBL)-Weg genannt. Alle Wege verlaufen über die sequentielle Prozessierung - und damit Aktivierung - von inaktiven Proformen von Proteasen. Da die jeweils aktivierte Protease die nächste Proform aktivieren kann, erhält man eine Verstärkung der auslösenden Reaktion vergleichbar mit der Gerinnungskaskade. Ein Überblick über das Complementsystem ist bei Sim und Laich (2000 Biochemical Society Transactions 28: 545) zu finden.

Zu den wichtigsten Proteinen, die während der Complementaktivierung entstehen, zählen C3a, C3b, C5a und C5b, auf die im Folgenden näher eingegangen wird.

C3b ist entscheidender Bestandteil einer zentralen Protease der Complementkaskade, genauer der C5-Konvertase, die sowohl beim klassischen als auch beim alternativen Weg der Aktivierung des Complementsystems beteiligt ist. Der MBL-Weg führt ebenfalls über die Konvertasen des klassischen Weges und somit auch über die C5-Konvertase. Die C5-Konvertase ist verantwortlich für den Fortgang der Complementkaskade und katalysiert die Spaltung von C5. Neben seiner Funktion als Bestandteil der C5-Konvertase wird C3b kovalent an die Oberfläche von z.B. Bakterien gebunden, die dadurch bevorzugt von z.B. Makrophagen aufgenommen werden können. Ähnliches gilt auch für die Klärung von Immunkomplexen.

C3a ist das kleine Fragment, das neben C3b durch die Spaltung von C3 entsteht. C3a ist ein verhältnismäßig schwaches Chemokin und zählt zu den Anaphylatoxinen.

C5b entsteht durch Spaltung von C5. Dieses Spaltprodukt ist der Ausgangspunkt für die Bildung des Membrane-Attack-Complex (MAC). Der MAC bildet eine Pore, durch die die Plasmamembran von Bakterien aber auch diejenige von körpereigenen Zellen perforiert werden kann. Dadurch kann es zur Lyse der perforierten Zellen kommen.

C5a ist das 74 Aminosäuren große, N-terminale Spaltprodukt der α-Kette des Plasmaproteins C5 und wird durch die proteolytische Aktivität der C5-Konvertase freigesetzt. C5a wird von seinem Rezeptor, der als C5a-Rezeptor oder auch CD88 oder C5R1 bezeichnet wird, mit hoher Affinität gebunden und löst eine Vielzahl proinflammatorischer Effekte aus. Es ist eines der stärksten Chemokine und gehört wie C3a zu den Anaphylatoxinen. Der C5a-Rezeptor ist auf einer Vielzahl von Zellen zu finden, insbesondere auf Neutrophilen, Makrophagen, Zellen der glatten Muskulatur und Endothelzellen.

Die Freisetzung von C5a wird direkt oder indirekt für eine Vielzahl von akuten und chronischen Erkrankungen bzw. Symptomen verantwortlich gemacht. Septischer Schock, SIRS (systemic/severe inflammatory response syndrome), MOF (multi organ failure) oder ARDS (acute respiratory dystress syndrome) seien als Beispiele für akute Erkrankungen bzw. Symptome und rheumatoide Arthritis als Beispiel für eine chronische Indikation genannt. Einige systemische Erkrankungen haben lokale Manifestationen zur Folge, die mit einem C5a Rezeptor-Antagonisten behandelt werden könnten.

Schließlich sind C5a und sein Rezeptor potentielle Zielmoleküle auch bei Infektionen und damit im Zusammenhang stehenden Erkrankungen, d.h. inflammatorischen Erkrankungen wie beispielsweise Myokarditis.

Infolge dieser umfänglichen Beteiligung von C5a und seinem Rezeptor an verschiedenen Erkrankungen und Symptomen wurden im Stand der Technik verschiedene Strategien zur Behandlung derselben entwickelt.

C5a-Rezeptor-Antagonisten, auf die im Folgenden detaillierter eingegangen wird, können neben der intrakorporalen Therapie grundsätzlich auch für eine extrakorporale Behandlung von Blut bzw. Organen verwendet werden. Beispielsweise soll auf die Behandlung von Dialyse-Patienten oder anderen Patienten verwiesen werden, die eine Schädigung dadurch erfahren, dass z.B. das Complementsystem an artifiziellen Oberflächen aktiviert wird. Außerdem kann eine derartige Behandlung eingesetzt werden, um den Reperfusionsschaden einzudämmen, der eintritt, wenn Patienten eine Revaskularisierung erfahren oder sich anderen Verfahren, die die Durchblutung steigern bzw. erneut ermöglichen, wie z.B. PTCA oder Thrombolyse, oder operativen Eingriffen unterziehen, bei denen die Zirkulation des Blutes ganz oder teilweise unterbrochen wurde, wie dies z.B. bei Aneurisma-Operationen der Fall ist. Ein neuer Aspekt ist die extrakorporale Behandlung von Organen, die für eine Transplantation verwendet werden sollen.

Der C5a-Rezeptor (C5a-Rezeptor) stellt ein besonders interessantes Zielmolekül für die Entwicklung eines Mittels zur Behandlung C5a vermittelter Erkrankungen und Symptome dar. Das wird dadurch belegt, dass Mäuse, denen der Rezeptor fehlt, keinen auffälligen Phänotyp besitzen (Hopken et al. 1996 Nature 383: 86). Daraus wird ersichtlich, dass die Blockade des C5a-Rezeptors vermutlich keine schädlichen Konsequenzen hat. Lediglich eine erhöhte Empfindlichkeit gegenüber Pseudomonadeninfektionen ist bei den Rezeptor negativen Mäusen beobachtet worden. Das bedeutet, dass die Complementkaskade mit ihren nützlichen Funktionen zur Abwehr von Krankheitserregern (MAC-Bildung) und dem Abbau von Immunkomplexen auch bei vollständiger Inaktivierung des Rezeptors weitestgehend ungehindert ablaufen kann. Trotzdem sind diese Tiere z.B. gegen einen Reperfusionsschaden geschützt. Damit erscheint die Inhibition des C5a-Rezeptors günstiger als z.B. ein Eingriff in die Complementkaskade auf der Ebene oberhalb der Spaltung von C5 oder darüber.

Verschiedene Ansätze zur Entwicklung von C5a-Rezeptor-Antagonisten wurden bisher verfolgt. Dabei wurden rekombinante Proteine, Peptide und auch eine geringere Zahl von Small Molecules in der Literatur offenbart.

Beispiele für C5a-Rezeptor-Antagonisten auf der Basis von rekombinanten Proteinen sind CGS 32359 (Ciba-Geigy, Pellas et al. 1998 Journal of Immunology 160: 5616), ΔpIII-A8 (Heller et al. 1999 Journal of Immunology 163: 985) bzw. Antikörper, die rekombinanten oder aber auch nicht-rekombinanten Ursprungs sein können (Huber-Lang et al. 2001 FASEB Journal 15: 568). Diese C5a-Rezeptor-Antagonisten sind Proteine und entsprechend kostspielig herzustellen. Sie zeichnen sich durch eine verhältnismäßig hohe Affinität und Spezifität aus, haben aber den Nachteil einer hohen Immunogenität. Zudem sind Proteine nur durch vergleichsweise aufwendige Verfahren wie z.B. intravenöse oder subkutane Injektionen effizient zu verabreichen.

Peptidische Antagonisten sind auf unterschiedlichen Wegen entwickelt worden. Z.B. wurden Sequenzinformationen aus dem C-terminalen Bereich von C5a zur Entwicklung von peptidischen Antagonisten verwendet. Peptide als therapeutisch nutzbare Antagonisten des C5a-Rezeptors haben im Vergleich zu Proteintherapeutika den Vorteil geringerer Produktionskosten und geringerer Immunogenität. Peptidische Antagonisten wurden in großer Zahl beschrieben. Beispiele für peptidische C5a-Rezeptor-Antagonisten bzw. partielle Agonisten sind unter anderem in folgenden Patentanmeldungen bzw. Patenten beschrieben: US 4692511, US 5663148, WO 9009162, WO 9211858, WO 9212168, WO 9221361, WO 9407518, WO 9407815, WO 9525957, WO 9606629, WO 9900406, WO 9913899, WO 03033528, EP01498422 und WO05010030. Nachteile von Peptiden sind aber z.B. eine i.d.R. geringe orale Verfügbarkeit oder ein hoher Syntheseaufwand, was insbesondere bei großen Peptiden bestehend aus mehr als zehn Aminosäuren sehr nachteilig ist. Auch können, insbesondere bei Peptiden, die auf proteinogenen Aminosäuren basieren, Amidbindungen in Peptiden gespalten werden, z.B. durch Proteasen in biologischen Flüssigkeiten.

Für die Therapie chronischer Erkrankungen ist insbesondere die Verwendung oral verfügbarer sogenannter Small Molecules wünschenswert. Darunter versteht man i.d.R. Verbindungen mit einem Molekulargewicht von bis 1000 g/mol, bevorzugterweise bis 500 g/mol, die nur wenige peptidtypische Strukturmerkmale wie z.B. Amidbindungen enthalten. Als Beispiele für derartige Entwicklungen sind unter anderem L-156602 (Merck; Tsuji et al. 1992 Bioscience Biotechnologie and Biochemistry 56: 2034-2036), TAN-2474 (Takeda; JP10182648), RPR120033 (Rhone-Poulenc, Wong et al. 1999 IDrugs 2: 686), W-54011 (Mitsubishi Pharma, Sumichika et al. 2002 Journal of Biological Chemistry 277: 49403) und NGD 2000-1 (Neurogen, Shaw und Hutchison, 220th ACS National Meeting, Washington, DC, August 2000) bekannt geworden. Die Entwicklungen dieser Inhibitoren spiegeln sich in einer Vielzahl von Patentschriften bzw. Anmeldungen wider, z.B. in den Folgenden: W00214265, WO0222556, WO0249993, W003082829, WO03082828, WO03082826, WO03084524, WO04018460, US2004/0048913, US6723743, US2004/0082577, WO04043925, US2004/0116424, US2004/0158067, US6777422, UA2004/0204446, WO03082829, WO05007087, US06858637, US6069172, W00179189, WO02068377, WO03029187, WO02068377 und WO04043925.

Im Folgenden werden die bisher bekannten Small Molecules, die als Agonisten oder Antagonisten mit dem C5a-Rezeptor wechselwirken, genauer beschrieben.

Die ersten Small-Molecule-C5a-Rezeptor-Antagonisten wurden von Merck Sharp and Dohme in den frühen 1990er Jahren entwickelt. Zunächst wurden positiv geladene Moleküle wie z.B. die Verbindungen 1 und 2 identifiziert (Merck, Lanza et al. 1992 J. Med. Chem. 35: 252). Die Verbindungen zeigten moderate Bindungsaktivitäten von 3.3 bzw. 12 µg/ml (= 8.9 bzw. 35.5 µM). Allerdings zeigten sich auch unerwünschte Eigenschaften (Verbindung 7 inhibierte die fMLF-induzierte Neutrophilen-Aktivierung) und keine weitere Entwicklung oder weitere Daten wurden beschrieben. Stattdessen identifizierte Merck eine andere Serie von C5a-bindenden Molekülen (Merck, Laszlo et al. 1997 Bioorg. Med. Chem. Lett. 7: 213) durch Screening einer Inhouse-Verbindungsbibliothek. Die affinsten Moleküle gegen den C5a-Rezeptor waren die Verbindungen 3 und 4. Beide Verbindungen sind aber Agonisten und Versuche, durch Modifikation der Struktur die agonistische Wirkung zu eliminieren, scheiterten. Auch bei anderen Leadserien wie z.B. Benzodiazepine (L-747 981, Flanagan et al. 210th ACS National Meeting, Chicago, Illinois, August 20th-24th 1995) oder Tetrahydroimidazopyridine (L-164 712, Kim et al. 210th ACS National Meeting, Chicago, Illinois, August 20th-24th 1995) gelang dies nicht. In den letzten Jahren ist keine Entwicklung der Firma Merck im Bereich der C5a-Rezeptor-Antagonisten bekannt geworden.

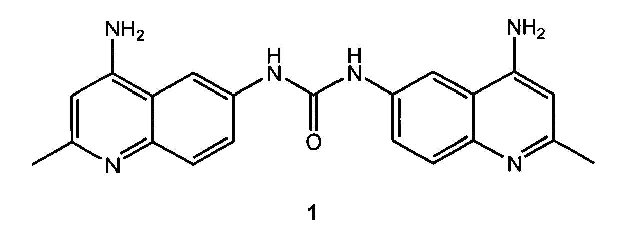

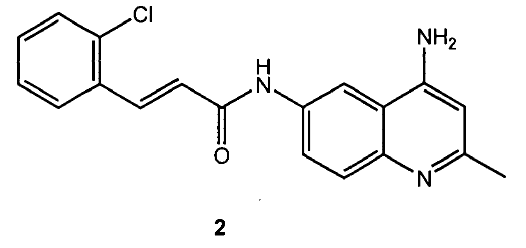

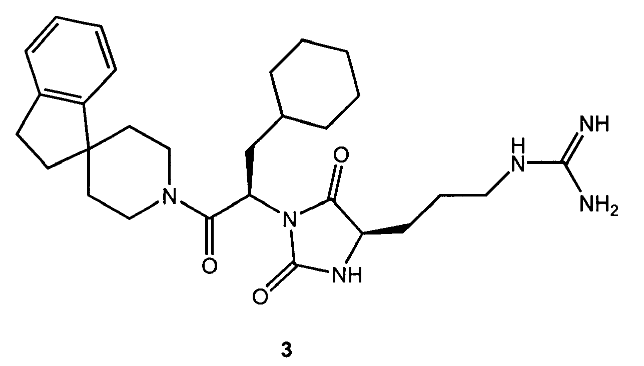

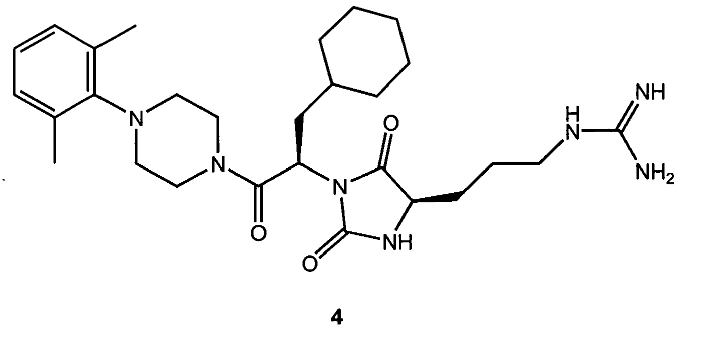

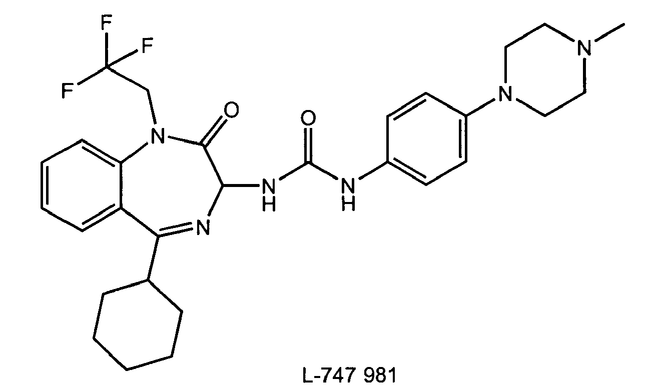

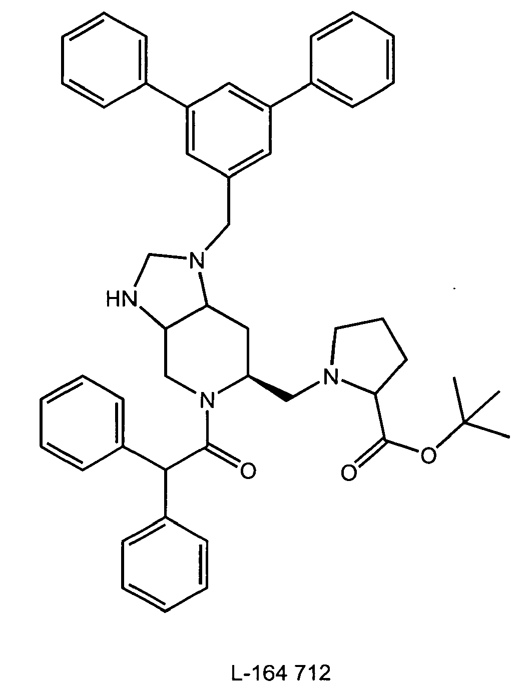

Auch die Firma Rhöne-Poulenc-Rorer hat C5a-Rezeptor-Antagonisten identifiziert und optimiert, aber keine weitere Entwicklung beschrieben (Astles et al. 1997 Bioorg. Med. Chem. Lett. 7: 907). Ein Grund dafür könnte sein, dass sogar die aktivste Verbindung 5 (RPR121154 bzw. RPR120033) nur moderate Aktivität von 0.8 µM zeigt. Die Verbindung enthält zudem eine Guanidin-Einheit, die sich häufig negativ auf orale Verfügbarkeit auswirkt (Reiner et al. 1992 Bioorg. Med. Chem. Lett. 12: 120) und darüber hinaus wurde beschrieben, dass die Verbindung cytotoxisch zu sein scheint (Sumichika et al. 2002 J. Biol. Chem. 277: 49403).

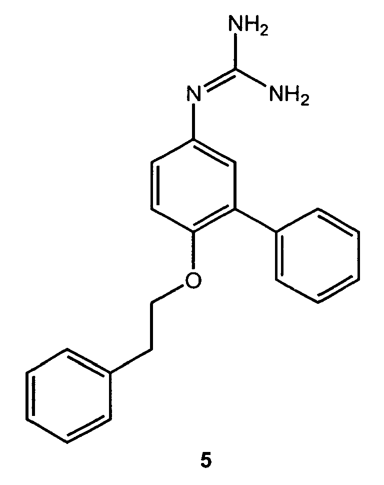

Auch die Firma Takeda beschrieb Verbindungen, die mit dem C5a-Rezeptor wechselwirken (z.B. TAN2474A und TAN2474B). Die Verbindungen besitzen aber potentiell reaktive Keton- und Chinon- oder Dihydrochinon-Gruppen. Eine Weiterentwicklung dieser Verbindungen ist nicht bekannt geworden.

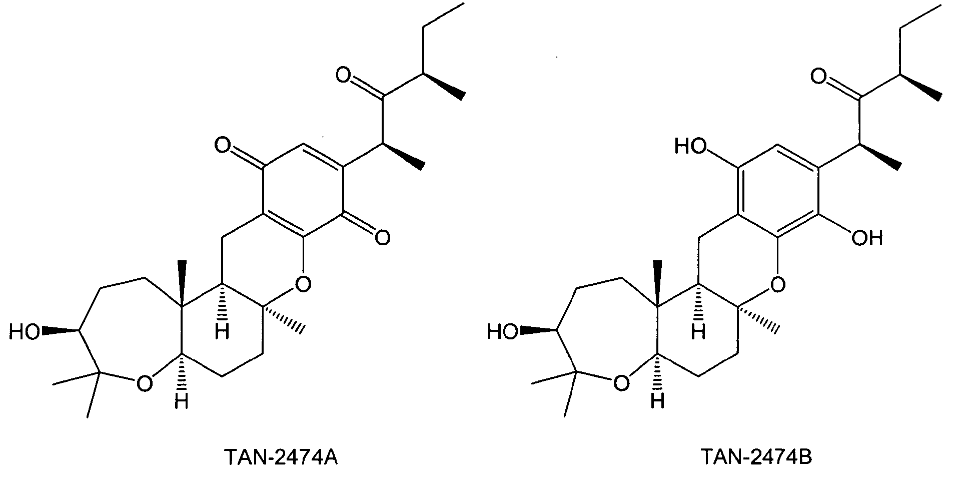

Weitere Small Molecules, von denen eine Wechselwirkung mit dem C5a-Rezeptor beschrieben wurde, sind Verbindungen der Firma DOMPÉ S.P.A., dargestellt z.B. durch die Verbindung **6** (US6069172, W00179189, WO02068377 oder WO03029187). Die Verbindung besitzt aber nur geringe Aktivität in einem Chemotaxis-Assay von 24 µM.

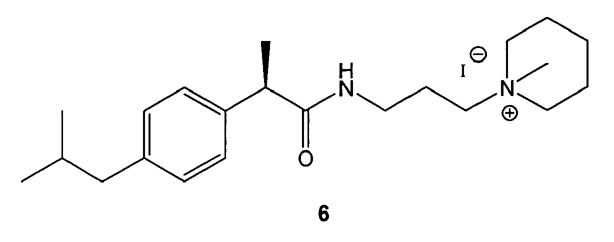

In den letzten Jahren hat die Firma Neurogen eine Reihe von Patentanmeldungen bzgl. C5a-Rezeptor-Antagonisten eingereicht, die z.B. Strukturen wie die Verbindungen **1-4** beinhalten, die z. B beschrieben sind in WO0249993, WO03082829, WO03082828, WO03082826, WO03084524, WO04018460, US2004/0048913, US6723743, US2004/0082577, WO04043925, US2004/0116424, US2004/0158067, US6777422, UA2004/0204446, WO03082829, WO05007087 und US06858637. Biologische Eigenschaften wurden nur für wenige Verbindungen beschrieben. Zu diesen Verbindungen gehören **9** und **10,** für die eine Aktivität in einem Assay, der auf der Inhibierung C5a-getriggerter Calcium-Mobilisierung beruht, von 24 bzw. 465 nM beschrieben wurde. Die Untersuchung der Verbindungen in einem funktionalen Assay der C5a induzierten Enzymfreisetzung von stabil transfizierten Ratten-basophilen Zellen, die den humanen C5a-Rezeptor exprimieren, (Beispiel 32) zeigt aber, dass **9** und **10** hier nur eine geringe Aktivität von 7152 nM bzw. >4760 nM aufweisen. Auch **8,** das unter die Ansprüche des Patents WO03082826 fällt, zeigt im Enzymfreisetzungsassay nur sehr geringe Aktivität (1917 nM).

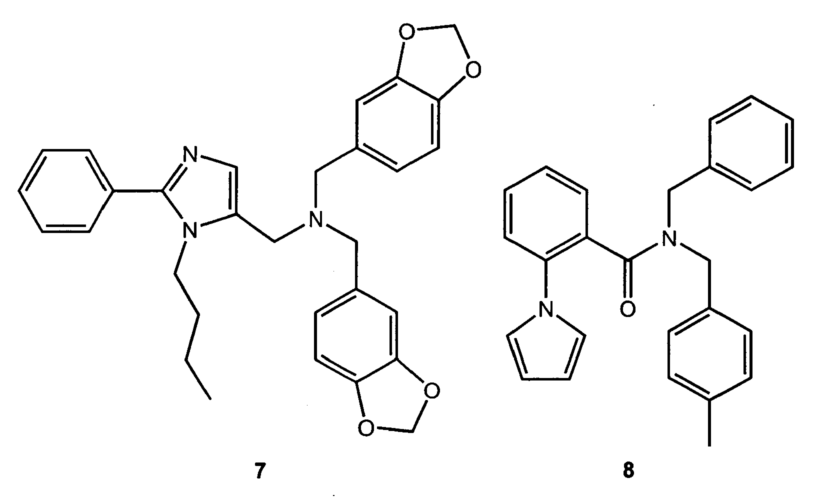

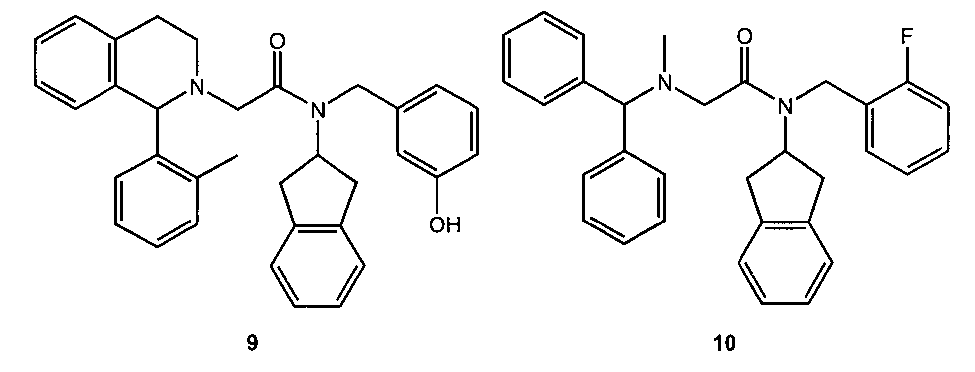

Die Firma Mitsubishi Pharma Corporation hat Patentanmeldungen mit zwei prinzipiell unterschiedlichen Grundstrukturen eingereicht, die durch **11** und **12** (W-54011) repräsentiert werden. Die Verbindung **11** besitzt neben einer geringen Aktivität (Beispiel 32) den Nachteil einer geringen Lagerstabilität (Beispiel 25). Für W-54011 wurde eine sehr hohe biologische Aktivität beschrieben: Man bestimmte einen IC₅₀-Wert von 3.1, 2.7 und 1.6 nM für die rhC5a-induzierte Calcium-Mobilisierung, Chemotaxis und ROS-Freisetzung (Sumichika et al. 2002 J. Biol. Chem. 277: 49403). Sumichika beschrieb die Verbindung als aktiver im Bindungsassay als das Peptidmimetikum PMX53 (ein hexameres, cyclisches Peptid mit der Formel Ac-F[OPchaWR]) und sogar als aktiver als einen anti-C5a-Rezeptor-monoklonalen Antikörper (Sumichika 2004 Curr. Opin. Invest. Drugs 5: 505). Im Enzymfreisetzungsassay gemäß Beispiel 32 besitzt W-54011 einen IC₅₀-Wert, der deutlich größer ist als der IC₅₀-Wert in den anderen, oben beschriebenen Assays, von 90 nM. Trotz dieser immer noch akzeptablen Aktivität besitzt die Verbindung eine Reihe von Nachteilen: Die Löslichkeit in wässrigem PBS-Puffer beträgt nur 0.4 µM und die chemische Synthese erfordert einen enantioselektiven Schritt oder eine Enantiomerentrennung, vorausgesetzt, es soll ein einzelnes Enantiomer eingesetzt werden. In den letzten Jahren wurden keine weiteren Daten bzgl. der Entwicklung der Verbindung W-54011 veröffentlicht.

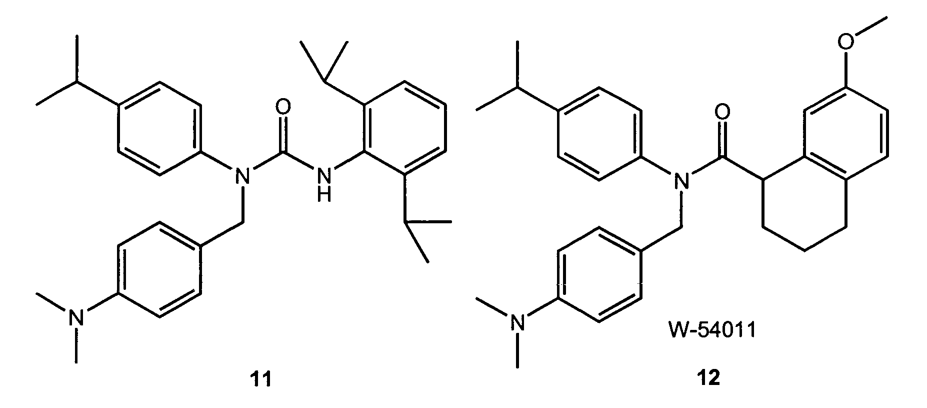

Zusammengefaßt läßt sich somit feststellen, dass alle bisher bekannten Small-Molecule-Inhibitoren - sofern näher beschrieben - zumindest einen der folgenden Nachteile aufweisen: eine geringe Spezifität, agonistische Wirkung, zu niedrige Affinität, geringe Wasserlöslichkeit, unzureichende Lagerstabilität, chemische Reaktivität, unzureichende metabolische Stabilität oder Inhibierung von P450 Enzymen. Für lediglich eines der C5a-inhibitorischen Small Molecules (NGD 2000-1 der Firma Neurogen; Shaw and Hutchison, 220th ACS National Meeting, Washington, DC, August 2000) wurden bisher klinische Studien durchgeführt. Bei diesen Studien konnte die Wirksamkeit der C5a Inhibition für die Indikation rheumatoide Arthritis gezeigt werden (Verbesserung ACR20), jedoch waren die Substanzeigenschaften (z.B. Cytochrom-Inhibition) nicht hinreichend, um höhere Dosen im Menschen zu verwenden und damit eine deutlichere und damit therapeutisch hinreichende Wirkung zu erzielen (Pressemitteilung der Firma Neurogen, www.neurogen.com, 16.06.2004). Es ist also im hohen Maße wünschenswert, dass neue, verbesserte C5a Rezeptor-Antagonisten bereitgestellt werden, die als Small Molecules für die orale Applikation geeignet sind.

Der vorliegenden Erfindung liegt die Aufgabe zugrunde, Antagonisten des C5a-Rezeptors bereitzustellen. Eine weitere der vorliegenden Erfindung zugrundeliegende Aufgabe besteht darin, dass Medikamente bereitgestellt werden, die bei der Behandlung von Krankheitsbildern verwendet werden können, an denen der C5a-Rezeptor und / oder C5a kausal, indirekt oder symptomatisch beteiligt sind/ist.

Die der vorliegenden Erfindung zugrundeliegende Aufgabe wird allgemein gelöst durch eine Verbindung mit einer molaren Masse kleiner als 700, einer aromatischen oder einer heteroaromatischen Gruppe, wobei die Verbindung eine antagonistische Aktivität gegen den C5a-Rezeptor von <5000 nM (IC₅₀) und eine agonistische Aktivität gegen den C5a-Rezeptor von <10% bei 1 µM zeigt.

Erfindungsgemäß wird die Aufgabe in einem ersten Aspekt gelöst durch eine Verbindung, bevorzugterweise einen C5a-Rezeptor-Antagonist, mit der folgenden Struktur (I): 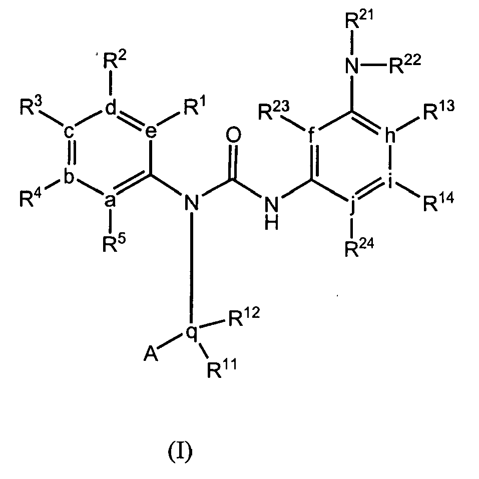 , wobei

A ausgewählt ist aus der Gruppe, die H, Alkyl, substituiertes Alkyl, Cycloalkyl, substituiertes Cycloalkyl, Heterocyclyl, substituiertes Heterocyclyl, Arylalkyl, substituiertes Arylalkyl, Aryl, substituiertes Aryl, Heteroaryl, substituiertes Heteroaryl, cyclisches Amino, und substituiertes cyclisches Amino umfasst,
a, b, c, d, e, f, h, i und j jeweils einzeln und unabhängig voneinander ausgewählt ist aus der Gruppe, die C und N umfasst,

R1, R2, R3, R4, R5, R11, R12, R13, R14, R21 und R22 jeweils einzeln und unabhängig voneinander ausgewählt ist aus der Gruppe, die H, Alkyl, substituiertes Alkyl, Alkenyl, substituiertes Alkenyl, Alkynyl, substituiertes Alkynyl, Cycloalkyl, substituiertes Cycloalkyl, Heterocyclyl, substituiertes Heterocyclyl, Aryl, substituiertes Aryl, Heteroaryl, substituiertes Heteroaryl, Arylalkyl, substituiertes Arylalkyl, Heteroarylalkyl, substituiertes Heteroarylalkyl, Alkoxyl, substituiertes Alkokyl, Aryloxy, substituiertes Aryloxy, Arylalkyloxy, substituiertes Arylalkyloxy, Acyloxy, substituiertes Acyloxy, Halogen, Hydroxyl, Nitro, Cyano, Acyl, substituiertes Acyl, Mercapto, Alkylthio, substituiertes Alkylthio, Amino, substituiertes Amino, Alkylamino, substituiertes Alkylamino, Dialkylamino, substituiertes Dialkylamino, cyclisches Amino, substituiertes cyclisches Amino, Carbamoyl (-CONH₂), substituiertes Carbamoyl, Carboxyl, Carbamat, Alkoxycarbonyl, substituiertes Alkoxycarbonyl, Acylamino, substituiertes Acylamino, Sulfamoyl (-SO₂NH₂), substituiertes Sulfamoyl, Haloalkyl, Haloalkyloxy, -C(O)H, Trialkylsilyl und Azido umfasst,
wobei jeweils 2 Reste einen aliphatischen oder aromatischen Ring miteinander ausbilden können, wobei die Reste ausgewählt sind aus der Gruppe umfassend A, R1, R2, R3, R4, R5, R11, R12, R13, R14, R21, R22, R23 und R24, wobei eine derartige Ringbildung in einem Molekül 0-, 1-, 2- oder 3-fach erfolgen kann,
und für den Fall, dass eines der Atome a, b, c, d, e, f, h, i und/oder j N ist, der dem N benachbarte Rest R1, R2, R3, R4, R5, R13, R14, R23, R24 fehlt oder ausgewählt ist aus der Gruppe, die O, Alkyl und substituiertes Alkyl umfasst,
wobei R23 und R24 jeweils einzeln und unabhängig voneinander ausgewählt ist aus der Gruppe, die Alkyl, substituiertes Alkyl, Alkenyl, substituiertes Alkenyl, Alkynyl, substituiertes Alkynyl, Cycloalkyl, substituiertes Cycloalkyl, Heterocyclyl, substituiertes Heterocyclyl, Aryl, substituiertes Aryl, Heteroaryl, substituiertes Heteroaryl, Alkoxyl, substituiertes Alkokyl, Aryloxy, substituiertes Aryloxy, Acyloxy, substituiertes Acyloxy, Halogen, Hydroxyl, Nitro, Cyano, Acyl, substituiertes Acyl, Mercapto, Alkylthio, substituiertes Alkylthio, Amino, substituiertes Amino, Alkylamino, substituiertes Alkylamino, Dialkylamino, substituiertes Dialkylamino, cyclisches Amino, substituiertes cyclisches Amino, Carbamoyl (-CONH₂), substituiertes Carbamoyl, Carboxyl, Carbamat, Alkoxycarbonyl, substituiertes Alkoxycarbonyl, Acylamino, substituiertes Acylamino, Sulfamoyl (-SO₂NH₂), substituiertes Sulfamoyl, Haloalkyl, Haloalkyloxy, -C(O)H, Trialkylsilyl und Azido umfasst,
wobei q ausgewählt ist aus der Gruppe umfassend C und N, oder q eine Bindung ist wobei wenn q N ist R11 fehlt, und wenn q eine Bindung ist, R11 und R12 fehlen.

In einer Ausführungsform weist die Verbindung die folgende Struktur (II) auf: 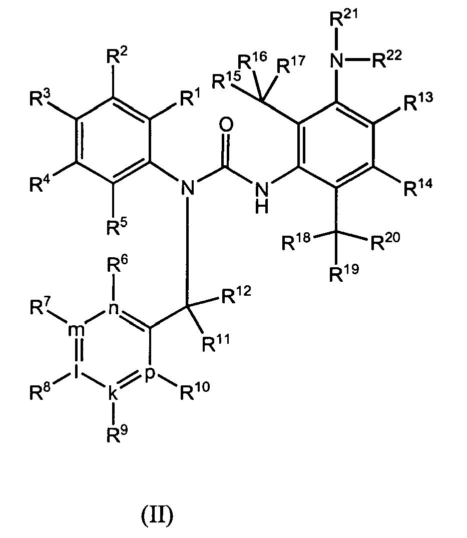 wobei R6, R7, R8, R9, R10, R15, R16, R17, R18, R19 und R20 jeweils einzeln und unabhängig voneinander ausgewählt ist aus der Gruppe, die H, Alkyl, substituiertes Alkyl, Alkenyl, substituiertes Alkenyl, Alkynyl, substituiertes Alkynyl, Cycloalkyl, substituiertes Cycloalkyl, Heterocyclyl, substituiertes Heterocyclyl, Aryl, substituiertes Aryl, Heteroaryl, substituiertes Heteroaryl, Arylalkyl, substituiertes Arylalkyl, Heteroarylalkyl, substituiertes Heteroarylalkyl, Alkoxyl, substituiertes Alkokyl, Aryloxy, substituiertes Aryloxy, Arylalkyloxy, substituiertes Arylalkyloxy, Acyloxy, substituiertes Acyloxy, Halogen, Hydroxyl, Nitro, Cyano, Acyl, substituiertes Acyl, Mercapto, Alkylthio, substituiertes Alkylthio, Amino, substituiertes Amino, Alkylamino, substituiertes Alkylamino, Dialkylamino, substituiertes Dialkylamino, cyclisches Amino, substituiertes cyclisches Amino, Carbamoyl (-CONH₂), substituiertes Carbamoyl, Carboxyl, Carbamat, Alkoxycarbonyl, substituiertes Alkoxycarbonyl, Acylamino, substituiertes Acylamino, Sulfamoyl (-SO₂NH₂), substituiertes Sulfamoyl, Haloalkyl, Haloalkyloxy, -C(O)H, Trialkylsilyl und Azido umfasst; und
k, l, m, n und p jeweils einzeln und unabhängig voneinander ausgewählt ist aus der Gruppe, die C und N umfasst,
- R23 durch den Molekülteil 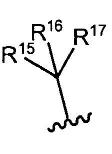 dargestellt ist,
- R24 durch den Molekülteil 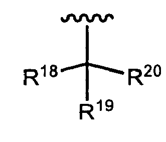 dargestellt ist,
- A durch den Molekülteil 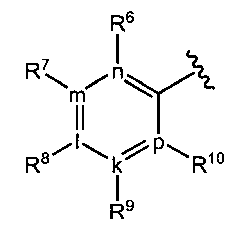 dargestellt ist
und q C ist.

In einer Ausführungsform, die bevorzugterweise eine spezielle Ausführungsform der vorhergehenden Ausführungsform ist, weist die Verbindung die folgende Struktur (III) auf 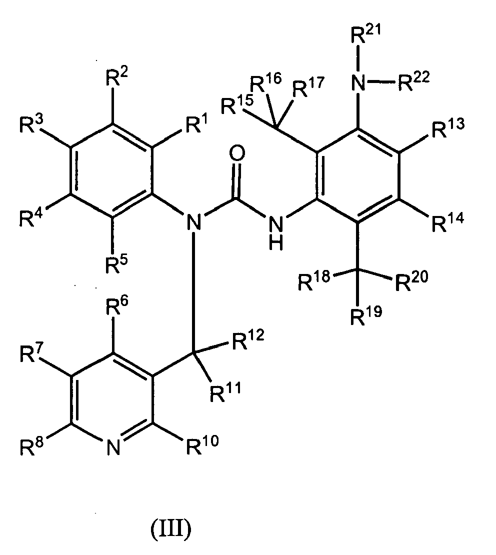 , wobei R6, R7, R8, R10, R15, R16, R17, R18, R19 und R20 jeweils einzeln und unabhängig voneinander ausgewählt ist aus der Gruppe, die H, Alkyl, substituiertes Alkyl, Alkenyl, substituiertes Alkenyl, Alkynyl, substituiertes Alkynyl, Cycloalkyl, substituiertes Cycloalkyl, Heterocyclyl, substituiertes Heterocyclyl, Aryl, substituiertes Aryl, Heteroaryl, substituiertes Heteroaryl, Arylalkyl, substituiertes Arylalkyl, Heteroarylalkyl, substituiertes Heteroarylalkyl, Alkoxyl, substituiertes Alkokyl, Aryloxy, substituiertes Aryloxy, Arylalkyloxy, substituiertes Arylalkyloxy, Acyloxy, substituiertes Acyloxy, Halogen, Hydroxyl, Nitro, Cyano, Acyl, substituiertes Acyl, Mercapto, Alkylthio, substituiertes Alkylthio, Amino, substituiertes Amino, Alkylamino, substituiertes Alkylamino, Dialkylamino, substituiertes Dialkylamino, cyclisches Amino, substituiertes cyclisches Amino, Carbamoyl (-CONH₂), substituiertes Carbamoyl, Carboxyl, Carbamat, Alkoxycarbonyl, substituiertes Alkoxycarbonyl, Acylamino, substituiertes Acylamino, Sulfamoyl (-SO₂NH₂), substituiertes Sulfamoyl, Haloalkyl, Haloalkyloxy, -C(O)H, Trialkylsilyl und Azido umfasst,
- R23 durch den Molekülteil 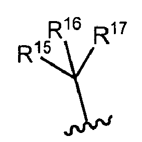 dargestellt ist,
- R24 durch den Molekülteil 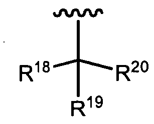 dargestellt ist;
   und -A durch den Molekülteil 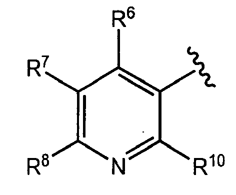 dargestellt ist.

In einer Ausführungsform, die eine bevorzugte Ausführungsform der ersten Ausführungsform des ersten Aspektes ist, weist die Verbindung die folgende Struktur (IIIB) auf 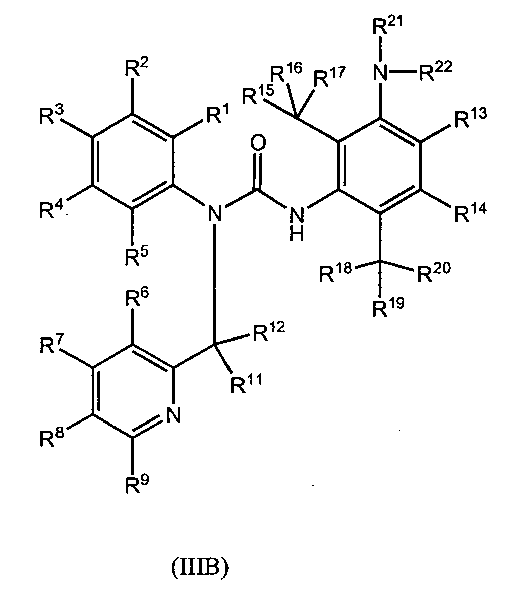 , wobei R6, R7, R8, R9, R15, R16, R17, R18, R19 und R20 jeweils einzeln und unabhängig voneinander ausgewählt ist aus der Gruppe, die H, Alkyl, substituiertes Alkyl, Alkenyl, substituiertes Alkenyl, Alkynyl, substituiertes Alkynyl, Cycloalkyl, substituiertes Cycloalkyl, Heterocyclyl, substituiertes Heterocyclyl, Aryl, substituiertes Aryl, Heteroaryl, substituiertes Heteroaryl, Arylalkyl, substituiertes Arylalkyl, Heteroarylalkyl, substituiertes Heteroarylalkyl, Alkoxyl, substituiertes Alkokyl, Aryloxy, substituiertes Aryloxy, Arylalkyloxy, substituiertes Arylalkyloxy, Acyloxy, substituiertes Acyloxy, Halogen, Hydroxyl, Nitro, Cyano, Acyl, substituiertes Acyl, Mercapto, Alkylthio, substituiertes Alkylthio, Amino, substituiertes Amino, Alkylamino, substituiertes Alkylamino, Dialkylamino, substituiertes Dialkylamino, cyclisches Amino, substituiertes cyclisches Amino, Carbamoyl (-CONH₂), substituiertes Carbamoyl, Carboxyl, Carbamat, Alkoxycarbonyl, substituiertes Alkoxycarbonyl, Acylamino, substituiertes Acylamino, Sulfamoyl (-SO₂NH₂), substituiertes Sulfamoyl, Haloalkyl, Haloalkyloxy, -C(O)H, Trialkylsilyl und Azido umfasst,
- R23 durch den Molekülteil 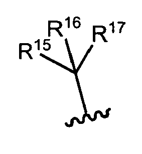 dargestelltist,
- R24 durch den Molekülteil 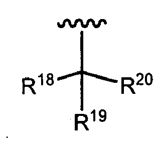 dargestellt ist;
   und -A durch den Molekülteil 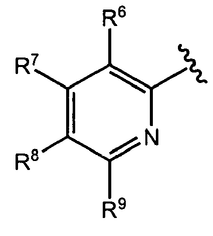 dargestellt ist.

In einer Ausführungsform, die eine bevorzugte Ausführungsform der ersten Ausführungsform des ersten Aspektes ist, weist die Verbindung die folgende Struktur (IIIC) auf 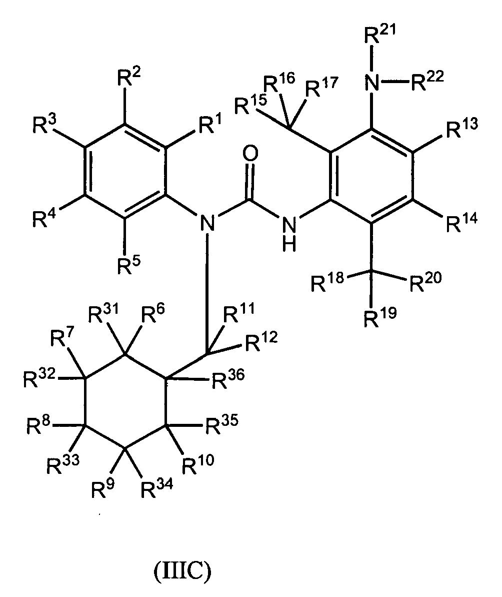 , wobei R6, R7, R8, R9, R10, R15, R16, R17, R18, R19, R20, R31, R32, R33, R34, R35 und R36 jeweils einzeln und unabhängig voneinander ausgewählt ist aus der Gruppe, die H, Alkyl, substituiertes Alkyl, Alkenyl, substituiertes Alkenyl, Alkynyl, substituiertes Alkynyl, Cycloalkyl, substituiertes Cycloalkyl, Heterocyclyl, substituiertes Heterocyclyl, Aryl, substituiertes Aryl, Heteroaryl, substituiertes Heteroaryl, Arylalkyl, substituiertes Arylalkyl, Heteroarylalkyl, substituiertes Heteroarylalkyl, Alkoxyl, substituiertes Alkokyl, Aryloxy, substituiertes Aryloxy, Arylalkyloxy, substituiertes Arylalkyloxy, Acyloxy, substituiertes Acyloxy, Halogen, Hydroxyl, Nitro, Cyano, Acyl, substituiertes Acyl, Mercapto, Alkylthio, substituiertes Alkylthio, Amino, substituiertes Amino, Alkylamino, substituiertes Alkylamino, Dialkylamino, substituiertes Dialkylamino, cyclisches Amino, substituiertes cyclisches Amino, Carbamoyl (-CONH₂), substituiertes Carbamoyl, Carboxyl, Carbamat, Alkoxycarbonyl, substituiertes Alkoxycarbonyl, Acylamino, substituiertes Acylamino, Sulfamoyl (-SO₂NH₂), substituiertes Sulfamoyl, Haloalkyl, Haloalkyloxy, -C(O)H, Trialkylsilyl und Azido umfasst,
- R23 durch den Molekülteil 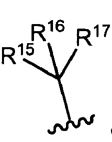 dargestellt ist,
- R24 durch den Molekülteil 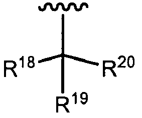 dargestellt ist;
   und -A durch den Molekülteil 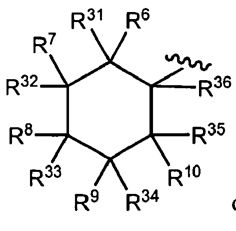 dargestellt ist.

In einer Ausführungsform, die die bevorzugte zweite Ausführungsform des ersten Aspektes der vorliegenden Erfindung ist, weist die Verbindung die folgende Struktur (IV) auf und ist bevorzugtererweise ein C5a-Rezeptorantagonist: 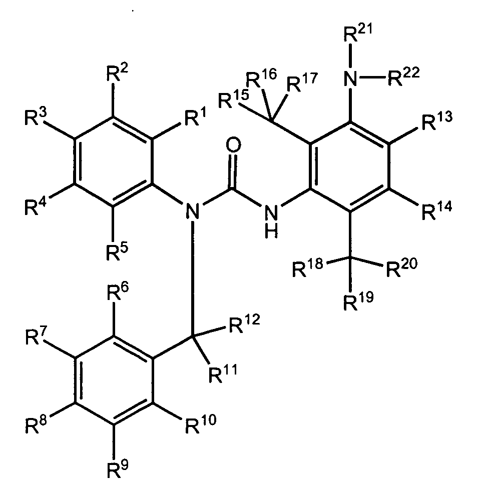 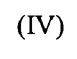 wobei R1, R2, R3, R4, R5, R6, R7, R8, R9, R10, R11, R12, R13, R14, R15, R16, R17, R18, R19, R20, R21 und R22 jeweils einzeln und unabhängig voneinander ausgewählt ist aus der Gruppe, die H, Alkyl, substituiertes Alkyl, Alkenyl, substituiertes Alkenyl, Alkynyl, substituiertes Alkynyl, Cycloalkyl, substituiertes Cycloalkyl, Heterocyclyl, substituiertes Heterocyclyl, Aryl, substituiertes Aryl, Heteroaryl, substituiertes Heteroaryl, Arylalkyl, substituiertes Arylalkyl, Heteroarylalkyl, substituiertes Heteroarylalkyl, Alkoxyl, substituiertes Alkokyl, Aryloxy, substituiertes Aryloxy, Arylalkyloxy, substituiertes Arylalkyloxy, Acyloxy, substituiertes Acyloxy, Halogen, Hydroxyl, Nitro, Cyano, Acyl, substituiertes Acyl, Mercapto, Alkylthio, substituiertes Alkylthio, Amino, substituiertes Amino, Alkylamino, substituiertes Alkylamino, Dialkylamino, substituiertes Dialkylamino, cyclisches Amino, substituiertes cyclisches Amino, Carbamoyl (-CONH₂), substituiertes Carbamoyl, Carboxyl, Carbamat, Alkoxycarbonyl, substituiertes Alkoxycarbonyl, Acylamino, substituiertes Acylamino, Sulfamoyl (-SO₂NH₂), substituiertes Sulfamoyl, Haloalkyl, Haloalkyloxy, -C(O)H, Trialkylsilyl und Azido umfasst.

In einer bevorzugten Ausführungsform der bevorzugten zweiten Ausführungsform des ersten Aspektes ist R1, R2, R3, R4 und R5 jeweils einzeln und unabhängig voneinander ausgewählt aus der Gruppe, die H, Alkyl, substituiertes Alkyl, Alkynyl, Cycloalkyl, Alkoxyl, substituiertes Alkokyl, Acyloxy, Halogen, Nitro, Cyano, Acyl, Alkylthio, substituiertes Alkylthio, Amino, substituiertes Amino, Alkylamino, substituiertes Alkylamino, Dialkylamino, cyclisches Amino, Carbamoyl (-CONH₂), Acylamino, und substituiertes Acylamino umfasst,
oder der Molekülteil ist 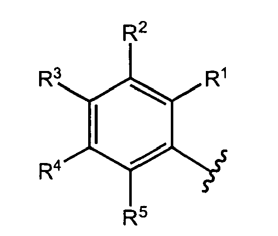 durch einen Molekülteil ersetzt, der ausgewählt ist aus der Gruppe, die die Molekülteile 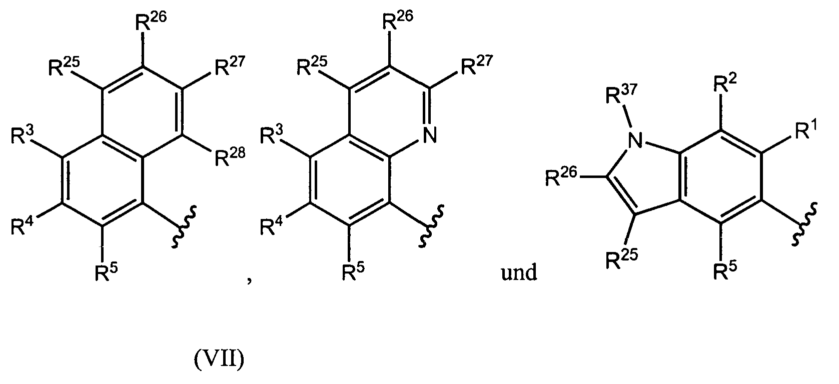 enthält,
wobei R1, R2, R3, R4 und R5 jeweils einzeln und unabhängig voneinander wie oben definiert ist,
wobei R25, R26, R27 und R28 jeweils einzeln und unabhängig voneinander ausgewählt ist aus der Gruppe, die H, Alkyl, substituiertes Alkyl, Alkenyl, substituiertes Alkenyl, Alkynyl, substituiertes Alkynyl, Cycloalkyl, substituiertes Cycloalkyl, Heterocyclyl, substituiertes Heterocyclyl, Aryl, substituiertes Aryl, Heteroaryl, substituiertes Heteroaryl, Arylalkyl, substituiertes Arylalkyl, Heteroarylalkyl, substituiertes Heteroarylalkyl, Alkoxyl, substituiertes Alkokyl, Aryloxy, substituiertes Aryloxy, Arylalkyloxy, substituiertes Arylalkyloxy, Acyloxy, substituiertes Acyloxy, Halogen, Hydroxyl, Nitro, Cyano, Acyl, substituiertes Acyl, Mercapto, Alkylthio, substituiertes Alkylthio, Amino, substituiertes Amino, Alkylamino, substituiertes Alkylamino, Dialkylamino, substituiertes Dialkylamino, cyclisches Amino, substituiertes cyclisches Amino, Carbamoyl (-CONH₂), substituiertes Carbamoyl, Carboxyl, Carbamat, Alkoxycarbonyl, substituiertes Alkoxycarbonyl, Acylamino, substituiertes Acylamino, Sulfamoyl (-SO₂NH₂), substituiertes Sulfamoyl, Haloalkyl, Haloalkyloxy, -C(O)H, Trialkylsilyl und Azido umfasst,
und R37 ausgewählt ist aus der Gruppe, die H, Alkyl, substituiertes Alkyl umfasst.

In einer bevorzugten Ausführungsform der bevorzugten zweiten Ausführungsform des ersten Aspektes ist R1, R2, R4 und R5 jeweils einzeln und unabhängig voneinander ausgewählt aus der Gruppe, die H, Alkyl, Alkoxyl und Halogen umfasst,
R3 ist ausgewählt aus der Gruppe, die H, Alkyl, substituiertes Alkyl, Alkynyl, Cycloalkyl, Alkoxyl, Acyl, Alkylthio, substituiertes Alkylthio, Alkylamino und substituiertes Alkylamino, umfasst,
oder der Molekülteil ist 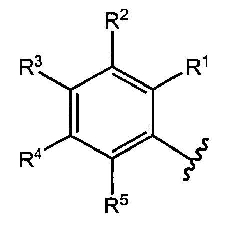 durch einen Molekülteil ersetzt, der ausgewählt ist aus der Gruppe, die die Molekülteile 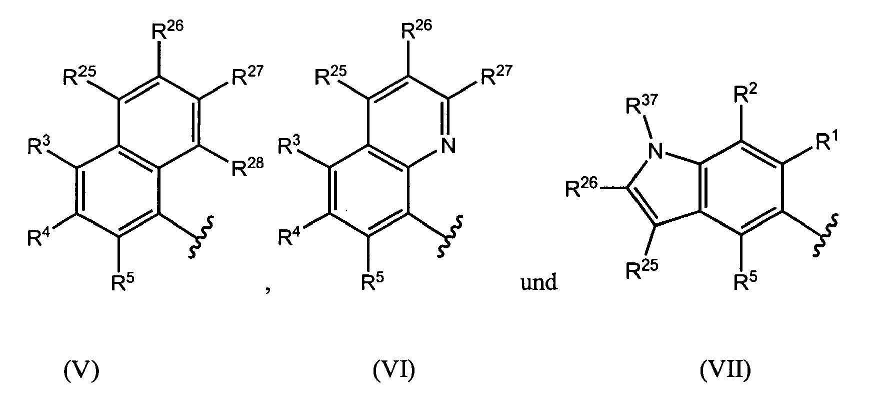 enthält,
wobei R1, R2, R4, R5 und R3 jeweils einzeln und unabhängig voneinander wie oben definiert ist,
wobei R25, R26, R27 und R28 jeweils einzeln und unabhängig voneinander ausgewählt ist aus der Gruppe, die H, Alkyl, Alkoxyl und Halogen umfasst,
und R37 ausgewählt ist aus der Gruppe, die H, Alkyl und substituiertes Alkyl umfasst.

In einer bevorzugten Ausführungsform der bevorzugten zweiten Ausführungsform des ersten Aspektes ist R1, R2, R4 und R5 jeweils einzeln und unabhängig voneinander ausgewählt aus der Gruppe, die H, Me, OMe, F, Cl und Br umfasst,
R3 ist ausgewählt aus der Gruppe, die Et-, iPr-, CF₃CH₂-, Cyclopropyl, HCC-, MeO-, MeS-, CF₃S-, MeNH-, und CF₃NH- umfasst,
oder der Molekülteil ist 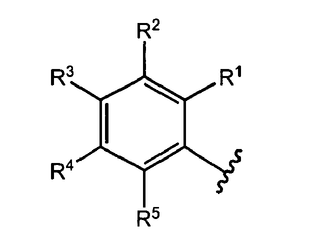 durch einen Molekülteil ersetzt, der ausgewählt ist aus der Gruppe, die die Molekülteile 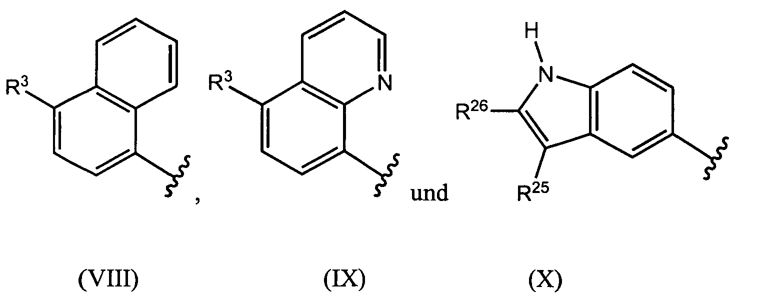 enthält,
wobei R3 wie oben definiert ist,
wobei R25 und R26 jeweils einzeln und unabhängig voneinander ausgewählt ist aus der Gruppe, die H, Me, OMe, F, Cl, Br und CF₃ umfasst.

In einer bevorzugten Ausführungsform der bevorzugten zweiten Ausführungsform des ersten Aspektes ist R6, R7, R8, R9 und R10 jeweils einzeln und unabhängig voneinander ausgewählt aus der Gruppe, die H, Alkyl, substituiertes Alkyl, Heterocyclyl, substituiertes Heterocyclyl, Aryl, substituiertes Aryl, Heteroaryl, substituiertes Heteroaryl, Alkoxyl, substituiertes Alkokyl, Acyloxy, Halogen, Nitro, Cyano, Acyl, Alkylthio, substituiertes Alkylthio, Amino, substituiertes Amino, Alkylamino, substituiertes Alkylamino, Dialkylamino, substituiertes Dialkylamino, cyclisches Amino, Carbamoyl (-CONH₂) und Acylamino umfasst,
oder der Molekülteil ist 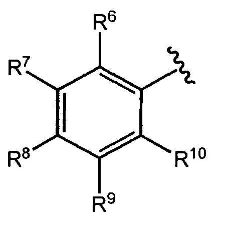 durch den folgenden Molekülteil ersetzt 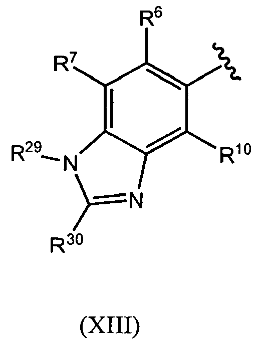 wobei R6, R7 und R10 jeweils einzeln und unabhängig voneinander wie oben definiert ist,
wobei R29 und R30 jeweils einzeln und unabhängig ausgewählt ist aus der Gruppe, die H, Alkyl, substituiertes Alkyl, Heterocyclyl, substituiertes Heterocyclyl, Aryl, substituiertes Aryl, Heteroaryl, substituiertes Heteroaryl, Alkoxyl, substituiertes Alkokyl, Acyloxy, Halogen, Nitro, Cyano, Acyl, Alkylthio, substituiertes Alkylthio, Amino, substituiertes Amino, Alkylamino, substituiertes Alkylamino, Dialkylamino, substituiertes Dialkylamino, cyclisches Amino, Carbamoyl (-CONH₂), und Acylamino umfasst.

In einer bevorzugten Ausführungsform der unmittelbar vorhergehenden Ausführungsform ist R6, R7, R9 und R10 jeweils einzeln und unabhängig voneinander ausgewählt aus der Gruppe, die H, Alkyl, substituiertes Alkyl, Heterocyclyl, substituiertes Heterocyclyl, Aryl, Heteroaryl, Alkoxyl, substituiertes Alkokyl, Halogen, Alkylthio, substituiertes Alkylthio, Amino, substituiertes Aminound cyclisches Amino umfasst,

R8 ist ausgewählt aus der Gruppe, die H, Alkyl, substituiertes Alkyl, Heterocyclyl, substituiertes Heterocyclyl, Aryl, Heteroaryl, substituiertes Heteroaryl, Alkoxyl, substituiertes Alkokyl, Halogen, Alkylthio, substituiertes Alkylthio, Amino, substituiertes Amino, Alkylamino, substituiertes Alkylamino, Dialkylamino, substituiertes Dialkylamino und cyclisches Amino umfasst,
oder der Molekülteil ist 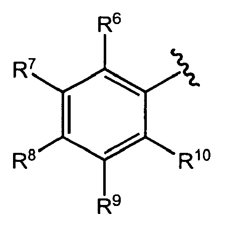 durch den folgenden Molekülteil ersetzt 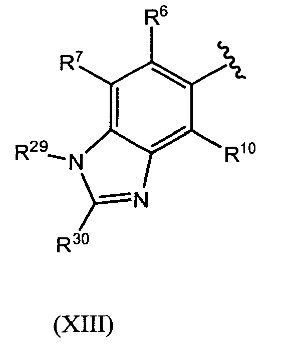 wobei R6, R7 und R10 jeweils einzeln und unabhängig voneinander wie oben definiert ist,
wobei R29 und R30 jeweils einzeln und unabhängig voneinander ausgewählt ist aus der Gruppe, die H, Alkyl, substituiertes Alkyl, Heterocyclyl, substituiertes Heterocyclyl, Aryl, Heteroaryl, Alkoxyl, substituiertes Alkokyl, Halogen, Alkylthio, substituiertes Alkylthio, Amino, substituiertes Amino und cyclisches Amino umfasst.

In einer alternativen Ausführungsform der unmittelbar vorhergehenden Ausführungsform ist R6, R7, R9 und R10 jeweils einzeln und unabhängig voneinander ausgewählt aus der Gruppe, die H-, Me-, -CF₃, -OMe, -OCF₃, -F, -Cl, -Br und -SCF₃ umfasst,
R8 ist ausgewählt aus der Gruppe, die H-, Me-, -CF₃, -OMe, -F, -Cl, -Br, -SMe, -NMe₂ und - NHMe umfasst,
oder der Molekülteil ist 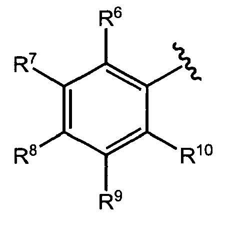 durch den folgenden Molekülteil ersetzt 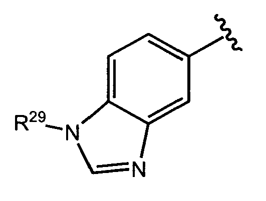 wobei R29 ausgewählt ist aus der Gruppe, die H-, Me-, -CH₂F, CHF₂, -CF₃ und -CF₃ umfasst.

In einer Ausführungsform der bevorzugten zweiten Ausführungsform des ersten Aspektes ist R11 und R12 jeweils einzeln und unabhängig voneinander ausgewählt aus der Gruppe, die H, Alkyl, substituiertes Alkyl und Halogen umfasst,
oder
R11 und R12 bilden zusammen einen Cycloalkylring,
oder der Molekülteil ist 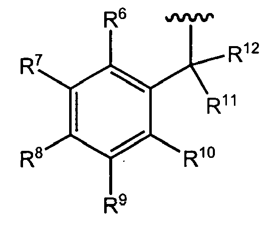 durch den Molekülteil 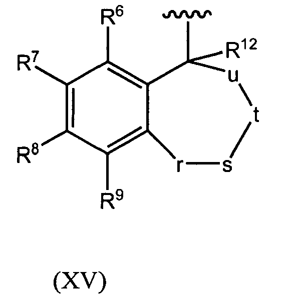 ersetzt,
wobei R12 ausgewählt ist aus der Gruppe, die H, Alkyl, substituiertes Alkyl, und Halogen umfasst,
wobei r, s, t und u jeweils einzeln und unabhängig ausgewählt-ist aus der Gruppe, die -CH₂-, -O-, -NAlkyl- und -NH- umfasst, oder wobei r, s, t, und u jeweils einzeln und unabhängig für eine chemische Bindung steht.

In einer bevorzugten Ausführungsform der unmittelbar vorangehenden Ausführungsform ist R11 und R12 jeweils einzeln und unabhängig ausgewählt-aus der Gruppe, die -H, -Me, -Et, - CF₃, und -F umfasst,
oder der Molekülteil_ist 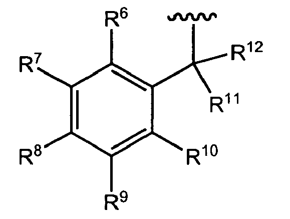 durch den Molekülteil 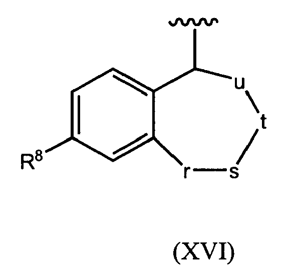 ersetzt, wobei r, s, t und u jeweils einzeln und unabhängig ausgewählt ist aus der Gruppe, die -CH₂- und -O- umfasst oder wobei r, s, t, und u jeweils einzeln und unabhängig für eine chemische Bindung steht.

In einer Ausführungsform der bevorzugten zweiten Ausführungsform des ersten Aspektes ist der Molekülteil 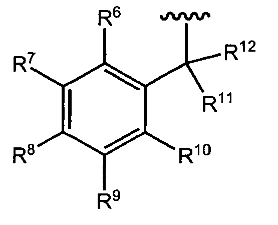 durch den Molekülteil 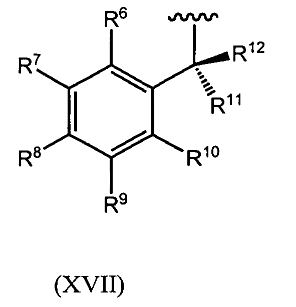 ersetzt,
wobei R11H ist,
wobei R12 ausgewählt ist aus der Gruppe, die H, Alkyl, substituiertes Alkyl und Halogen umfasst.

In einer bevorzugten Ausführungsform der unmittelbar vorangehenden Ausführungsform ist R11 H, und
R12 ist ausgewählt aus der Gruppe, die -H, -Me, -Et, -CF₃ und -F umfasst.

In einer Ausführungsform der bevorzugten zweiten Ausführungsform des ersten Aspektes ist R13 und R14 jeweils einzeln und unabhängig voneinander ausgewählt aus der Gruppe, die H, Alkyl, substituiertes Alkyl, Alkoxyl, substituiertes Alkokyl, Halogen, Cyano, Alkylthio, Amino und substituiertes Amino umfasst.

In einer bevorzugten Ausführungsform der unmittelbar vorangehenden Ausführungsform ist R13 und R14 jeweils einzeln und unabhängig voneinander ausgewählt aus der Gruppe, die - H, -Me, -CF₃, -OMe, -F, -Cl und -Br umfasst.

In einer Ausführungsform der bevorzugten zweiten Ausführungsform des ersten Aspektes ist R15, R16, R17, R18, R19 und R20 jeweils einzeln und unabhängig voneinander ausgewählt aus der Gruppe, die H, Alkyl, substituiertes Alkyl, Alkoxyl, substituiertes Alkokyl, Acyloxy, Halogen, Alkylthio, substituiertes Alkylthio, Amino, substituiertes Amino und Carbamoyl (-CONH₂) umfasst,
oder
zwei oder drei der Reste aus der Gruppe umfassend R15, R16 und R17 und/oder aus der Gruppe umfassend R18, R19, und R20 bilden zusammen Alkynyl, substituiertes Alkynyl, Cycloalkyl, substituiertes Cycloalkyl, Heterocyclyl, Aryl, Heteroaryl oder Keto.

In einer bevorzugten Ausführungsform der unmittelbar vorangehenden Ausführungsform ist R15, R16, R17, R18, R19 und R20 jeweils einzeln und unabhängig voneinander ausgewählt aus der Gruppe, die -H, -Me, -CF₃, und -F umfasst.

In einer Ausführungsform der bevorzugten zweiten Ausführungsform des ersten Aspektes ist R21 und R22 jeweils einzeln und unabhängig voneinander ausgewählt aus der Gruppe, die H, Alkyl, substituiertes Alkyl, Acyl, substituiertes Acyl, Alkylthio und substituiertes Alkylthio umfasst,
oder der Molekülteil ist 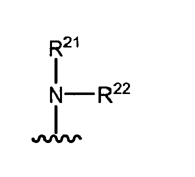 durch einen Rest ersetzt, der ausgewählt ist aus der Gruppe, die Nitro, Nitroso (NO) und Azido umfasst.

In einer bevorzugten Ausführungsform der unmittelbar vorangehenden Ausführungsform ist R21 und R22 jeweils einzeln und unabhängig voneinander ausgewählt aus der Gruppe, die - H, -Me und -CF₃ umfasst,
oder der Molekülteil ist 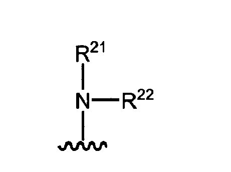 ersetzt durch Nitroso (NO).

In einer Ausführungsform der bevorzugten Ausführungsformen des ersten Aspektes, insbesondere der bevorzugten ersten Ausführungsform des ersten Aspektes, weist die Verbindung eine der folgenden Strukturen auf 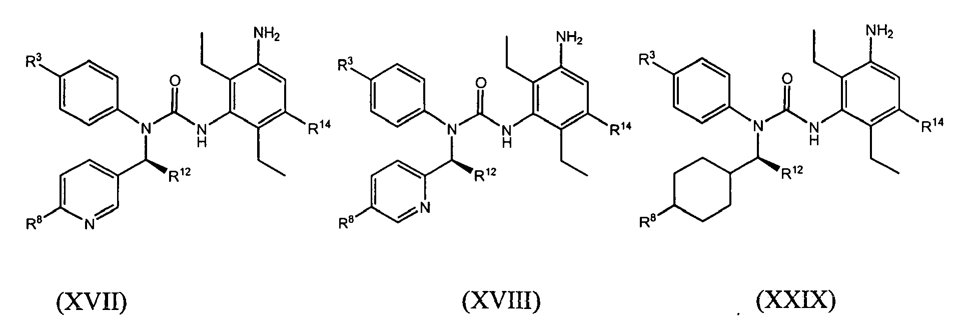 und
R3 ist ausgewählt aus der Gruppe, die Et-, iPr-, CF₃CH₂-, Cyclopropyl, HCC-, MeO-, MeS-, CF₃S-, MeNH- und CF₃NH- umfasst,
R8 ist ausgewählt aus der Gruppe, die H-, Me-, -CF₃, -OMe, -F, -Cl, -Br, -SMe, -NMe₂ und - NHMe umfasst,
R12 ist ausgewählt aus der Gruppe, die H- und Me- umfasst, und
R14 ist ausgewählt aus der Gruppe, die H- und -Cl umfasst.

In einer Ausführungsform der bevorzugten zweiten Ausführungsform des ersten Aspektes weist die Verbindung die folgende Struktur auf 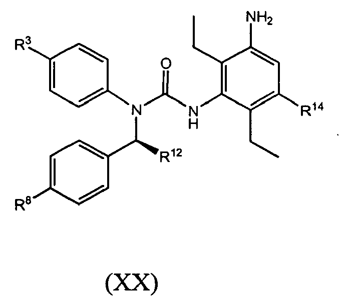 und
R3 ist ausgewählt aus der Gruppe, die Et-, iPr-, CF₃CH₂-, Cyclopropyl, HCC-, MeO-, MeS-, CF₃S-, MeNH- und CF₃NH- umfasst,
R8 ist ausgewählt aus der Gruppe, die H-, Me-, -CF₃, -OMe, -F, -Cl, -Br, -SMe, -NMe₂, und-NHMe umfasst,
R12 ist ausgewählt aus der Gruppe, die H- und Me- umfasst, und
R14 ist ausgewählt aus der Gruppe, die H-und -Cl umfasst,

In einer Ausführungsform der bevorzugten zweiten Ausführungsform des ersten Aspektes weist die Verbindung eine der folgenden Strukturen auf: 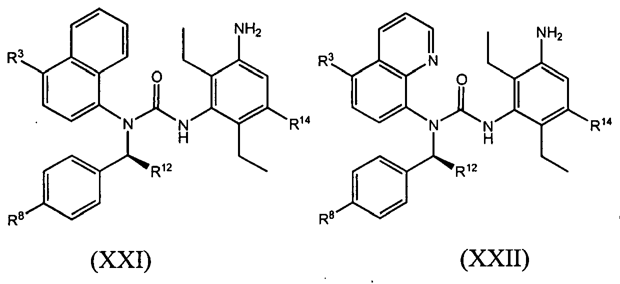 oder 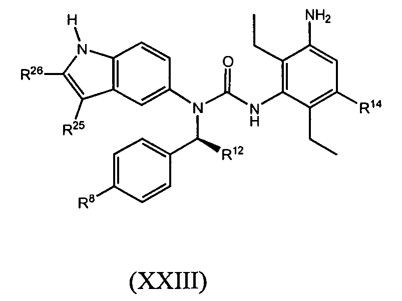 und
R3, R25 und R26 ist jeweils einzeln und unabhängig voneinander ausgewählt aus der Gruppe, die H-, Et-, iPr-, CF₃CH₂-, Cyclopropyl, HCC-, MeO-, MeS-, CF₃S-, MeNH-, CF₃NH- und umfasst,
R8 ist ausgewählt aus der Gruppe, die H-, Me-, -CF₃, -OMe, -F, -Cl, -Br, -SMe, -NMe₂ und - NHMe umfasst,
R12 ist ausgewählt aus der Gruppe, die H- und Me- umfasst, und
R14 ist ausgewählt aus der Gruppe, die H-und -Cl umfasst.

In einer weiteren Ausführungsform der bevorzugten zweiten Ausführungsform des ersten Aspektes weist die Verbindung eine der folgenden Strukturen auf: 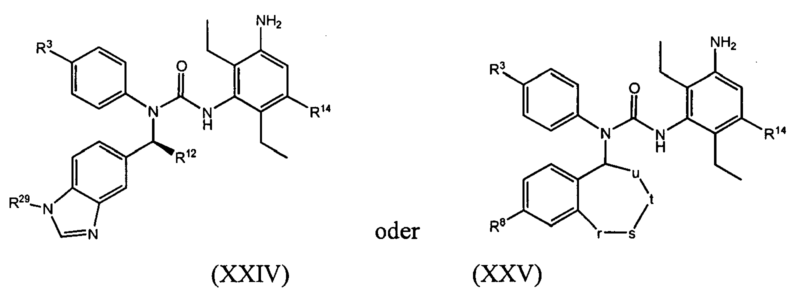 und R3 ist ausgewählt aus der Gruppe, die Et-, iPr-, CF₃CH₂-, Cyclopropyl, HCC-, MeO-, MeS-, CF₃S-, MeNH-und CF₃NH- umfasst,
R8 ist ausgewählt aus der Gruppe, die H-, Me-, -CF₃, -OMe, -F, -Cl, -Br, -SMe, -NMe₂ und - NHMe umfasst,
R29 ist ausgewählt aus der Gruppe, die H-, Me-, -CH₂F, CHF₂, -CF₃ und -CF₃ umfasst.
R14 ist ausgewählt aus der Gruppe, die H- und -Cl umfasst,
R12 ist ausgewählt aus der Gruppe, die H- und Me- umfasst, und
r, s, t und u ist jeweils einzeln und unabhängig ausgewählt aus der Gruppe, die -CH₂- und -O-umfasst oder wobei r, s, t und u jeweils einzeln und unabhängig für eine chemische Bindung steht.

In einer bevorzugten Ausführungsform ist die Verbindung ausgewählt aus der Gruppe umfassend
3-(3-Amino-2,6-düsopropyl-phenyl)-1-(4-isopropyl-phenyl)-1-(4-methoxy-benzyl)-harnstoff
3-(3-Amino-2,6-diethyl-phenyl)-1-(4-isopropyl-phenyl)-1-(4-methoxy-benzyl)-harnstoff
2-{2,4-Diethyl-3-[3-(4-isopropyl-phenyl)-3-(4-methoxy-benzyl)-ureido]-phenylamino}- acetamid
{2,4-Diethyl-3-[3-(4-isopropyl-phenyl)-3-(4-methoxy-benzyl)-ureido]-phenylamino}-essigsäuremethylester
3-(3-Amino-5-chlor-2,6-diethyl-phenyl)-1-(4-isopropyl-phenyl)-1-(4-methoxy-benzyl)-harnstoff
3-(3-Amino-5-chlor-2,6-diethyl-phenyl)-1-(4-ethyl-phenyl)-1-(4-methoxy-benzyl)-harnstoff
3-(3-Amino-5-chlor-2,6-diethyl-phenyl)-1-(4-isopropyl-phenyl)-1-(4-trifluormethoxy-benzyl)-harnstoff
3-(3-Amino-5-chlor-2,6-diethyl-phenyl)-1-(4-isopropyl-phenyl)-1-(4-methyl-benzyl)-harnstoff
3-(3-Amino-5-chlor-2,6-diethyl-phenyl)-1-(4-ethyl-benzyl)-1-(4-isopropyl-phenyl)-harnstoff
3-{3-Chlor-2,6-diethyl-5-[(furan-2-ylmethyl)-amino]-phenyl}-1-(4-isopropyl-phenyl)-1-(4-methoxy-benzyl)-harnstoff
2-{5-Chlor-2,4-diethyl-3-[3-(4-isopropyl-phenyl)-3-(4-methoxy-benzyl)-ureido]-phenylamino}-N,N-dimethyl-acetamid
3-{3-Chlor-2,6-diethyl-5-[(1-methyl-1H-imidazol-2-ylmethyl)-amino]-phenyl}-1-(4-isopropyl-phenyl)-1-(4-methoxy-benzyl)-harnstoff
3-(3-Amino-2,6-diethyl-phenyl)-1-(4-isopropyl-phenyl)-1-(4-methyl-benzyl)-harnstoff
(R)-3-(3-Amino-5-chlor-2,6-diethyl-phenyl)-1-(4-isopropyl-phenyl)-1-(1,2,3,4-tetrahydro-naphthalen-1-yl)-harnstoff
(S)-3 -(3-Amino-5-chlor-2,6-diethyl-phenyl)-1-(4-isopropyl-phenyl)-1-(1,2,3,4-tetrahydro-naphthalen-1-yl)-harnstoff
3-(3-Amino-5-chlor-2,6-diethyl-phenyl)-1-benzyl-1-(4-isopropyl-phenyl)-harnstoff
3-(3-Amino-5-chlor-2,6-diethyl-phenyl)-1-indan-5-yl-1-(4-methoxy-benzyl)-harnstoff
2-{5-Chlor-2,4-diethyl-3-[3-(4-isopropyl-phenyl)-3-(4-methoxy-benzyl)-ureido]-phenylamino}-acetamid
3-(3-Amino-5-chlor-2,6-diethyl-phenyl)-1-(4-isopropyl-phenyl)-1-(6-methoxy-pyridin-3-ylmethyl)-harnstoff
3-(3-Amino-2,6-diethyl-phenyl)-1-(4-isopropyl-phenyl)-1-(6-methoxy-pyridin-3-ylmethyl)-harnstoff
3-(3-Amino-5-chlor-2,6-diethyl-phenyl)-1-(4-isopropyl-phenyl)-1-(4-trifluormethyl-benzyl)-harnstoff
3-(3-Amino-2,6-diethyl-phenyl)-1-(4-isopropyl-phenyl)-1-(4-trifluormethyl-benzyl)-harnstoff
3-(3-Amino-2,6-diethyl-phenyl)-1-(4-isopropyl-phenyl)-1-phenethyl-harnstoff
3-(3-Amino-5-chlor-2,6-diethyl-phenyl)-1-(4-isopropyl-phenyl)-1-(2-p-tolyl-ethyl)-harnstoff
3-(3-Amino-5-chlor-2,6-diethyl-phenyl)-1-(4-ethyl-phenyl)-1-(4-methyl-benzyl)-harnstoff
3-(3-Amino-2,6-diethyl-phenyl)-1-(4-ethyl-phenyl)-1-(4-methyl-benzyl)-harnstoff
{5-Chlor-2,4-diethyl-3-[3-(4-isopropyl-phenyl)-3-(4-methoxy-benzyl)-ureido]-phenylamino}-essigsäuremethylester
(R)-3-(3-Amino-5-chlor-2,6-diethyl-phenyl)-1-(4-isopropyl-phenyl)-1-[1-(4-methoxyphenyl)-ethyl]-harnstoff
(R)-3-(3-Amino-5-chlor-2,6-diethyl-phenyl)-1-(4-isopropyl-phenyl)-1-(1-p-tolyl-ethyl)-harnstoff
3-(3-Amino-5-chlor-2,6-diethyl-phenyl)-1-(2-fluor-4-methoxy-benzyl)-1-(4-isopropylphenyl)-harnstoff
3-(3-Amino-2,6-diethyl-phenyl)-1-(2-fluor-4-methoxy-benzyl)-1-(4-isopropyl-phenyl)-harnstoff
3-(3-Amino-5-chlor-2,6-diethyl-phenyl)-1-cyclohexylmethyl-1-(4-isopropyl-phenyl)-harnstoff
3-(3-Ammo-2,6-diethyl-phenyl)-1-cyclohexylmethyl-1-(4-isopropyl-phenyl)-hamstoff
3-(3-Amino-5-chlor-2,6-diethyl-phenyl)-1-(4-isopropyl-phenyl)-1-(2-methoxy-benzyl)-harnstoff
3-(3-Amino-2,6-diethyl-phenyl)-1-(4-isopropyl-phenyl)-1-(2-methoxy-benzyl)-harnstoff
3-(3-Amino-5-chlor-2,6-diethyl-phenyl)-1-(4-isopropyl-phenyl)-1-(4-methanesulfonyl-benzyl)-harnstoff
3-(3-Amino-5-chlor-2,6-diethyl-phenyl)-1-(2,3-dihydro-benzofuran-5-ylmethyl)-1-(4-isopropyl-phenyl)-harnstoff
(S)-3-(3-Amino-5-chlor-2,6-diethyl-phenyl)-1-(4-isopropyl-phenyl)-1-[1-(4-methoxyphenyl)-ethyl]-harnstoff
3-(3-Amino-2,6-diethyl-phenyl)-1-(2,3-dihydro-benzofuran-5-ylmethyl)-1-(4-isopropylphenyl)-harnstoff
(R)-3-(3-Amino-2,6-diethyl-phenyl)-1-(4-isopropyl-phenyl)-1-(1-p-tolyl-ethyl)-harnstoff
(S)-3-(3-Amino-2, 6-diethyl-phenyl)-1-(4-isopropyl-phenyl)-1-(1-p-tolyl-ethyl)-harnstoff
(R)-3-(3-Amino-2,6-diethyl-phenyl)-1-(4-isopropyl-phenyl)-1-[1-(4-methoxy-phenyl)-ethyl]-harnstoff
(S)-3-(3-Amino-2,6-diethyl-phenyl)-1-(4-isopropyl-phenyl)-1-[1-(4-methoxy-phenyl)-ethyl]-harnstoff
3-(3-Amino-5-chlor-2,6-diethyl-phenyl)-1-(4-chlor-benzyl)-1-(4-isopropyl-phenyl)-harnstoff
(S)-3-(3-Amino-5-chlor-2,6-diethyl-phenyl)-1-(4-isopropyl-phenyl)-1-(1-p-tolyl-ethyl)-harnstoff
3-(3-Amino-5-chlor-2,6-diethyl-phenyl)-1-(4-isopropyl-phenyl)-1-pyridin-3-ylmethyl-harnstoff
3-(3-Amino-5-chlor-2,6-diethyl-phenyl)-1-(4-isopropyl-phenyl)-1-(5-methyl-pyrazin-2-ylmethyl)-harnstoff
3-(3-Amino-5-chlor-2,6-diethyl-phenyl)-1-(4-isopropyl-phenyl)-1-(6-methyl-pyridin-3-ylmethyl)-harnstoff
3-(3-Amino-5-chlor-2,6-diethyl-phenyl)-1-(2,4-dimethoxy-pyrimidin-5-ylmethyl)-1-(4-isopropyl-phenyl)-harnstoff
(S)-3-(3-Amino-5-chlor-2,6-diethyl-phenyl)-1-[1-(4-chlor-phenyl)-ethyl]-1-(4-isopropylphenyl)-harnstoff
(S)-3-(3-Amino-5-chlor-2,6-dimethyl-phenyl)-1-(4-isopropyl-phenyl)-1-(1-p-tolyl-ethyl)-hamstoff
(S)-3-(3-Amino-5-chlor-2,6-diethyl-phenyl)-1-[1-(4-chlor-phenyl)-ethyl]-1-(4-ethyl-phenyl)-harnstoff
(S)-3-(3-Amino-5-chlor-2,6-diethyl-phenyl)-1-[1-(4-fluor-phenyl)-ethyl]-1-(4-isopropylphenyl)-harnstoff
(S)-3-(3-Amino-5-chlor-2,6-diethyl-phenyl)-1-(4-ethyl-phenyl)-1-[1-(4-fluor-phenyl)-ethyl]-harnstoff
(S)-3-(3-Amino-5-chlor-2,6-diethyl-phenyl)-1-[1-(4-chlor-phenyl)-ethyl]-1-(4-methylsulfanyl-phenyl)-harnstoff
3-(3-Amino-5-chlor-2,6-diethyl-phenyl)-1-indan-1-yl-1-(4-isopropyl-phenyl)-harnstoff
3-(3-Amino-5-chlor-2,6-diethyl-phenyl)-1-(4-ethynyl-phenyl)-1-(4-methoxy-benzyl)-harnstoff
(S)-3-(3-Amino-5-chlor-2,6-diethyl-phenyl)-1-(4-chlor-phenyl)-1-[1-(4-chlor-phenyl)-ethyl]-harnstoff
3-(3-Amino-5-chlor-2,6-diethyl-phenyl)-1-(4-isopropyl-phenyl)-1-(6-trifluormethyl-pyridin-3-ylmethyl)-harnstoff
3-(3-Amino-5-chlor-2,6-diethyl-phenyl)-1-(5-chlor-indan-1-yl)-1-(4-isopropyl-phenyl)-harnstoff
3-(3-Amino-5-chlor-2,6-diethyl-phenyl)-1-chroman-4-yl-1-(4-isopropyl-phenyl)-harnstoff
3-(3-Amino-2,6-diethyl-phenyl)-1-indan-1-yl-1-(4-isopropyl-phenyl)-harnstoff
3-(3-Amino-5-chlor-2,6-diethyl-phenyl)-1-(4-ethyl-phenyl)-1-(1-ethyl-1H-pyrazol-4-ylmethyl)-harnstoff
(S)-3-(3-Amino-5-chlor-2,6-diethyl-phenyl)-1-[1-(4-chlor-phenyl)-ethyl]-1-(4-methoxyphenyl)-harnstoff
3-(3-Amino-5-chlor-2,6-diethyl-phenyl)-1-(4-chlor-benzyl)-1-(4-trifluormethylsulfanyl-phenyl)-harnstoff
(S)-3-(3-Amino-5-chlor-2,6-diethyl-phenyl)-1-[1-(4-chlor-phenyl)-ethyl]-1-(3-fluor-4-methoxy-phenyl)-harnstoff
(S)-3-(3-Amino-S-chlor-2,6-diethyl-phenyl)-1-[1-(4-chlor-phenyl)-ethyl]-1-(4-methanesulfinyl-phenyl)-harnstoff
3-(3-Amino-5-chlor-2,6-diethyl-phenyl)-1-(4-ethyl-phenyl)-1-(4-fluor-benzyl)-harnstoff
(S)-3-(3-Amino-5-chlor-2,6-diethyl-phenyl)-1-[1-(4-chlor-phenyl)-ethyl]-1-(4-ethyl-3-fluorphenyl)-harnstoff
3-(3-Amino-5-chlor-2,6-diethyl-phenyl)-1-(4-chlor-benzyl)-1-(4-ethyl-phenyl)-harnstoff
3-(3-Amino-5-chlor-2,6-diethyl-phenyl)-1-(4-bromo-benzyl)-1-(4-ethyl-phenyl)-harnstoff
3-(3-Amino-5-chlor-2,6-diethyl-phenyl)-1-(6-chlor-pyridin-3-ylmethyl)-1-(4-isopropylphenyl)-harnstoff
3-(3-Amino-5-chlor-2,6-diethyl-phenyl)-1-(4-isopropyl-phenyl)-1-thiochroman-4-yl-harnstoff
(S)-3-(3-Amino-5-chlor-2,6-diethyl-phenyl)-1-[1-(4-chlor-phenyl)-ethyl]-1-(2-fluor-4-methoxy-phenyl)-harnstoff
3-(3-Amino-5-chlor-2,6-diethyl-phenyl)-1-(4-chlor-benzyl)-1-[4-(2,2,2-trifluor-ethyl)-phenyl]-harnstoff
3-(3-Amino-5-chlor-2,6-diethyl-phenyl)-1-(5-chlor-indan-1-yl)-1-(4-ethyl-phenyl)-harnstoff
3-(3-Amino-5-chlor-2,6-diethyl-phenyl)-1-(5-chlor-pyridin-2-ylmethyl)-1-(4-isopropylphenyl)-harnstoff
3-(3-Amino-5-chlor-2,6-diethyl-phenyl)-1-(4-ethyl-phenyl)-1-(6-methyl-pyridin-3-ylmethyl)-harnstoff
3-(3-Amino-2,6-diethyl-phenyl)-1-(4-ethyl-phenyl)-1-(6-methyl-pyridin-3-ylmethyl)-harnstoff
3-(3-Amino-5-chlor-2,6-diethyl-phenyl)-1-(4-chlor-benzyl)-1-naphthalen-1-yl-hamstoff
(S)-3-(3-Amino-5-chlor-2,6-diethyl-phenyl)-1-[1-(4-chlor-phenyl)-ethyl]-1-(2-hydroxymethyl-4-methoxy-phenyl)-hamstoff
3-(3-Amino-5-chlor-2,6-diethyl-phenyl)-1-(4-ethyl-phenyl)-1-(4-pyrazol-1-yl-benzyl)-harnstoff
(S)-3-(3-Amino-6-tert-butyl-2-methyl-phenyl)-1-[1-(4-chlor-phenyl)-ethyl]-1-(4-ethylphenyl)-harnstoff
3-(3-Amino-5-chlor-2,6-diethyl-phenyl)-1-(4-ethyl-phenyl)-1-(2-nitro-1-phenyl-ethyl)-harnstoff
3-(3-Amino-5-chlor-2,6-diethyl-phenyl)-1-(4-cyano-benzyl)-1-(4-ethyl-phenyl)-harnstoff
3-(3-Amino-5-chlor-2,6-diethyl-phenyl)-1-(4-chlor-benzyl)-1-(3,4-dimethoxy-phenyl)-harnstoff
3-(3-Amino-5-chlor-2,6-diethyl-phenyl)-1-(4-chlor-benzyl)-1-(4-methoxy-naphthalen-1-yl)-harnstoff
1-(4-Amino-benzyl)-3-(3-amino-5-chlor-2,6-diethyl-phenyl)-1-(4-ethyl-phenyl)-harnstoff
3-(3-Amino-5-chlor-2,6-diethyl-phenyl)-1-(4-chlor-benzyl)-1-(4-difluormethoxy-phenyl)-harnstoff
3-(3-Amino-5-chlor-2,6-diethyl-phenyl)-1-(4-ethyl-phenyl)-1-(1-methyl-1H-benzoimidazol-5-ylmethyl)-harnstoff
3-(3-Amino-2,6-diethyl-phenyl)-1-(4-chlor-benzyl)-1-(4-methylamino-phenyl)-harnstoff
3-(3-Amino-5-chlor-2,6-diethyl-phenyl)-1-(4-chlor-benzyl)-1-(4-methylamino-phenyl)-harnstoff
3-(3-Amino-5-chlor-2,6-diethyl-phenyl)-1-(2-dimethylamino-pyrimidin-5-ylmethyl)-1-(4-ethyl-phenyl)-harnstoff
3-(3-Amino-5-chlor-2,6-diethyl-phenyl)-1-(4-ethyl-phenyl)-1-[1-(1-methyl-1H-benzoimidazol-5-yl)-ethyl]-harnstoff
3-(3-Amino-5-chlor-2,6-diethyl-phenyl)-1-(4-ethyl-phenyl)-1-[1-(6-methyl-pyridin-3-yl)-ethyl]-harnstoff
3-(3-Amino-5-chlor-2,6-diethyl-phenyl)-1-(2-fluor-4-methoxy-phenyl)-1-(6-methyl-pyridin-3-ylmethyl)-harnstoff
3-(3-Amino-5-chlor-2,6-diethyl-phenyl)-1-(4-chlor-benzyl)-1-(5-methoxy-chinolin-8-yl)-harnstoff
3-(3-Amino-5-chlor-2,6-diethyl-phenyl)-1-(6-methyl-pyridin-3-ylmethyl)-1-[4-(2,2,2-trifluorethyl)-phenyl]-harnstoff
3-(3-Amino-5-chlor-2,6-diethyl-phenyl)-1-(4-chlor-benzyl)-1-(4-dimethylamino-phenyl)-harnstoff
3-(3-Amino-5-chlor-2,6-diethyl-phenyl)-1-benzyl-1-(4-dimethylamino-phenyl)-harnstoff
3-(3-Amino-5-chlor-2,6-diethyl-phenyl)-1-benzyl-1-(4-methylamino-phenyl)-harnstoff
3-(3-Amino-5-chlor-2,6-diethyl-phenyl)-1-(4-chlor-benzyl)-1-(8-methoxy-2,3-dimethyl-chinoxalin-5-yl)-harnstoff
3-(3-Amino-5-chlor-2,6-diethyl-phenyl)-1-[1-(4-chlor-phenyl)-ethyl]-1-(4-methylamino-phenyl)-harnstoff
3-(3-Amino-5-chlor-2,6-diethyl-phenyl)-1-(4-methylamino-phenyl)-1-(1-phenyl-ethyl)-harnstoff
3-(3-Chlor-2,6-diethyl-5-nitroso-phenyl)-1-[1-(4-chlor-phenyl)-ethyl]-1-(4-methylamino-phenyl)-harnstoff
3-(3-Amino-5-chlor-2,6-diethyl-phenyl)-1-(4-methoxy-naphthalen-1-yl)-1-(6-methyl-pyridin-3-ylmethyl)-harnstoff
3-(3-Amino-5-chlor-2,6-diethyl-phenyl)-1-(4-chlor-benzyl)-1-(4-ethylamino-phenyl)-harnstoff
3-(3-Amino-5-chlor-2,6-diethyl-phenyl)-1-[4-(2-amino-ethyl)-benzyl]-1-(4-ethyl-phenyl)-harnstoff
3-(3-Amino-5-chlor-2,6-diethyl-phenyl)-1-(4-methylamino-phenyl)-1-[1-(1-methyl-1H-benzoimidazol-5-yl)-ethyl]-harnstoff
3-(3-Amino-5-chlor-2,6-diethyl-phenyl)-1-(4-chlor-benzyl)-1-(4-methylamino-naphthalen-1-yl)-harnstoff
N- {4-[3-(3-Amino-5-chlor-2,6-diethyl-phenyl)-1-(4-chlor-benzyl)-ureido]-phenyl} -acetamid
3-(3-Amino-5-chlor-2,6-diethyl-phenyl)-1-[1-(4-chlor-phenyl)-ethyl]-1-(H-indol-5-yl)-harnstoff

In einer ganz besonders bevorzugten Ausführungsform ist die Verbindung ausgewählt aus der Gruppe umfassend
3-(3-Amino-2,6-diisopropyl-phenyl)-1-(4-isopropyl-phenyl)-1-(4-methoxy-benzyl)-harnstoff
3-(3-Amino-2,6-diethyl-phenyl)-1-(4-isopropyl-phenyl)-1-(4-methoxy-benzyl)-harnstoff
3-(3-Amino-5-chlor-2,6-diethyl-phenyl)-1-(4-isopropyl-phenyl)-1-(4-methoxy-benzyl)-harnstoff
3-(3-Ammo-5-chlor-2,6-diethyl-phenyl)-1-(4-ethyl-phenyl)-1-(4-methoxy-benzyl)-hamstoff
3-(3-Amino-5-chlor-2,6-diethyl-phenyl)-1-(4-isopropyl-phenyl)-1-(4-methyl-benzyl)-harnstoff
3-(3-Amino-S-chlor-2,6-diethyl-phenyl)-1-(4-ethyl-benzyl)-1-(4-isopropyl-phenyl)-harnstoff
3-(3-Amino-2,6-diethyl-phenyl)-1-(4-isopropyl-phenyl)-1-(4-methyl-benzyl)-harnstoff
(R)-3-(3-Amino-5-chlor-2,6-diethyl-phenyl)-1-(4-isopropyl-phenyl)-1-(1,2,3,4-tetrahydro-naphthalen-1-yl)-harnstoff
(S)-3-(3-Amino-5-chlor-2,6-diethyl-phenyl)-1-(4-isopropyl-phenyl)-1-(1,2,3,4-tetrahydro-naphthalen-1-yl)-harnstoff
3-(3-Amino-5-chlor-2,6-diethyl-phenyl)-1-benzyl-1-(4-isopropyl-phenyl)-harnstoff
3-(3-Amino-5-chlor-2,6-diethyl-phenyl)-1-(4-isopropyl-phenyl)-1-(6-methoxy-pyridin-3-ylmethyl)-harnstoff
3-(3-Amino-5-chlor-2,6-diethyl-phenyl)-1-(4-isopropyl-phenyl)-1-(4-trifluormethyl-benzyl)-harnstoff
3-(3-Amino-2,6-diethyl-phenyl)-1-(4-isopropyl-phenyl)-1-(4-trifluormethyl-benzyl)-harnstoff
3-(3-Amino-5-chlor-2,6-diethyl-phenyl)-1-(4-isopropyl-phenyl)-1-(2-p-tolyl-ethyl)-harnstoff
3-(3-Amino-5-chlor-2,6-diethyl-phenyl)-1-(4-ethyl-phenyl)-1-(4-methyl-benzyl)-harnstoff
3-(3-Amino-2,6-diethyl-phenyl)-1-(4-ethyl-phenyl)-1-(4-methyl-benzyl)-harnstoff
(R)-3-(3-Amino-5-chlor-2,6-diethyl-phenyl)-1-(4-isopropyl-phenyl)-1-[1-(4-methoxyphenyl)-ethyl]-harnstoff
(R)-3-(3 -Amino-5-chlor-2,6-diethyl-phenyl)- 1 -(4-isopropyl-phenyl)- 1 -(1 -p-tolyl-ethyl)-harnstoff
3-(3-Amino-5-chlor-2,6-diethyl-phenyl)-1-(2-fluor-4-methoxy-benzyl)-1-(4-isopropylphenyl)-harnstoff
3-(3-Amino-2,6-diethyl-phenyl)-1-(2-fluor-4-methoxy-benzyl)-1-(4-isopropyl-phenyl)-hamstoff
3-(3-Amino-2,6-diethyl-phenyl)-1-cyclohexylmethyl-1-(4-isopropyl-phenyl)-harnstoff
3-(3-Amino-5-chlor-2,6-diethyl-phenyl)-1-(4-isopropyl-phenyl)-1-(2-methoxy-benzyl)-harnstoff
3-(3-Amino-5-chlor-2,6-diethyl-phenyl)-1-(2,3-dihydro-benzofuran-5-ytmethyt)-1-(4-isopropyl-phenyl)-harnstoff
(S)-3-(3-Amino-5-chlor-2,6-diethyl-phenyl)-1-(4-isopropyl-phenyl)-1-[ 1-(4-methoxyphenyl)-ethyl]-harnstoff
3-(3-Amino-2,6-diethyl-phenyl)-1-(2,3-dihydro-benzofuran-5-ylmethyl)-1-(4-isopropylphenyl)-harnstoff
(S)-3-(3-Amino-2,6-diethyl-phenyl)-1-(4-isopropyl-phenyl)-1-(11-p-tolyl-ethyl)-hamstoff
(S)-3-(3-Amino-2,6-diethyl-phenyl)-1-(4-isopropyl-phenyl)-1-[1-(4-methoxy-phenyl)-ethyl]-harnstoff
3-(3-Amino-5-chlor-2,6-diethyl-phenyl)-1-(4-chlor-benzyl)-1-(4-isopropyl-phenyl)-harnstoff
(S)-3-(3-Amino-5-chlor-2,6-diethyl-phenyl)-1-(4-isopropyl-phenyl)-1-(1-p-tolyl-ethyl)-harnstoff
3-(3-Amino-5-chlor-2,6-diethyl-phenyl)-1-(4-isopropyl-phenyl)-1-pyridin-3-ylmethyl-harnstoff
3-(3-Amino-5-chlor-2,6-diethyl-phenyl)-1-(4-isopropyl-phenyl)-1-(5-methyl-pyrazin-2-ylmethyl)-harnstoff
3-(3-Amino-5-chlor-2,6-diethyl-phenyl)-1-(4-isopropyl-phenyl)-1-(6-methyl-pyridin-3-ylmethyl)-harnstoff
3-(3-Amino-5-chlor-2,6-diethyl-phenyl)-1-(2,4-dimethoxy-pyrimidin-5-ylmethyl)-1-(4-isopropyl-phenyl)-harnstoff
(*S*)-3-(3-Amino-5-chlor-2,6-diethyl-phenyl)-1-[1-(4-chlor-phenyl)-ethyl]-1-(4-isopropylphenyl)-harnstoff
(*S*)-3-(3-Amino-5-chlor-2,6-dimethyl-phenyl)-1-(4-isopropyl-phenyl)-1-(1-p-tolyl-ethyl)-harnstoff
(S)-3-(3-Amino-5-chlor-2,6-diethyl-phenyl)-1-[1-(4-chlor-phenyl)-ethyl]-1-(4-ethyl-phenyl)-harnstoff
(S)-3-(3-Amino-5-chlor-2,6-diethyl-phenyl)-1-[1-(4-fluor-phenyl)-ethyl]-1-(4-isopropylphenyl)-harnstoff
(S)-3-(3-Amino-5-chlor-2,6-diethyl-phenyl)-1-(4-ethyl-phenyl)-1-[1-(4-fluor-phenyl)-ethyl]-harnstoff
(S)-3-(3-Amino-5-chlor-2,6-diethyl-phenyl)-1-[1-(4-chlor-phenyl)-ethyl]-1-(4-methylsulfanyl-phenyl)-harnstoff
3-(3-Amino-5-chlor-2,6-diethyl-phenyl)-1-indan-1-yl-1-(4-isopropyl-phenyl)-harnstoff
3-(3-Amino-5-chlor-2,6-diethyl-phenyl)-1-(4-ethynyl-phenyl)-1-(4-methoxy-benzyl)-harnstoff
(S)-3-(3-Amino-5-chlor-2,6-diethyl-phenyl)-1-(4-chlor-phenyl)-1-[1-(4-chlor-phenyl)-ethyl]-harnstoff
3-(3-Amino-5-chlor-2,6-diethyl-phenyl)-1-(4-isopropyl-phenyl)-1-(6-trifluormethyl-pyridin-3-ylmethyl)-harnstoff
3-(3-Amino-5-chlor-2,6-diethyl-phenyl)-1-(5-chlor-indan-1-yl)-1-(4-isopropyl-phenyl)-harnstoff
3-(3-Amino-5-chlor-2,6-diethyl-phenyl)-1-chroman-4-yl-1-(4-isopropyl-phenyl)-harnstoff
3-(3-Amino-2,6-diethyl-phenyl)-1-indan-1-yl-1-(4-isopropyl-phenyl)-harnstoff
3-(3-Amino-5-chlor-2,6-diethyl-phenyl)-1-(4-ethyl-phenyl)-1-(1-ethyl-1H-pyrazol-4-ylmethyl)-harnstoff
(S)-3-(3-Amino-5-chlor-2,6-diethyl-phenyl)-1-[1-(4-chlor-phenyl)-ethyl]-1-(4-methoxyphenyl)-harnstoff
3-(3-Amino-5-chlor-2,6-diethyl-phenyl)-1-(4-chlor-benzyl)-1-(4-trifluormethylsulfanyl-phenyl)-harnstoff
(S)-3-(3-Amino-5-chlor-2,6-diethyl-phenyl)-1-[1-(4-chlor-phenyl)-ethyl]-1-(3-fluor-4-methoxy-phenyl)-harnstoff
(S)-3-(3-Amino-5-chlor-2,6-diethyl-phenyl)-1-[1-(4-chlor-phenyl)-ethyl]-1-(4-methanesulfinyl-phenyl)-harnstoff
3-(3-Amino-5-chlor-2,6-diethyl-phenyl)-1-(4-ethyl-phenyl)-1-(4-fluor-benzyl)-harnstoff
(S)-3-(3-Amino-5-chlor-2,6-diethyl-phenyl)-1-[1-(4-chlor-phenyl)-ethyl]-1-(4-ethyl-3-fluorphenyl)-harnstoff
3-(3-Amino-5-chlor-2,6-diethyl-phenyl)-1-(4-chlor-benzyl)-1-(4-ethyl-phenyl)-harnstoff
3-(3-Amino-5-chlor-2,6-diethyl-phenyl)-1-(4-bromo-benzyl)-1-(4-ethyl-phenyl)-harnstoff
3-(3-Amino-5-chlor-2,6-diethyl-phenyl)-1-(6-chlor-pyridin-3-ylmethyl)-1-(4-isopropylphenyl)-harnstoff
(S)-3-(3-Amino-5-chlor-2,6-diethyl-phenyl)-1-[1-(4-chlor-phenyl)-ethyl]-1-(2-fluor-4-methoxy-phenyl)-harnstoff
3-(3-Amino-5-chlor-2,6-diethyl-phenyl)-1-(4-chlor-benzyl)-1-[4-(2,2,2-trifluor-ethyl)-phenyl]-harnstoff
3-(3-Amino-5-chlor-2,6-diethyl-phenyl)-1-(5-chlor-indan-1-yl)-1-(4-ethyl-phenyl)-harnstoff
3-(3-Amino-5-chlor-2,6-diethyl-phenyl)-1-(5-chlor-pyridin-2-ylmethyl)-1-(4-isopropylphenyl)-harnstoff
3-(3-Amino-5-chlor-2,6-diethyl-phenyl)-1-(4-ethyl-phenyl)-1-(6-methyl-pyridin-3-ylmethyl)-harnstoff
3-(3-Amino-2,6-diethyl-phenyl)-1-(4-ethyl-phenyl)-1-(6-methyl-pyridin-3-ylmethyl)-harnstoff
3-(3-Amino-5-chlor-2,6-diethyl-phenyl)-1-(4-chlor-benzyl)-1-naphthalen-1-yl-harnstoff
(S)-3-(3-Amino-6-tert-butyl-2-methyl-phenyl)-1-[1-(4-chlor-phenyl)-ethyl]-1-(4-ethylphenyl)-harnstoff
3-(3-Amino-5-chlor-2,6-diethyl-phenyl)-1-(4-cyano-benzyl)-1-(4-ethyl-phenyl)-harnstoff
3-(3-Amino-5-chlor-2,6-diethyl-phenyl)-1-(4-chlor-benzyl)-1-(3,4-dimethoxy-phenyl)-harnstoff
3-(3-Amino-5-chlor-2,6-diethyl-phenyl)-1-(4-chlor-benzyl)-1-(4-methoxy-naphthalen-1-yl)-harnstoff
3-(3-Amino-5-chlor-2,6-diethyl-phenyl)-1-(4-chlor-benzyl)-1-(4-difluormethoxy-phenyl)-harnstoff
3-(3-Amino-5-chlor-2,6-diethyl-phenyl)-1-(4-ethyl-phenyl)-1-(1-methyl-1H-benzoimidazol-5-ylmethyl)-harnstoff
3-(3-Amino-2,6-diethyl-phenyl)-1-(4-chlor-benzyl)-1-(4-methylamino-phenyl)-harnstoff
3-(3-Amino-5-chlor-2,6-diethyl-phenyl)-1-(4-chlor-benzyl)-1-(4-methylamino-phenyl)-harnstoff
3-(3-Amino-5-chlor-2,6-diethyl-phenyl)-1-(4-ethyl-phenyl)-1-[1-(1-methyl-1H-benzoimidazol-5-yl)-ethyl]-harnstoff
3-(3-Amino-5-chlor-2,6-diethyl-phenyl)-1-(4-ethyl-phenyl)-1-[1-(6-methyl-pyridin-3-yl)-ethyl]-harnstoff
3-(3-Amino-5-chlor-2,6-diethyl-phenyl)-1-(2-fluor-4-methoxy-phenyl)-1-(6-methyl-pyridin-3-ylmethyl)-harnstoff
3-(3-Amino-5-chlor-2,6-diethyl-phenyl)-1-(4-chlor-benzyl)-1-(5-methoxy-chinolin-8-yl)-harnstoff
3-(3-Amino-5-chlor-2,6-diethyl-phenyl)-1-(6-methyl-pyridin-3-ylmethyl)-1-[4-(2,2,2-trifluorethyl)-phenyl]-harnstoff
3-(3-Amino-5-chlor-2,6-diethyl-phenyl)-1-(4-chlor-benzyl)-1-(4-dimethylamino-phenyl)-harnstoff
3-(3-Amino-5-chlor-2,6-diethyl-phenyl)-1-benzyl-1-(4-methylamino-phenyl)-harnstoff
3-(3-Amino-5-chlor-2,6-diethyl-phenyl)-1-[1-(4-chlor-phenyl)-ethyl]-1-(4-methylamino-phenyl)-harnstoff
3-(3-Amino-5-chlor-2,6-diethyl-phenyl)-1-(4-methylamino-phenyl)-1-(1-phenyl-ethyl)-harnstoff
3-(3-Chlor-2,6-diethyl-5-nitroso-phenyl)-1-[1-(4-chlor-phenyl)-ethyl]-1-(4-methylamino-phenyl)-harnstoff
3-(3-Amino-5-chlor-2,6-diethyl-phenyl)-1-(4-methoxy-naphthalen-1-yl)-1-(6-methyl-pyridin-3-ylmethyl)-harnstoff
3-(3-Amino-5-chlor-2,6-diethyl-phenyl)-1-(4-chlor-benzyl)-1-(4-ethylamino-phenyl)-harnstoff
3-(3-Amino-5-chlor-2,6-diethyl-phenyl)-1-[4-(2-amino-ethyl)-benzyl]-1-(4-ethyl-phenyl)-harnstoff
3-(3-Amino-5-chlor-2,6-diethyl-phenyl)-1-(4-methylamino-phenyl)-1-[1-(1-methyl- 1H-benzoimidazol-5-yl)-ethyl]-harnstoff
N- {4-[3-(3-Amino-5-chlor-2,6-diethyl-phenyl)-1-(4-chlor-benzyl)-ureido]-phenyl}-acetamid
3-(3-Amino-5-chlor-2,6-diethyl-phenyl)-1-[1-(4-chlor-phenyl)-ethyl]-1-(1H-indol-5-yl)-harnstoff

Erfindungsgemäß wird die Aufgabe in einem zweiten Aspekt gelöst durch eine pharmazeutische Zusammensetzung umfassend mindestens eine Verbindung gemäß dem ersten Aspekt und zusätzlich ein pharmazeutisch akzeptables Trägermittel.

Erfindungsgemäß wird die Aufgabe in einem dritten Aspekt gelöst durch die Verwendung mindestens einer Verbindung nach dem ersten Aspekt zur Herstellung eines Medikamentes.

In einer Ausführungsform des dritten Aspektes wird das Medikament für die Prävention und/oder Behandlung einer Erkrankung verwendet, bei der das Komplementsystem aktiviert ist und/oder bei der die Inhibierung des Komplementsystems eine Linderung der Symptome hervorruft.

In einer Ausführungsform des dritten Aspektes wird das Medikament für die Prävention und/oder Behandlung einer Erkrankung verwendet, bei der die Inhibierung der Aktivierung des C5a Rezeptors allein und/oder in Kombination mit anderen Therapeutika eine Linderung der Symptome hervorruft.

In einer alternativen Ausführungsform des dritten Aspektes sind die Erkrankung und/oder die zu behandelnden Symptome ausgewählt aus der Gruppe umfassend Autoimmunerkrankungen, akute und chronische inflammatorische Erkrankungen, Traumata, lokale Entzündungen, Schock und Verbrennungen.

In einer Ausführungsform des dritten Aspektes sind die Erkrankungen schwere Verbrennungen.

In einer Ausführungsform des dritten Aspektes sind die Erkrankung durch Verbrennungen verursachte Folgeschäden, wobei die Folgeschäden Organversagen, Schock, SIRS (severe/systemic inflammatory response syndrome), Sepsis, Ödembildung, Komplikationen bei der operativen Entfernung von angegriffenen Hautarealen und Vemarbung von Haut oder Organen umfassen.

In einer Ausführungsform des dritten Aspektes ist die Erkrankung ausgewählt aus der Gruppe umfassend septischer Schock, hämorrhagischer Schock, SIRS (systemic/severe inflammatory response syndrom), MOF (Multiorganversagen), akute respiratorische Insuffizienz (ARDS), Gehirnschlag, Herzinfarkt, Reperfusionsschaden und akute Verletzungen des zentralen Nervensystems.

In einer bevorzugten Ausführungsform des dritten Aspektes tritt der Reperfusionsschaden an einem oder mehreren Organen, Organsystemen oder Körperteilen auf, wobei diese ausgewählt sind aus der Gruppe umfassend Leber, Niere, Darm, Lunge, Herz, Milz, Harnblase, Magen, Muskeln, Haut, Extremitäten, Gehirn und Bauchspeicheldrüse.

In einer noch bevorzugteren Ausführungsform des dritten Aspektes werden akute Folgen und/oder die chronischen Folgen eines Reperfusionsschaden behandelt, wobei bevorzugterweise akute Folgen das akute Organversagen oder die Bildung nekrotischer Bereiche sind, und bevorzugterweise chronische Folgen Veränderungen wie die dilatative Kardiomyopathie oder Fibrosen, bevorzugtererweise Narbenbildung nach einem Trauma, Narbenbildung beim Herzinfarkt oder bei der Transplantation sind, wobei die Folgen auch bevorzugterweise, eine eingeschränkte Organfunktion sind.

In einer bevorzugten Ausführungsform des dritten Aspektes tritt der Reperfusionsschaden nach einem Myokardinfarkt auf.

In einer Ausführungsform des dritten Aspektes tritt der Reperfusionsschaden an der Niere auf.

In einer Ausführungsform des dritten Aspektes tritt der Reperfusionsschaden nach oder während einer Aneurismaoperation auf.

In einer Ausführungsform des dritten Aspektes ist die Erkrankung ausgewählt aus der Gruppe umfassend Asthma, Myokarditis, entzündliche Darmerkrankungen (IBD: inflammatory bowel disease; Morbus Crohn und Colitis ulcerosa), entzündliche Erkrankungen des Auges, Glomerulonephritis, entzündliche Gefäßerkrankungen, lokale Manifestationen systemischer Erkrankungen.

In einer bevorzugten Ausführungsform des dritten Aspektes sind die entzündlichen Erkrankungen des Auges aus der Gruppe ausgewählt, die Uveitis, altersabhängige Makulardegenration, diabetische Retinopathie, diabetisches makulares Ödem, okularen Pemphigoid, Keratoconjunctivitis, Stevens-Johnson Syndrom und Graves Ophthalmophatie umfasst.

In einer Ausführungsform des dritten Aspektes ist die entzündliche Erkrankung des Auges die altersabhängige Makulardegeneration.

In einer Ausführungsform des dritten Aspektes ist die Erkrankung eine lokale Manifestation systemischer Erkrankungen, wobei die systemische Erkrankung aus der Gruppe ausgewählt ist, die Rheuma, SLE und Typ I und Typ II Diabetes umfasst.

In einer Ausführungsform des dritten Aspektes ist die Manifestation ausgewählt aus der Gruppe, die Manifestationen am Auge, am oder im Gehirn, an den Gefäßen, am Herzen, an der Lunge, an den Nieren, an der Leber, des gastrointestinalen Traktes, der Milz, der Haut, am Knochensystem, am lymphatischen System und im Blut umfasst.

In einer bevorzugten Ausführungsform des dritten Aspektes ist die chronisch inflammatorische Erkrankung eine Autoimmunkrankheit, wobei die Autoimmunkrankheit bevorzugterweise aus der Gruppe ausgewählt ist umfassend Alopecia areata, autoimmun-hämolytische Anämie (AIHA) vom Kältetyp (Kälteagglutininkrankheit), autoimmun-hämolytische Anämie (AIHA) vom Wärmetyp, pernizöse Anämie (Morbus Biermer, Addison Anämie), Antiphospholipid-Syndrom (APS), Arteriitis temporalis, Atherosklerose, Autoimmun-Adrenalitis (autoimmune Nebennierenrinden-Atrophie, Morbus Addison), chronisches Erschöpfungssyndrom (CFIDS), chronisch-inflammatorische, demyelinisierende Polyneuropathie, Churg-Strauss Syndrom, Cogan-Syndrom, Colitis ulcerosa, CREST-Syndrom, Diabetes mellitus Typ I, Dermatitis Herpetiformis During, Dermatomyositis, Fibromyalgie, chronisch autoimmune Gastritis, Goodpasture Syndrom (Anti-GBM vermittelte Glomerulonephritis), Guillain-Barré-Syndrom (GBS; Polyradikuloneuritis), Hashimoto Thyroiditis, autoimmune Hepatitis, idiopathische pulmonale Fibrose, Immun-thrombozytopenische Purpura (Morbus Werlhof), autoimmune Infertilität, autoimmune Innenohrschwerhörigkeit (AIED), juvenile rheumatoide Arthritis, autoimmune Kardiomyopathie, Lambert-Eaton Syndrom, Lichen sclerosis, Lupus erythematodes insbesondere die diskoide Form, Lyme-Arthritis (Lyme-Krankheit), Mischkollagenose, Morbus Basedow (Graves Disease), Morbus Behçet, Morbus Bechterew (Spondylitis ankylosans), Morbus Ménière, Morbus Reiter, Multiple Sklerose (MS, Encephalomyelitis), Myasthenia gravis (Myasthenie), sympathische Ophtalmie, vernarbendes Pemphigoid, bullöses Pemphigoid, Pemphigus vulgaris, Polyarteriitis nodosa, Polychondritis (Panchondritis), Polyglanduläres Autoimmun-(PGA)-Syndrom, Polymyalgia rheumatica, Polymoysitis, Primäre biliäre Zirrhose (primäre Autoimmun-Cholangitis), Psoriasis, rheumatisches Fieber, rheumatoide Arthritis, Sarkoidose (Morbus Boeck, Besnier-Boeck-Schaumann Krankheit), Sjörgensen-Syndrom, Sklerodermie, Sprue/Zöliakie, Stiff-Man-Syndrom (Moersch-Woltmann-Syndrom), systemischer Lupus erythematodes, Takayasu Arteriitis (Aortenbogen-Syndrom), transiente Gluten-Intoleranz, Urticaria, autoimmune Uveitis, Vaskulitiden und Vitiligo (Weißfleckkrankheit).

In einer Ausführungsform des dritten Aspektes ist die entzündliche Gefäßerkrankung aus der Gruppe ausgewählt, die Vaskulitis, Vascular Leakage und Artherosklerose umfasst.

In einer bevorzugten Ausführungsform des dritten Aspektes ist die Vaskulitis ausgewählt aus der Gruppe umfassend primäre Vaskulitis und sekundäre Vaskulitis.

In einer noch bevorzugteren Ausführungsform des dritten Aspektes ist die primäre Vaskulitis eine solche, die aus der Gruppe von Vaskulitiden ausgewählt ist, die Morbus Wegener, das Churg-Strauss-Syndrom und mikroskopische Polyangiitis umfasst.

In einer Ausführungsform des dritten Aspektes ist die sekundäre Vaskulitits eine solche, die aus der Gruppe von Vaskulitiden ausgewählt ist, die durch Medikamente induzierte Vaskulitiden und durch andere Erkrankungen hervorgerufene Vaskultiden umfasst.

In einer bevorzugten Ausführungsform des dritten Aspektes sind die anderen Erkrankungen ausgewählt aus der Gruppe umfassend AIDS, Hepatitis B, Hepatitis C und Cytomegalie-Virus-Infektion.

In einer Ausführungsform des dritten Aspektes ist die Urticaria aus der Gruppe ausgewählt, die spontane und physikalische Urticaria sowie besondere Formen der Urticaria umfasst.

In einer Ausführungsform des dritten Aspektes wird die physikalische Urticaria aus der Gruppe ausgewählt, die Urticaria factitia, Kälteurticaria, Hitzeurticaria, Druckurticaria und Lichturticaria umfasst.

In einer Ausführungsform des dritten Aspektes wird die spontane Urticaria aus der Gruppe ausgewählt, die akute Urticaria und chronische Urticaria umfasst.

In einer Ausführungsform des dritten Aspektes ist die spontane Urticaria dadurch gekennzeichnet, dass bei den betroffenen Patienten Autoantikörper gegen IgE oder den IgE-Rezeptor nachzuweisen sind.

In einer Ausführungsform des dritten Aspektes sind die besonderen Formen der Urticaria cholinergische Urticaria, adrenergische Urticaria, Kontakturticaria und Urticaria, die durch Wasser ausgelöst wird (aquagenic urticaria).

In einer Ausführungsform des dritten Aspektes wird das Medikament zur Prävention und/oder Unterstützung chirurgischer Eingriffe verwendet.

In einer bevorzugten Ausführungsform des dritten Aspektes werden das Medikament bzw. die Verbindung zur Unterstützung und/oder zur Prävention und/oder Nachsorge eines chirurgischen Eingriffs verwendet, wobei der chirurgische Eingriff ausgewählt ist aus der Gruppe, die CABG, PCTA, PTA, MidCAB, OPCAB, Thrombolyse, Organtransplantation, Aneurysmaoperationen und Gefäßverschluss (clamping) umfasst, bevorzugterweise ist ein Aspekt dabei, die bei extrakorporaler Zirkulation (z.B. Herzlungen-Maschine) möglicherweise folgenden neurokognitiven Dysfunktionen zu vermindern bzw. zu verhindern.

In einer Ausführungsform des dritten Aspektes wird das Medikament als Unterstützung für die thrombolytische Behandlung verwendet.

In einer Ausführungsform des dritten Aspektes wird das Medikament im Rahmen einer Dialyse-Behandlung, gegebenenfalls vor, während oder danach, verwendet.

In einer Ausführungsform des dritten Aspektes wird das Medikament zur Vorbeugung von Schädigungen eines transplantierten und/oder zu transplantierenden Organs verwendet.

In einer Ausführungsform des dritten Aspektes kann das Medikament zur Konservierung oder Unterstützung der Konservierung von Organen, die für die Transplantation bestimmt sind, verwendet werden.

In einer Ausführungsform des dritten Aspektes wird das Medikament zur Vorbeugung oder Behandlung von Abstoßungsreaktionen eines transplantierten Organs verwendet.

In einer Ausführungsform des dritten Aspektes ist das transplantierte bzw. zu transplantierende Organ ausgewählt aus der Gruppe, die Nieren, Leber, Lunge, Herz, Haut, Hornhaut, Bauchspeicheldrüse und Darm umfasst.

In einer Ausführungsform des dritten Aspektes handelt es sich bei dem Organ um eine Eigenspende, bevorzugterweise um eine Eigenspende von Haut zur Behandlung bei Brandverletzten oder um eine Eigenspende von Blut.

In einer Ausführungsform des dritten Aspektes wird das Medikament zur Vorbeugung von fibrotischen Ereignissen eingesetzt, bevorzugtererweise zur Behandlung zur Verhinderung oder Verminderung der Bildung von Narbengewebe.

In einer bevorzugten Ausführungsform des dritten Aspektes kann das fibrotische Ereignis in einem oder mehreren der folgenden Organe auftreten: Leber, Lunge, Nieren, Haut, Herz und anderen Organen.

In einer Ausführungsform des dritten Aspektes wird das Medikament zur Vorbeugung oder Behandlung der IgA-Nephropathie verwendet.

Erfindungsgemäß wird die Aufgabe in einem vierten Aspekt gelöst durch die Verwendung einer Verbindung nach dem ersten, zweiten und/oder dritten Aspekt zur kosmetischen Behandlung eines menschlichen oder tierischen Körpers.

In einer Ausführungsform des dritten Aspektes ist die entzündliche Darmerkrankung ausgewählt aus der Gruppe umfassend Morbus Crohn oder Ulcerative colitis.

In einer Ausführungsform des dritten Aspektes wird die Erkrankung durch intrazelluläre Parasiten oder Viren hervorgerufen.

In einer bevorzugten Ausführungsform des dritten Aspektes sind die intrazellulären Parasiten aus der Gruppe ausgewählt umfassend Leischmanien, Rickettsien, Chlamydien, Coxiella, Plasmodien, Brucella, Mycobakterien, Listerien, Toxoplasmen und Trypanosomen.

In einer Ausführungsform des dritten Aspektes erfolgt die Prävention und/oder Behandlung mit einem Medikament gemäß dem dritten und/oder vierten Aspekt in Kombination mit einem oder mehreren antiinflammatorischen und/oder einem oder mehreren immunsupressiven Therapeutika.

In einer Ausführungsform des dritten Aspektes erfolgt die Prävention und/oder Behandlung mit einem Medikament gemäß dem dritten und/oder vierten Aspekt in Kombination mit einem oder mehreren immunsuppressiven Therapeutika.

In einer bevorzugten Ausführungsform des dritten Aspektes bestehen die Kombinationen aus einem Medikament gemäß dem dritten und/oder vierten Aspekt und einem Medikament ausgewählt aus der Gruppe umfassend Calcineurin-Inhibitoren oder einem Medikament umfassend eine oder mehrere Substanzen ausgewählt aus der Gruppe umfassend Cyclosporin A, Methotrexate, Azathioprin, FK506 (Tacrolimus), Rapamycin, Leflunomid, Mycophenolatmofetil, Brequinar, Mizoribin, Thalidomid oder Deoxyspergualin.

In einer Ausführungsform des dritten Aspektes erfolgt die Prävention und/oder Behandlung mit einem Medikament gemäß dem dritten und/oder vierten Aspekt in Kombination mit einem oder mehreren Antihistaminika.

In einer bevorzugten Ausführungsform des dritten Aspektes ist das Antihistaminikum ausgewählt aus der Gruppe, die Meclozin, Clemastin, Dimetinden, Bamipin, Ketotifen, Cetirizin, Lovecetirizin, Loratidin, Desloratidin, Azelastin, Mizolastin, Levocabastin, Terfenadin, Fexofenadin und Ebastin umfasst.

In einer Ausführungsform des dritten Aspektes erfolgt die Prävention und/oder Behandlung mit einem erfindungsgemäßen Medikament in Kombination mit einem oder mehreren Glukokortikoiden.

In einer bevorzugten Ausführungsform des dritten Aspektes ist das Glukokortikoid ausgewählt aus der Gruppe, die Betamethason, Effervescent, Budesonide, Kortison, Dexamethason Elixir, Hydrokortison, Methylprednisolon, Prednisolon, Prednison und Triamcinolone umfasst.

In einer Ausführungsform des dritten Aspektes erfolgt die Prävention und/oder Behandlung mit einem Medikament gemäß dem dritten oder vierten Aspekt in Kombination mit einem oder mehreren Antibiotika.

In einer bevorzugten Ausführungsform des dritten Aspektes ist das Antibiotikum ausgewählt aus der Gruppe, die Amynoglykoside, β-Lactam-Antibiotika, Glycopeptidantibiotika, Gyrasehemmer, Lincosamide, Makrolidantibiotika, Nitroimidazolderivate, Polypeptidantibiotika, Sulfonamide, Trimethoprim und Tetracycline umfasst.

In einer Ausführungsform des dritten Aspektes erfolgt die Prävention und/oder Behandlung mit einem Medikament gemäß dem dritten und/oder vierten Aspekt in Kombination mit einem oder mehreren antiinflammatorischen Biologika.

In einer bevorzugten Ausführungsform des dritten Aspektes ist das antiinflammatorische Therapeutikum ausgewählt aus der Gruppe, die IL-10, Erlizumab, TolerMab, Rituximab, Gomiliximab, Basiliximab, Daclizumab, HuMax-TAC, Visilizumab, HuMaxCD4, Clenoliximab, MAX 16H5, TNX 100, Toralizumab, Alemtuzumab, CY 1788, Galiximab, Pexelizumab, Eculizumab, ETI 104, FG 3019, Bertilimumab, 249417 (anti-Faktor IX), Abciximab, YM 337, Omalizumab, Talizumab, Fontolizumab, J695 (anti-IL12), HuMax IL-15, Mepolizumab, Elsilimomab, HuDREG, Adalimumab, Infliximab, Certolizumab, Afelimomab, CytoFab, AME 527, Vapaliximab, Avastin, Vitaxin, Belimumab, MLN 1202, Volociximab, F200 (anti-α5β1), Efalizumab, m60.11 (anti-CD11b), Etanercept, Onerecept, Natalizumab und Siplizumab umfasst.

In einer Ausführungsform des dritten Aspektes erfolgt die Prävention und/oder Behandlung mit einem Medikament gemäß dem dritten oder vierten Aspekt in Kombination mit der photodynamischen Therapie mit Visodyne.

In einer Ausführungsform des dritten Aspektes ist die Erkrankung AMD und die Prävention und/oder Behandlung der AMD erfolgt mit einem Medikament gemäß dem dritten oder vierten Aspekt in Kombination mit einem Medikament, das ausgewählt ist aus der Gruppe umfassend Visodyne, VEGF-Inhibitoren und α5β1-Inhibitoren.

In einer Ausführungsform des dritten Aspektes erfolgt die Prävention und/oder Behandlung mit einem Medikament gemäß dem dritten oder vierten Aspekt in Kombination mit einem Medikament ausgewählt aus der Gruppe umfassend Acetylsalicylsäure, Ibuprofen, Diclofenac und Naproxen.

In einer Ausführungsform des dritten Aspektes erfolgt die Prävention und/oder Behandlung mit einem Medikament gemäß dem dritten oder vierten Aspekt in Kombination mit einem Medikament, das aus der Gruppe ausgewählt ist, die Antagonisten des Bradykininrezeptors 1 und Antagonisten des Bradykininrezeptors 2 umfasst.

In einer bevorzugten Ausführungsform des dritten Aspektes dient die Prävention und/oder Behandlung der Behandlung von akuten inflammatorischen Erkrankungen, wobei die Erkrankung ausgewählt ist aus der Gruppe umfassend Sepsis, schwere Verbrennungen, Reperfusionsschaden, Myokardinfarkt, Organabstoßung und hämorrhagischer Schock.

In einer noch bevorzugteren Ausführungsform des dritten Aspektes dient die Prävention und/oder Behandlung der Behandlung von chronischen Autoimmunerkrankungen und/oder der Behandlung von Infektionserkrankungen.

Erfindungsgemäß wird die Aufgabe in einem fünften Aspekt gelöst durch die Kombination aus einem Antagonisten des Bradykininrezeptors 2 und einem C5a-Rezeptor-Antagonisten zur Therapie schwerer Verbrennungen.

Wie hierin verwendet, bezeichnet der Begriff Medikament nach dem dritten oder vierten Aspekt ein solches Medikament, wie es gemäß der hierin enthaltenen Offenbarung unter Verwendung der erfindungsgemäßen Verbindungen hergestellt ist oder hergestellt werden kann bzw. wie es im Zusammenhang mit dem zweiten, dritten bzw. vierten Aspekt hierin definiert ist.

Der vorliegenden Erfindung liegt dabei genauer die überraschende Erkenntnis zugrunde, dass die bi- und/oder trisubstituierten Harnstoffverbindungen gemäß der vorliegenden Erfindung, die zumindest einen aromatischen Substituenten an einem jeden der beiden N-Atome des Harnstoffes aufweisen, eine hohe Aktivität als C5a-Rezeptor-Antagonisten besitzen. Weiterhin wurde überraschenderweise festgestellt, dass die vorteilhaften Eigenschaften der Verbindungen insbesondere auf die Anwesenheit der Gruppe NR21R22 an einem der beiden aromatischen Substituenten der erfindungsgemäßen Harnstoffderivate zurückzuführen ist. Diese Gruppe NR21R22 begründet im Übrigen auch den Unterschied zu den in den internationalen Patentanmeldungen WO0214265 und WO0222556 der Firma Mitsubishi Pharma beschriebenen Verbindungen. In einer bevorzugten Ausführungsform der erfindungsgemäßen Verbindungen handelt es sich bei der Gruppe NR21R22 um eine Aminogruppe.

Neben einer hohen Aktivität als C5a-Rezeptor-Antagonisten können die beschriebenen Verbindungen eine Reihe von weiteren günstigen Eigenschaften besitzen, so z.B. eine gegenüber dem Stand der Technik erhöhte Spezifität, verringerte agonistische Wirkung, erhöhte Affinität, erhöhte Wasserlöslichkeit, erhöhte Lagerstabilität, verringerte chemische Reaktivität, erhöhte mikrosomale Stabilität, oder verringerte Inhibierung von P450 Enzymen.

Dies gilt insbesondere auch gegenüber den in den vorstehenden internationalen Patentanmeldungen WO0214265 und WO0222556 beschriebenen Verbindungen.

Ohne im Folgenden darauf festgelegt sein zu wollen gehen die vorliegenden Erfinder davon aus, dass die NR21R22-Gruppe an dem Aromaten für eine Reihe der bei den erfindungsgemäßen Verbindungen beobachteten Vorteilen verantwortlich oder an deren Vermittlung beteiligt ist: Diese Gruppe kann die Wasserlöslichkeit der Verbindungen erhöhen, da sie vorteilhaft mit Wassermolekülen wechselwirken kann. Durch die Eigenschaft, Wasserstoffbrücken zu bilden, kann die Gruppe auch eine Bindungswechselwirkung mit biologischen Rezeptoren eingehen, was sich z.B. in erhöhter antagonistischer Aktivität äußert oder äußern kann. Die Gruppe kann durch eine höhere Hydrophilie auch die Rezeptorspezifität erhöhen oder zu einer geringeren Cytochrom-Inhibierung führen, da hydrophobe Substanzen häufig dazu neigen, unspezifische Bindungen einzugehen und von Cytochromen umgesetzt zu werden.

Ein weiterer Aspekt der vorliegenden Erfindung ist, dass bestimmte bevorzugte Verbindungen ein Stereozentrum enthalten. Bevorzugterweise ist ein Stereozentrum in alpha-Position zu einem aromatischen Ring positioniert und besonders bevorzugterweise besitzt das Stereozentrum die (S)-Konfiguration.

(S)-Konfiguration wird z.B. erreicht, wenn der Molekülteil 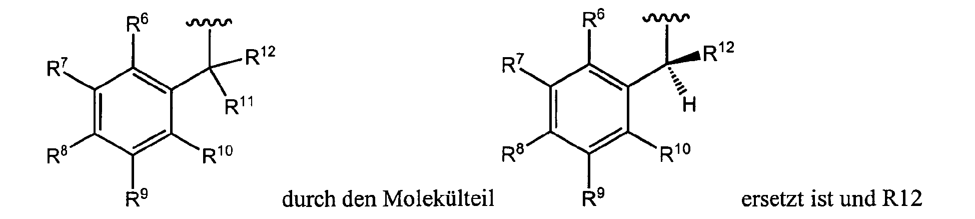 ausgewählt ist aus der Gruppe, die Alkyl, substituiertes Alkyl und Halogen umfaßt.

Die geeignete Wahl eines Stereozentrums kann sich z.B. positiv auf die antagonistische Aktivität eines Moleküls auswirken.

Trotz der positiven Eigenschaften verschiedener Verbindungen mit einem Stereozentrum können aber ausdrücklich auch Verbindungen ohne Stereozentrum sehr günstige Eigenschaften besitzen. Wenn ein Stereozentrum vorhanden ist, liegt es bevorzugterweise in der (S)-Konfiguration. Vor.

Eine weiteres Merkmal der vorliegenden Erfindung ist, dass verschiedene Verbindungen einen H-Donor an der Position von R3 in der Struktur (IV) enthalten. In soweit betrifft die vorliegenden Erfindung insbesondere auch jene Verbindungen, die unter die hierin offenbarten allgemeineren oder generischen Formeln fallen, die einen H-Donor an einer Position besitzen, die der Position von R3 in der Struktur (IV) entspricht.]

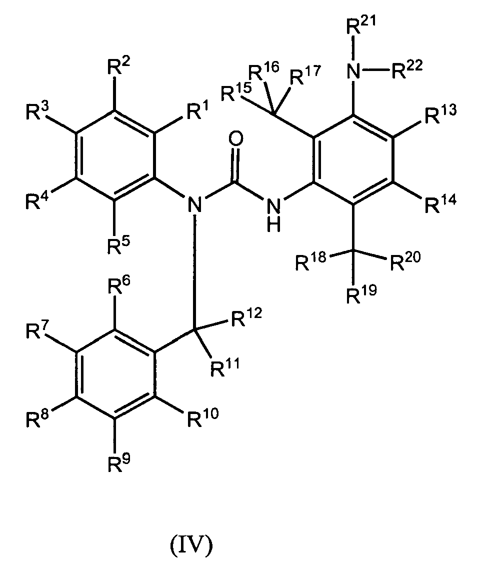

Trotz der positiven Eigenschaften verschiedener Verbindungen mit einem H-Donor an der Position von R3 können aber ausdrücklich auch Verbindungen ohne einen H-Donor an der besagten Position sehr günstige Eigenschaften besitzen.

Positive Eigenschaften der erfindungsgemäßen Verbindungen können ausdrücklich auch durch weitergehende strukturelle Merkmale der Verbindungen hervorgerufen werden. So kann durch die geeignete Wahl von Substituenten eine höhere chemische Stabilität erzielt werden, als dies mit Verbindungen gemäß des Standes der Technik erreicht wurde. In Beispiel 25 wird ein solches Beispiel beschrieben, in dem eine Verbindung gemäß des Standes der Technik, die nicht lagerstabil ist, durch Modifikation von 5 verschiedenen Resten in eine Verbindung gemäß der vorliegenden Erfindung überführt wird, die hohe Lagerstabilität aufweist.

Die Verbindungen gemäß der vorliegenden Erfindung wurden in einem primären Test auf ihre 1C₅₀-Werte in einem funktionellen Testsystem geprüft (Köhl 1997 The Anaphylatoxins. In: Dodds, A.W., Sim, R.B. (Eds.), Complement: A Practical Approach. Oxford: 135). Bevorzugterweise gelten alle Verbindungen im Sinne der vorliegenden Erfindung als nennenswert inhibitorisch aktiv, die einen IC₅₀-Wert von weniger als 200 nM in einem funktionellen Assay, wie er in Beispiel 32 beschrieben ist, zeigen.

Einige besonders bevorzugte Beispiele für Verbindungen gemäß der vorliegenden Erfindung sind in Tabelle 1 und im Folgenden angegeben (es ist jeweils der IC₅₀-Wert für die Inhibition C5a-getriggerter Enzymfreisetzung gemäß Beispiel 32 angegeben): 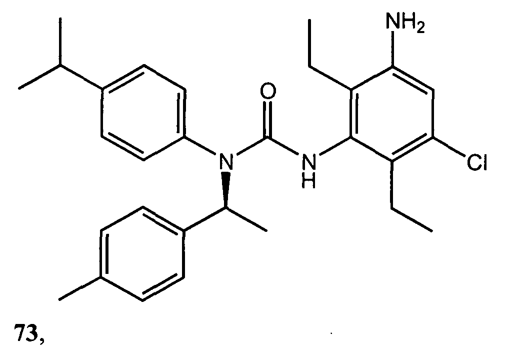 (S)-3-(3-Amino-5-chlor-2,6-diethyl-phenyl)-1-(4-isopropyl-phenyl)-1-(1-p-tolyl-ethyl)-harnstoff; 32 nM 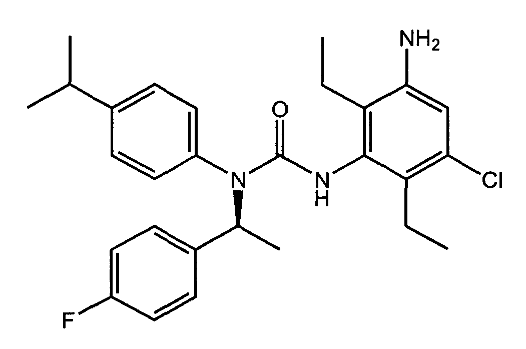 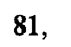 (S)-3-(3-Amino-5-chlor-2,6-diethyl-phenyl)-1-[1-(4-fluor-phenyl)-ethyl]-1-(4-isopropylphenyl)-harnstoff; 38 nM 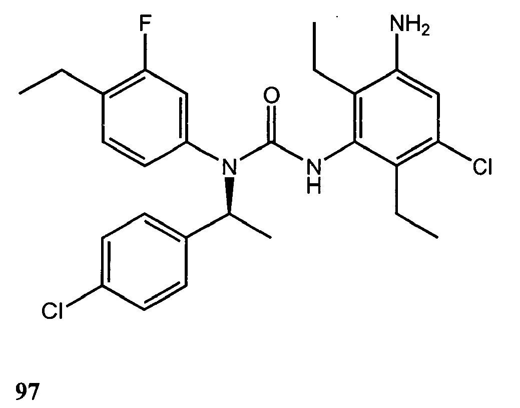 (S)-3-(3-Amino-5-chlor-2,6-diethyl-phenyl)-1-[1-(4-chlor-phenyl)-ethyl] -1-(4-ethyl-3-fluorphenyl)-harnstoff; 35 nM 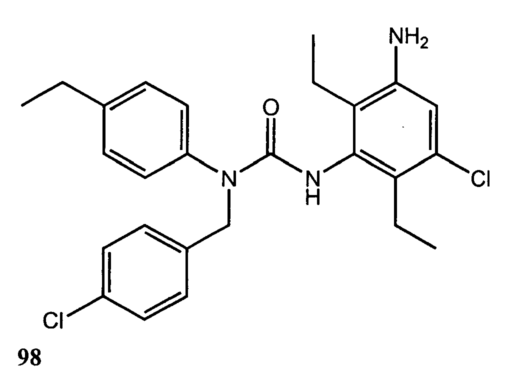 3-(3-Amino-5-chlor-2,6-diethyl-phenyl)-1-(4-chlor-benzyl)-1-(4-ethyl-phenyl)-harnstoff; 86 nM 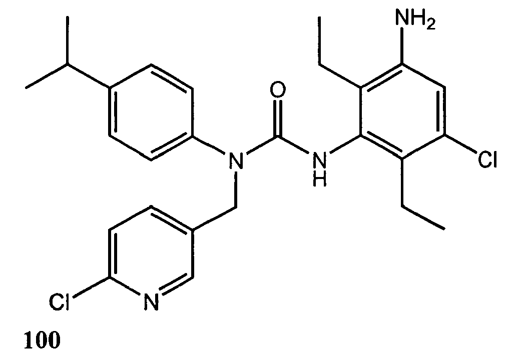 3-(3-Amino-5-chlor-2,6-diethyl-phenyl)-1-(6-chlor-pyridin-3-ylmethyl)-1-(4-isopropylphenyl)-hamstoff; 62 nM 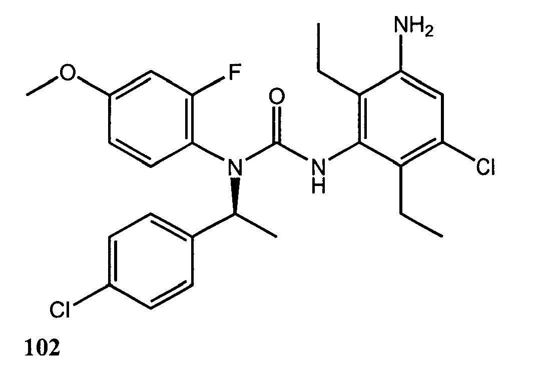 (S)-3-(3-Amino-5-chlor-2,6-diethyl-phenyl)-1-[1-(4-chlor-phenyl)-ethyl]-1-(2-fluor-4-methoxy-phenyl)-harnstoff; 31 nM 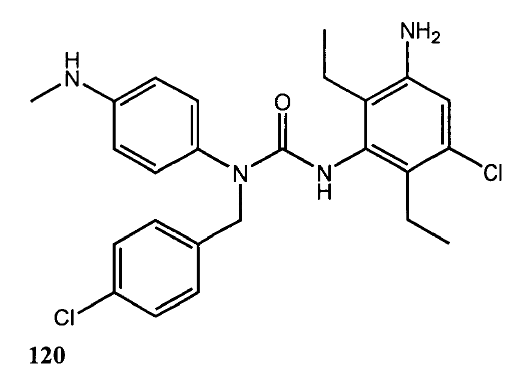 3-(3-Amino-5-chlor-2,6-diethyl-phenyl)-1-(4-chlor-benzyl)-1-(4-methylamino-phenyl)-harnstoff
19 nM 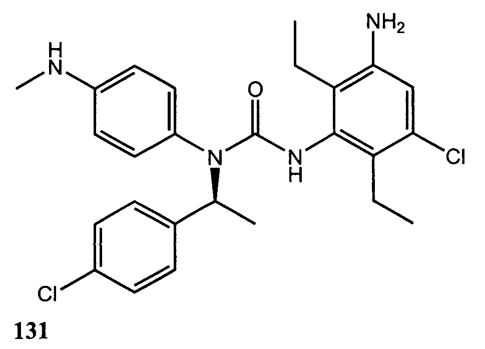 (S)-3-(3-Amino-5-chlor-2,6-diethyl-phenyl)-1-[1-(4-chlor-phenyl)-ethyl]-1-(4-methylamino-phenyl)-harnstoff; 3 nM 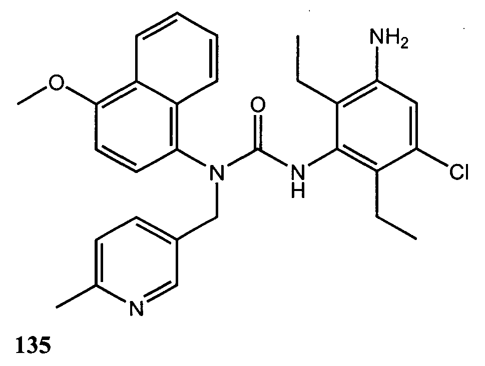 3-(3-Amino-5-chlor-2,6-diethyl-phenyl)-1-(4-methoxy-naphthalin-1-yl)-1-(6-methyl-pyridin-3-ylmethyl)-harnstoff; 55 nM

Es zeigt sich, dass viele Verbindungen gemäß der vorliegenden Erfindung eine deutlich höhere antagonistische Aktivität besitzen, als dies für die Verbindung W-54011 (89 nM) gemäß des Standes der Technik der Fall ist.

In einer bevorzugten Ausführungsform zeigen die erfindungsgemäßen Verbindungen das Fehlen einer agonistischen Aktivität in einem zellulären Assay bis zu einer Konzentration von mindestens 7100 nM wie in Beispiel 33 gezeigt. Ein erfindungsgemäßer Antagonist, wie hierin bevorzugt verwendet, zeigt keinen Agonismus, wenn der Antagonist bei einer Konzentration von mindestens 7100 nM bevorzugterweise weniger als 5% der maximal durch C5a zu erreichenden Glucosaminidase-Ausschüttung erreicht. Besonders bevorzugt ist die Ausschüttung von bis zu 1% der maximal von C5a ausgelösten Enzymfreisetzung bei einer Konzentration einer erfindungsgemäßen Verbindung von 7100 nM in einem derartigen Assay. Beispiel 33 zeigt die Ergebnisse von Messungen einer Auswahl der erfindungsgemäßen Verbindungen mittels eines Verfahrens, mit dem der Agonismus bzgl. des C5a-Rezeptors bestimmt wird. Keine der untersuchten Verbindungen ist ein Agonist.

Nachfolgend werden einige Begrifflichkeiten angegeben, deren Bedeutung für bevorzugte Ausführungsformen der vorliegenden Erfindung, insbesondere jene, wie sie detaillierter hierin angegeben sind, herangezogen werden sollen. Obwohl diese Begrifflichkeiten gelegentlich als Definitionen bezeichnet werden, ist der Begriffsinhalt der verschiedenen Begriffe nicht notwendigerweise darauf beschränkt.

Der Begriff "enthält" bedeutet in bevorzugten Ausführungsformen, dass das jeweilige Strukturelement enthalten ist, aber die Struktur nicht darauf beschränkt ist.

Der Begriff "substituiert" bedeutet in bevorzugten Ausführungsformen, dass ein oder mehrere Wasserstoffatome einer Gruppe oder Verbindung die substituiert ist, durch ein oder mehrere andere(s) Atom(e), Gruppe(n) von Atomen, Molekül(e) oder Molekülgruppe(n) ersetzt ist (sind). Ein solches Atom, Gruppe von Atomen, Moleküle, sowie Molekülgruppen wird/werden hierbei selbst als Substituenten oder Substitutionen bezeichnet. Bei der Substitution ist vorrausgesetzt, dass die normale Valenz des jeweiligen Atomes nicht überschritten wird und dass die Substitution in einer stabilen Verbindung resultiert. Durch die Substitution zweier Wasserstoff-Atome kann eine Carbonyl-Gruppe (C=O) entstehen. Carbonyl-Substituenten sind bevorzugterweise nicht innerhalb von aromatischen Einheiten enthalten.

Substituenten oder Substitutionen können bevorzugt einzeln oder in beliebiger Kombination aus der Gruppe ausgewählt sein die H, Alkyl, substituiertes Alkyl, Alkenyl, substituiertes Alkenyl, Alkynyl, substituiertes Alkynyl, Cycloalkyl, substituiertes Cycloalkyl, Heterocyclyl, substituiertes Heterocyclyl, Aryl, substituiertes Aryl, Heteroaryl, substituiertes Heteroaryl, Arylalkyl, substituiertes Arylalkyl, Heteroarylalkyl, substituiertes Heteroarylalkyl, Alkoxyl, substituiertes Alkokyl, Aryloxy, substituiertes Aryloxy, Arylalkyloxy, substituiertes Arylalkyloxy, Acyloxy, substituiertes Acyloxy, Halogen, Hydroxyl, Nitro, Cyano, Acyl, substituiertes Acyl, Mercapto, Alkylthio, substituiertes Alkylthio, Amino, substituiertes Amino, Alkylamino, substituiertes Alkylamino, Dialkylamino, substituiertes Dialkylamino, cyclisches Amino, substituiertes cyclisches Amino, Carbamoyl (-CONH₂), substituiertes Carbamoyl, Carboxyl, Carbamat, Alkoxycarbonyl, substituiertes Alkoxycarbonyl, Acylamino, substituiertes Acylamino, Sulfamoyl (-SO₂NH₂), substituiertes Sulfamoyl, Haloalkyl, Haloalkyloxy, -C(O)H, Trialkylsilyl und Azido umfasst._Jeder Substituent kann wiederum selbst durch einen oder mehrere weitere Substituenten substituiert sein. Dies gilt speziell für Alkyl, Cycloalkyl, Heterocyclyl, Aryl, Heteroaryl und Aryloxy. Weiterhin gelten jegliche hierin angegebenen Definitionen auch für Substituenten.

Unter dem Begriff "Alkyl" versteht man in einer Ausführungsform der vorliegenden Erfindung ein gesättigtes aliphatisches Radikal bestehend aus 1 bis 10 Kohlenstoffatomen oder ein einfach oder mehrfach ungesättigtes aliphatisches Kohlenwasserstoffradikal, welches zwischen 2 und 12 Kohlenstoffatome sowie mindestens eine Doppel- oder Dreifachbindung enthält. Der Begriff "Alkyl" schließt sowohl geradkettige als auch verzweigte Alkylreste ein. Bevorzugt sind geradkettige Alkylreste mit 1 bis 8 Kohlenstoffatomen. Besonders bevorzugt sind geradkettige Alkylreste mit 1 bis 6 Kohlenstoffatomen sowie verzweigte Alkylreste mit 3 bis 6 Kohlenstoffatomen. Weiterhin beinhaltet der Begriff "Alkyl" jegliche Analoga, die sich aus Kombinationsbezeichnungen der Vorsilben "Alk" oder "Alkyl" zusammensetzen lassen.

In einer bevorzugten Ausführungsform bezeichnet die Abkürzung Me einen Mehtyl-Rest oder ein Methylradikal, die Abkürzung Et einen Ethyl-Rest oder ein Ethylradiakal und die Abkürzung Pr einen Propyl-Rest oder ein Propylradikal.

Beispielsweise beschreibt der Begriff "Alkoxy" oder "Alkylthio" eine Alkylgruppe, die über einen Sauerstoff bzw. einen Schwefel gebunden ist. Unter "Alkanoyl" versteht man eine Alkylgruppe, die über eine Carbonylgruppe (C=O) gebunden ist.

Unter dem Begriff "Cycloalkyl" versteht man in einer Ausführungsform der vorliegenden Erfindung zyklische Derivate einer Alkylgruppe nach obiger Definition, die optional ungesättigt und/oder substituiert sein kann. Bevorzugt sind gesättigte Cycloalkylgruppen, insbesondere Cycloalkylgruppen mit 3 bis 8 Kohlenstoffatomen. Besonders bevorzugt sind Cycloalkylgruppen, die 3 bis 6 Kohlenstoffatome enthalten.

Der Begriff "Aryl" bezeichnet in einer Ausführungsform der vorliegenden Erfindung einen aromatischen Rest mit 6 bis 14 Kohlenstoffatomen, wobei "substituiertes Aryl" für Arylreste steht, die einen oder mehrere Substituenten tragen.

Jede der obig definierten Gruppen "Alkyl", "Cycloalkyl", und "Aryl" schließt die jeweiligen halogenierten Derivate mit ein, die ein oder mehrere Halogenatome tragen können. Die halogenierten Derivate beinhalten jegliches Halogenradikal nach der folgenden Definition.

"Halogen" bezeichnet in einer Ausführungsform der vorliegenden Erfindung ein Halogenradikal aus der Gruppe Fluor, Chlor, Brom und Jod. Bevorzugt hierbei sind Fluor, Chlor und Brom.

Der Begriff "Heteroaryl" bezeichnet in einer Ausführungsform der vorliegenden Erfindung ein 5- bis 8-gliedriges, bevorzugt 5- bis 6-gliedriges, monozyklisches oder 8- bis 11-gliedriges bizyklisches, aromatisches, heterozyklisches Radikal, wobei jeder Heterozyklus sowohl aus Kohlenstoffatomen, sowie 1 bis 4 Heteroatomen aus der Reihe N, O oder S bestehen kann. Der Heterozyklus kann durch jedes Atom des Zyklus' verbunden sein, so dass eine stabile Struktur resultiert. Bevorzugte Heteroarylradikale im Rahmen dieser Erfindung sind beispielsweise Furyl, Thienyl, Pyrrolyl, Oxazolyl, Thiazolyl, Imidazolyl, Pyrazolyl, Isoxazolyl, Oxadiazolyl, Triazolyl, Tetrazolyl, Thiadiazolyl, Pyridinyl, Pyridazinyl, Pyrimidinyl, Pyrazinyl, Indolizinyl, Indolyl, Isoindolyl, Benzofuranyl, Benzothienyl, Indazolyl, Benzimidazolyl, Benzthiazolyl, Benzoxazolyl, Purinyl, Chinolizinyl, Chinolinyl, Isochinolinyl, Cinnolinyl, Phthalazinyl, Chinazolinyl, Chinoxalinyl, Naphthridinyl, Pteridinyl, Carbazolyl, Acridinyl, Phenazinyl, Phenothiazinyl und Phenoxazinyl.

Unter dem Begriff "Heterocyclyl" versteht man in einer Ausführungsform der vorliegenden Erfindung ein 5- bis 8-gliedriges, bevorzugt 5- bis 6-gliedriges, monozyklisches oder 8- bis 11-gliedriges bizyklisches, heterozyklisches Radikal, welches entweder gesättigt oder ungesättigt, jedoch nicht aromatisch ist. Jeder Heterozyklus besteht sowohl aus Kohlenstoffatomen, sowie 1 bis 4 Heteroatomen aus der Reihe N, O oder S. Der Heterozyklus kann durch jedes Atom des Zyklus verbunden sein, so dass eine stabile Struktur resultiert. Bevorzugte Heteroarylradikale im Rahmen dieser Erfindung sind beispielsweise Pyrrolinyl, Pyrrolidinyl, Pyrazolinyl, Pyrazolidinyl, Piperidinyl, Morpholinyl, Thiomorpholinyl, Pyranyl, Thiopyranyl, Piperazinyl, Indolinyl, Azetidinyl, Tetrahydropyranyl, Tetrahydrothiopyranyl, Tetrahydrofuranyl, Hexahydropyrimidinyl, Hexahydropyridazinyl, 2,5-Dioxo-hexahydropyrimidin-4-yl, 2,6-Dioxo-piperidin-4-yl, 2-Oxo-hexahydro-pyrimidin-4-yl, 2,6-Dioxohexahydro-pyrimidin-4-yl, 3,6-Dioxo-piperazin-2-yl, 1,4,5,6-Tetrahydropyrimidin-2-ylamin, Dihydro-oxazolyl, 1,2-thiazinyl-1,1-dioxid, 1,2,6-Thiadiazinanyl-1,1-dioxid, Isothiazolidinyl-1,1-dioxid und Imidazolidinyl-2,4-dion.

Werden die Begriffe "Heterocyclyl", "Heteroaryl" und "Aryl" zusammen mit anderen Ausdrücken oder Begriffen benutzt gelten weiterhin obige Definitionen. Zum Beispiel beschreibt der Begriff "Aroyl" einen Phenyl- oder Naphthylrest, der an eine Carbonylgruppe (C=O) gebunden ist.

Jede Aryl- oder Heteroarylverbindung beinhaltet außerdem die teilweise oder vollständig hydrierten Derivate. Zum Beispiel kann Chinolinyl auch Decahydrochinolinyl und Tetrahydrochinolinyl einschließen. Naphthyl kann auch die hydrierten Derivate wie beispielsweise Tetrahydronaphthyl mit einschließen.

Im Rahmen dieser Erfindung sind mit den Ausdrücken "Stickstoff" oder "N" und "Schwefel" oder "S" auch jegliche oxidierten Derivate des Stickstoffs wie Nitrone, N-Oxide oder des Schwefels wie Sulfoxide, Sulfone sowie quarternierte Formen basischen Stickstoffs wie HCl- oder andere dem Fachmann bekannte Salze mit eingeschlossen.

Der Begriff Bindung, insbesondere wie hierin im Zusammenhang mit Substituenten verwendet, bezeichnet bevorzugterweise Einfachbindungen, sofern nichts Gegenteiliges angegeben ist.

Radikale können sowohl Mono-, als auch Di-, Tri- oder Tetraradikale sein. Dadurch ist es möglich, dass sich auch die Bedeutung verschiedener Begriffe leicht ändert. So bedeutet ein Diradikal, das als "Propyl" beschrieben wird, automatisch "Propyplen" (z.B. -(CH₂)₃-).

Beschreibungen, die die Grenzen eines Bereichs wie beispielsweise "1 bis 5" spezifizieren, bedeuten jede ganze Zahl von 1 bis 5. Im einzelnen 1, 2, 3, 4 und 5. Mit anderen Worten beinhaltet jeder Bereich, der durch zwei ganze Zahlen beschrieben ist, sowohl die beiden ganzen Zahlen der Definitionsgrenzen, als auch alle ganzen Zahlen innerhalb dieses Bereichs.

Die vorliegende Erfindung beinhaltet alle Isotope von Atomen in den beschriebenen Verbindungen. Isotope sind Atome, die die gleiche Ordnungszahl aber verschiedene Massenzahl haben. Beispielsweise sind das Tritium und das Deuterium Isotope von Wasserstoff. Beispiele für Kohlenstoff-Isotope sind ¹¹C, ¹³C und ¹⁴C.

Bei den hierin angegebenen Gruppendefinitionen bezeichnet der Begriff "und jeweilige Derivate davon", dass alle Derivate der in der Gruppe angeführten Einzelverbindungen, Gruppen von Verbindungen, Molekülteile, Radikale oder chemische Gruppen jeweils als Derivate vorliegen können. Im Übrigen ist es im Rahmen der vorliegenden Erfindung, dass bei Verwendung der einzelnen Gruppendefinitionen die entsprechenden Derivate davon mit umfasst sind.

Die Begrifflichkeit "einzeln und unabhängig voneinander" oder "jeweils einzeln und unabhängig" bezeichnet hierin, dass die zwei oder mehr angeführten Substituenten so ausgebildet werden können, wie in der entsprechenden Passage beschrieben. Diese Formulierungen sollen dabei lediglich unnötige Wiederholungen vermeiden und offenbart, dass ein jeglicher der angesprochenen Substituenten die beschriebene Ausgestaltung aufweisen kann, wobei die Ausgestaltung für jeden einzelnen Substituenten für sich erfolgt oder vorliegt und nicht durch die Auswahl einer oder mehrerer der anderen Substituenten beeinflusst wird. Dabei ist es im Rahmen der vorliegenden Erfindung, dass eine jegliche Kombination, einschließlich einer jeden Unterkombination, der solchermaßen kollektiv definierten Reste oder Substituenten miteinander und/oder untereinander damit hierein offenbart wird.

Es ist allgemein im Rahmen der vorliegenden Erfindung, dass die für die einzelnen erfindungsgemäßen Verbindungen, insbesondere die generischen Strukturen, angegebenen Substituenten für alle generischen Formeln mit den entsprechenden Substituenten gelten, sofern nichts gegenteiliges ausgeführt wird.

Eine jegliche Verbindung der vorliegenden Erfindung, die ein oder mehrere asymmetrische Kohlenstoffatome enthält, kann als Racemat oder racemische Mischung, einzelnes Enantiomer, diasteromere Mischung, einzelnes Diastereomer oder jeweils ein Gemisch dergleichen auftreten. Alle derartigen isomeren Formen dieser Verbindungen sind ausdrücklich in der vorliegenden Erfindung umfasst. Ein jedes stereogene Kohlenstoffatom kann in der S- oder R-Konfiguration oder einem Gemisch beider Konfigurationen vorliegen.

Es ist im Rahmen der vorliegenden Erfindung, dass die erfindungsgemäßen Verbindungen Vorstufen von pharmazeutisch aktiven Verbindungen sind. Solche pharmazeutisch aktiven Verbindungen können aus den erfindungsgemäßen Verbindungen durch Metabolismus und jegliche Form von Abbau oder Zerfall entstehen. Entsprechende Reaktionen sind dem Fachmann bekannt (Yan et al. 2001 Curr. Top. Med. Chem. 1:403; Fura et al. 2004 J. Med. Chem. 47:4339; Lin et al. 1997 Pharmacological Reviews 49:403 und die in diesen Stellen zitierte Literatur). Weiterhin ist es im Rahmen der vorliegenden Erfindung, dass diese Vorstufen zu den hierin offenbarten Verbindungen umgewandelt werden. Diese Vorstufen können beispielsweise so genannte Prodrugs sein. Entsprechende allgemein anwendbare Konzepte zur Herstellung von Prodrugs aus den erfindungsgemäßen Verbindungen sind dem Fachmann bekannt (Anand et al. 2002 Expert Opin. Biol. Ther. 2:607; Majumdar et al. 2004 Adv. Drug Deliv. Rev. 56:1437; Wang et al. 1999 Curr. Pharm. Des. 5:265; Shan et al. 1997 J. Pharm. Sci. 86:765, und die in diesen Stellen zitierte Literatur).

Die vorliegende Erfindung betrifft auch Zusammensetzungen oder Formulierungen, insbesondere pharmazeutische Zusammensetzungen oder Formulierungen, die zumindest eine der erfindungsgemäßen Verbindungen enthalten. Häufig werden pharmazeutische Wirkstoffe mit anderen pharmazeutisch akzeptablen Bestandteilen gemischt, um eine verbesserte Wirkung wie beispielsweise verbesserten Transport, verbesserte Haltbarkeit, verbessertes zeitliches Verhalten bei der Freisetzung und dergleichen zu gewährleisten. Dem Fachmann ist eine Vielzahl entsprechender Formulierungen bekannt. Als Bestandteile derartiger Formulierungen sind unter anderem inerte Verdünnungsmittel, Calciumcarbonat, Natriumcarbonat, Lactose, Calciumphosphat, Natriumphosphat, Stärke, Alginate, Gelatine, Magnesiumstearat und Talk bekannt. Bestimmte Bestandteile können beigefügt werden, um eine zeitverzögerte Freisetzung der pharmazeutischen Wirkstoffe zu ermöglichen. Beispiele dafür sind Glycerolmonostearat und Glyceroldistearat. Zur oralen Applikation werden insbesondere Hartgelatinekapseln verwendet, wobei der pharmazeutisch aktive Bestandteil mit Calciumcarbonat, Calciumphosphat oder Kaolin gemischt wird. Bei Weichgelatinekapseln werden die pharmazeutisch aktiven Wirkstoffe z.B. mit Ölen gemischt (Erdnussöl, flüssiges Paraffin, Olivenöl). Für die Applikation in wässerigen Lösungen können die pharmazeutisch wirksamen Bestandteile insbesondere mit folgenden Bestandteilen gemischt werden: Carboxymethylcellulose, Methylcellulose, Hydropropylmethylcellulose, Natriumalgenat, Polyvinylpyrrolidon, Lecithin, Polymerisierungsprodukte von Alkylenoxiden und Fettsäuren wie beispielsweise Polyoxyethylenstearat, Heptadecaethylenoxycetanol, Polyoxyethylensorbitolmonooleat und Polyoxyethylensorbitanmonooleat. Zur Konservierung können verschiedene Zusatzstoffe zum Einsatz kommen. Beispiele dafür sind Ethyl- oder n-Propyl-p-hydroxybenzonat.

Bestimmte Formulierungen werden eingesetzt, um spezielle Applikationsformen zu ermöglichen. Die erfindungsgemäßen Verbindungen können dabei als pharmazeutisch akzeptable Salze oder Solvate vorliegen. Abhängig von der im einzelnen zu behandelnden Erkrankung können die Verbindungen gemäß der vorliegenden Erfindung systemisch oder lokal, bei systemischen Erkrankungen bevorzugt systemisch bzw. bei lokalen Erkrankungen (z.B. Alopecia areata) lokal verabreicht und entsprechend formuliert werden. Verfahren zum Formulieren und Verabreichen sind beispielsweise in "Remington's Pharmaceutical Sciences", 1990, 18. Auflage, Mack Publishing Co., Easton, PA beschrieben. Die Verabreichung einer Verbindung gemäß der vorliegenden Erfindung kann auf verschiedene Art und Weise erfolgen, einschließlich, aber nicht beschränkt auf, oral, buccal, subkutan, intravenös, intranasal, transdermal, intraperitonial, intramuskulär, intrapulmonär, vaginal, rektal, intraokular, periokular, intraorbital, intrakapsulär, intrasynovial, intrazisternal oder topisch, um nur einige anzuführen.

Für die nasale Anwendungsform werden die Verbindungen pur eingesetzt oder mit den dafür üblichen Zusatzstoffen wie Stabilisatoren oder inerten Verdünnungsmitteln vermischt und durch übliche Methoden, wie wäßrigen, alkoholischen oder öligen Suspensionen oder wäßrigen, alkoholischen oder öligen Lösungen in geeignete Darreichungsformen gebracht. Wäßrigen intranasalen Zubereitungen können Chelatbildner, wie z.B. Ethylendiamin-N,N,N',N'-Tetraessigsäure, Citronensäure, Weinsäure oder deren Salze zugefügt werden. Die Applikation der Nasallösungen kann mittels Dosierzerstäuber erfolgen oder als Nasaltropfen mit viskositätserhöhendem Anteil bzw. Nasengels oder Nasencremes.

Für die inhalativen Anwendungen können Vernebler oder Druckgaspackungen unter Verwendung inerter Trägergase benutzt werden.

Ein Präparat für die topische Anwendung kann als wäßrige oder ölige Lösung, Lotion, Emulsion oder Gelee, Salbe oder Fettsalbe oder, falls möglich, in Sprayform vorliegen, wobei gegebenenfalls durch Zusatz eines Polymers die Haftung verbessert werden kann.

Ein geeigneter Dosisbereich für topische und inhalative Anwendungsformen der erfindungsgemäßen Verbindungen sind Lösungen mit 0.01 bis 5 mg/ml. Bei systemischen Applikationsformen sind 0.01 bis 50 mg/kg geeignet.

Für Injektionszwecke kann die erfindungsgemäße Verbindung als wässrige Lösung mit oder ohne Lösungsvermittlern, bevorzugterweise in physiologisch kompatiblen Puffern wie beispielsweise Hank's Lösung, Ringer-Lösung oder physiologischem Salinepuffer formuliert sein. Für die transmukosale Verabreichung werden Penetranzien verwendet, die die erfindungsgemäße Verbindung dabei unterstützen, die zu penetrierende Barriere zu überwinden. Derartige Penetranzien sind im Stand der Technik bekannt.

Die Verwendung von pharmazeutisch akzeptablen Trägern, um die Verbindung gemäß der vorliegenden Erfindung in Dosierung oder pharmazeutische Zusammensetzung zu formulieren, die insbesondere für die systemische Verabreichung geeignet sind, ist im Rahmen der vorliegenden Erfindung. Mit einer geeigneten Auswahl des Trägers und einem geeigneten Herstellungsverfahren kann die Zusammensetzung der vorliegenden Erfindung, insbesondere wenn diese als Lösungen vorliegen, parenteral, wie beispielsweise durch intravenöse Injektion verabreicht werden. Die erfindungsgemäßen Verbindungen können leicht unter Verwendung pharmazeutisch akzeptabler Träger, die in der Technik gut bekannt sind, in Dosierungen formuliert werden, die für die orale Verabreichung geeignet sind. Derartige Träger erlauben, dass die Verbindungen der vorliegenden Erfindung als Tabletten, Pillen, Kapseln, Dragees, Flüssigkeiten, Gele, Sirups, Aufschlämmungen, Suspensionen und dergleichen, für orale Aufnahme durch einen zu behandelnden Patienten formuliert werden können.

Zusätzlich können Suspensionen der aktiven Verbindung als geeignete ölige Injektionssuspensionen hergestellt werden. Geeignete lipophile Lösungsmittel oder Vehikel umfassen fette Öle wie beispielsweise Sesamöl oder Rizinusöl, oder synthetische Fettsäureester, wie beispielsweise Ethyloleat oder Triglyceride oder Liposomen. Wässrige Injektionssuspensionen können Verbindungen enthalten, die die Viskosität der Suspension erhöhen, wie beispielsweise Carboxymethylzellulose, Sorbitol, Dextran oder dergleichen. Optional kann die Suspension auch geeignete Stabilisatoren oder Agenzien enthalten, die die Löslichkeit der Verbindung erhöhen, um die Herstellung von hochkonzentrierten Lösungen zu erlauben.

Pharmazeutische Zusammensetzungen, die eine Verbindung gemäß der vorliegenden Erfindung enthalten, für die orale Verwendung, können erhalten werden durch Kombinieren der aktiven Verbindung(en) mit festen Bindemitteln, optionalem Mahlen der sich ergebenden Mischung und Verarbeiten der Mischung als Granulat, nachdem geeignete Hilfsmittel, sofern erwünscht, hinzugesetzt sind, um Tabletten oder Drageekerne zu erhalten.

Geeignete Bindemittel sind, insbesondere, Füllmittel wie Zucker, einschließlich Lactose, Saccharose, Manitol, Sorbitol und dergleichen; Zellulosepräparate, wie beispielsweise Maisstärke, Weizenstärke, Reisstärke, Kartoffelstärke, Gelatine, Gummitragant, Methylzellulose, Hydroxypropylmethylzellulose, Natriumcarboxymethylzellulose, Polyvinylpyrrolidon (PVP) und dergleichen, ebenso wie Mischungen von zwei oder mehreren davon. Sofern erwünscht, können Sprengmittel hinzugefügt werden, wie beispielsweise quervernetztes Polyvinylpyrrolidon, Alginisäure oder ein Salz davon, wie beispielsweise Natriumalginat und dergleichen.

Drageekerne, die eine pharmazeutische Zusammenfassung oder eine Verbindung gemäß der vorliegenden Erfindung umfassen, werden mit geeigneten Beschichtungen versehen. Zu diesem Zweck können konzentrierte Zuckerlösungen verwendet werden, die optional enthalten können: Gummi arabikum, Talk, Polyvinylpyrrolidon, Carbopol-Gel, Polyethylenglycol, Titandioxid, geeignete organische Lösungsmittel oder Lösungsmittelmischungen und dergleichen. Farbstoffe oder Pigmente können zu den Tabletten- oder Drageebeschichtungen hinzugefügt werden, um sie zu identifizieren oder verschiedene Kombinationen oder unterschiedliche Dosierungen der aktiven Verbindung zu kennzeichnen.

Pharmazeutische Zusammensetzungen, die eine Verbindung gemäß der vorliegenden Verbindung umfassen, die oral verwendet werden können, umfassen bevorzugterweise Pushfit-Kapseln, die aus Gelatine hergestellt sind, ebenso wie Weichkapseln, die aus Gelatine und einem Weichmacher hergestellt sind, wie beispielsweise Glycerin oder Sorbitol. Die Push-fit-Kapseln können den aktiven Wirkstoff in Mischung mit einem Füllstoff wie beispielsweise Lactose, Bindemitteln wie beispielsweise Stärke und/oder Schmiermitteln, wie beispielsweise Talk oder Magnesiumsterat enthalten und optional Stabilisatoren. Bei Weichkapseln können die aktiven Verbindungen in geeigneten Flüssigkeiten gelöst oder suspendiert sein, wie beispielsweise Ölen, flüssigem Paraffin oder flüssigen Polyethylenglykolen. Zusätzlich können Stabilisatoren hinzugefügt sein.

Die Therapie mit einem erfindungsgemäßen Medikament kann mit <100 mg/kg, <50 mg/kg, <10 mg/kg, <1 mg/kg oder < 0.1 mg/kg erfolgen.

In einer Ausführungsform umfassen die pharmazeutischen Zusammensetzungen gemäß der vorliegenden Erfindung wenigstens eine Verbindung gemäß der vorliegenden Erfindung in einer Form, die für die Verabreichung in einen Patienten geeignet ist. Bevorzugterweise liegt eine Verbindung gemäß der vorliegenden Erfindung allgemein und insbesondere in einer pharmazeutischen Zusammensetzung gemäß der vorliegenden Erfindung in einer wasserlöslichen Form vor, wie beispielsweise einem pharmazeutisch akzeptablen Salz, was im Rahmen der vorliegenden Erfindung sowohl saures als auch basisches Additionssalz sein kann, das auch hierin allgemein als pharmazeutisch akzeptables Salz bezeichnet wird. "Saures Additionssalz", insbesondere "pharmazeutisch akzeptables saures Additionssalz" bezeichnet jene Salze, die die biologische Wirksamkeit der freien Base beibehalten und nicht biologisch oder medizinisch oder anderweitig unerwünscht sind. Die Salze werden bevorzugterweise ausgebildet mit anorganischen Salzen wie beispielsweise Chlorwasserstoff, Bromwasserstoff, Schwefelsäure, Salpetersäure, Phosphorsäure und dergleichen, oder organischen Säuren wie Essigsäure, Propionsäure, Glycolsäure, Bernsteinsäure, Oxalsäure, Maleinsäure, Malonsäure, Fumarsäure, Weinsäure, Zitronensäure, Benzolsäure, Zimtsäure, Mandelsäure, Methansulfonsäure, Ethansulfonsäure, p-Toluensulfonsäure, Salicylsäure und dergleichen. "Basische Additionssalze", insbesondere "pharmazeutisch akzeptable basische Additionssalze" umfassen jene, die von anorganischen Basen abgeleitet sind, wie beispielsweise Natrium-, Kalium-, Lithium-, Ammonium-, Calcium-, Magnesium-, Eisen-, Zink-, Kupfer-, Mangan-, Aluminiumsalze und dergleichen. Insbesondere bevorzugt sind Amonium-, Kalium, Calcium-, Natrium- und Magnesiumsalze. Salze, die von pharmazeutisch akzeptablen organischen nicht-toxischen Basen abgeleitet sind, umfassen bevorzugterweise auch primäre, sekundäre und tertiäre Amine, substituierte Amine, einschließlich natürlicherweise vorkommende substituierte Amine, zyklische Amine und basische Ionenaustauscherharze. Bevorzugte Amine zur Salzbildung sind Isopropylamin, Trimethylamin, Diethylamin, Dicyclohexylamin, Triethylamin, Tripropylamin und Ethanolamin.

Ein "Patient" für die Zwecke der vorliegenden Erfindung, d. h. ein Lebewesen, dem eine Verbindung gemäß der vorliegenden Erfindung oder eine pharmazeutische Zusammensetzung gemäß der vorliegenden Erfindung verabreicht wird, umfasst sowohl Menschen als auch Tiere und andere Organismen. Somit umfasst der Anwendungsbereich der Verbindungen und der pharmazeutischen Zusammensetzungen gemäß der vorliegenden Erfindung insbesondere sowohl den Bereich der Human- als auch den der Veterinärmedizin sowie Diagnostika und diagnostische Verfahren ebenso wie Staging-Verfahren für diese beiden Bereiche. Die veterinärmedizinischen Anwendungen umfassen, sind aber nicht darauf beschränkt, Affen, Hunde, Rinder, Katzen, Schweine, Esel, Pferde, Nutztiere, ebenso wie andere Haustiere, einschließlich Reptilien, wie beispielsweise Schildkröten, Schlangen und Leguane, Vögel, wie beispielsweise Finken und Mitglieder der Papageienfamilie, Lagomorpha, wie beispielsweise Kaninchen, Nagetiere wie beispielsweise Ratten, Mäuse, Meerschweinchen, Hamster, Amphibien, Fische und Arthropoden. Patienten können auch Zootiere sein. Bevorzugterweise ist der Patient ein Säugetier und am bevorzugtesten ist der Patient ein Mensch.

In einem weiteren Aspekt betrifft die vorliegende Erfindung ein Verfahren zur Behandlung und/oder Therapie von Erkrankungen, wobei das Verfahren die Verabreichung mindestens einer der erfindungsgemäßen Verbindungen oder einer Verbindung gemäß der vorliegenden Erfindung an einen Patienten umfasst. Bei der Verbindung und/oder der Zusammensetzung wird diese bevorzugt in einer Menge und/oder Form verabreicht, dass sie geeignet ist, die Erkrankung oder die damit verbundenen Symptome zu verhindern, zu lindern oder zu therapieren. Weiterhin ist bevorzugt der Patient ein solcher, der der Behandlung oder der Prävention bedarf.

Erkrankungen, auf die bevorzugt im Zusammenhang mit den verschiedenen Aspekten der vorliegenden Erfindung Bezug genommen wird, sind dabei bevorzugt jene, an denen C5a oder der C5a-Rezeptor direkt, d.h. kausal, indirekt oder symptomatisch beteiligt ist. Bevorzugt sind auch inflammatorische Erkrankungen bei denen eine Aktivierung des Complementsystems vorliegt.

Ohne im Folgenden darauf festgelegt sein zu wollen, gehen die vorliegenden Erfinder derzeit von zwei grundlegenden Pathogenitätsmechanismen von C5a aus. Der erste Mechanismus beinhaltet Vorgänge, die direkt durch die Einwirkung von C5a auf Zellen, die den C5a-Rezeptor exprimieren, ausgelöst werden. Zelltypen, die den Rezeptor exprimieren, sind unter anderem die folgenden: Astrocyten, Microglia, Neuronen, Granulozyten (Neutrophile, Basophile, Eosinophile), Mastzellen, Endothelzellen, Epithelzellen, Makrophagen, T-Zellen, dentritische Zellen, Hepatozyten, Zellen in der Niere (z.B. in den Glomeruli und dem Tubolointerstitium), der Lunge und Zellen der glatten Muskulatur. Die direkte Wirkung von C5a ist sehr vielfältig und abhängig vom betrachteten Zelltyp. Beispiele für die direkte Wirkung sind Chemotaxis und Degranulierung von Granulozyten. Die Degranulierung wiederum führt im Folgenden als indirekte Wirkung zu einer Schädigung von umliegendem Gewebe (z.B. durch Matrixmetalloproteasen, reaktiven Sauerstoff, Elastase usw.). Eine weitere direkte Wirkung ist die Verzögerung der Apoptose von Neutrophilen durch C5a (Perianayagam et al. 2004 European Journal of Clinical Investigations 34: 50) oder die Produktion von Plasminogen Aktivator Inhibitor-1 in Mastzellen und Basophilen (Wojita 2002 Blood 100: 517).

Der zweite grundlegende Pathogenitätsmechanismus beinhaltet Vorgänge, die indirekt über C5a vermittelt sind. Darunter fällt unter anderem die Beeinflussung der adaptiven Immunabwehr durch C5a und Veränderungen im Cytokinmuster, die durch die Einwirkung von C5a auf cytokinsekretierende Zellen beobachtet werden können (Jauneau et al. 2003 FEBS Letters 537: 17). Diese Effekte werden auch durch Zellen vermittelt, die nicht dem angeborenen Immunsystem zuzuordnen sind. Der C5a-Rezeptor wurde dementsprechend auch z.B. bei T-Zellen (Nataf et al. 1999 Journal of Immunology 162: 4018), B-Zellen oder Antigen-präsentierenden Zellen (APCs) gefunden. Das veränderte Cytokinmuster kann zu einer unterschiedlichen Ausdifferenzierung von T-Zellen in Th1 oder Th2 Zellen primär durch eine dosisabhängige Veränderung der IL-12 Ausschüttung (Hawlisch et al. 2004 Molecular Immunology 41: 123) führen. Weitere wichtige Cytokine, die die T-Zelldifferenzierung beeinflussen, sind z.B. IL-2, IL-4, IL-5, IL-10, IL-23, IL-27. Ähnliche Ergebnisse sind auch aus genetisch veränderten Maus-Stämmen bekannt, bei denen das C5 Gen fehlt. Derartige Tiere produzieren deutlich weniger IL-12 als entsprechende Vergleichsstämme.

Die von C5a beeinflusste Veränderung des Cytokinmusters kann auch dazu führen, dass intrazelluläre Parasiten (z.B. Viren, Leischmanien, Rickettsien, Chlamydien, Coxiella, Plasmodien, Brucella, Mycobakterien, Listerien, Toxoplasman, Trypanosomen) weniger gut durch das Immunsystem bekämpft werden können. Hawlisch et al. (2005 Immunity 22: 415) zeigen, dass Mäuse, die den C5a-Rezeptor nicht exprimieren, deutlich resistenter gegenüber Leischmanieninfektionen sind, als entsprechende Vergleichstiere, die den Rezeptor funktionell exprimieren. Damit ist es möglich, Krankheiten, die durch intrazelluläre Parasiten bzw. Viren verursacht werden, zu behandeln bzw. unterstützend zu behandeln, indem man einen erfindungsgemäßen C5a-Rezeptor-Antagonisten zur Therapie oder als unterstützende Therapie verwendet.

Die Folge der Beeinflussung des Cytokinmusters ist nicht auf eine Verschiebung des Gleichgewichts zwischen Th1 und Th2 Zellen beschränkt. Für viele Erkrankungen ist aber gezeigt worden, dass Veränderungen dieses Gleichgewichts entscheidend für die Pathogenese sind (Bamias et al. 2001 Current Opinion Investigational Drugs 11: 1279; Lucey et al. 1996 Clinical Microbiology Reviews 9: 532).

Letztlich wird eine Vielzahl von Prozessen indirekt durch C5a beeinflusst. Hierbei können natürlich auch solche Zellen und Organe betroffen sein, die keinen oder nur wenig C5a-Rezeptor exprimieren.

Es ist häufig nicht möglich, beide Pathogenitätsmechanismen so zu trennen, dass eine eindeutige Zuordnung von Erkrankungen zu einem der beiden Prozesse möglich ist. Man kann aber annehmen, dass der Anteil des ersten Pathogenitätsmechanismus in akuten Entzündungsreaktionen von größerer Bedeutung ist, während der zweite Pathogenitätsmechanismus in chronischen und Immun- bzw. Autoimmunerkrankungen eine Rolle spielt. Dass die Übergänge fließend sind, zeigt z.B. die akute Indikation Sepsis, bei der im Tiermodell durch C5a-Rezeptor Blockade (Knock-Out Tiere bzw. Antikörper oder Antagonisten) sowohl die Störung der Chemotaxis und des oxidativen Burst von Neutrophilen aufgehoben werden kann, als auch das Cytokinmuster (cytokine storm) positiv beeinflusst wird (Huber-Lang et al. 2002 FASEB Journal 16: 1567; Ward 2004 Nature Review Immunology 4: 133; Riedenmann et al. 2003 Immunity 19: 193, Riedenmann et al. 2003 Nature Medicine 9: 517; Czermak et al. 1999 Nature Medicine 5: 788, Huber-Lang et al. 2001 FASEB Journal 15: 568, Huber-Lang et al. 2001 Journal of Immunology 166: 1193).

Die Quelle von C5a ist dabei sekundär. Es ist möglich, dass C5a über die Aktivierung des Complementsystems (z.B. klassischer, alternativer oder MBL-Weg) oder direkt von bestimmten Zellen (z.B. phagocytotischen Zellen) freigesetzt wird (Huber-Lang 2002 American Journal of Pathology 161: 1849).

Beispiele für akute Indikationen bzw. Indikationen, die in akuten Schüben ablaufen können, mit C5a assoziiert und damit mit einer erfindungsgemäßen Verbindung oder einem erfindungsgemäßen Medikament behandelt werden können, sind Asthma (Köhl 2001 Molecular Immunology 38: 51), Inflammatory Bowel Disease (Morbus Crohn, Colitis ulcerosa) (Woodruff et al. 2003 Journal of Immunology 171: 5514), Sepsis bzw. septischer Schock (Huber-Lang et al. 2001 Faseb Journal 15: 568), schwere Brandverletzungen (Piccolo et al. 1999 Experimental and Molecular Pathology 66: 220) und die akuten Folgen schwerer Brandverletzungen (Organversagen, Schock, Sepsis, SIRS), Multiple Sklerose (Mullerladner et al. 1996 Journal of Neurological Science 144: 135) und Reperfusionsschaden an unterschiedlichen Organen wie z.B. Herz (Herzinfarkt), Milz, Harnblase, Bauchspeicheldrüse, Magen, Lunge, Leber, Niere, Extremitäten, Gehirn (Schlaganfall), Muskeln oder Darm (Riley et al. 2000 Journal of Thoriacic and Cardiovascular Surgery 120: 350).

Auf der Grundlage der beiden beschriebenen Pathogenitätsmechanismen können auch alle inflammatorischen oder immuno-inflammatorischen Erkrankungen unter Verwendung der erfindungsgemäßen Verbindungen behandelt oder präventiv behandelt werden. Einen Überblick über inflammatorische Erkrankungen, an denen C5a beteiligt ist, bietet Köhl (2001 Molecular Immunology 38: 51).

Immunkomplex-assoziierte Erkrankungen (Heller et al. 1999 Journal of Immunology 163: 985) sind ebenfalls Erkrankungen, die mit einem erfindungsgemäßen C5a-Rezeptor-Antagonisten behandelt werden können. Ein Beispiel für eine Immunkomplex-assoziierte oder Immunkomplex-vermittelte Erkrankung ist die Glomerulonephritis.

Entsprechend gehören zu diesen Erkrankungen, die ebenfalls mit einer erfindungsgemäßen Verbindung bzw. Medikament behandelt werden können, auch durch Infektionen hervorgerufene Erkrankungen wie bspw. Myokarditis. Bei einigen Erkrankungen des Auges wie z.B. Uveitis, altersabhängige Makulardegenration, diabetische Retinopathie, diabetisches makulares Ödem, okulares Pemphigoid, Keratoconjunctivitis, Stevens-Johnson Syndrom und Graves Ophthalmophatie ist ebenfalls ein therapeutischer Ansatz mit einem C5a-Rezeptor-Antagonist möglich.

Bei einem Tiermodell der altersabhängigen Makulardegeneration (AMD) konnte gezeigt werden, dass der proinflammatorische Teil der Erkrankung unter anderem durch C5a vermittelt wird (Ambati et al. 2003 Nature Medicine 9: 1390). Deswegen ist auch diese Indikation bevorzugt für die Therapie mit einer erfindungsgemäßen Verbindung.

Durch den neuartigen Ansatz, vermittels der erfindungsgemäßen C5a-Rezeptor-Antagonisten auf T-Zell Populationen bzw. andere Zelltypen, die durch ein verändertes Cytokinmuster angesprochen werden, einzuwirken, ist es möglich, z.B. auch primär T-Zell vermittelte Immunantworten zu beeinflussen. Ein derartiger Ansatz eröffnet die Möglichkeit, ein großes Spektrum schwer therapierbarer Immun- bzw. Autoimmunerkrankheiten mit den erfindungsgemäßen Verbindungen bzw. Medikamenten zu behandeln. Die Gruppe der Autoimmunerkrankungen umfasst insbesondere die folgenden Krankheiten, ist aber nicht darauf beschränkt: Alopecia areata, autoimmun-hämolytische Anämie (AIHA) vom Kältetyp (Kälteagglutininkrankheit), autoimmun-hämolytische Anämie (AIHA) vom Wärmetyp, pernizöse Anämie (Morbus Biermer, Addison Anämie), Antiphospholipid-Syndrom (APS), Arteriitis temporalis, Atherosklerose, Autoimmun-Adrenalitis (autoimmune Nebennierenrinden-Atrophie, Morbus Addison), chronisches Erschöpfungssyndrom (CFIDS), chronisch-inflammatorische, demyelinisierende Polyneuropathie, Churg-Strauss Syndrom, Cogan-Syndrom, Colitis ulcerosa, CREST-Syndrom, Diabetes mellitus Typ I, Dermatitis Herpetiformis During, Dermatomyositis, Fibromyalgie, chronisch autoimmune Gastritis, Goodpasture Syndrom (Anti-GBM vermittelte Glomerulonephritis), Guillain-Barre-Syndrom (GBS; Polyradikuloneuritis), Hashimoto Thyroiditis, autoimmune Hepatitis, idiopathische pulmonale Fibrose, Immun-thrombozytopenische Purpura (Morbus Werlhof), autoimmune Infertilität, autoimmune Innenohrschwerhörigkeit (AIED), juvenile rheumatoide Arthritis, autoimmune Kardiomyopathie, Lambert-Eaton Syndrom, Lichen sclerosis, Lupus erythematodes (insbesondere die diskoide Form), Lyme-Arthritis (Lyme-Krankheit), Mischkollagenose, Morbus Basedow (Graves Disease), Morbus Behçet, Morbus Bechterew (Spondylitis ankylosans), Morbus Crohn, Morbus Ménière, Morbus Reiter, Multiple Sklerose (MS, Encephalomyelitis), Myasthenia gravis (Myasthenie), sympathische Ophtalmie, vernarbendes Pemphigoid, bullöses Pemphigoid, Pemphigus vulgaris, Polyarteriitis nodosa, Polychondritis (Panchondritis), polyglanduläres Autoimmun-(PGA)-Syndrom, Polymyalgia rheumatica, Polymoysitis, primäre biliäre Zirrhose (primäre Autoimmun-Cholangitis), Psoriasis, rheumatisches Fieber, rheumatoide Arthritis, Sarkoidose (Morbus Boeck, Besnier-Boeck-Schaumann Krankheit), Sjörgensen-Syndrom, Sklerodermie, Sprue/Zöliakie, Stiff-Man-Syndrom (Moersch-Woltmann-Syndrom), systemischer Lupus erythematodes, Takayasu Arteriitis (Aortenbogen-Syndrom), transiente Gluten-Intoleranz, Urticaria, autoimmune Uveitis, Vaskulitiden und Vitiligo (Weißfleckkrankheit).

Die Vaskulitiden sind als eine Form der Immun- bzw. Autoimmunerkrankungen zu betrachten. Sie stellen genauer eine Gruppe unterschiedlicher entzündlicher Gefäßerkrankungen dar. Man unterscheidet primäre Vaskulitiden und sekundäre Vaskulitiden. Die primären Vaskulitiden sind Erkrankungen, die z.B. durch beim Patienten vorhandene Autoantikörper hervorgerufen werden können. Eine besonderers bevorzugte Gruppe der Vaskulitiden ist jene, die durch cytoplasmatische anti-Neutrophile Antikörper (ANCA) hervorgerufen werden. Zu dieser Gruppe zählen Morbus Wegener (Wegener's Granulomatose), Churg-Strauss Syndrom und mikroskopische Polyangiitis. Zu der Gruppe der sekundären Vaskulitiden gehören Medikamenten-induzierte Vaskulitiden und Vaskulitiden, die durch andere Erkrankungen, wie beispielsweise AIDS, Hepatitis B und C, Cytomegalie-Virus Infektion hervorgerufen werden können.

Im Rahmen der primären Erkrankungen kommt es z.B. zu einer leukocytoklastischen Vaskulitis bzw. zu einer Gewebeinfiltration mit Eosinophilen, das auch als Churg-Strauss-Syndrom bezeichnet wird. Die Erkrankungen zeigen unter anderem eine Ablagerung von Immunkomplexen und Aktivierung des Complementsystems. Zudem führen die an Neutrophile bindenden Antikörper zu einer Aktivierung der Neutrophilen und anschliessend zur Freisetzung von reaktivem Sauerstoff. Dadurch wird zusätzlich eine Schädigung z.B. der Endothelzellen hervorgerufen. Neutrophile und andere Leukozyten tragen den C5a-Rezeptor und können über die Bindung von C5a aktiviert werden.

Untherapiert kann eine Vaskulitis wie die Wegenersche Granulomatose schnell zum Tode führen. Meist sterben die Patienten an akutem Lungen- oder Nierenversagen. Die Behandlung nach dem Stand der Technik erfolgt über eine unspezifische Suppression der überschiessenden Immunantwort durch Cyclophosphamid, Glucocorticoide, Methotrexat, Mucophenolatmofetil, Azathioprin oder Leflunomid. Diese Therapien sind mit zum Teil erheblichen Nebenwirkungen verbunden wie beispielsweise erhöhte Infektanfälligkeit oder Abfall der Zahl weißer Blutkörperchen. Die vor diesem Hintergrund wünschenswerte zielgerichtetere und sicherere Therapie wird durch die erfindungsgemäßen Verbindungen und deren Verwendung zur Therapie dieser Erkrankungen bereitgestellt.

Unter dem Begriff Urticaria wird eine Reihe von unterschiedlichen Formen der Nesselsucht zusammengefasst. Man unterscheidet spontane (akute und chronische Urticaria) und physikalische Urticaria (Urticaria factitia, Kälteurticaria, Hitzeurticaria, Druckurticaria, Lichturticaria) (Zuberbier et al. 2001 Journal of Investigative Dermatology Symposium Proceedings 6: 123). Darüber hinaus gibt es besondere Formen der Urticaria wie cholinergische Urticaria, adrenergische Urticaria, Kontakturticaria und durch Wasser ausgelöste Urticaria.

Insbesondere für die chronische Urticaria ist experimentell gezeigt worden, dass das Complementsystem und insbesondere C5a an der Histaminfreisetzung im Krankheitsverlauf beteiligt ist (Kaplan et al. 2004 Current Reviews of Allergy and Clinical Immunology 114: 465). Eine Therapie der Urticaria mit C5a-Inhibitoren erscheint als neuartiger Ansatz äußerst sinnvoll.

Ein weiterer Aspekt der indirekten Wirkung von C5a ist beispielsweise die Fibrose. Die von C5a mit ausgelöste Chemotaxis von Neutrophilen und anderen Leukozyten zu den Orten einer Entzündung kann dort zum Teil in Folge der Infiltration vermehrt Fibrose auslösen. Jedoch kann C5a auch direkt auf Zellen aus den betroffenen Organen einwirken und als Folge vermehrt fibrotische Ereignisse auslösen. C5a-Inhibition verringert den Grad der Fibrose in einer Vielzahl von Organen und Erkrankungen. Beispiele dafür sind Leberfibrose, Lungenfibrose, Fibrose in Nieren, Haut und anderen Organen. Fibrotische Ereignisse finden auch beim Myokardinfarkt statt und können einen entscheidenden Grund für eine verminderte Auswurfleistung des Herzens nach Abheilen des Infarktes darstellen. Ebenso ist eine Minderfunktion oder ein Funktionsverlust von Nieren und anderen Organen nach der Transplantation unter anderem auf Fibrosen zurückzuführen. Deshalb können die erfindungsgemäßen C5a-Rezeptor-Antagonisten verwendet werden, um fibrotische Ereignisse zu verhindern oder zu verringern.

Aus den beiden genannten Pathogenitätsmechanismen ergeben sich weitere Anwendungen der erfindungsgemäßen Verbindungen: zur Unterstützung und Nachbehandlung von Patienten, die eine Transplantation von Organen wie z.B. Nieren, Leber, Lunge, Herz, Haut (insbesondere Eigenspende bei z.B. Brandverletzten), Hornhaut, Bauchspeicheldrüse oder Darm erhalten. Dabei kann positiv auf die akute Abstoßungsreaktion (durch die Verminderung des Reperfusionsschadens) und auf die chronische Abstoßungsreaktion (durch Modulation des Cytokinmusters) eingewirkt werden. Bei jedem dieser Prozesse spielt C5a direkt oder indirekt eine Rolle. Deshalb sind Transplantationen ein potentielles Anwendungsgebiet eines erfindungsgemäßen C5a-Rezeptor-Antagonisten.

Eine andere Option ist die Verwendung einer erfindungsgemäßen Substanz oder Verbindung, bzw. einer, mindestens eine davon enthaltenden Zusammensetzung, zur Konservierung von Organen, die bei Transplantationen verwendet werden. In einer Ausführungsform ist vorgesehen, den Spender mit einem C5a Rezeptor-Antagonisten vorzubehandeln, das Organ mit einem Antagonisten zu behandeln und/oder den Empfänger zu behandeln. Bevorzugterweise wird dabei eine erfindungsgemäße Verbindung verwendet, bevorzugtererweise bei allen Ebenen der Verwendung die gleiche Verbindung. Eine Kombination der Behandlung von Organ, Spender und/oder Empfänger erscheint ebenfalls sinnvoll. Derartige Organe können z.B. Nieren, Leber, Lunge, Herz, Haut (insbesondere Eigenspende bei z.B. Brandverletzten), Hornhaut, Bauchspeicheldrüse oder Darm sein.

Prinzipiell stellt jeder chirurgische Eingriff ein Trauma dar, das je nach seiner Schwere sinnvoll mit einem C5a-Rezeptor-Antagonisten gemäß der vorliegenden Erfindung behandelt werden kann. Beispiele hierfür sind CABG, PTCA, PTA, MidCAB, OPCAB, Thrombolyse, Organtransplantation, Aneurysmaoperationen und Gefäßverschluss (clamping). Ein Aspekt ist dabei, die bei extrakorporaler Zirkulation (z.B. Herz-Lungen-Maschine oder bei der Dialyse) möglicherweise folgenden neurokognitiven Dysfunktionen bzw. den lokalen und/oder systemischen Reperfusionsschaden zu vermindern bzw. zu verhindern. Dies kann insbesondere prophylaktisch durch die Behandlung der Patienten vor dem Eingriff bzw. vor der Reperfusion geschehen.

Einige systemische Erkrankungen haben lokale Manifestationen zur Folge, die mit einem C5a Rezeptor-Antagonisten behandelt werden könnten. Beispiele hierfür sind die lokalen Manifestationen von Rheuma, SLE und Typ I und Typ II Diabetes, die am Auge, am oder im Gehirn, an den Gefäßen, am Herzen, an der Lunge, an den Nieren, an der Leber, am gastrointestinalen Trakt, der Milz, der Haut, am Knochensystem, am lyphatischen System und im Blut auftreten können.
Zudem können C5a Rezeptor-Antagonisten im hämorrhagischen Schock oder zur Prävention und/oder Behandlung desselben sinnvoll eingesetzt werden, wenn größere Flüssigkeitsmengen zur Kreislaufstabilisierung zugeführt werden müssen. Der Zustand des hämorrhagischen Schocks ist vergleichbar mit einer systemischen Ischämie. Wenn der Kreislauf durch Zugabe von Flüssigkeit wieder stabilisiert wird, ergibt sich eine Situation, die vergleichbar mit dem Reperfusionsschaden ist.

Eine Therapie mit einem erfindungsgemäßen Medikament kann allein oder in Kombination mit anderen Therapeutika erfolgen. Besonders geeignet für eine Kombinationstherapie sind dabei antiinflammatorische und immunsupressive Therapeutika. Wie hierin bevorzugt verwendet bezeichnet der Begriff der Kombinationstherapie die Kombination zweier oder mehrerer therapeutisch aktiver Verbindungen. Die Kombination kann dabei auf verschiedenen Ebenen erfolgen und umfasst, ist aber darauf nicht beschränkt, die folgenden. Die Kombination kann eine pharmazeutische Formulierung umfassend die beiden oder mindestens zwei der mehreren therapeutisch aktiven Verbindungen sein. Eine weitere Ausführungsform kann darin bestehen, dass die beiden oder mindestens zwei der mehreren therapeutisch aktiven Verbindungen zwar in zwei oder mehreren verschiedenen pharmazeutischen Formulierungen enthalten sind, aber die Formulierungen in einer Verpackung enthalten sind, typischerweise versehen mit einer Anweisung, in welcher zeitlicher Beziehung diese Formulierungen anzuwenden, zu verabreichen oder einzunehmen sind. Schließlich kann eine Kombination im Sinne einer bevorzugten Ausführungsform auch darin bestehen, dass die beiden oder mindestens zwei der mehreren therapeutisch aktiven Verbindungen in verschiedenen Formulierungen und in verschiedenen Verpackungen enthalten sind. Bevorzugterweise findet sich zumindest in einer der beiden Verpackungen eine Anweisung, in welcher zeitlicher Beziehung diese Formulierungen anzuwenden, zu verabreichen oder einzunehmen sind. Im Zusammenhang mit den Kombinationstherapien, wie hierin bevorzugt ausgeführt, ist es im Rahmen der vorliegenden Erfindung, dass der Begriff der verschiedenen Formulierungen auch jene Ausführungsformen umfasst, bei denen unterschiedliche Formulierungen vorhanden sind und der Unterschied in den Formulierungen lediglich durch die in der Formulierung enthaltene therapeutisch wirksame Verbindung bedingt wird.

Für die meisten der Erkrankungen, die ihrem Pathogenitätsmechanismus nach mit einem erfindungsgemäßen C5a-Rezeptor-Antagonisten behandelt werden können, gibt es zur Zeit bereits therapeutische Ansätze. In vielen Fällen sind diese Ansätze jedoch unbefriedigend und/oder die Nebenwirkungen der verabreichten Medikamente sind hoch. Es ist daher wünschenswert, durch die Kombination eines erfindungsgemäßen C5a-Rezeptor-Antagonisten mit einer bestehenden oder in der Entwicklung befindlichen Therapie eine Verbesserung des therapeutischen Effekts zu erzielen bzw. die Dosis eines nebenwirkungsreichen Therapeutikums durch die Kombination mit erfindungsgemäßen C5a-Rezeptor-Antagonisten zu senken. Im folgenden werden einige der bestehenden Therapien und der dabei verwendeten Medikamente bzw. Wirkstoffe dargestellt, die im Rahmen der vorliegenden Erfindung mit den erfindungsgemäßen Verbindungen und/oder Medikamenten verwendet werden können.

Für die Indikation Transplantation werden insbesondere immunsuppressive Therapeutika eingesetzt, um die chronische Transplantatabstossung zu verhindern. Der Wirkmechanismus der gängigen Immunsuppressia unterscheidet sich deutlich vom Wirkmechanismus der erfindungsgemäßen Verbindungen. Es ist somit ein additiver oder synergistischer Effekt der zwei Therapieansätze zu erwarten. Eine Kombination aus gängigen Immunsupressiva und den erfindungsgemäßen Verbindungen ist daher sehr sinnvoll. Beispiele für gängige Immunsuppressiva sind: Calcineurin-Inhibitoren oder andere Substanzen wie z.B. Cyclosporin A, Methotrexate, Azathioprin, FK506 (Tacrolimus), Rapamycin, Leflunomid, Mycophenolatmofetil, Brequinar, Mizoribin und Deoxyspergualin. Diese Immunsupressiva finden auch für andere Indikationen Verwendung. Eine Kombination aus den erfindungsgemäßen Verbindungen und Immunsupressiva ist von daher auch unter anderem für folgende Indikationen sinnvoll: Alopecia areata, autoimmun-hämolytische Anämie (AIHA) vom Kältetyp (Kälteagglutininkrankheit), autoimmun-hämolytische Anämie (AIHA) vom Wärmetyp, pernizöse Anämie (Morbus Biermer, Addison Anämie), Antiphospholipid-Syndrom (APS), Arteriitis temporalis, Atherosklerose, Autoimmun-Adrenalitis (autoimmune Nebennierenrinden-Atrophie, Morbus Addison), chronisches Erschöpfungssyndrom (CFIDS), chronisch-inflammatorische, demyelinisierende Polyneuropathie, Churg-Strauss Syndrom, Cogan-Syndrom, Colitis ulcerosa, CREST-Syndrom, Diabetes mellitus Typ I, Dermatitis Herpetiformis During, Dermatomyositis, Fibromyalgie, chronisch autoimmune Gastritis, Goodpasture Syndrom (Anti-GBM vermittelte Glomerulonephritis), Guillain-Barré-Syndrom (GBS; Polyradikuloneuritis), Hashimoto Thyroiditis, autoimmune Hepatitis, idiopathische pulmonale Fibrose, Immun-thrombozytopenische Purpura (Morbus Werlhof), autoimmune Infertilität, autoimmune Innenohrschwerhörigkeit (AIED), juvenile rheumatoide Arthritis, autoimmune Kardiomyopathie, Lambert-Eaton Syndrom, Lichen sclerosis, Lupus erythematodes (insbesondere die diskoide Form), Lyme-Arthritis (Lyme-Krankheit), Mischkollagenose, Morbus Basedow (Graves Disease), Morbus Behçet, Morbus Bechterew (Spondylitis ankylosans), Morbus Crohn, Morbus Ménière, Morbus Reiter, Morbus Wegener, Multiple Sklerose (MS, Encephalomyelitis), Myasthenia gravis (Myasthenie), sympathische Ophtahnie, vernarbendes Pemphigoid, bullöses Pemphigoid, Pemphigus vulgaris, Polyarteriitis nodosa, Polychondritis (Panchondritis), polyglanduläres Autoimmun-(PGA)-Syndrom, Polymyalgia rheumatica, Polymoysitis, primäre biliäre Zirrhose (primäre Autoimmun-Cholangitis), Psoriasis, rheumatisches Fieber, rheumatoide Arthritis, Sarkoidose (Morbus Boeck, Besnier-Boeck-Schaumann Krankheit), Sjörgensen-Syndrom, Sklerodermie, Sprue/Zöliakie, Stiff-Man-Syndrom (Moersch-Woltmann-Syndrom), systemischer Lupus erythematodes, Takayasu Arteriitis (Aortenbogen-Syndrom), transiente Gluten-Intoleranz, Urticaria, autoimmune Uveitis, Vaskulitiden und Vitiligo (Weißfleckkrankheit).

Bei Urticaria werden insbesondere Antihistaminika eingesetzt. Es ist bekannt, dass z.B. bei der chronischen Urticaria C5a eine entscheidende Rolle bei der Aktivierung von Mastzellen über Autoantikörper, die den IgE-Rezeptor I oder den Fc Teil von IgE binden, spielt. Deswegen ist eine Kombination von Antihistaminika und den erfindungsgemäßen Therapeutika sinnvoll und verspricht zudem eine Linderung der Krankheitssymptome bei Patienten, die nicht oder unvollständig auf Antihistaminika ansprechen.

Glukokortikoide (z.B. Prednisolon) werden bei einer sehr großen Zahl verschiedenster Indikationen eingesetzt. Insbesondere für Erkrankungen mit inflammatorischem oder Autoimmun-Anteil können diese Substanzen genutzt werden. Beispiele für Erkrankungen, bei denen die Kombinationstherapie aus Glukokortikoiden und C5a-Rezeptor-Antagonisten sinnvoll ist, sind: rheumatoide Arthritis, systemischer Lupus erythematodes, Colitis ulcerosa, Morbus Crohn, COPD, Uveitis, Keratokonjunktivitis, Asthma, Morbus Bechterew, Multiple Sklerosis, Morbus Wegener und viele andere Immun- und Autoimmunerkrankungen.

Bakterielle und andere Infektionen können das Immunsystem des Menschen überfordem und/oder eine systemische Infektion kann eine massive Aktivierung der Abwehrmechanismen nach sich ziehen, was zu nachteiligen Reaktionen bei den Betroffenen führen kann. Das prominenteste Beispiel ist der septische Schock, der z.B. durch eine systemische bakterielle Infektion oder durch die Freisetzung einer hinreichenden Menge z.B. von LPS aus lokalen Infektionsherden hervorgerufen werden kann. Ein wichtiger Grund für die nachteiligen systemischen Effekte einer Infektion liegt in der Freisetzung von C5a. Deshalb bieten sich insbesondere antimikrobielle Therapien mit Antibiotika für eine Kombination mit C5a-Rezeptor-Antagonisten an. Beispiele für die für eine Kombinationstherapie geeigneten Antibiotika-Klassen sind Amynoglykoside, β-Lactam-Antibiotika, Glycopeptidantibiotika, Gyrasehemmer, Lincosamide, Makrolidantibiotika, Nitroimidazolderivate, Polypeptidantibiotika, Sulfonamide, Trimethoprim und Tetracycline.

Antiinflammatorische therapeutische Antikörper oder andere antiinflammatorische Proteine, Nucleinsäuren und deren Derivate sowie Peptide und kleine Moleküle, die z.B. die Wirkung bzw. Effekte von proinflammatorischen Molekülen inhibieren bzw. die Wirkung von antiinflammatorischen Molekülen unterstützen (z.B. IL-10), sind ebenfalls bevorzugt für eine Kombinationstherapie mit erfindungsgemäßen C5a-Rezeptor-Antagonisten. Die meisten Stoffe aus der hierin gegebenen Aufzählung können in der Klasse der Biologika zusammengefasst werden und sind Stoffe, die meist einen sehr spezifischen Mechanismus aufweisen.

Häufig ist es sinnvoll, mehrere Wege innerhalb eines biologischen Systems zu modifizieren, um eine verbesserte Wirkung zu erzielen. Deshalb ist besonders bevorzugt die Kombination von erfindungsgemäßen C5a-Rezeptor-Antagonisten mit Inhibitoren von proinflammatorischen Molekülen.

Beispiele für proinflammatorische Moleküle, deren Wirkung in Kombination mit den erfindungsgemäßen C5a-Rezeptor-Antagonisten inhibiert werden kann, um eine Verbesserung der therapeutischen Wirkung zu erreichen, sind unter anderem: IL-1, IL-5, IL-6, IL-8, IL-10, IL-12, IL-13, IL-15, IL-16, IL-17, IL-18, TNFα, α4β7, α5β1, BlyS, Cadherin, CCR2, CD11a, CD11b, CD125, CD130, CD16, CD18, CD2, CD20, CD22, CD23, CD25, CD28, CD3, CD30, CD4, CD40, CD40L, CD44, CD45R, CD54, CD62E, CD62L, CD8, CD80, CD95, CEP, Gastrin-R, Complement C1 bzw. C1-Esterase, Complement Factor 5, Complement Factor D, Complement MBL, Complement Rezeptor 1, CRTH2 Rezeptor, CTGF, E- und P-Selectin, Eotaxin, Factor IX, FGF-20, Fgl-2, GM-CSFr, GP IIb/IIIa Rezeptor, HMG1, IgE, Thymocyten, IFNγ, IFNr, IP-10, MCP-1, M-CSF Rezeptor, MIF, MMP9, PDGF-D, P-Selectin, TGFβ1, Tissue Factor, TrkA (tyrosine kinase receptor), VAP-1, VCAM-1, VEGF, VLA1 und vWF. Die Inhibition der proinflammatorischen Moleküle aus dieser Liste kann z.B. über Antikörper, lösliche Rezeptoren oder andere inaktivierenden natürliche bzw. künstliche Bindungspartner, Aptamere, Spiegelmere, RNAi, antisense-Moleküle oder kleine Moleküle realisiert werden.

Bei der AMD ist neben der Drusenbildung die Neogenese von Blutgefässen ein wichtiges Krankheitsmerkmal. Unter anderem durch das Einwachsen der Blutgefäße kommt es zu einem Verlust der Sehkraft beim Patienten. Zur Zeit werden AMD-Patienten mit der photodynamischen Therapie behandelt. Außerdem sind Antikörper gegen VEGF und Integrin α5β1 für die Therapie von AMD in der Entwicklung. Eine Kombination dieser vorstehend genannten Therapieansätze mit einer erfindungsgemäßen C5a-Rezeptor-Antagonistentherapie ist deswegen besonders bevorzugt.

Allgemein antiinflammatorische oder schmerzlindernde Therapeutika wie Acetylsalicylsäure, Ibuprofen, Diclofenac, Naproxen, eignen sich ebenfalls besonders, um zur Kombinationstherapie mit erfindungsgemäßen C5a-Rezeptor-Antagonisten verwendet zu werden. Betroffene von Rheumaerkrankungen stellen ein Beispiel für eine Patientenkollektiv dar, das von einer derartigen Kombinationsbehandlung profitiert.

Wie die Bestandteile der Complementkaskade sind auch die Bestandteile der Kininkaskade und des Gerinnungssystems im Blutplasma lokalisiert. Durch artifizielle Oberflächen oder andere Auslöser, wie z.B. bei Verbrennungen oder Sepsis, wird neben dem Complementsystem auch das Kininsystem aktiviert. Dieses legt eine enge Verknüpfung von Complementkaskade, Kininkaskade und Gerinnungssystem nahe. Eine enge Verknüpfung der drei Wege ist auch darin ersichtlich, das der C1-Esterase-Inhibitor (C1Inh, ein natürlich vorkommendes Protein) nicht nur das Complementsystem hemmt, sondern auch das Kininsystem und den intrinsischen Weg der Blutgerinnung. Deswegen ist es insbesondere bevorzugt, Antagonisten der Endprodukte des Kininsystems wie z.B. Bradykinin, desArg-Bradykinin, Kalidin und desArg-Kalidin, mit erfindungsgemäßen C5a-Inhibitoren zu kombinieren. Ein Beispiel für einen derartigen Antagonisten, der für eine Kombination geeignet erscheint, ist Icatibant (ein BR₂-Antagonist). Genauso bevorzugt ist auch die Kombination mit Inhibitoren der Kininkaskade bzw. des Gerinnungssystems. Beispiele für Indikationen, bei denen eine Kombination zweier oder drei der Inhibitoren aus der Kinin-, der Gerinnungs- und Complementkaskade besonders sinnvoll ist, sind z.B. der Reperfusionsschaden (Herz, Lunge, Leber, Niere, Darm, Gehirn oder Haut), schwere Verbrennungen und septischer Schock. Als ganz besonders bevorzugt gilt die Kombination von Bradykinin-Rezeptor-Antagonisten mit C5a-Rezeptor-Antagonisten. Insbesondere die Kombination von Therapeutika, die die Kinin-Kaskade hemmen bzw. Inhibitoren der Kininrezeptoren BR₁ und BR₂, wie z.B. Icatibant, mit den erfindungsgemäßen Verbindungen ist bevorzugt. Der Einfluss von Bradykinin auf die erworbene Immunantwort ist gezeigt worden (Aliberti et al. 2003 Journal of Immunology 170: 5349), und es wird deutlich, dass sich insbesondere BR₂ Antagonisten für eine Kombinationstherapie mit erfindungsgemäßen Verbindungen eignen, um so über zwei unterschiedliche Angriffspunkte spezifisch auf die erworbene Immunantwort einwirken zu können. Ein neuer Aspekt dieser Erfindung ist demnach die Beeinflussung der erworbenen Immunantwort mit einer Kombinationstherapie aus C5a-Rezeptor-Antagonisten und BR₂-Antagonisten. Auch im Zusammenhang mit intracellulären Parasiten wurde gezeigt, dass Bradykininrezeptoren eine wichtige Rolle spielen (Trypanosoma cruzi, Scharfstein et al. 2003 FASEB Journal 17: 73). Auch für diese Indikationen bietet sich die Kombination eines C5a-Inhibitors mit einem Bradykinin-Antagonisten an.

Weitere mögliche Angriffspunkte für eine Kombinationstherapie mit einem C5a-Rezeptor-Antagonisten sind die p38 MAP Kinase, die Phosphodiesterase 4 (PDE-4), der NO-Haushalt (NO-Synthase) und das IL-1β Converting Enzyme (Caspase-1).

Für die Kombination eines C5a-Rezeptor-Antagonisten mit einem anderen Mediakament kommen nicht nur die erfindungsgemäßen C5a-Rezeptorantagonisten sondern auch die C5a-Rezeptor-Antagonisten in Frage, die unter die Ansprüche der Anmeldung WO2005/010030 fallen, deren Offenbarung hierin durch Bezugnahme aufgenommen wird. Besonders bevorzugt sind dabei Verbindungen der folgenden Struktur:

**X1--X2--X3--X4--X5--X6--X7--X8** (C5a)

wobei
X1 ein Radikal mit einer Masse von etwa 1-300 ist und wobei X1 bevorzugterweise ausgewählt ist aus der Gruppe, die R5-, R5-CO-, R5-N(R6)-CO-, R5-O-CO-, R5-SO₂-, R5-N(R6)-SO₂-, R5-N(R6)-, R5-N(R6)-CS-, R5-N(R6)-C(NH)-, R5-CS-, R5-P(O)OH-, R5-B(OH)-, R5-CH=N-O-CH₂-CO- umfasst, wobei R5 und R6 einzeln und unabhängig voneinander ausgewählt sind aus der Gruppe, die H, F, Hydroxy, Alkyl, substituiertes Alkyl, Cycloalkyl, substituiertes Cycloalkyl, Heterocyclyl, substituiertes Heterocyclyl, Arylalkyl, substituiertes Arylalkyl, Aryl, substituiertes Aryl, Heteroaryl, substituiertes Heteroaryl, Acyl, substituiertes Acyl, Alkoxy, Alkoxyalkyl, substituiertes Alkoxyalkyl, Aryloxyalkyl und substituiertes Aryloxyalkyl umfasst,
X2 ein Radikal ist, das die biologischen Bindungseigenschaften einer Phenylalanin-Einheit mimikt,
X4 einzeln und unabhängig voneinander ein Spacer ist, wobei der Spacer bevorzugterweise aus der Gruppe ausgewählt ist, die Aminosäuren, Aminosäure-Analoga und Aminosäure-Derivate umfasst,
X5 ein Radikal ist, das die biologischen Bindungseigenschaften einer Cyclohexylalanin-Einheit mimikt,
X6 ein Radikal ist, das die biologischen Bindungseigenschaften einer Tryptophan-Einheit mimikt,
X7 ein Radikal ist, das die biologischen Bindungseigenschaften einer Norleucin- oder Phenylalanin-Einheit mimikt,
X8 ein Radikal ist, wobei das Radikal optional in Struktur I enthalten ist und wenn es enthalten ist, ausgewählt ist aus der Gruppe, die H, NH₂, OH, NH-OH, Amino, substituiertes Amino, Alkoxy, substituiertes Alkoxy, Hydrazino, substituiertes Hydrazino, Aminooxy, substituiertes Aminooxy, Alkyl, substituiertes Alkyl, Cycloalkyl, substituiertes Cycloalkyl, Heterocyclyl, substituiertes Heterocyclyl, Heteroaryl, substituiertes Heteroaryl, Arylalkyl, substituiertes Arylalkyl, Aryl, substituiertes Aryl, Aminosäure, Aminosäurederivat und Aminosäureanalogon umfasst;
X3 folgende Struktur aufweist: 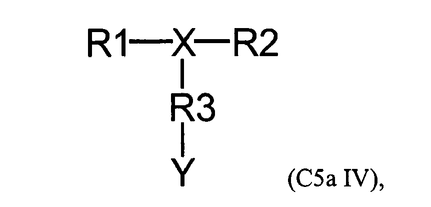 worin
X C(R4) oder N ist,
R1 optional vorhanden ist und wenn R1 vorhanden ist, R1 ein Radikal ist, das ausgewählt ist aus der Gruppe, die >N-R1B, >C(R1B)(R1C) und >O umfasst, wobei R1B und R1C unabhängig voneinander ausgewählt sind aus der Gruppe, die H, Alkyl, substituiertes Alkyl, Cycloalkyl, substituiertes Cycloalkyl, Heterocyclyl, substituiertes Heterocyclyl, Aryl, substituiertes Aryl, Heteroaryl, substituiertes Heteroaryl, Arylakyl, substituiertes Arylalkyl, Cycloalkylalkyl und substituiertes Cycloalkylalkyl umfasst;
R2 optional vorhanden ist und wenn R2 vorhanden ist, R2 ein Radikal ist, das ausgewählt ist aus der Gruppe, die C=O, C=S, SO₂, PO(OH), B(OH), CH₂, CH₂CO, CHF und CF₂ umfasst; R4 ein Radikal ist, wobei das Radikal ausgewählt ist aus der Gruppe, die H, F, CH₃, CF₃, Alkyl und substituiertes Alkyl umfasst;
die Bindung von Struktur (C5a IV) an die Molekülbestandteile X2 und X4 bevorzugt über R1 und R2 erfolgt;
R3 ein Radikal ist, das aus der Gruppe ausgewählt ist, die H, Alkyl, substituiertes Alkyl, Cycloalkyl, substituiertes Cycloalkyl, Cycloalkylalkyl, substituiertes Cycloalkylalkyl, Heterocyclyl, substituiertes Heterocyclyl, Heterocyclylalkyl, substituiertes Heterocyclylalkyl, Aryl, substituiertes Aryl, Arylalkyl, substituiertes Arylalkyl, Heteroaryl, substituiertes Heteroaryl, Heteroarylalkyl, substituiertes Heteroarylalkyl, Acyl, substituiertes Acyl, Alkoxyalkyl, substituiertes Alkoxyalkyl, Aryloxyalkyl, substituiertes Aryloxyalkyl, Sulfhydrylalkyl, substituiertes Sulfhydrylalkyl, Hydroxyalkyl, substituiertes Hydroxyalkyl, Carboxyalkyl, substituiertes Carboxyalkyl, Carboxamidoalkyl, substituiertes Carboxamidoalkyl, Carboxyhydrazinoalkyl, Ureidoalkyl Aminoalkyl, substituiertes Aminoalkyl, Guanidinoalkyl und substituiertes Guanidinoalkyl umfasst; und
Y optional vorhanden ist und wenn Y vorhanden ist, Y ein Radikal ist, das ausgewählt ist aus der Gruppe, die H, -N(YB1)-CO-YB2, -N(YB1)-CO-N(YB2)(YB3), -N(YB1)-C(N-YB2)-N(YB3)(YB4), -N(YB1)(YB2), -N(YB1)-SO₂-YB2, O-YB1, S-YB1, -CO-YB1, -CO-N(YB1)(YB2) und -C=N-O-YB1 umfasst, wobei YB1, YB2, YB3 und YB4 einzeln und unabhängig voneinander ausgewählt sind aus der Gruppe, die H, CN, NO₂, Alkyl, substituiertes Alkyl, Cycloalkyl, substituiertes Cycloalkyl, Heterocyclyl, substituiertes Heterocyclyl, Aryl, substituiertes Aryl, Heteroaryl, substituiertes Heteroaryl, Arylakyl, substituiertes Arylalkyl, Cycloalkylalkyl und substituiertes Cycloalkylalkyl umfasst.

Besonders bevorzugt sind C5a-Rezeptor-Antagonisten der Struktur:

X1--X2--X3--X4--X5--X6--X7--X8

wobei:
X1 ein Radikal mit einer Masse von etwa 1-300 ist und wobei X1 bevorzugterweise ausgewählt ist aus der Gruppe, die R5-, R5-CO-, R5-N(R6)-CO-, R5-O-CO-, R5-SO₂-, R5-N(R6)-SO₂-, R5-N(R6)-, R5-N(R6)-CS-, R5-N(R6)-C(NH)-, R5-CS-, R5-P(O)OH-, R5-B(OH)-, R5-CH=N-O-CH₂-CO- umfasst, wobei R5 und R6 einzeln und unabhängig voneinander ausgewählt sind aus der Gruppe, die H, F, Hydroxy, Alkyl, substituiertes Alkyl, Cycloalkyl, substituiertes Cycloalkyl, Heterocyclyl, substituiertes Heterocyclyl, Arylalkyl, substituiertes Arylalkyl, Aryl, substituiertes Aryl, Heteroaryl, substituiertes Heteroaryl, Acyl, substituiertes Acyl, Alkoxy, Alkoxyalkyl, substituiertes Alkoxyalkyl, Aryloxyalkyl und substituiertes Aryloxyalkyl umfasst,
X2 ein Aminosäurederivat einer Aminosäure ist, die ausgewählt ist aus der Gruppe, die Phenylalanin, 2-Fluor-Phenylalanin, 3-Fluor-Phenylalanin, 4-Fluor-Phenylalanin, 2-Chlorphenylalanine, 3-Chlorphenylalanine, 4-Chlorphenylalanine, 1-Naphtylalanin, 2-Thienylalanin, 3-Thienylalanin, 3,3-Diphenylalanin, Tyrosin, Tryptophan, Histidin und jeweilige Derivate davon umfasst;
oder X2 und X1 sind zusammengenommen PhCH₂CH₂CO- oder PhCH₂-;
X3 und X4 wie sie oben im Zusammenhang mit den Verbindungen mit der Formel (C5a) definiert sind;
X5 ein Aminosäurederivat einer Aminosäure ist, die ausgewählt ist aus der Gruppe, die D-Cyclohexylalanin, D-Cyclohexylglycin, D-Homo-Cyclohexylalanin, Octahydroindol-2-carbonsäure, 2-Methyl-D-Phenylalanin und jeweilige Derivate davon umfasst;
X6 ein Aminosäurederivat einer Aminosäure ist, die ausgewählt ist aus der Gruppe, die Tryptophan, Phenylalanin, Tyrosin, Histidin, 1-Naphtylalanin, Benzothienylalanin, 2-Aminoindan-2-carbonsäure, 2-Thienylalanin, 3-Thienylalanin, 2-Fluor-Phenylalanin, 3-Fluor-Phenylalanin, 4-Fluor-Phenylalanin, 2-Chlorphenylalanine, 3-Chlorphenylalanine, 4-Chlorphenylalanine und jeweilige Derivate davon umfasst;
X7 ein Aminosäurederivat einer Aminosäure ist, die ausgewählt ist aus der Gruppe, die Norvalin, Norleucin, Homo-Leucin, Leucin, Isoleucin, Valin, Cystein, Cystein(Me), Cystein(Et), Cystein(Pr), Methionin, Allylglycin, Propargylglycin, Cyclohexylglycin, Cyclohexylalanin, Phenylalanin, Tyrosin, Tryptophan, Histidin, 1-Naphtylalanin, 2-Thienylalanin, 3-Thienylalanin und jeweilige Derivate davon umfasst.
X8 ein Radikal ist, wobei das Radikal optional in Struktur I enthalten ist und wenn es enthalten ist, ausgewählt ist aus der Gruppe, die H, NH₂, OH, NH-OH, Amino, substituiertes Amino, Alkoxy, substituiertes Alkoxy, Hydrazino, substituiertes Hydrazino, Aminooxy, substituiertes Aminooxy, Alkyl, substituiertes Alkyl, Cycloalkyl, substituiertes Cycloalkyl, Heterocyclyl, substituiertes Heterocyclyl, Heteroaryl, substituiertes Heteroaryl, Arylalkyl, substituiertes Arylalkyl, Aryl, substituiertes Aryl, Aminosäure, Aminosäurederivat und Aminosäureanalogon umfasst.

Wie hierin verwendet sind Aminosäure-Derivate bevorzugterweise Verbindungen, die aus Aminosäuren dadurch entstehen, daß diese am N- und/oder C-Terminus modifiziert werden. Nicht limitierende Beispiele sind Umsetzungen der Carboxylgruppe zu Salzen, Estern, Acylhydraziden, Hydroxamsäuren oder Amiden und der Amino-Gruppe zu Amiden, Harnstoffen, Thioharnstoffen, Thioamiden, Sulfonamiden, Phosphorsäureamiden, Borsäureamiden oder Alkylaminen. Teile von Verbindungen, die durch Modifizierungen von Aminosäuren am C- und/oder N-Terminus entstehen, können auch als Aminosäure-Einheiten bezeichnet werden. Darüber hinaus können, die Aminosäuren auch an ihren Seitenketten derivatisiert sein. Sofern eine derivatisierte Aminosäure eine solche ist, bei der die Seitenkette einfach oder mehrerfach derivatisiert ist, wird auf diese Art der Derivatisierung üblicherweise hierin speziell hingewiesen. Eine bevorzugte Derivatisierung der Seitenkette kann insbesondere dort erfolgen, wo die Seitenkette eine funktionelle Gruppe trägt. Eine bevorzugte funktionelle Gruppe ist beispielsweise eine Aminogruppe, Carboxylgruppe, Thiolgruppe oder Alkoholgruppe.

Wie hierin verwendet sind Aminosäure-Analoga bevorzugterweise Verbindungen, die aus Aminosäuren dadurch entstehen, daß die Amino- und/oder Carboxylgruppe durch andere Gruppen, die diese mimiken können, ersetzt werden. Nicht limitierende Beispiele sind der Einbau von Thioamiden, Harnstoffen, Thioharnstoffen, Acylhydraziden, Estern, Alkylaminen, Sulfonamiden, Phosphorsäureamiden, Ketonen, Alkoholen, Boronsäureamiden, Benzodiazepinen und anderen aromatischen oder nichtaromatischen Heterocyclen (für eine Übersicht siehe M. A. Estiarte, D. H. Rich in Burgers Medicinal Chemistry, 6th Edition, Volume 1, Part 4, John Wiley & Sons, New York, 2002).

Die biologischen Bindungseigenschaften einer Aminosäureeinheit wie hierin bezeichnet sind bevorzugterweise jene Bindungseigenschaften, die die jeweilige Aminosäure bei der Wechselwirkung mit einem biologischen Molekül aufweist. Dabei sind biologische Moleküle insbesondere solche, die eine biologische Funktion ausüben. Beispiele für derartige biologische Moleküle, aber nicht darauf beschränkt, sind beispielsweise Protein- oder Peptidbasierte Rezeptoren.

Gruppen oder Einheiten, die die biologischen Bindungseigenschaften einer Aminosäure mimiken oder nachahmen, sind bevorzugterweise definiert als Gruppen, die mindestens eine gleiche oder ähnliche Wechselwirkung mit einem Rezeptor oder Wechselwirkungspartner, bevorzugterweise einem biologischen Rezeptor oder biologischen Wechselwirkungspartner eingehen können wie die Aminosäure selbst. Bei der Auswahl solcher Gruppen ist es bevorzugt, die verbreitetsten im Sinne von bevorzugtesten Wechselwirkungen der infragestehenden Aminosäure mit biologischen Rezeptoren zu betrachten. So ist das Sauerstoffatom der Carbonylgruppe einer Aminosäure befähigt, als Wasserstoffbrücken-Akzeptor zu fungieren, wohingegen das NH-Proton Wechselwirkungen als Wasserstoffbrücken-Donor eingehen kann. Zusätzlich können Aminosäuren Wechselwirkungen mit Rezeptoren über Ihre Seitenketten eingehen. Phenylalanin und Tryptophan können sowohl hydrophobe Wechselwirkungen über die Seitenketten-Methylengruppe oder die aromatischen Gruppen als auch π-π-Wechselwirkungen über die aromatischen Gruppen eingehen. Zusätzlich kann die Indolgruppe des Tryptophans über die NH-Gruppe als Wasserstoffbrücken-Donor fungieren. Cyclohexylalanin und Norleucin können grundsätzlich hydrophobe Wechselwirkungen mit biologischen Rezeptoren über ihre Alkyl- bzw. Cycloalkyl-Seitenketten eingehen. Bei allen Aminosäuren können nicht nur die gesamte Seitenkette, sondern auch Teile davon grundsätzlich wichtige Wechselwirkungen eingehen.

Wenn eine Gruppe oder eine Einheit, die die biologische Bindungseigenschaft einer Aminosäure mimiken oder nachahmen soll bzw. diese Eigenschaft aufweisen soll, mindestens eine der vorstehenden Wechselwirkungen der jeweiligen Aminosäure eingehen kann, so kann sie deren biologische Bindungseigenschaften mimiken.

Bei den hierin angegebenen Gruppendefinitionen bezeichnet der Begriff "und jeweilige Derivate davon", dass alle Derivate der in der Gruppe angeführten Einzelverbindungen, Gruppen von Verbindungen, Molekülteile, Radikale oder chemische Gruppen jeweils als Derivate vorliegen können.

Ganz besonders bevorzugte C5a-Rezeptor-Antagonisten sind dabei ebenfalls die folgenden Moleküle zu verstehen: Ac-Phe-[Orn-Pro-cha-Trp-Phe], Ac-Phe-[Orn-Hyp-cha-Trp-Phe], HOCH2(CHOH)4-C=N-O-CH2-CO-Phe-[Orn-Pro-cha-Trp-Nle], X-Phe-[Orn-Pro-cha-Trp-Nle]; X = 2-Acetamido-1-Methyl-Glucuronyl, Ac-Phe-[Orn-Hyp(COCH2OCH2CH2OCH2CH2OCH3)-cha-Trp-Nle], Ac-Phe-[Orn-Hyp(CONH-CH2CH(OH)-CH2OH)-cha-Trp-Nle], Ac-Phe-[Orn-Pro-cha-Trp-Ecr], Ac-Phe-[Orn-Pro-cha-Trp-Nle], Ac-Phe-[Orn-Pro-cha-Trp-Met], Ac-Phe-[Orn-Pro-cha-Trp-Nva], Ac-Phe-[Orn-Pro-cha-Trp-Hle], Ac-Phe-[Orn-Pro-cha-Trp-Eaf], Ac-Phe-[Orn-Pro-cha-Trp-Ebd], Ac-Phe-[Orn-Pro-cha-Trp-Eag], Ac-Phe-[Orn-Pro-cha-Trp-Pmf], Ac-Phe-[Orn-Pro-cha-Trp-2Ni], Ac-Phe-[Orn-Pro-cha-Trp-Thi], Ph-CH2-CH2-CO-[Orn-Pro-cha-Trp-Nle], H-Phe-[Orn-Pro-cha-Trp-Nle], Ac-Lys-Phe-[Orn-Pro-cha-Trp-Nle], H-Phe-[Orn-Ser-cha-Trp-Nle], Ac-Phe-Orn-Pro-cha-Trp-Phe-NH2, Ac-Phe-Orn-Aze-cha-Bta-Phe-NH2, Ac-Phe-Orn-Pro-cha-Bta-2Ni-NH2, Ac-Phe-Orn-Pro-cha-Bta-Cha-NH2, Ac-Phe-Orn-Pip-cha-Trp-Phe-NH2, Ph-CH2-[Orn-Pro-cha-Trp-Nle], Ph-CH2-[Orn-Pro-cha-Trp-Phe], Ac-Phe-[Orn-Pro-cha-Trp-1Ni], Ph-CH(OH)-CH2-CO-[Orn-Pro-cha-Trp-Nle], Ac-Phe-Orn-Pro-cha-Trp-Phe-NH2, Ac-Phe-Orn-Pro-cha-Bta-Phe-NH2, Ac-Phe-Orn-Pro-cha-Trp-2Ni-NH2, Ac-Phe-Orn-Pro-cha-Trp-Cha-NH2, Ac-Thi-Orn-Aze-cha-Bta-Phe-NH2, Ac-Thi-Orn-Pip-cha-Bta-Phe-NH2, Ac-Phe-Orn-Pro-cha-Trp-Eap-NH2, Me2-Phe-Orn-Pro-cha-Trp-Phe-NH2, Ph2-CH-CH2-CO-Orn-Pro-cha-Trp-Phe-NH2, Ac-Ebw-Orn-Pro-cha-Trp-Phe-NH2, Ac-Phe-Orn-Pro-cha-Trp-NH-CH2-CH2-Ph, Ac-Phe-Orn-Aze-cha-Bta-NH-CH2-CH2-Ph, H-Phe-Orn-Pro-cha-Trp-Phe-NH2, H-Me-Phe-Orn-Pro-cha-Trp-Phe-NH2, Bu-NH-CO-Phe-Orn-Pro-cha-Trp-Phe-NH2, Ac-Thi-Orn-Pro-cha-Trp-Phe-NH2, Ac-Ebw-Orn-Pro-cha-Trp-Phe-NH2, Ac-Phe-Orn-Ala-cha-Trp-Phe-NH2, Ac-Phe-Orn-Pro-cha-Trp-Thi-NH2, Ac-Phe-Orn-Aze-cha-Pcf-Phe-NH2, Ac-Phe-Orn(Ac)-Pro-cha-Trp-Phe-NH2, Ac-Phe-Orn-Aze-cha-Trp-Phe-NH2, Ac-Phe-Trp-Pro-cha-Trp-Phe-NH2, Ph-NH-CO-Phe-Orn-Pro-cha-Trp-Phe-NH2, Bu-O-CO-Phe-Orn-Pro-cha-Trp-Phe-NH2, Ac-Phe-Lys-Pro-cha-Trp-Phe-NH2, Ac-Phe-Arg-Pro-cha-Trp-Phe-NH2, Ac-Phe-Gln-Pro-cha-Trp-Phe-NH2, Ac-Phe-Orn-Pip-cha-Trp-Phe-NH2, Ac-Phe-Orn-Hyp-cha-Trp-Phe-NH2, Ac-Phe-Orn-Pro-cha-Trp-1Ni-NH2, Ac-Phe-Orn-Aze-cha-Bta-Phe-NH-Me, CH3-SO2-Phe-Orn-Aze-cha-Bta-Phe-NH2, Ac-Phe-Orn-Aze-cha-Pff Phe-NH2, Ac-Phe-Orn-Aze-cha-Mcf-Phe-NH2, Ac-Phe-Orn(Ac)-Aze-cha-Bta-Phe-NH2, Ac-Ebw-Orn-Pro-cha-Trp-Phe-NH2, Ac-Phe-Trp-Pro-cha-Trp-Phe-NH2, Ac-Phe-Arg-Pro-cha-Trp-Phe-NH2, Ac-Phe-Orn-Pip-cha-Trp-Phe-NH2, 3PP-Orn-Aze-cha-Bta-Phe-NH2, Ac-Phe-Orn-Tic-cha-Trp-Phe-NH2, Ac-Phe-Orn-Ser-cha-Trp-Phe-NH2, Ac-Phe-Orn-Pro-chg-Trp-Phe-NH2, Ac-Phe-Orn-Pro-hch-Trp-Phe-NH2, Ac-Phe-Orn-Pro-cha-Trp-Phg-NH2, Ac-Phe-Bta-Aze-cha-Bta-Phe-NH2, Ac-Phe-Trp-Pro-cha-Bta-Phe-NH2, Ac-Phe-Orn-Pip-cha-Trp-Phe-OH, Ac-Phe-Orn-Tic-cha-Trp-Phe-OH, Ac-Phe-Orn-Ser-cha-Trp-Phe-OH, Ac-Phe-Orn-Pro-chg-Trp-Phe-OH, Ac-Phe-Eec-Pro-cha-Bta-Phe-NH2, Ac-Phe-Nle-Pro-cha-Bta-Phe-NH2, Ac-Phe-Har-Pro-cha-Bta-Phe-NH2, Ac-Phe-Arg-Pro-cha-Bta-Phe-NH2, Ac-Phe-Cys(Acm)-Pro-cha-Bta-Phe-NH2, Ac-Phe-Mpa-Pro-cha-Bta-Phe-NH2, Ac-Eby-Orn-Pro-cha-Bta-Phe-NH2, Ac-Phg-Orn-Pro-cha-Bta-Phe-NH2, Ac-Phe-Paf Pro-cha-Bta-Phe-NH2, H2N-CO-Phe-Orn-Pro-cha-Bta-Phe-NH2, Me-O-CO-Phe-Orn-Pro-cha-Bta-Phe-NH2, (-CO-CH2-NH-CO-)-Phe-Orn-Pro-cha-Bta-Phe-NH2, Ac-Phe-Orn-Pro-hch-Trp-Phe-OH, (-CO-CH2-CH2-CO-)-Phe-Orn-Pro-cha-Bta-Phe-NH2, tBu-CO-Phe-Orn-Pro-cha-Bta-Phe-NH2, Ac-Lys-Phe-Orn-Aze-cha-Bta-Phe-NH2, Ac-Gly-Phe-Orn-Aze-cha-Bta-Phe-NH2, Ac-Arg-Phe-Orn-Aze-cha-Bta-Phe-NH2, Ac-His-Phe-Orn-Aze-cha-Bta-Phe-NH2, Ac-Ser-Phe-Orn-Aze-cha-Bta-Phe-NH2, Ac-Guf Phe-Orn-Aze-cha-Bta-Phe-NH2, Ac-Dab-Phe-Orn-Aze-cha-Bta-Phe-NH2, FH2C-CO-Phe-Orn-Pro-cha-Bta-Phe-NH2, Ac-Phe-Orn(Et2)-Pro-cha-Trp-Phe-NH2, Ac-Phe-[Orn-Hyp-cha-Trp-Nle], 3PP-[Orn-Hyp-cha-Trp-Nle], Ac-Phe-[Orn-Pro-cha-Trp-Tyr], Ac-Phe-[Orn-Pro-omf Trp-Nle], Ac-Phe-Orn-Pro-hle-Bta-Phe-NH2, Ac-Phe-Arg(CH2-CH2)-Pro-cha-Bta-Phe-NH2, Ac-Ala-Phe-Orn-Aze-cha-Bta-Phe-NH2, Ac-Arg-Phe-Orn-Aze-cha-Bta-Phe-NH2, Ac-Cit-Phe-Orn-Aze-cha-Bta-Phe-NH2, Ac-Gly-Phe-Orn-Aze-cha-Bta-Phe-NH2, Ac-Gly-Phe-Orn-Azechg-Bta-Phe-NH2, Ac-Gly-Phe-Orn-Aze-hch-Bta-Phe-NH2, Ac-Gly-Thi-Orn-Aze-cha-Bta-Phe-NH2, Ac-His-Phe-Orn-Aze-cha-Bta-Phe-NH2, Ac-Hyp-Phe-Orn-Aze-cha-Bta-Phe-NH2, Ac-Lys-Phe-Orn-Aze-cha-Bta-Phe-NH2, Ac-Mff-Orn-Pro-cha-Bta-Phe-NH2, Ac-Mff-Orn-Pro-hle-Bta-Phe-NH2, Ac-Mff-Orn-Pro-hle-Mcf-Mff-NH2, Ac-Mmy-Orn-Pro-hle-Pff-Phe-NH2, Ac-NMF-Orn-Pro-cha-Bta-Phe-NH2, Ac-Off-Orn-Pro-cha-Bta-Phe-NH2, Ac-Off-Orn-Pro-hle-Bta-Phe-NH2, Ac-Orn-Phe-Orn-Aze-cha-Bta-Phe-NH2, Ac-Pff-Orn-Pro-cha-Bta-Phe-NH2, Ac-Pff-Orn-Pro-hle-Bta-Phe-NH2, Ac-Pff-Orn-Pro-hle-Mcf-Pff-NH2, Ac-Phe-[Cys-Pro-cha-Bta-Phe-Cys]-NH2, Ac-Phe-[Orn-Asn-cha-Trp-Nle], Ac-Phe-[Orn-Aze-cha-Trp-Nle], Ac-Phe-[Orn-Chy-cha-Trp-Nle], Ac-Phe-[Orn-HyA-cha-Trp-Phe], Ac-Phe-[Orn-Hyp-hle-Bta-Phe], Ac-Phe-[Orn-Hyp-hle-Mcf-Phe], Ac-Phe-[Orn-Hyp-hle-Pff-Nle], Ac-Phe-[Orn-Hyp-hle-Pff-Phe], Ac-Phe-[Orn-Hyp-hle-Trp-Phe], Ac-Phe-[Orn-Hyp-Mmf Trp-Nle], Ac-Phe-[Orn-Hyp-Mmf Trp-Phe], Ac-Phe-[Orn-NMD-cha-Trp-Nle], Ac-Phe-[Orn-Pip-hle-Bta-Phe], Ac-Phe-[Orn-Pro-cha-Pff-Nle], Ac-Phe-[Orn-Pro-cha-Pff-Phe], Ac-Phe-[Orn-Pro-cha-Trp-1Ni], Ac-Phe-[Orn-Pro-cha-Trp-Cha], Ac-Phe-[Orn-Pro-cha-Trp-Chg], Ac-Phe-[Orn-Pro-cha-Trp-Ecr], Ac-Phe-[Orn-Pro-cha-Trp-Leu], Ac-Phe-[Orn-Pro-cha-Trp-nle], Ac-Phe-[Orn-Pro-cha-Trp-Phe], Ac-Phe-[Orn-Pro-hle-Bta-Nle], Ac-Phe-[Orn-Pro-hle-Bta-Phe], Ac-Phe-[Orn-Pro-hle-Pff-Phe], Ac-Phe-[Orn-Pro-hle-Trp-Nle], Ac-Phe-[Orn-Ser-cha-Trp-Nle], Ac-Phe-[Orn-Ser-cha-Trp-Nle], Ac-Phe-[Orn-Ser-hle-Trp-Nle], Ac-Phe-[Orn-Thr-cha-Trp-Nle], Ac-Phe-[Orn-Tic-cha-Trp-Nle], Ac-Phe-[Orn-Tic-cha-Trp-Nle], Ac-Phe-Ala-Pro-cha-Bta-Phe-NH2, Ac-Phe-Arg-Pro-hle-Bta-Phe-NH2, Ac-Phe-Arg-Pro-hle-Mcf-Phe-NH2, Ac-Phe-Cit-Hyp-hle-Bta-Phe-NH2, Ac-Phe-Cit-Pro-cha-Bta-Phe-NH2, Ac-Phe-Cit-Pro-hle-Bta-Phe-NH2, Ac-Phe-Cit-Ser-hle-Bta-Phe-NH2, Ac-Phe-Dab-Aze-cha-Bta-Phe-NH2, Ac-Phe-Dab-Aze-hle-Bta-Phe-NH2, Ac-Phe-Dab-Pro-cha-Bta-Phe-NH2, Ac-Phe-Dap-Pro-cha-Bta-Phe-NH2, Ac-Phe-Ech-Pro-cha-Bta-Phe-NH2, Ac-Phe-Eep-Pro-cha-Bta-Phe-NH2, Ac-Phe-Fcn-Aze-cha-Bta-Phe-NH2, Ac-Phe-Fcn-Pro-cha-Bta-Phe-NH2, Ac-Phe-Fco-Pro-cha-Bta-Phe-NH2, Ac-Phe-Fco-Pro-cha-Bta-Phe-NH2, Ac-Phe-Fcp-Aze-cha-Bta-Phe-NH2, Ac-Phe-Ffa-Aze-cha-Bta-Phe-NH2, Ac-Phe-Ffa-Pro-cha-Bta-Phe-NH2, Ac-Phe-Ffa-Pro-hle-Bta-Phe-NH2, Ac-Phe-G23-Pro-cha-Bta-Phe-NH2, Ac-Phe-Guf-Pro-cha-Bta-Phe-NH2, Ac-Phe-Har-Aze-cha-Bta-Phe-NH2, Ac-Phe-His-Pro-cha-Bta-Phe-NH2, Ac-Phe-L22-Pro-cha-Bta-Phe-NH2, Ac-Phe-OrA-Pro-cha-Bta-Phe-NH2, Ac-Phe-OrE-Pro-cha-Bta-Phe-NH2, Ac-Phe-Orn-Aze-hle-Bta-Phe-NH2, Ac-Phe-Orn-Chy-cha-Bta-Phe-NH2, Ac-Phe-Orn-Chy-hle-Pff Phe-NH2, Ac-Phe-Orn-G24-cha-Bta-Phe-NH2, Ac-Phe-Orn-G25-cha-Bta-Phe-NH2, Ac-Phe-Orn-G26-cha-Bta-Phe-NH2, Ac-Phe-Orn-G27-cha-Bta-Phe-NH2, Ac-Phe-Orn-G30-cha-Bta-Phe-NH2, Ac-Phe-Orn-G31-cha-Bta-Phe-NH2, Ac-Phe-Orn-Hse-cha-Bta-Phe-NH2, Ac-Phe-Orn-Hyp-hle-Bta-Phe-NH2, Ac-Phe-Orn-Hyp-hle-Pff-Phe-NH2, Ac-Phe-Orn-NMA-cha-Bta-Phe-NH2, Ac-Phe-Orn-NMS-cha-Bta-Phe-NH2, Ac-Phe-Orn-Pro-cha-1Ni-Phe-NH2, Ac-Phe-Orn-Pro-cha-Bta-1Ni-NH2, Ac-Phe-Orn-Pro-cha-Bta-Bhf-NH2, Ac-Phe-Orn-Pro-cha-Bta-Dff-NH2, Ac-Phe-Orn-Pro-cha-Bta-Eaa-NH2, Ac-Phe-Orn-Pro-cha-Bta-L19, Ac-Phe-Orn-Pro-cha-Bta-Mcf NH2, Ac-Phe-Orn-Pro-cha-Bta-Mff-NH2, Ac-Phe-Orn-Pro-cha-Bta-NH-CH(CH2OH)-CH2-Ph, Ac-Phe-Orn-Pro-Cha-Bta-NH-NBn-CO-NH2, Ac-Phe-Orn-Pro-cha-Bta-Opa-NH2, Ac-Phe-Orn-Pro-cha-Bta-Pcf NH2, Ac-Phe-Orn-Pro-cha-Bta-Pmf-NH2, Ac-Phe-Orn-Pro-cha-Bta-Thi-NH2, Ac- Phe-Orn- Pro-cha-Otf-Phe- NH2, Ac-Phe-Orn-Pro-ctb-Bta-Phe-NH2, Ac-Phe-Orn-Pro-ctb-Eaa-Phe-NH2, Ac-Phe-Orn-Pro-ctb-Mcf Phe-NH2, Ac-Phe-Orn-Pro-ctb-Pff-Phe-NH2, Ac-Phe-Orn-Pro-hch-Trp-Phe-OH, Ac-Phe-Orn-Pro-hle-1Ni-Phe-NH2, Ac-Phe-Orn-Pro-hle-6FW-Phe-NH2, Ac-Phe-Orn-Pro-hle-Bta-1Ni-NH2, Ac-Phe-Orn-Pro-hle-Bta-2Ni-NH2, Ac-Phe-Orn-Pro-hle-Bta-5Ff-NH2, Ac-Phe-Orn-Pro-hle-Bta-Aic-NH2, Ac-Phe-Orn-Pro-hle-Bta-Cha-NH2, Ac-Phe-Orn-Pro-hle-Bta-Chg-NH2, Ac-Phe-Orn-Pro-hle-Bta-Eaa-NH2, Ac-Phe-Orn-Pro-hle-Bta-Egy-NH2, Ac-Phe-Orn-Pro-hle-Bta-Pcf-NH2, Ac-Phe-Orn-Pro-hle-Bta-Pff-NH2, Ac-Phe-Orn-Pro-hle-Bta-Phe-NH2, Ac-Phe-Orn-Pro-hle-Bta-phe-OH, Ac-Phe-Orn-Pro-hle-Bta-Tyr-NH2, Ac-Phe-Orn-Pro-hle-Dff-Phe-NH2, Ac-Phe-Orn-Pro-hle-Eaa-Phe-NH2, Ac-Phe-Orn-Pro-hle-Egc-Phe-NH2, Ac-Phe-Orn-Pro-hle-Egy-Phe-NH2, Ac-Phe-Orn-Pro-hle-Egz-Phe-NH2, Ac-Phe-Orn-Pro-hle-Mcf-2Ni-NH2, Ac-Phe-Orn-Pro-hle-Mcf-Cha-NH2, Ac-Phe-Orn-Pro-hle-Mcf-Pff-NH2,Ac-Phe-Orn-Pro-hle-Mcf-Phe-NH2, Ac-Phe-Orn-Pro-hle-Mff-Phe-NH2,Ac-Phe-Orn-Pro-hle-Mmy-Phe-NH2, Ac-Phe-Orn-Pro-hle-Ocf-Phe-NH2, Ac-Phe-Orn-Pro-hle-Off-Phe-NH2, Ac-Phe-Orn-Pro-hle-Otf-Phe-NH2, Ac-Phe-Orn-Pro-hle-Pff-2Ni-NH2, Ac-Phe-Orn-Pro-hle-Pff-Cha-NH2, Ac-Phe-Orn-Pro-hle-Pff-Eaa-NH2, Ac-Phe-Orn-Pro-hle-Pff-Mmy-NH2, Ac-Phe-Orn-Pro-hle-Pff-Pff-NH2, Ac-Phe-Orn-Pro-hle-Pff-Phe-NH2, Ac-Phe-Orn-Pro-hle-Phe-Phe-NH2, Ac-Phe-Orn-Pro-hle-Tff-Phe-NH2, Ac-Phe-Orn-Pro-hle-Trp-Phe-NH2,Ac-Phe-Orn-Pro-ile-Trp-Phe-NH2, Ac-Phe-Orn-Pro-omf-Bta-Phe-NH2, Ac-Phe-Orn-Ser-cha-Bta-Phe-NH2, Ac-Ser-Phe-Orn-Aze-cha-Bta-Phe-NH2, Ac-Thi-[Orn-Pro-hle-Bta-Phe], Ac-Thi-Orn-Pro-cha-Bta-Phe-NH2, Ac-Thi-Orn-Pro-cha-Bta-Thi-NH2, Ac-Thr-Phe-Orn-Aze-cha-Bta-Phe-NH2, Bzl-[Orn-Pro-cha-Bta-Nle], CH3CH2CO-Phe-Orn-Pro-cha-Bta-Phe-NH2, Def-[Orn-Ser-hle-Trp-Nle], Eby-Phe-[Orn-Hyp-cha-Trp-Phe], Eth-Phe-[Orn-Pro-hle-Pff-Nle], FAc-Phe-Fib-Aze-cha-Bta-Phe-NH2, FAc-Phe-Orn-Aze-cha-Bta-Phe-NH2, FAc-Phe-Orn-Pro-cha-Bta-Phe-NH2, Fai-Phe-[Orn-Hyp-cha-Trp-Phe], Faz-Orn-Pro-cha-Bta-Phe-NH2, Fbi-Phe-[Orn-Pro-cha-Trp-Nle], Fbn-Phe-[Orn-Hyp-cha-Trp-Phe], Fbn-Phe-[Orn-Pro-cha-Trp-Nle], Fbn-Phe-[Orn-Pro-cha-Trp-Nle], Fbn-Phe-Cit-Pro-hle-Bta-Phe-NH2, Fbo-Phe-[Orn-Pro-cha-Trp-Nle], Fbp-[Orn-Pro-cha-Trp-Nle], Fci-[Phe-Orn-Hyp-cha-Trp-Phe], Fck-[Phe-Orn-Pro-cha-Trp-Nle], Fck-Phe-[Orn-Pro-cha-Trp-Nle], Fha-Phe-[Orn-Hyp-cha-Trp-Phe], Fhb-[Phe-Orn-Hyp-cha-Trp-Phe], Fhi-Phe-[Orn-Hyp-cha-Trp-Phe], Fhu-Phe-[Orn-Pro-h!e-Pff-Nle], Fhu-Phe-Orn-Pro-cha-Bta-Phe-NH2, Fid-Phe-Orn-Pro-cha-Bta-Phe-NH2, H-Amf[Orn-Aze-hle-Pff-Nle], H-Bal-Phe-[Orn-Hyp-hle-Trp-Nle], H-Bal-Phe-[Orn-Pro-hle-Pff-Nle], H-Eby-[Orn-Hyp-hle-Trp-Nle], H-Gly-Phe-Orn-Pro-cha-Bta-Phe-NH2, H-Nip-Phe-Cit-Pro-hle-Bta-Phe-NH2, Hoo-Phe-[Orn-Hyp-hle-Pff-Nle], Hoo-Phe-Cit-Pro-hle-Pff-Phe-NH2, Hoo-Phe-Orn-Hyp-hle-Pff Phe-NH2, Hoo-Phe-Orn-Pro-hle-Bta-Phe-NH2, Hoo-Phe-Orn-Pro-hle-Mcf-Phe-NH2, Hoo-Phe-Orn-Pro-hle-Pff-Phe-NH2, H-Phe-[Lys-Hyp-hle-Pff-Nle], H-Phe-[Orn-Hym-hle-Mcf-Nle], H-Phe-[Orn-Hym-hle-Pff-Phe], H-Phe-[Orn-Hyp-cha-Trp-Nle], H-Phe-[Orn-Hyp-cha-Trp-Phe], H-Phe-[Orn-Hyp-ctb-Pff-Nle], H-Phe-[Orn-Hyp-ctb-Trp-Nle], H-Phe-[Orn-Hyp-ctb-Trp-Phe], H-Phe-[Orn-Hyp-hle-Mcf-Leu], H-Phe-[Orn-Hyp-hle-Pff-Chg], H-Phe-[Orn-Hyp-hle-Pff-Hle], H-Phe-[Orn-Hyp-hle-Pff-Leu], H-Phe-[Orn-Hyp-hle-Pff-Nle], H-Phe-[Orn-Hyp-hle-Pff-Phe], H-Phe-[Orn-Hyp-hle-Trp-Hle], H-Phe-[Orn-Hyp-hle-Trp-Leu], H-Phe-[Orn-Hyp-hle-Trp-Nle], H-Phe-[Orn-Hyp-hle-Trp-Nva], H-Phe-[Orn-Hyp-hle-Trp-Phe], H-Phe-[Orn-NMS-cha-Trp-Nle], H-Phe-[Orn-NMS-hle-Pff-Phe], H-Phe-[Orn-Pro-cha-Pff-Nle], H-Phe-[Orn-Pro-cha-Pff-Phe], H-Phe-[Orn-Pro-cha-Trp-Nle], H-Phe-[Orn-Pro-hle-Mcf-Phe], H-Phe-[Orn-Pro-hle-Ocf-Phe], H-Phe-[Orn-Pro-hle-Pff-Nle], H-Phe-[Orn-Pro-hle-Pff-Phe], H-Phe-[Orn-Pro-hle-Trp-Nle], H-Phe-[Orn-Ser-cha-Trp-Nle], H-Phe-[Orn-Ser-cha-Trp-Phe], H-Phe-[Orn-Ser-hle-Eaa-Nle], H-Phe-[Orn-Ser-hle-Mcf-Leu], H-Phe-[Orn-Ser-hle-Ocf-Nle], H-Phe-[Orn-Ser-hle-Pff-Leu], H-Phe-[Orn-Ser-hle-Pff-Nle], H-Phe-[Orn-Ser-hle-Pff-Phe], H-Phe-[Orn-Ser-hle-Trp-Nle], H-Phe-Cit-Pro-hle-Bta-Phe-NH2, Ohf [Orn-Hyp-hle-Trp-Nle], Tmg-Phe-[Orn-Hyp-cha-Trp-Phe].

Eine weitere Gruppe von Verbindungen und/oder daraus abgeleitete, diese enthaltende Medikamente haben als Wirkort Integrine, insbesonders alpha5betal-Integrine, sind somit Integrin-Antagonisten, insbesondere alpha5beta1-Antagonisten, die mit den erfindungsgemäßen C5aR-Antagonisten und/oder diese umfassenden Medikamenten in Kombination zur Prävention und/oder Behandlung verwendet werden können.

Mindestens drei Klassen von Reagentien wurden als Integrin-, insbesondere alpha5beta1-Integrin-Antagonisten entwickelt. Diese beinhalten Antikörper wie z.B. monoklonale Antikörper, polyklonale Antikörper und Antikörperfragmente (Kim et al. 2000 Am. J. Path. 156: 1345), natürliche Peptide wie zB. venom-abgeleitete"disintegrin-Peptide" (Marcinkiewicz et al. 1999 Biochemistry 38: 13302), synthetische Peptide (z.B. Koivunen et al. 1994 JBC 124: 373, US6001965) und nicht-peptidische Small Molecules wie z.B. Spiro-Verbindungen (WO9733887) oder Benzyl-Verbindungen (WO9532710).

Weitere Small Molecules, die als Integrin-, insbesondere alpha5beta1-Integrin-Antagonisten wirken können, besitzen die folgende Struktur: 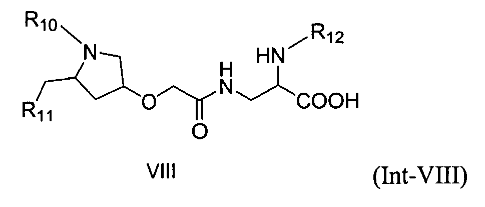 wobei R₁₀ -CO-R₁₃ oder -CO-O-R₁₃ ist,
wobei R₁₁ ein substituiertes Pyridin-2-ylamin ist,
wobei R₁₂ -CO-R₁₃ oder -SO₂-R₁₃ ist und
wobei R₁₃ ein Radikal ist, das ausgewählt ist aus der Gruppe, die Alkyl, substituiertes Alkyl, Cycloalkyl, Aryl, substituiertes Aryl, Heteroaryl, substituiertes Heteroaryl umfasst.

Eine weitere Gruppe von Verbindungen und/oder daraus abgeleitete, diese enthaltende Medikamente haben als Wirkort Kinine und insbesondere Bradykinin, sind somit Kini- oder Bradykinin-Antagonisten, die mit den erfindungsgemäßen C5aR-Antagonisten und/oder diese umfassenden Medikamenten in Kombination zur Prävention und/oder Behandlung verwendet werden können.

Bradykinin-Antagonisten können ausgewählt sein aus der Gruppe der B1-Inhibitoren und umfassen bevorzugterweise :
Ac-Lys-Arg-Pro-Pro-Gly-Phe-Ser-D-beta-Nal-Ile
Ac-Lys-Arg-Pro-Pro-Gly-N-MePhe-Ser-D-beta-Nal-Ile
AcLys-Lys-Arg-Pro-Pro-Gly-NMePhe-Ser-D-betaNal-Ile
(Gobeil et al. 1999 Hypertension 33: 823)

AcOrn-Arg-Oic-Pro-Gly-NMePhe-Ser-D-betaNal-Phe
(Gabra und Sirois 2003 Peptides 24: 1131)

Lys-Lys-Arg-Pro-Hyp-Gly-Igl-Ser-DIgl-Oic
Lys-Lys-Arg-Pro-Hyp-Gly-CpG-Ser-DTic-CpG
CpG=Cyclopentylglycin
(Stewart et al. 1996 Immunopharmacology 33: 51)
2-[1-(3,4-Dichlor-benzenesulfonyl)-3-oxo-1,2,3,4-tetrahydro-chinoxalin-2-yl]-N- {2-[4-(4,5-dihydro-1H-imidazol-2-yl)-phenyl]-ethyl} -acetamid
N- {2-[4-(4,5-Dihydro-1H-imidazol-2-yl)-phenyl]-ethyl}-2-[1-(naphthalin-2-sulfonyl)-3-oxo-1,2,3,4-tetrahydro-chinoxalin-2-yl]-acetamid
3-(3,4-Dichlor-phenyl)-N-{1-[4-(4,5-dihydro-1H-imidazol-2-yl)-benzyl]-2-oxo-2-pyrrolidin-1-yl-ethyl} -3-(naphthalin-2-sulfonylamino)-propionamid
4'-(1-{3-[(2,2-Difluor-cyclopropanecarbonyl)-amino]-4-methyl-pyridin-2-ylamino} -ethyl)-5-methyl-biphenyl-2-carbonsäuremethylester
N-(4-Chlor-2- {1-[3'-fluoro-2'-(3-methyl-[1,2,4]oxadiazol-5-yl)-biphenyl-4-yl]-ethylamino}-pyridin-3-yl)-3,3,3-trifluor-propionamid
(Hess et al. 2004 J. Pharmacol. Exp. Ther. 310: 488)
3-Benzo[1,3]dioxol-5-yl-N-[2-[4-(2,6-dimethyl-pipendin-1-ylmethyl)-phenyl]-1-(isopropylmethyl-carbamoyl)-ethyl]-3-(6-methoxy-naphthalin-2-sulfonylamino)-propionamid
(Gougat et al. 2004 Pharmacol. Exp. Ther. 309: 661)
{2-(2,2-Diphenyl-ethylamino)-5-[4-(4-isopropyl-piperazine-1-carbonyl)-piperidine-1-sulfonyl]-phenyl}-morpholin-4-yl-methanon
{2-(2,2-Diphenyl-ethylamino)-5-[4-(4-methyl-piperazine-1-carbonyl)-piperidine-1-sulfonyl]-phenyl} -morpholin-4-yl-methanon
(Ritchie et al. 2004 J. Med. Chem. 47: 4642)

Bradykinin-Antagonisten können aus der Gruppe der B2-Inhibitoren ausgewählt sein: 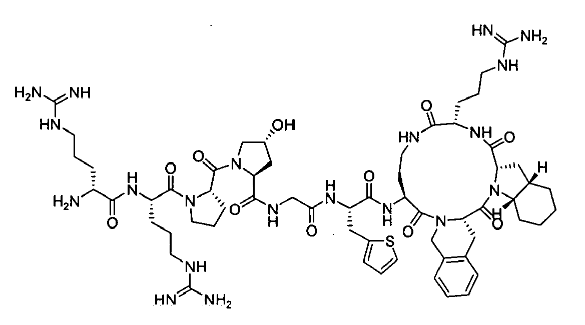 (Meini et al. 1999 J. Pharmacol. Exp. Ther. 289: 1250)
D-Arg-Arg-Pro-Hyp-Gly-Igl-Ser-D-F5f-Igl-Arg (B-10056)
Arg- ⁰Arg¹- Pro²-Hyp³ -Gly⁴- Igl⁵ -Ser⁶- D- Igl⁷ -Oic⁸ -Arg⁹ (B-9430)
(Stewart et al. 1999 Immunopharmacology 43: 155)
[D-Arg-Arg-Pro-Hyp-Gly-Phe-Cys-D-Phe-Leu-Arg]₂BSH
BSH=bis-Succinimidohexan (CP-0127Bradycor) 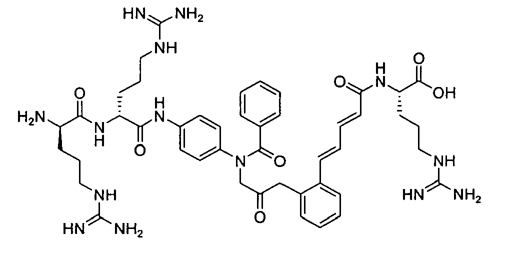 FR 167344 ; 4-{2-[({[3-(3-Brom-2-methyl-imidazo[1,2-a]pyridin-8-yloxymethyl)-2,4-dichlorphenyl]-methyl-carbamoyl}-methyl)-carbamoyl]-vinyl}-N,N-dimethyl-benzamid
FR 173657; FK3657; 3-(6-Acetylamino-pyridin-3-yl)-N-({[2,4-dichlor-3-(2-methyl-chinolin-8-yloxymethyl)-phenyl]-methyl-carbamoyl}-methyl)-acrylamid
LF-160687; Anatibant; 1-[2,4-Dichlor-3-(2,4-dimethyl-chinolin-8-yloxymethyl)-benzenesulfonyl]-pyrrolidin-2-carbonsäure [3-(4-carbamimidoyl-benzoylamino)-propyl]-amid 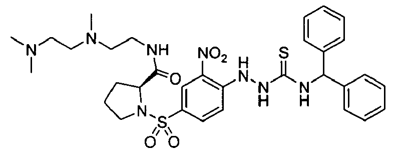 Bradizide
(Heitsch 2002 Curr Med Chem 9: 91)
LF-160335; 4-(4-{1-[2,4-Dichlor-3-(2,4-dimethyl-chinolin-8-yloxymethyl)-benzenesulfonyl]-pyrrolidin-2-carbonyl} -piperazin-1-carbonyl)-benzamidin (Pruneau, et al. 1998 Br. J. Pharmacol. 125 : 365)
2-[5-(4-Cyano-benzoyl)-1-methyl-1H-pyrrol-2-yl]-N-[2,4-dichlor-3-(2-methyl-chinolin-8-yloxymethyl)-phenyl]-N-methyl-acetamid
(R., C. In Medicinal Chemistry - 28th National Symposium (Part II) - Overnight Report; IDdb meeting Report: San Diego, 2002)

Weitere Verbindungen, die als Kinin-Rezeptor-Antagonisten, bevorzugterweise als Bradykinin-Antagonisten, wirken können und somit errfindungsgemäß mit den Verbindungen, d.h. C5aR-Antagonisten, der vorliegenden Erfindung in Kombination verwendet werden können, sind Verbindungen der folgenden Struktur (I):

**Z-P-A-B-C-E-F-K-(D)Q-G-M-F'-I** (BI)

, wobei:
Z a₁) Wasserstoff, (C₁-C₈)-Alkyl, (C₁-C₈)-Alkanoyl, (C₁-C₈)-Alkoxycarbonyl, (C₃-C₈)-Cycloalkyl, (C₄-C₉) -Cycloalkanoyl oder (C₁-C₈)-Alkylsulfonyl ist,
   wobei 1, 2 oder 3 Wasserstoffatome optional und unabhängig voneinander ersetzt sind durch 1, 2 oder 3 identische oder unterschiedliche Radikale, die ausgewählt sind aus der Gruppe, die Carboxyl, NHR (1), [(C₁-C₄)-Alkyl]NR(1) oder [(C₆-C₁₀)-Aryl-(C₁₋C₄)-Alkyl]NR(1) enthält, wobei R(1) Wasserstoff ist oder eine Urethan-Schutzgruppe, (C₁-C₄)-Alkyl, (C₁-C₈)-Alkylamino, (C₆-C₁₀)-Aryl-(C₁-C₄)-Alkylamino, Hydroxyl, (C₁-C₄)-Alkoxy, Halogen, Di-(C₁-C₈)-Alkylamino, Di-[(C₆-C₁₀)-Aryl-(C)-C₄)]-Alkylamino, Carbamoyl, Phthalimido, 1,8-Naphthalimido, Sulfamoyl, (C₁-C₄) Alkoxycarbonyl, (C₆-C₁₄)-Aryl und (C₆-C₁₄)-Aryl-(C₁-C₅)-Alkyl umfasst,
   oder wobei 1 Wasserstoffatom in jedem Falle optional ersetzt ist durch ein Radikal, das ausgewählt ist aus der Gruppe, die (C₃-C₈)-Cycloalkyl, (C₁-C₆)-Alkylsulfonyl, (C₁-C₆)-Alkylsulfinyl, (C₆-C₁₄)-Aryl-(C₁-C₄)-Alkylsulfonyl, (C₆-C₁₄)-Aryl-(C₁-C₄)-Alkylsulfinyl, (C₆-C₁₄)-Aryl, (C₆-C₁₄)-Aryloxy, (C₃-C₁₃)-Heteroaryl und (C₃-C₁₃)-Heteroaryloxy enthält,
   und 1 oder 2 Wasserstoffatome ersetzt sind durch 1 oder 2 identische oder unterschiedliche Radikale, die ausgewählt sind aus der Gruppe, die Carboxyl, Amino, (C₁-C₈)-Alkylamino, Hydroxyl, (C₁-C₄)-Alkoxy, Halogen, Di-(C₁-C₈)-Alkylamino, Carbamoyl, Sulfamoyl, (C₁-C₄)-Alkoxycarbonyl, (C₆-C₁₄)-Aryl und (C₆-C₁₄)-Aryl-(C₁-C₅)-Alkyl umfasst;
   a₂) (C₆-C₁₄)-Aryl, (C₇-C₁₅)-Aroyl, (C₆-C₁₄)-Arylsulfonyl, (C₃-C₁₃)-Heteroaryl oder (C₃-C₁₃)-Heteroaroyl ist; oder
   a₃) Carbamoyl ist, das optional substituiert sein kann am Stickstoffatom durch (C₁-C₈)-Alkyl, (C₆-C₁₄)-Aryl oder (C₆-C₁₄)-Aryl-(C₁-C₅)-Alkyl;
   wobei in den Radikalen, die unter a₁), a₂) and a₃) definiert sind, die Aryl-, Heteroaryl-, Aroyl-, Arylsulfonyl- und Heteroaroyl-Gruppen optional substituiert sind durch 1, 2, 3 oder 4 Radikale, die einzeln und unabhängig voneinander ausgewählt sind aus der Gruppe, die Carboxyl, Amino, Nitro, (C₁-C₈)-Alkylamino, Hydroxyl, (C₁-C₆)-Alkyl, (C₁-C₆)-Alkoxy, (C₆-C₁₄)-Aryl, (C₇-C₁₅)-Aroyl, Halogen, Cyano, Di-(C₁-C₈)-Alkylamino, Carbamoyl, Sulfamoyl und (C₁-C₆)-Alkoxycarbonyl enthält;
P eine chemische Bindung oder ein Radikal der Struktur BII ist,

   -NR(2)―(U)―CO- (BII)

   wobei
   R(2) Wasserstoff, Methyl oder eine Urethan-Schutzgruppe ist,
U (C₃-C₈)-Cycloalkyliden, (C₆-C₁₄)-Aryliden, (C₃-C₁₃)-Heteroaryliden, (C₆-C₁₄)-Aryl-(C₁₋C₆)-Alkylidene ist, welche jeweils und unabhängig voneinander optional substituiert sein können, oder [CHR(3)]ₙ ist,
   wobei n eine Zahl im Bereich 1-8, bevorzugterweise 1-6 angibt,
R(3) einzeln und unabhängig ausgewählt ist aus der Gruppe, die Wasserstoff, (C₁-C₆)-Alkyl, (C₃-C₈)-Cycloalkyl, (C₆-C₁₄)-Aryl, (C₃-C₁₃)-Heteroaryl enthält, welche mit der Ausnahme von Wasserstoff jeweils optional monosubstituiert sind durch Amino, substituiertes Amino, Amidino, substituiertes Amidino, Hydroxyl, Carboxyl, Carbamoyl, Guanidino, substituiertes Guanidino, Ureido, substituiertes Ureido, Mercapto, Methylmercapto, Phenyl, 4-Chlorphenyl, 4-Fluorphenyl, 4-Nitrophenyl, 4-Methoxyphenyl, 4-Hydroxyphenyl, Phthalimido, 1,8-Naphthalimido, 4-Imidazolyl, 3-Indolyl, 2-Thienyl, 3-Thienyl, 2-Pyridyl, 3-Pyridyl oder Cyclohexyl,
   wobei substituiertes Amino bevorzugterweise -N(A')―Z ist, substituiertes Amidino bevorzugterweise ―(NH=)C―NH―Z ist, substituiertes Guanidino bevorzugterweise - N(A')C[=N(A')]― NH―Z ist und substituiertes Ureido bevorzugterweise -CO― N(A')―Z ist, wobei A' unabhängig voneinander Wasserstoff oder Z ist, wobei Z definiert ist wie unter a₁) oder a₂);
   oder
   wobei R(2) und R(3), zusammen mit den Atomen, die sie tragen, ein mono-, bi- oder tricyclisches Ringsystem mit 2 bis 15 Kohlenstoffatomen bilden;
A definiert ist als P;
B eine basische Aminosäure in der L-or D-Konfiguration ist, die optional an der Seitenkette substituiert ist ;
C eine Verbindung der Formel BIII a oder BIII b ist 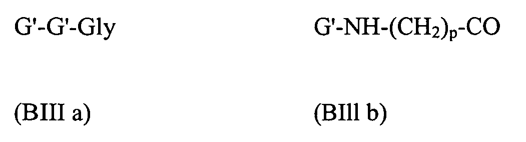 wobei
p eine Zahl im Bereich von 2 bis 8 angibt, und
G' jeweils unabhängig voneinander ein Radikal der Formel IV ist

   -NR(4)-CHR(5)-CO- (BIV)

   wobei
   R(4) und R(5), zusammen mit den Atomen, die sie tragen, ein mono-, bi- oder tricyclisches Ringsystem mit 2 bis 15 Kohlenstoffatomen bilden
E das Radikal einer neutralen, sauren oder basichen, aliphatischen oder alicyclischaliphatischen Aminosäure ist;
F unabhängig voneinander ein Radikal einer neutralen, sauren oder basichen, aliphatischen oder aromatichen Aminosäure ist, die an der Seitenkette substituiert sein kann, oder eine chemische Bindung ist;
(D)Q D-Tic, D-Phe, D-Oic, D-Thi oder D-Nal, das optional substituiert sein kann durch Halogen, Methyl oder Methoxy oder ein Radikal der Formel (BV) ist 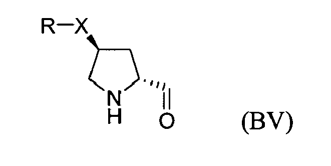 wobei
X Sauerstoff, Schwefel oder eine chemische Bindung ist;
R Wasserstoff, (C₁-C₈)-Alkyl, (C₃-C₈)-Cycloalkyl, (C₆-C₁₄)-Aryl, (C₆-C₁₄)-Aryl-(C₁-C₄)-Alkyl ist, wobei das alicycliche System optional substituiert sein kann durch Halogen, Methyl oder Methoxy;
G definiert ist wie G' oben oder eine chemische Bindung ist;
F' definiert ist wie F, ein Radikal -NH-(CH₂)_{q}- mit q=2 bis 8 ist, oder, falls G keine Bindung ist, eine Bindung ist;
I -OH, -NH₂ oder NHC₂H₅ ist;
K das Radikal -NH-(CH₂)ₓ-CO- ist, wobei x=1-4, oder eine Bindung ist, und
M wie F definiert ist,
   oder ein physiologisch tolerables Salz davon.

Bevorzugterweise ist das Peptid ein Peptid der Formel BI, wobei
Z Wasserstoff ist oder definiert ist wie oben unter a₁), a₂) oder a₃),
P eine Bindung oder ein Radikal der Formel BII ist

-NR(2)-(U)―CO― (BII)

wobei
U CHR(3) ist und
R(3) definiert ist wie oben,
R(2) H oder CH₃ ist,
A eine Bindung ist.

Besonders bevorzugterweise ist das Peptid ein Peptid gemäss der vorangehenden Beschreibungen, ganz besonders bevorzugterweise gemäß der letzten Beschreibung,
wobei
Z Wasserstoff ist oder definiert ist wie oben unter a₁), a₂) oder a₃),
P eine Bindung oder ein Radikal der Formel BII ist

-NR(2)―(U)-CO- (BII)

wobei
U CHR(3) ist und
R(3) einzeln und unabhängig voneinander ausgewählt ist aus der Gruppe umfassend Wasserstoff, (C1-C6)-Alkyl, (C3-C8)-Cycloalkyl, (C6-C14)-Aryl, (C3-C13)-Heteroaryl, welche mit der Ausnahme von Wasserstoff jeweils optional monosubstituiert sind durch Amino, substituiertes Amino, Hydroxyl, Carboxyl, Carbamoyl, Guanidino, substituiertes Guanidino, Ureido, Mercapto, Methylmercapto, Phenyl, 4-Chlorphenyl, 4-Fluorphenyl, 4-Nitrophenyl, 4-Methoxyphenyl, 4-Hydroxyphenyl, Phthalimido, 4-Imidazolyl, 3-Indolyl, 2-Thienyl, 3-Thienyl, 2-Pyridyl, 3-Pyridyl oder Cyclohexyl,
oder wobei R(2) und R(3), zusammen mit den Atomen, die sie tragen, ein mono-, bi- oder tricyclisches Ringsystem mit 2 bis 15 Kohlenstoffatomen bilden,
R(2) H oder CH3 ist;
A eine Bindung ist;
(D)Q D-Tic ist oder ein physikalisch toleriertes Salz davon.

Besonders bevorzugterweise ist das Peptid ein Peptid gemäss der vorangehenden Beschreibungen,wobei dieses ausgewählt ist aus der Gruppe :
H-D-Arg-Arg-Pro-Hyp-Gly- Thi-Ser-D-Tic-Oic-Arg-OH (HOE 140)
para-guanidobenzoyl-Arg-Pro-Hyp-Gly-Thi-Ser-D-Tic-Oic-Arg-OH
H-D-Arg-Arg-Pro-Hyp-Gly-Phe-S er-D-HypE(transpropyl)-Oic-Arg-OH
H-D-Arg-Arg-Pro-Hyp-Gly-Cpg-Ser-D-Cpg-Cpg-Arg-OH
H-D-Arg-Arg-Pro-Pro-Gly-Thi-Ser-D-Tic-Oic-Arg-OH
H-Arg(Tos)-Pro-Hyp-Gly-Thi-Ser-D-Tic-Oic-Arg-OH
H-Arg(Tos)-Pro-Hyp-Gly-Phe-Ser-D-Tic-Oic-Arg-OH
H-D-Arg -Arg-Pro-Hyp-Gly-Phe-Ser-D-Tic-Oic-Arg-OH
Fmoc-D-Arg-Arg-Pro-Hyp-Gly-Thi-Ser-D-Tic-Oic-Arg-OH
Fmoc-Aoc-D-Arg-Arg-Pro-Hyp-GIy-Thi-Ser-D-Tic-Oic-Arg-OH
Fmoc-ε-aminocaproyl-D-Arg-Arg-Pro-Hyp-Gly-Thi-Ser-D-Tic-Oic-Arg-OH
benzoyl-D-Arg-Arg-Pro-Hyp-Gly-Thi-Ser-D-Tic-Oic-Arg-OH
cyclohexylcarbonyl-D-Arg-Arg-Pro-Hyp-Gly-Thi-Ser-D-Tic-Oic-Arg-OH
Fmoc-Aeg(Fmoc)-D-Arg-Arg-Pro-Hyp-Gly-Thi-Ser-D-Tic-Oic-Arg-OH
Fmoc-Aeg(Fmoc)-Arg-Pro-Hyp-Gly-Thi-Ser-D-Tic-Oic-Arg-OH
indol-3-yl-acetyl-D-Arg-Arg-Pro-Hyp-Gly-Thi-Ser-D-Tic-Oic-Arg-OH
dibenzylacetyl-D-Arg-Arg-Pro-Hyp-Gly-Thi-Ser-D-Tic-Oic-Arg-OH
oder ein physikalisch tolerierbares Salz davon.

Besonders bevorzugterweise ist das Peptid ein Peptid gemäss der vorangehenden Beschreibung, wobei dieses ausgewählt ist aus
H-D-Arg-Arg-Pro-Hyp-Gly- Thi-Ser-D-Tic-Oic-Arg-OH (HOE 140)
para-Guanidobenzoyl-Arg-Pro-Hyp-Gly-Thi-Ser-D-Tic-Oic-Arg-OH; oder ein physikalisch toleriertes Salz davon.

Besonders bevorzugterweise ist das Peptid ein Peptid gemäss der vorangehenden Beschreibungen, mit folgender Struktur
H-D-Arg-Arg-Pro-Hyp-Gly- Thi-Ser-D-Tic-Oic-Arg-OH (HOE 140)

Es wird von den Fachleuten verstanden werden, dass verschiedene Erkrankungen zu verschiedenen Oberbegriffen, wie hierin verwendet, zugeordnet werden können. Diese Zuordnung stellt keine Limitierung dar, vielmehr kann die jeweilige Erkrankung für sich selbst genommen mittels der erfindungsgemäßen Verbindungen behandelt oder verhindert werden. Es wird weiter von den Fachleuten verstanden werden, dass die in Klammern hierin angegebenen Erkrankungen synonym verwendet werden oder besondere Formen der angegebenen Erkrankung darstellen.

### Figurenbeschreibung:

Die Erfindung wird nun anhand der folgenden Figuren und Beispiele näher erläutert werden, aus denen sich weitere Merkmale, Ausführungsformen und Vorteile ergeben. Dabei zeigt
- Fig. 1:: RP-HPLC-Chromatogramme von **11** und **13** unter verschiedenen Lagerungsbedingungen sowie mögliche Route bei der Zersetzung von **11**; genauer
- Fig. 1A): nach der Reinigung per HPLC (Lösung in MeCN/H₂O/TFA),
- Fig. 1 B): nach dem Lyophilisieren (0 Tage Lagerung als Feststoff),
- Fig. 1 C): nach 7 Tagen Lagerung als Feststoff; und
- Fig. 2: *in vivo* Wirkung einer beispielhaften, erfindungsgemäßen Verbindung in einem Modell der C5a induzierten Neutropänie. Aufgetragen ist auf der X-Achse der zeitliche Verlauf des Experiments. Die Y-Achse stellt den prozentualen Anteil der Neutrophilen im Blutkreislauf dar.

### Beispiele:

### Beispiel 1: Material und Methoden

Die im Folgenden beschriebenen Materialien und Methoden sowie allgemeinen Arbeitsvorschriften wurden im Rahmen der hierin beschriebenen weiteren Beispiele durchgeführt.

### Lösungsmittel:

Alle verwendeten Lösungsmittel wurden in der angegebenen Qualität ohne weitere Reinigung eingesetzt:
Acetonitril (Gradient grade, J.T. Baker); Dichlormethan (zur Synthese, Merck Eurolab); Diethylether (zur Synthese, Merck Eurolab); *N,N-*Dimethylformamid (LAB, Merck Eurolab); Dioxan (zur Synthese, Aldrich); Methanol (zur Synthese, Merck Eurolab).
Wasser wurde unter Verwendung einer Vollentsalzungsanlage (Milli-Q Plus, Millipore) entmineralisiert.

### Reagenzien:

Die verwendeten Reagenzien wurden von den Firmen Advanced ChemTech (Bamberg, Deutschland), Sigma-Aldrich-Fluka (Deisenhofen, Deutschland), Bachem (Heidelberg, Deutschland), J.T. Baker (Phillipsburg, USA), Lancaster (Mühlheim/Main, Deutschland), Merck Eurolab (Darmstadt, Deutschland), Neosystem (Strassburg, Frankreich), Novabiochem (Bad Soden, Deutschland, ab 2003 Merck Biosciences, Darmstadt, Deutschland), Acros (Geel, Belgien, Vertriebsgesellschaft Fisher Scientific GmbH, Schwerte, Deutschland), Peptech (Cambridge, MA, USA), Synthetech (Albany, OR, USA), Pharmacore (High Point, NC, USA) und Anaspec (San Jose, CA, USA) bezogen und ohne weitere Aufreinigung verwendet. Nicht kommerziell erhältliche unnatürliche Aminosäuren oder Carbonsäuren zur N-terminalen Modifizierung wurden nach Standardvorschriften hergestellt. So erhielt man Fmoc-cis-Hyp-OH durch Umsetzung von H-cis-Hyp-OH mit Fmoc-OSu [Paquet et al. 1982 Canadian Journal of Chemistry 60: 976-980A]. Fmoc-Phe(4-STrt-Amidino)-OH synthetisierte man durch eine bekannte Vorschrift [Pearson et al. 1996 Journal of Medicinal Chemistry 39:1372]. Seitenketten-modifizierte Cystein-Derivate wurden durch Alkylierung von Fmoc-Cystein-OH mit Alkylhalogeniden dargestellt.

Bei den Konzentrationen der Reagenzien in Prozent handelt es sich, sofern nicht anders angegebenen, um Volumenprozent (v/v).

### RP-HPLC-MS-Analysen:

Analytische Chromatographie erfolgte unter Verwendung eines Hewlett Packard Serie 1100-Systems (Entgaser G1322A, quaternäre Pumpe G1311A, automatischer Probengeber G1313A, thermostatiertes Säulenfach G 1316A, Variabler UV-Detektor G1314A) und gekoppelter ESI-MS (Finnigan LCQ Ion-Trap-Massenspektrometer). Dazu wurde eine Steuersoftware der Firma Finnigan verwendet (Navigator Ver 1.1 sp1). Als Stossgas in der Ionenfalle diente Helium. Die Trennung erfolgte an RP-18-Säulenmaterial (Vydac 218 TP5215, 2.1 x 150 mm, 5 µm, C18, 300 A mit Vorsäule (Merk)) bei 30°C und einem Fluss von 0.3 ml/min unter Anwendung eines linearen Gradienten für alle Chromatogramme (5:95% B innerhalb von 25 min, wobei A: 0.05% TFA in Wasser und B: 0.05% TFA in CH₃CN). Die UV-Detektion erfolgte bei λ = 220 nm. Retentionszeiten (Rₜ) sind im Dezimalsystem angegeben (z.B. 1.9 min = 1 min 54 sec) und beziehen sich auf die Detektion im Massenspektrometer. Die Totzeit zwischen Injektion und UV-Detektion (HPLC) betrug 1.65 min, zwischen UV-Detektion und Massendetektion 0.21 min. Die Genauigkeit des Massenspektrometers beträgt ca. ± 0.2 amu.

Analytik mittels HPLC-MS, Injektion von 5 µl, Gradient linear von 95:5 zu 5:95 in 9.5 min (A; Wasser mit 0.05% TFA, B: Acetonitril mit 0.05% TFA), RP-Säule der Firma Phenomenex, Typ Luna (C-18), 3 µm, 50x2.00 mm, Fluss 0.3 ml, bei Raumtemperatur HPLC; ThermoFinnigan Surveyor mit PDA-Detektor (210-350 nm), MS; ThermoFinnigan Advantage bzw. LCQ-Classic (beide Iontrap), ESI-Ionisation, als Stossgas in der Ionenfalle diente Helium. Excalibur Vers. 1.3 bzw. 1.2. Retentionszeiten (Rt) werden im Dezimalsystem angegeben (z.B. 1.9 min = 1 min 54 sec).

### Präparative HPLC:

Präparative HPLC-Trennungen wurden auf Vydac R18-RP-Säulen mit Gradienten folgender Lösungsmittel durchgeführt: A: 0.05% TFA in Wasser und B: 0.05% TFA in CH₃CN.

**Tabelle 1: Verwendete Abkürzungen:**

| | |
|---|---|
| Abb. | Abbildung |
| AAV | Allgemeine Arbeitsvorschrift |
| Ac | Acetyl |
| CABG | Coronary Artery Bypass Grafting |
| d | Dublett |
| DCM | Dichlormethan |
| DIPEA | N,N-Diisopropylethylamin |
| DMF | *N*,*N*-Dimethylformamid |
| DMEM | Dulbecco's Modified Eagle Medium |
| DMSO | Dimethylsulfoxid |
| eq. | Äquivalent(e) |
| fMLF | Formylmethionine-Leucin-Phenylalanine |
| h | Stunde(n) |
| HEPES | *N*-2-2-Hydroxyethyl-1-piperazin-*N*'-2-ethanolsulfonsäure |
| HOBt | 1-Hydroxybenzotriazol |
| HPLC | high-pressure liquid chromatography |
| m | Multiplett |
| Me | Methyl |
| MidCAB | minimal invasive direct coronary artery bypass |
| min | Minute |
| ml | Milliliter |
| NMP | *N*-Methylpyrrolidon |
| NMR | nuclear magnetic resonance |
| OPCAB | off pump coronary artery bypass |
| Ph | Phenyl |
| PTCA | perkutane transluminale coronare Angioplastie |
| PTA | Perkutane transluminale Angioplastie |
| q | Quartett |
| RT | Raumtemperatur |
| s | Singulett |
| ^{t}Bu | tert-Butyl |
| THF | Tetrahydrofuran |
| TFA | Trifluoressigsäure |
| t | Triplett |
| WSCxHCl | 1-Ethyl-3-(3'-dimethylaminopropyl)carbodiimid-Hydrochlorid |

### Beispiel 2: Allgemeine Arbeitsvorschriften zur Synthese von Verbindungen

### AAV-1: Synthese sekundärer Amine

AAV-1A: Synthese sekundärer Amine durch reduktive Aminierung von Aldehyden oder Ketonen mit NaBH₃CN oder NaBH₄ und Titantetraisopropylat

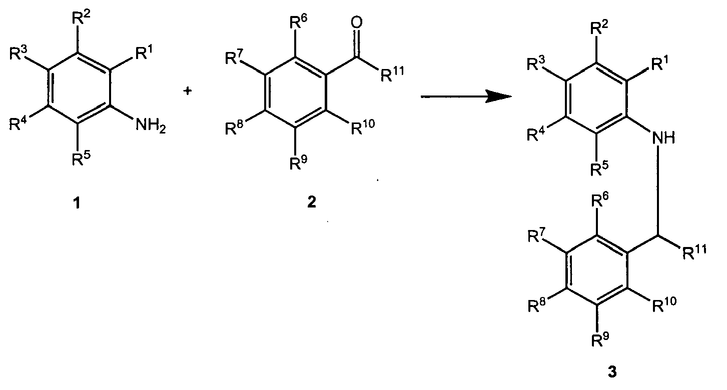

Die Carbonylverbindung **2** wird in THF gelöst. Man gibt 1.1 eq. Titantetraisopropylat zu und versetzt die erhaltene Lösung mit 1.0 eq. des Amins **1.** Nach eintägigem Rühren bei Raumtemperatur werden 3.0 eq. Natriumcyanoborhydrid oder Natriumborhydrid gelöst in Ethanol zugetropft. Man rührt für 2 bis 5 Stunden und entfernt dann das Lösungsmittel am Rotationsverdampfer. Nach Zugabe von ca. 20-80 ml 2N NaOH-Lösung und ca. 20-80 ml Dichlormethan zentrifugiert man den weißen Niederschlag ab und extrahiert die wässrige Phase dreimal mit Dichlormethan. Die vereinigten organischen Phasen werden über Magnesiumsulfat getrocknet und das Lösungsmittel im Vakuum entfernt. Man erhält das gewünschte sekundäre Amin **3,** das entweder direkt für weitere Reaktionen eingesetzt oder per Flashchromatographie oder HPLC gereinigt wird.

AAV-1B: Synthese sekundärer Amine durch Kupplung einer Boronsäure mit einem primären Amin 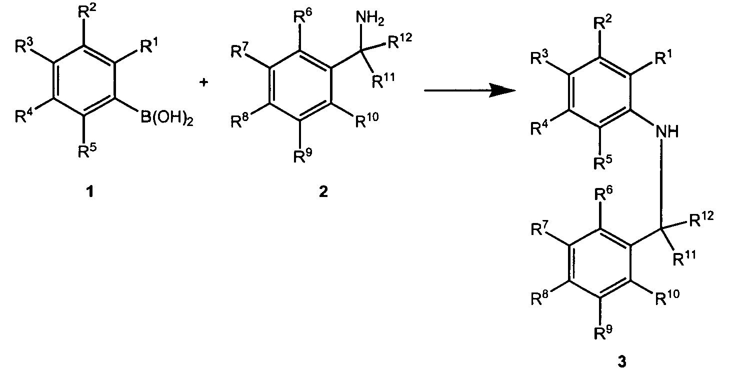 Das primäre Amin **2** wird in Dichlorethan gelöst. Man gibt 1.0 eq. Kupfer-II-acetat und optional 1.0 eq. 2,6-Lutidin zu und versetzt die erhaltene Lösung mit 1.2 eq. der Boronsäure **1.** Nach eintägigem Rühren bei Raumtemperatur wird das Lösungsmittel am Rotationsverdampfer entfernt. Nach Zugabe von ca. 20-80 ml 2N NaOH-Lösung und ca. 20-80 ml Dichlormethan extrahiert man die wässrige Phase dreimal mit Dichlormethan. Die vereinigten organischen Phasen werden über Magnesiumsulfat getrocknet und das Lösungsmittel im Vakuum entfernt. Man erhält das gewünschte sekundäre Amin 3, das entweder direkt für weitere Reaktionen eingesetzt oder per Flashchromatographie oder HPLC gereinigt wird.

AAV-1C: Synthese sekundärer Amine durch Alkylierung primärer Amine mit Alkylhalogeniden 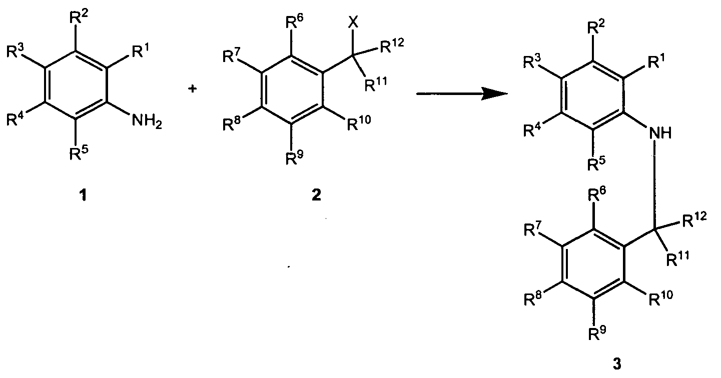 Das Amin **1** wird in THF gelöst. Man tropft eine Lösung von 0.8 bis 1.2 eq. des Alkylhalogenids **2** zu und versetzt mit einer Spatelspitze Kaliumcarbonat. Nach eintägigem Rühren bei 60°C entfernt man das Lösungsmittel am Rotationsverdampfer, gibt ca. 20 bis 80 ml 1M HCl-Lösung und ca. 20 bis 80 ml Dichlormethan zu und extrahiert die wässrige Phase dreimal mit Dichlormethan. Die vereinigten organischen Phasen werden über Magnesiumsulfat getrocknet und das Lösungsmittel im Vakuum entfernt. Man erhält das gewünschte sekundäre Amin **3,** das entweder direkt für weitere Reaktionen eingesetzt oder per Flashchromatographie oder HPLC gereinigt wird.

AAV-1D: Synthese sekundärer Amine durch Synthese und Reduktion von Carbonsäureamiden 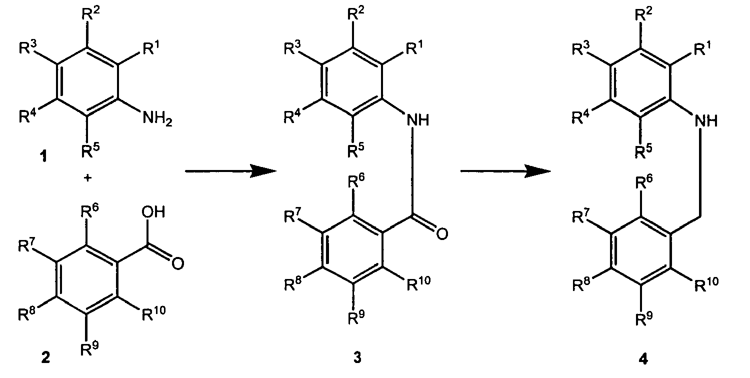 Die Carbonsäure **2** wird in getrocknetem Dichlormethan gelöst. Man gibt 1.5 eq. WSCxHCl sowie 2.0 eq. N-Ethylmorpholin zu und versetzt die erhaltene Lösung mit 1.3 eq. des Amins **1.** Nach 2- bis 16-stündigem Rühren bei Raumtemperatur werden ca. 20 bis 80 ml ges. NH₄Cl-Lösung zugegeben und die wässrige Phase dreimal mit Dichlormethan extrahiert. Die vereinigten organischen Phasen werden über Magnesiumsulfat getrocknet und das Lösungsmittel im Vakuum entfernt. Man erhält das gewünschte Amid **3,** das entweder direkt für die folgende Reduktion zum Amin **4** eingesetzt oder per Flashchromatographie oder HPLC gereinigt wird.

Zur Synthese des sekundären Amins **4** wird das Amid **3** unter Argonatmosphäre in THF gelöst und mit 3 bis 5 eq. Lithiumaluminiumhydrid in THF versetzt. Man erhitzt auf 55°C und rührt für 1 bis 4 Tage. Zur Aufarbeitung wird vorsichtig unter Eisbadkühlung zunächst Isopropanol, dann Methanol und zum Schluß Wasser zugesetzt. Man entfernt das Lösungsmittel am Rotationsverdampfer. Nach Zugabe von ca. 20 bis 80 ml 2N NaOH-Lösung und ca. 20 bis 80 ml Dichlormethan zentrifugiert man den weißen Niederschlag ab und extrahiert die wässrige Phase dreimal mit Dichlormethan. Die vereinigten organischen Phasen werden über Magnesiumsulfat getrocknet und das Lösungsmittel im Vakuum entfernt. Man erhält das gewünschte sekundäre Amin **4,** das entweder direkt für weitere Reaktionen eingesetzt oder per Flashchromatographie oder HPLC gereinigt wird.

### AAV-2: Überführung sekundärer Amine in Harnstoffe

AAV-2A: Überführung primärer Amine in das entsprechende Carbonylchlorid bzw. Isocyanat und anschließende Umsetzung mit sekundären Aminen zu Harnstoffen 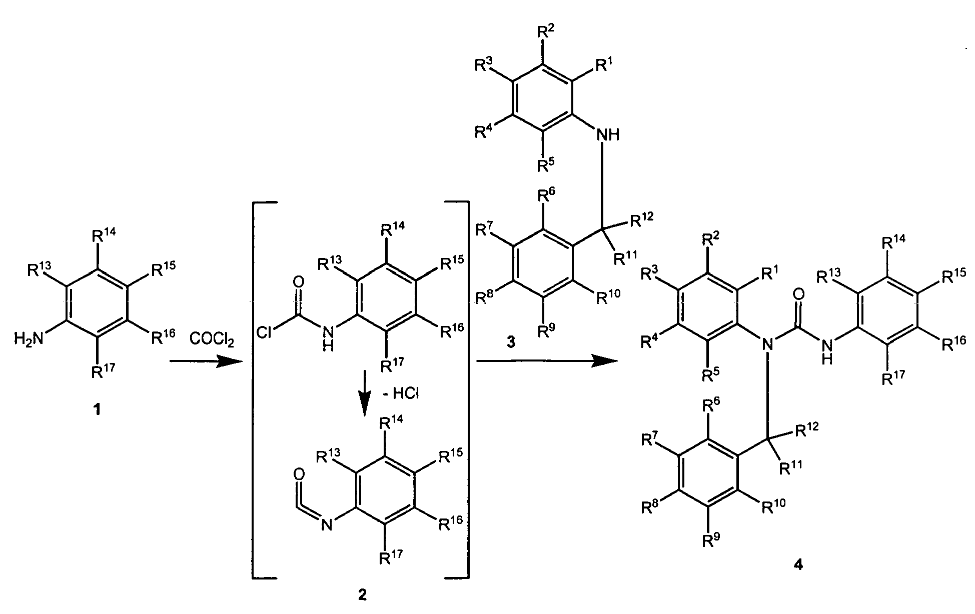 Das primäre Amin **1** wird in Acetonitril gelöst. Man gibt 2.0 eq. Natriumcarbonat sowie 1.0 bis 1.3 eq. einer 20%igen Lösung von Phosgen in Toluol zu und rührt bei Raumtemperatur. Nach 2 bis 7 Stunden werden 1.0 bis 1.5 eq. des sekundären Amins **3** gelöst in MeCN zugetropft. Man rührt für 16 Stunden und entfernt dann das Lösungsmittel am Rotationsverdampfer. Nach Zugabe von ca. 1 bis 20 ml ges. NH₄Cl-Lösung oder Wasser und ca. 1 bis 20 ml Dichlormethan oder EE extrahiert man die wässrige Phase 3 bis 6x mit Dichlormethan oder EE. Die vereinigten organischen Phasen werden über Magnesiumsulfat getrocknet und das Lösungsmittel im Vakuum entfernt. Man erhält den gewünschten Harnstoff **4,** der entweder direkt für weitere Reaktionen eingesetzt oder per Flashchromatographie oder HPLC gereinigt wird.

AAV-2B: Überführung sekundärer Amine in das entsprechende Carbonylchlorid und anschließende Umsetzung mit primären Aminen zu Harnstoffen 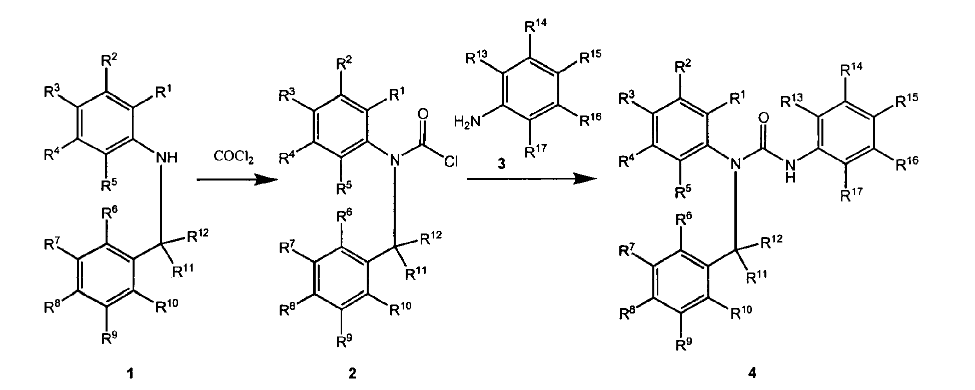 Die Synthese nach AAV-2B erfolgt analog zu AAV-1A, wobei primäres und sekundäres Amin ausgetauscht werden.

### AAV-3: Reduktion von Nitroverbindungen zu Aminen

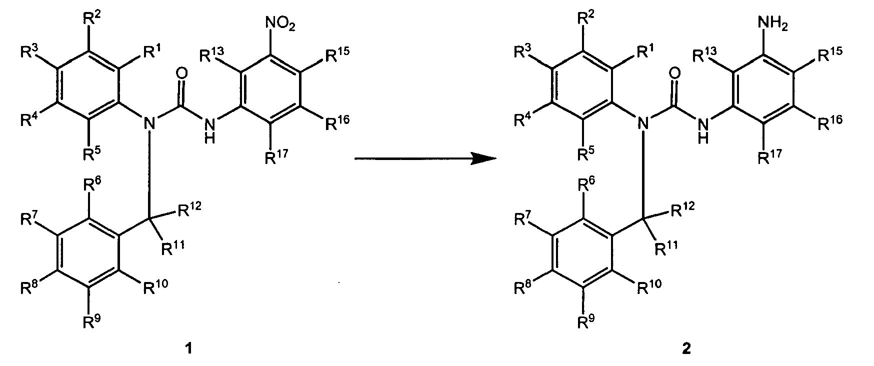

Die angegebene Struktur stellt lediglich ein Beispiel dar. Analog können auch andere aromatische oder nichtaromatische Nitroverbindungen anderer Struktur in die entsprechenden Amine überführt werden.

AAV-3A: Reduktion von Nitroverbindungen zu Aminen mit Wasserstoff und Pd/C Die Nitroverbindung **1** wird in MeOH gelöst. Man gibt einige Spatelspitzen 5% Pd/C zu und setzt die Lösung unter H₂-Atmosphäre. Nach 10 bis 60minütigem Rühren bei Raumtemperatur wird filtriert und das Lösungsmittel am Rotationsverdampfer entfernt. Man erhält das gewünschte Amin **2,** das entweder direkt für weitere Reaktionen eingesetzt oder per Flashchromatographie oder HPLC gereinigt wird.

AAV-3B: Reduktion von Nitroverbindungen zu Aminen mit Zinndichlorid Die Nitroverbindung **1** wird in EE gelöst und mit 2 bis 4 eq. Zinndichlorid-Dihydrat versetzt. Man erhitzt für 1 bis 6 Stunden auf 80°C, bis HPLC- oder DC-Kontrolle die Vollständigkeit der Reaktion anzeigt. Zur Aufarbeitung wird zunächst das Lösungsmittel am Rotationsverdampfer entfernt. Nach Zugabe von ca. 1 bis 50 ml 2N NaOH-Lösung und ca. 1 bis 50 ml Dichlormethan extrahiert man die wässrige Phase dreimal mit Dichlormethan. Die vereinigten organischen Phasen werden über Magnesiumsulfat getrocknet und das Lösungsmittel im Vakuum entfernt. Man erhält das gewünschte Amin 2, das entweder direkt für weitere Reaktionen eingesetzt oder per Flashchromatographie oder HPLC gereinigt wird.

AAV-3C: Reduktion von Nitroverbindungen zu Aminen mit Eisenpulver Die Nitroverbindung **1** wird in Essigsäure gelöst und mit einigen Spatelspitzen Eisenpulver versetzt. Man rührt für 1 bis 6 Stunden bei Raumtemperatur, bis HPLC- oder DC-Kontrolle die Vollständigkeit der Reaktion anzeigt. Alternativ kann die Reaktion auch in EtOH/NH₄C1 ges. 10:1 bei 60°C durchgeführt werden. Zur Aufarbeitung wird filtriert, das Lösungsmittel am Rotationsverdampfer entfernt, ca. 1 bis 50 ml 2N NaOH-Lösung oder H₂O zugegeben und die wässrige Phase 2 bis 3x mit Dichlormethan extrahiert. Die vereinigten organischen Phasen werden über Magnesiumsulfat getrocknet und das Lösungsmittel im Vakuum entfernt. Man erhält das gewünschte Amin **2,** das entweder direkt für weitere Reaktionen eingesetzt oder per Flashchromatographie oder HPLC gereinigt wird.

### Beispiel 3: Synthese von 3-Chlor-2,6-diethyl-5-nitro-phenylamin (2)

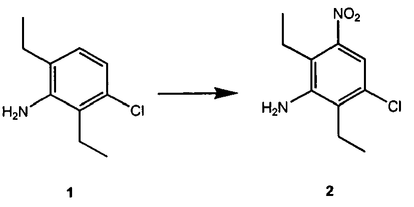

1.83 ml (10.9 mmol) 3-Chlor-2,6-diethyl-phenylamin (1) wird im Eisbad bei 0°C in 8 ml konz. H₂SO₄ gelöst. Man gibt 562 µl (12.0 mmol; 1.1 eq.) 90%ige HNO₃ zu und rührt für 2.5 Stunden bei 0°C. Zur Aufarbeitung wird die Reaktionslösung auf ca. 200 ml Eis gegossen und die erhaltene Lösung 8x mit 50 bis 100 ml Ethylacetat extrahiert. Die vereinigten organischen Phasen werden über Magnesiumsulfat getrocknet und das Lösungsmittel im Vakuum entfernt. Man erhält 2.1 g Rohprodukt. Flashchromatographische Trennung liefert 1.24 g (5.42 mmol; 50%) 2.

### Beispiel 4: Synthese von 106

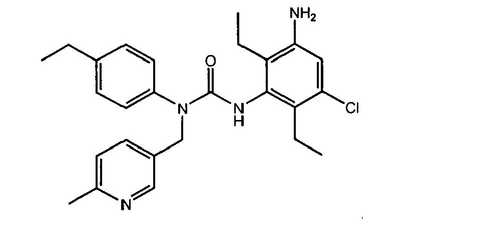

Stufe 1: 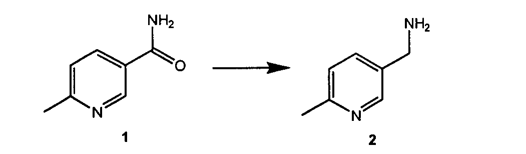 3.00 g (22.0 mmol) 6-Methylnicotinamid wird unter Argonatmosphäre in 10 ml THF gelöst und mit 34 ml (34 mmol; 1.5 eq.) 1 M Lithiumaluminiumhydrid-Lösung in THF versetzt. Nach 45 min gibt man weiteres Lithiumaluminiumhydrid (1.22 g; 1.5 eq.) in 40 ml THF zu. Man erhitzt auf 60°C und rührt einen Tag. Zur Aufarbeitung wird vorsichtig unter Eisbadkühlung zunächst Isopropanol, dann Methanol und zum Schluß Wasser zugesetzt. Man entfernt das Lösungsmittel am Rotationsverdampfer. Nach Zugabe von 100 ml 2N NaOH-Lösung und 100 ml Dichlormethan zentrifugiert man den weißen Niederschlag ab und extrahiert die wässrige Phase 4x mit 60 ml Dichlormethan. Die vereinigten organischen Phasen werden über Magnesiumsulfat getrocknet und das Lösungsmittel im Vakuum entfernt. Man erhält 1.57 g (12.9 mmol; 58%) des gewünschten Amins **2,** das direkt für weitere Reaktionen eingesetzt wird.

Stufe 2: 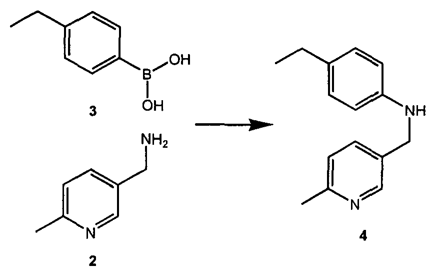 900 mg (7.37 mmol) der ungereinigten Verbindung **2** wird gemäß AAV-1B ohne Lutidin mit 4-Ethylphenylboronsäure **(3)** umgesetzt. Man erhält 788 mg Rohprodukt. Nach flashchromatographischer Reinigung erhält man 60.4 mg (0.27 mmol; 4%) des gewünschten Amins **4.**

Stufe 3: 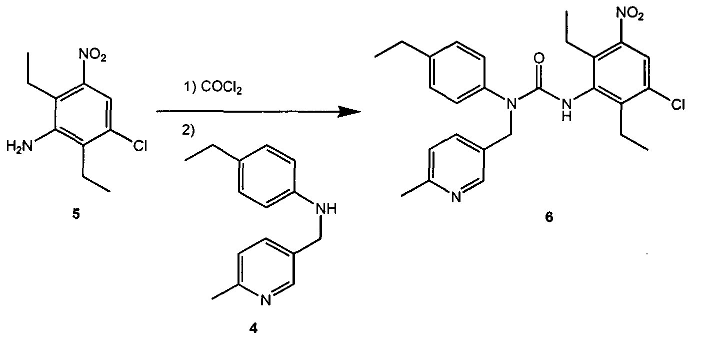 Man setzt 61.3 mg (0.267 mmol) 3-Chlor-2,6-diethyl-5-nitro-phenylamin **(5)** nach AAV-2A mit 60.4 mg (0.267 mmol; 1.0 eq.) (4-Ethyl-phenyl)-(6-methyl-pyridin-3-ylmethyl)-amin **(4)** um. Das erhaltene Rohprodukt wird ohne Reinigung für den nächsten Reaktionsschritt eingesetzt.

Stufe 4: 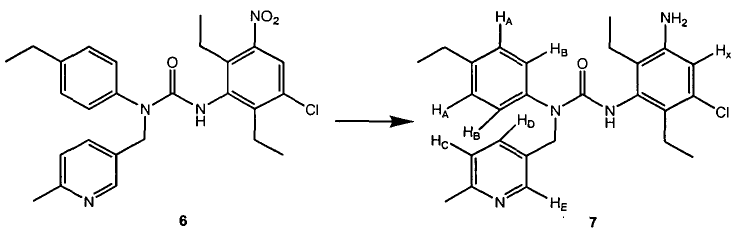 Die in Stufe 3 erhaltene Nitroverbindung **6** wird gemäß AAV-3A reduziert und das erhaltene Rohprodukt per HPLC gereinigt. Man erhält 32.64 mg (0.072 mmol; 27%, 2 Stufen) der gewünschten Verbindung **7.**
¹H NMR (CD₃OD): δ (ppm) = 1.06 (m, 6H, 2x CH₃CH₂), 1.24 (t, 3H, CH₃CH₂), 2.50-2.73 (3q, 3x CH₂CH₃), 2.76 (s, 3H, CH₃-Ar), 5.01 (s, 2H, N-CH₂), 6.99 (s, 1H, Hₓ), 7.26, 7.37 (2d, 4H, 2x H_{A}, 2x H_{B}), 7.87 (d, 1H, H_{C}), 8.37 (dd, 1H, H_{D}), 8.54 (d, 1H, H_{E}).

### Beispiel 5: Synthese von 53

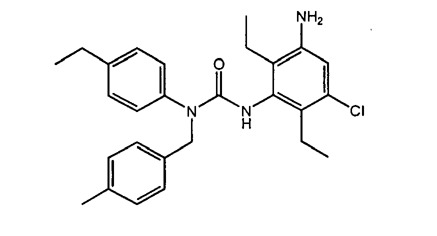

Stufe 1: 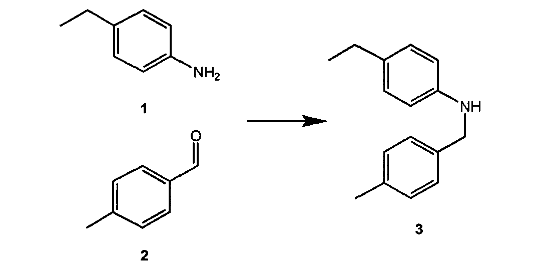 1.50 g (12.34 mmol) 4-Ethylanilin **(1)** wird gemäß AAV-1A mit 4-Methyl-benzaldehyd **(2)** umgesetzt. Man erhält 2.32 g (10.3 mmol; 83%) der Verbindung 3 als Rohprodukt, das ohne Reinigung für den nächsten Reaktionsschritt eingesetzt wird.

Stufe 2: 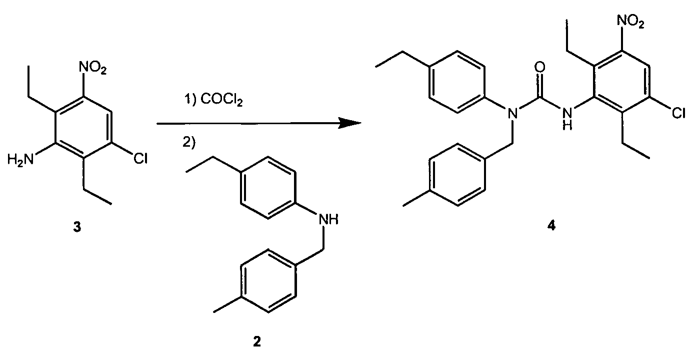 Man setzt 70 mg (0.306 mmol) 3-Chlor-2,6-diethyl-5-nitro-phenylamin **(3)** nach AAV-2A mit 105 mg (0.459 mmol; 1.5 eq.) (4-Ethyl-phenyl)-(4-methyl-benzyl)-amin **(2)** um. Das erhaltene Rohprodukt wird ohne Reinigung für den nächsten Reaktionsschritt eingesetzt.

Stufe 3: 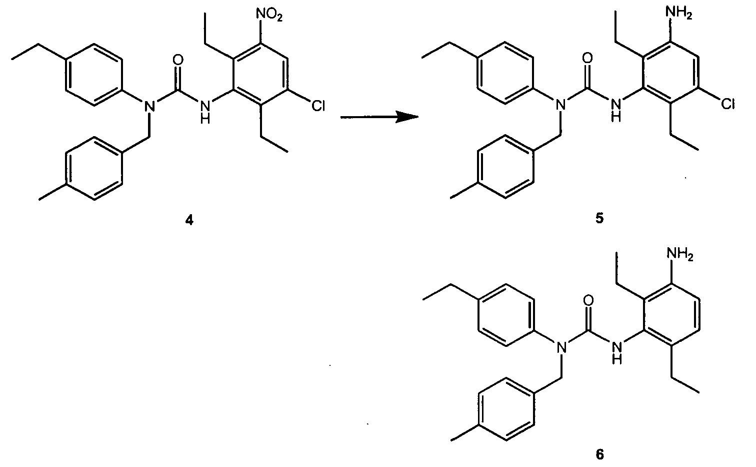 Die in Stufe 2 erhaltene Nitroverbindung **4** wird gemäß AAV-3A reduziert und das erhaltene Rohprodukt per HPLC gereinigt. Man erhält 5.1 mg der Verbindung **5** sowie 7.0 mg der durch reduktive Dechlorierung entstandenen Verbindung **6.**

### Beispiel 6: Synthese von 77

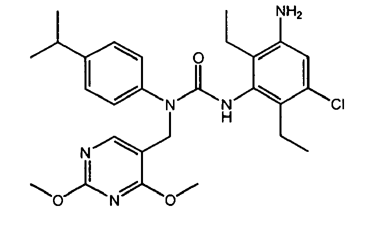

Stufe 1: 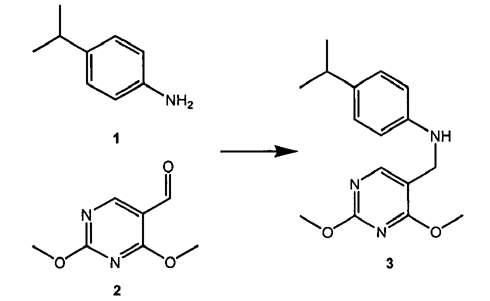 160.9 mg (1.19 mmol) 4-Isopropylanilin **(1)** wird gemäß AAV-1A mit 2,4-Dimethoxy-pyrimidin-5-carbaldehyd **(2)** umgesetzt. Man erhält 318 mg (1.11 mmol; 93%) der Verbindung **3** als Rohprodukt, das ohne Reinigung für den nächsten Reaktionsschritt eingesetzt wird.

Stufe 2: 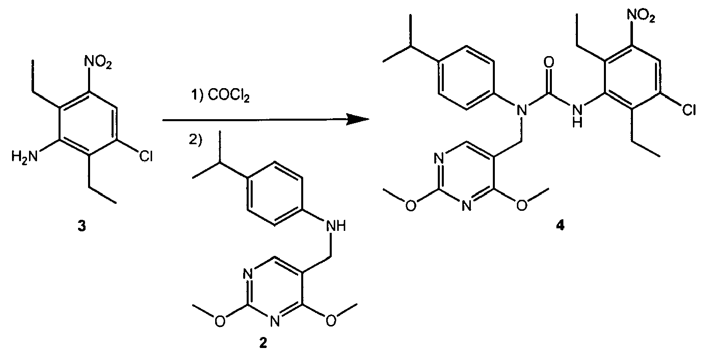 Man setzt 90 mg (0.395 mmol) 3-Chlor-2,6-diethyl-5-nitro-phenylamin **(3)** nach AAV-2A mit 168.5 mg (0.586 mmol; 1.5 eq.) (2,4-Dimethoxy-pyrimidin-5-ylmethyl)-(4-ethyl-phenyl)-amin **(2)** um. Das erhaltene Rohprodukt wird ohne Reinigung für den nächsten Reaktionsschritt eingesetzt.

Stufe 3: 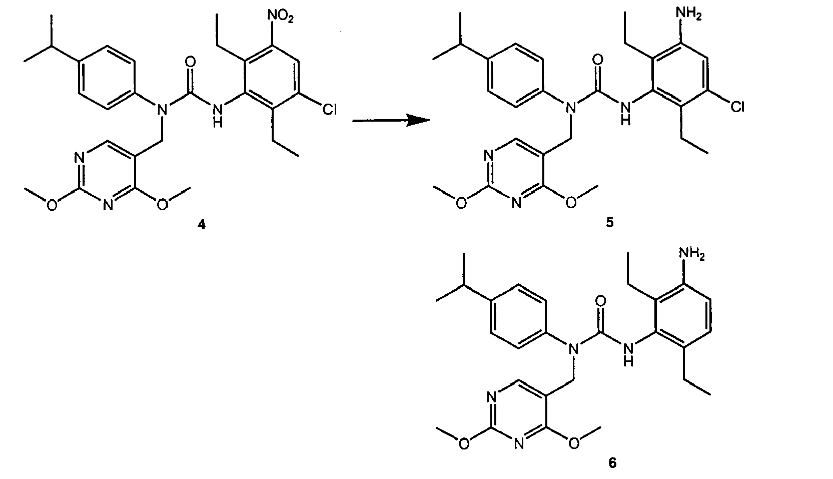 Die in Stufe 2 erhaltene Nitroverbindung **4** wird gemäß AAV-3A reduziert und das erhaltene Rohprodukt per HPLC gereinigt. Man erhält 72.5 mg der Verbindung **5** sowie 2.8 mg der durch reduktive Dechlorierung entstandenen Verbindung **6.**

### Beispiel 7: Synthese von 111

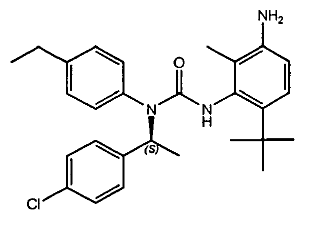

Stufe 1: 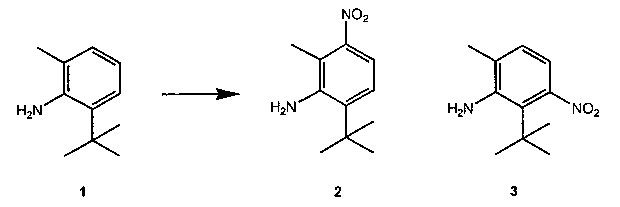 700 mg (4.29 mmol) 6-*tert*-Butyl-*o*-toluidin **(1)** wird im Eisbad bei 0°C in 8 ml konz. H₂SO₄ gelöst. Man gibt 221.6 µl (4.72 mmol; 1.1 eq.) konz. HNO₃ zu und rührt für 3 Stunden bei 0°C. Zur Aufarbeitung wird die Reaktionslösung auf ca. 75 ml Eis gegossen und die erhaltene Lösung 5x mit Ethylacetat extrahiert. Die vereinigten organischen Phasen werden über Magnesiumsulfat getrocknet und das Lösungsmittel im Vakuum entfernt. Man erhält 844 mg (4.06 mmol; 95%) eines Gemisches der Verbindungen **2** und **3** als Rohprodukt. Trennung von 200 mg des Rohproduktes per HPLC liefert 72 mg (0.35 mmol; 34%) **2** sowie 36 mg (0.17 mmol; 17%) **3.**

Stufe 2: 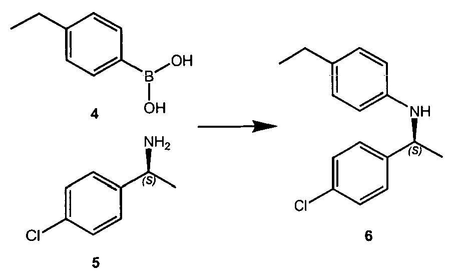 2.25 ml (16.1 mmol) (*S*)-4-Chlor-alpha-Methylbenzylamin **(5)** wird gemäß AAV-1B mit 1.88 ml (16.1 mmol; 1.0 eq.) Lutidin und 3.62 g (24.2 mmol; 1.5 eq.) 4-Ethylphenylboronsäure **(4)** umgesetzt. Nach flashchromatographischer Reinigung (Hexan / Ethylacetat 12:1) erhält man 1.96 g (7.54 mmol; 47%) des gewünschten Amins **6**.

Stufe 3: 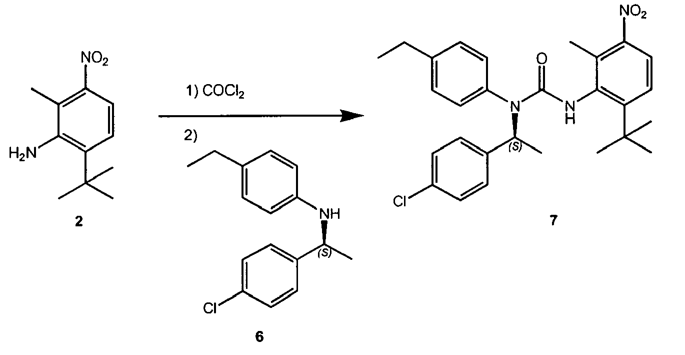 Man setzt 72 mg (0.35 mmol) 6-*tert*-Butyl-2-methyl-3-nitro-phenylamin **(2)** nach AAV-2A mit 81 mg (0.31 mmol; 0.9 eq.) (*S*)-[1-(4-Chlorphenyl)-ethyl]-(4-ethyl-phenyl)-amin **(6)** um. Das erhaltene Rohprodukt (129 mg) wird ohne Reinigung für den nächsten Reaktionsschritt eingesetzt.

Stufe 4: 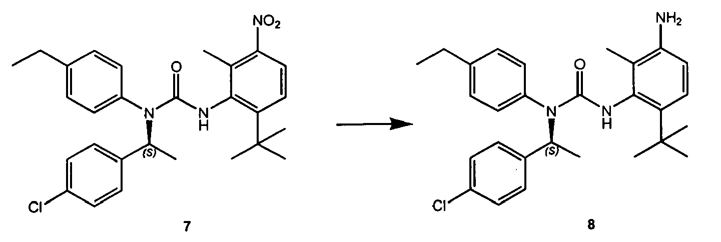 Die in Stufe 3 erhaltene Nitroverbindung **7** wird gemäß AAV-3B reduziert und das erhaltene Rohprodukt per HPLC gereinigt. Man erhält 13.4 mg der gewünschten Verbindung **8.**

### Beispiel 8: Synthese von 112

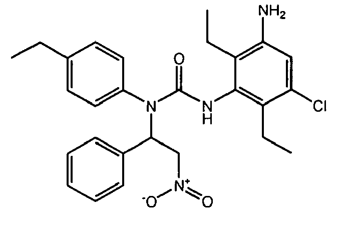

Stufe 1: 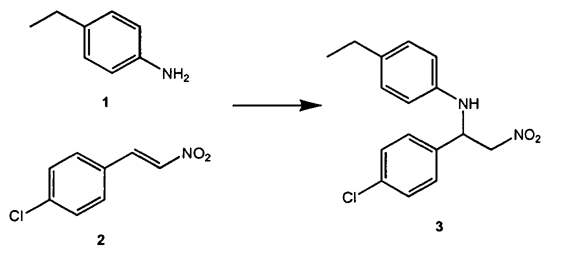 250 mg (1.36 mmol) 1-(4-Chlorphenyl)-2-nitroethen **(2)** wird mit 136 µl (1.09 mmol; 0.8 eq.) 4-Ethylanilin **(1)** in 2 ml Acetonitril gelöst. Man rührt 2 Tage bei 45°C und engt im Vakuum ein. Das erhaltene Rohprodukt wird ohne Reinigung für den nächsten Reaktionsschritt eingesetzt.

Stufe 2: 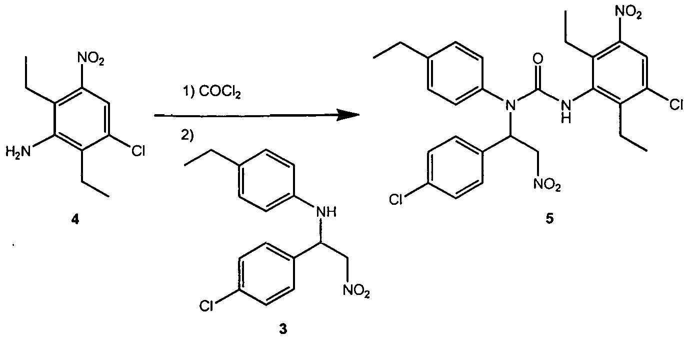 Man setzt 75 mg (0.328 mmol) 3-Chlor-2,6-diethyl-5-nitro-phenylamin **(4)** nach AAV-2A mit 130 mg (0.43 mmol; 1.3 eq.) [1-(4-Chlorphenyl)-2-nitroethyl]-(4-ethylphenyl)-amin **(3),** das als Rohprodukt aus Stufe 1 erhalten wurde, um. Reinigung per HPLC liefert 22 mg (0.039 mmol; 12%) **5.**

Stufe 3: 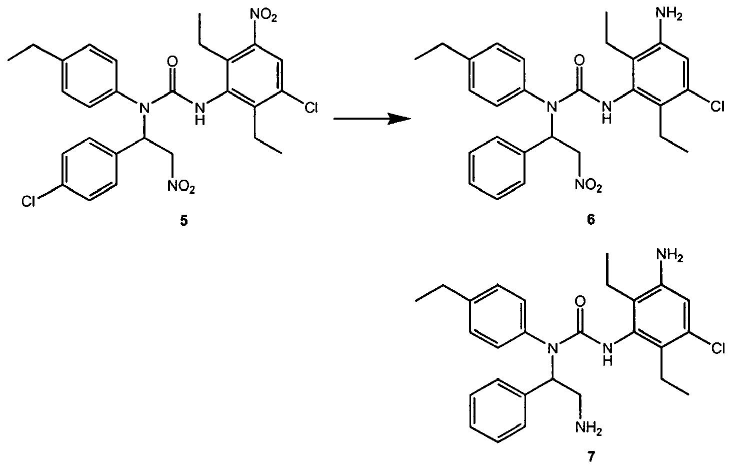 12.7 mg (0.0227 mmol) der Dinitroverbindung **5** wird gemäß AAV-3A reduziert und das erhaltene Rohprodukt per HPLC gereinigt. Man erhält 1.41 mg (0.0030 mmol; 13%) des Diamins 7 sowie 1.56 mg (0.0032 mmol; 14%) der Verbindung **6.**

### Beispiel 9: Synthese von 118

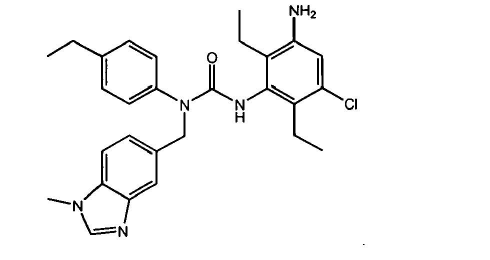

Stufe 1: 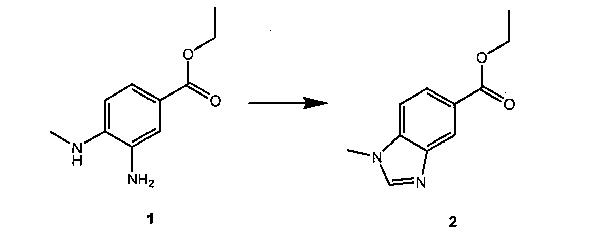 150 mg (0.77 mmol) Ethyl-3-amino-4-(methylamino)benzoat **(1)** wird in 10 ml Ameisensäure gelöst. Man erhitzt auf 60°C und rührt einen Tag. Nach Entfernung des Lösungsmittels am Rotationsverdampfer erhält man 235 mg des gewünschten Amins als Rohprodukt, das direkt für weitere Reaktionen eingesetzt wird.

Stufe 2: 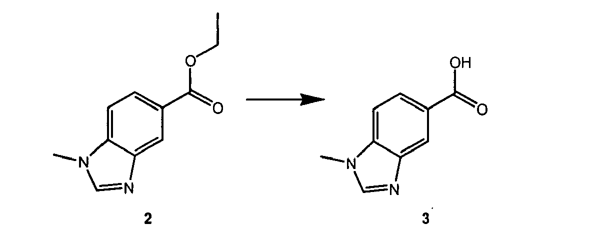 235 mg des Ethylesters **2**, der als Rohprodukt aus Stufe 1 erhalten wurde, löst man in einem Gemisch aus 20 ml 1N LiOH und 8 ml Dioxan. Nach 4stündigem Rühren bei Raumtemperatur wird 11 ml 2N HCl-Lösung bis pH 7 zugesetzt und das Dioxan am Rotationsverdampfer entfernt. Die wässrige Phase wird lyophilisiert. Man erhält 530 mg eines Rohproduktes, das direkt für die nächste Reaktion eingesetzt wird.

Stufe 3: 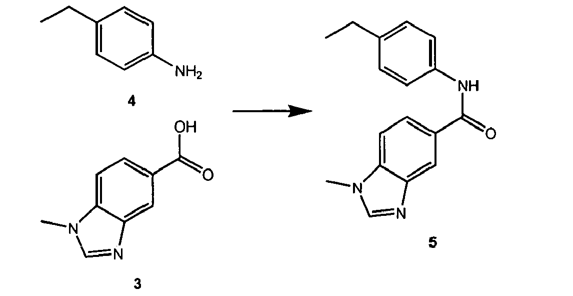 Das Rohprodukt aus Stufe 2 (theoret. Menge: 0.77 mmol) wird in 14 ml DMF aufgeschlämmt und mit 1038 mg (5.4 mmol; 7.0 eq.) WSCxHCl, 802 mg (3.0 mmol; 3.9 eq.) HOBt und 300 µl (2.4 mmol; 3.1 eq.) 4-Ethylanilin versetzt. Nach 1tägigem Rühren bei Raumtemperatur wird das DMF am Rotationsverdampfer entfernt. Man gibt ca. 50 ml 2N NaOH-Lösung zu und extrahiert die wässrige Phase fünfmal mit Dichlormethan. Die vereinigten organischen Phasen werden über Magnesiumsulfat getrocknet und das Lösungsmittel im Vakuum entfernt. Man erhält 1.04 g eines braunen Öls, das in ca. 50 ml DCM gelöst und fünfmal gegen 30 ml 1M HCl-Lösung extrahiert wird. Die wässrige Phase stellt man mit ca. 30 ml 1N NaOH-Lösung auf pH 14 ein und extrahiert erneut fünfmal mit ca. 50 ml DCM. Die organischen Phasen werden über Magnesiumsulfat getrocknet und das Lösungsmittel im Vakuum entfernt. Man erhält 5 als Rohprodukt, das direkt für weitere Reaktionen eingesetzt wird.

Stufe 4: 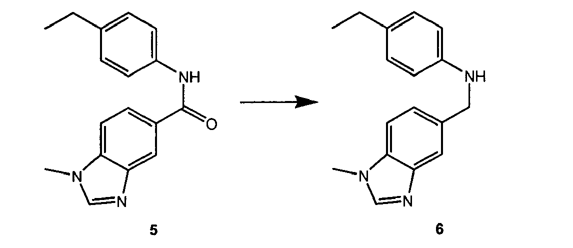 Das Rohprodukt aus Stufe 3 (theoret. Menge: 0.77 mmol) wird gemäß AAV-1D bei Raumtemperatur für einen Tag umgesetzt. Nach flashchromatographischer Reinigung erhält man 57.4 mg (0.22 mmol; 29% über 4 Stufen) des gewünschten Amins **6.**

Stufe 5: 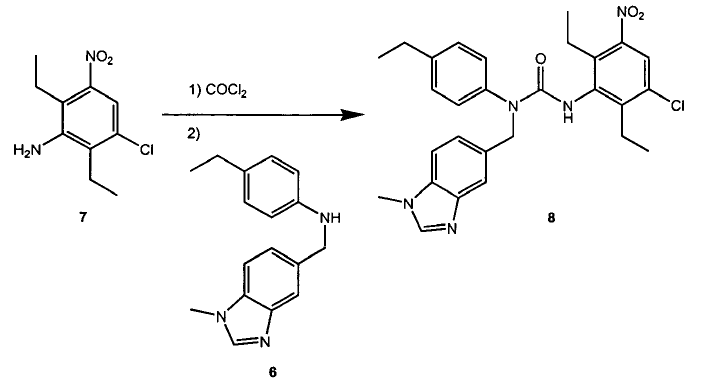 Man setzt 34.4 mg (0.151 mmol) 3-Chlor-2,6-diethyl-5-nitro-phenylamin **(7)** nach AAV-2A mit 40 mg (0.151 mmol; 1.0 eq.) (4-Ethyl-phenyl)-(1-methyl-1H-benzoimidazol-5-ylmethyl)-amin **(6)** um. Das erhaltene Rohprodukt der Verbindung **8** wird ohne Reinigung für den nächsten Reaktionsschritt eingesetzt.

Stufe 6: 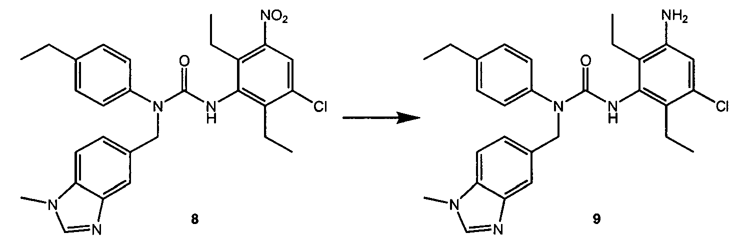 Die in Stufe 5 erhaltene Nitroverbindung **8** wird gemäß AAV-3B reduziert und das erhaltene Rohprodukt per HPLC gereinigt. Man erhält 2.37 mg der gewünschten Verbindung **9.**

### Beispiel 10: Synthese von 131

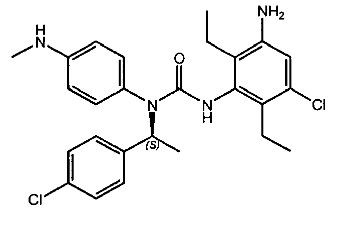

Stufe 1: 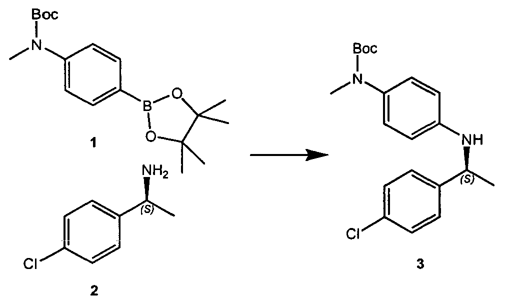 500 mg (1.50 mmol) Methyl-[4-(4,4,5,5-tetramethyl-[1,3,2]dioxaborolan-2-yl)-phenyl]-carbaminsäure-tert-butyl-ester **(1)** wird gemäß AAV-1B mit 252 µl (1.80 mmol; 1.2 eq.) (S)-1-(4-Chlorphenyl)-ethylamin **(2)** umgesetzt, wobei man als Base 418 µl (3.0 mmol; 2.0 eq.) Triethylamin einsetzt. Nach flashchromatographischer Reinigung (Hexan / Ethylacetat) erhält man 133 mg (0.37 mmol; 25%) des gewünschten Amins **3.**

Stufe 2: 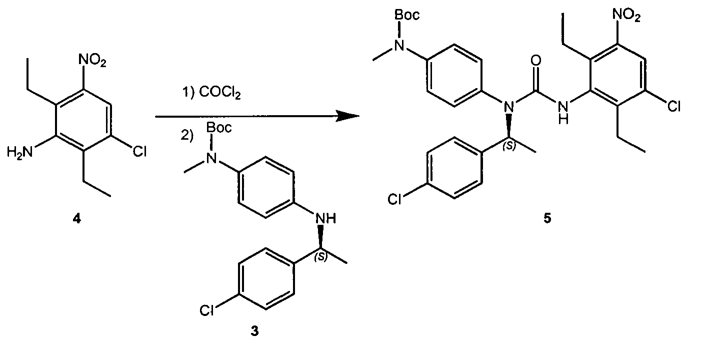 Man setzt 70 mg (0.307 mmol) 3-Chlor-2,6-diethyl-5-nitro-phenylamin **(4)** nach AAV-2A mit 132 mg (0.366 mmol; 1.2 eq.) {4-[1-(4-Chlorphenyl)-ethylamino]-phenyl}-methyl-carbaminsäure-tert-butyl-ester **(3)** um. Das erhaltene Rohprodukt wird per HPLC gereinigt. Man erhält 105 mg (0.171 mmol; 56%) der gewünschten Verbindung **5.**

Stufe 3: 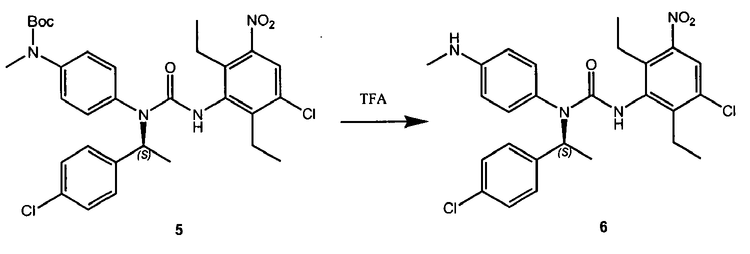 80 mg (0.130 mmol) der in Stufe 2 erhaltenen Boc-geschützten Verbindung **5** wird in ca. 5 ml DCM/TFA/H₂O 50:50:0.05 gelöst und für 10 min bei Raumtemperatur gerührt. Man entfernt das Lösungsmittel im Vakuum und enthält 73 mg (0.142 mmol; 109%) des gewünschten Amins **6,** das ohne Reinigung für den nächsten Reaktionsschritt eingesetzt wird.

Stufe 4: 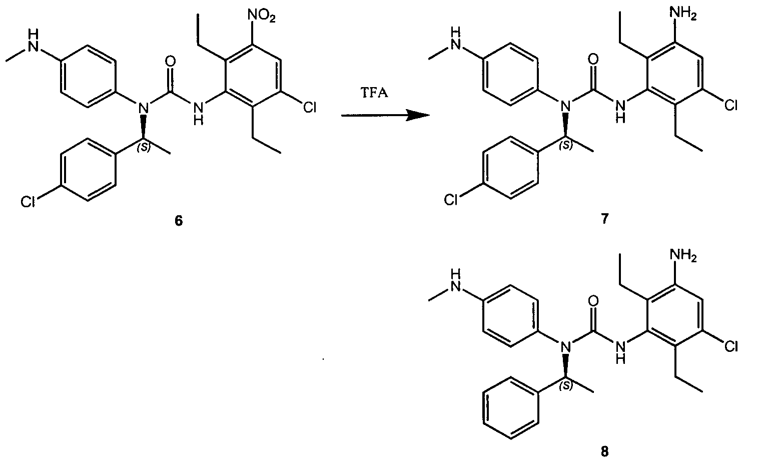 50 mg (0.097 mmol) der in Stufe 3 erhaltenen Nitroverbindung **6** wird gemäß AAV-3A für 3 min reduziert und das erhaltene Rohprodukt per HPLC gereinigt. Man erhält 12.5 mg (0.026 mmol; 27%) der gewünschten Verbindung **7** sowie 2.7 mg (0.006 mmol; 6%) der durch reduktive Dechlorierung entstandenen Verbindung **8.**

### Beispiel 11: Synthese von 141

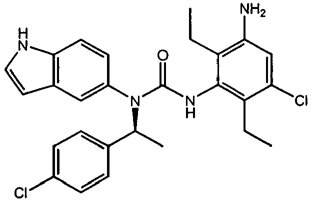

Stufe 1: 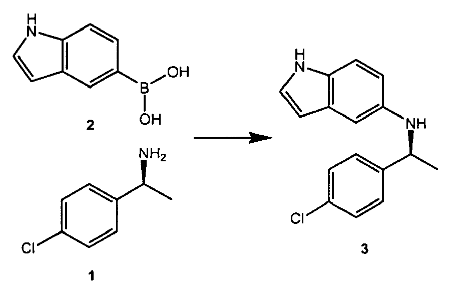 140.2 µl (1.0 mmol) der Verbindung **1** wird gemäß AAV-1B mit 151 µl (1.3 eq.) Lutidin, 236 mg (1.3 eq.) Kupferacetat und 210 mg (1.3 eq.) 5-Indolylboronsäure **(2)** umgesetzt. Man erhält 240 mg Rohprodukt. Nach flashchromatographischer Reinigung erhält man 26 mg (0.10 mmol; 10%) des gewünschten Amins **3.**

Stufe 2: 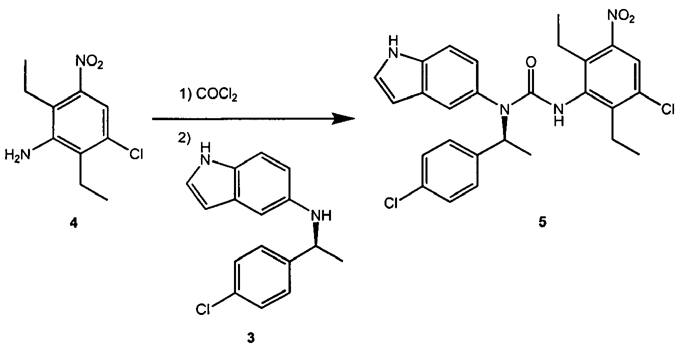 Man setzt 26 mg (0.116 mmol, 1.2 eq.) 3-Chlor-2,6-diethyl-5-nitro-phenylamin **(4)** nach AAV-2A mit 26 mg (0.096 mmol; 1.0 eq.) [1-(4-Chlor-phenyl)-ethyl]-(1H-indol-5-yl)-amin **(3)** bei 40°C um. Das erhaltene Rohprodukt wird per HPLC gereinigt. Man erhält 6 mg (0.011 mmol; 10%) **5.**

Stufe 3: 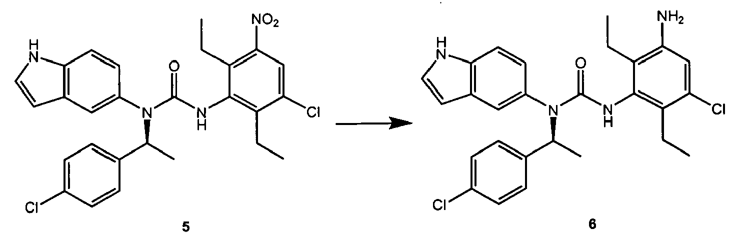 Die in Stufe 2 erhaltene Nitroverbindung **5** wird gemäß AAV-3C reduziert und das erhaltene Rohprodukt per HPLC gereinigt. Man erhält 2.70 mg (0.0054 mmol; 50%) der gewünschten Verbindung **6.**

### Beispiel 12: Synthese von 27

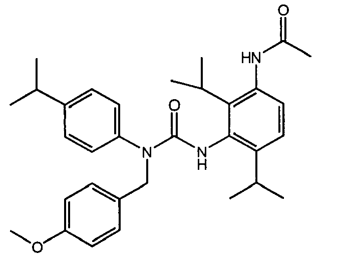

Stufe 1: 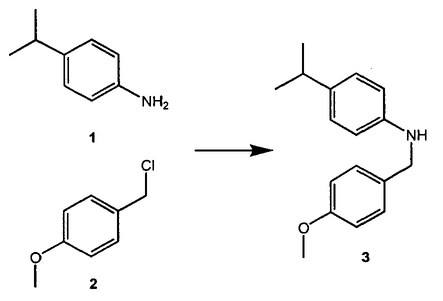 1.46 ml (18 mmol) 4-Isopropylanilin **(1)** wird gemäß AAV-1C mit 4-Methoxy-benzylchlorid (2) umgesetzt. Man erhält 1.1 g (4.3 mmol; 72%) der Verbindung **3** nach HPLC-Reinigung.

Stufe 2: 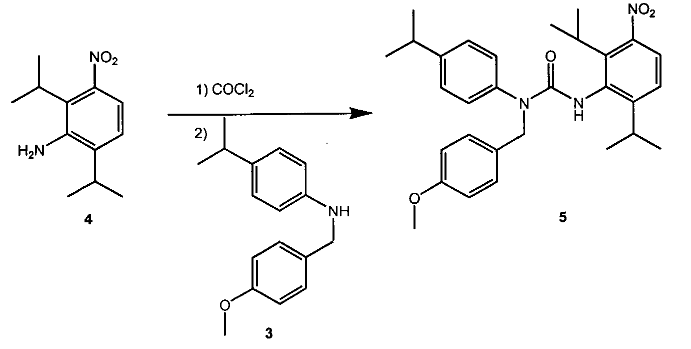 Man setzt 90 mg des Nitroanilins **4** mit 134 mg (0.525 mmol; 1.5 eq.) Amin **3** nach AAV-2A um. Das erhaltene Rohprodukt wird ohne Reinigung für den nächsten Reaktionsschritt eingesetzt.

Stufe 3: 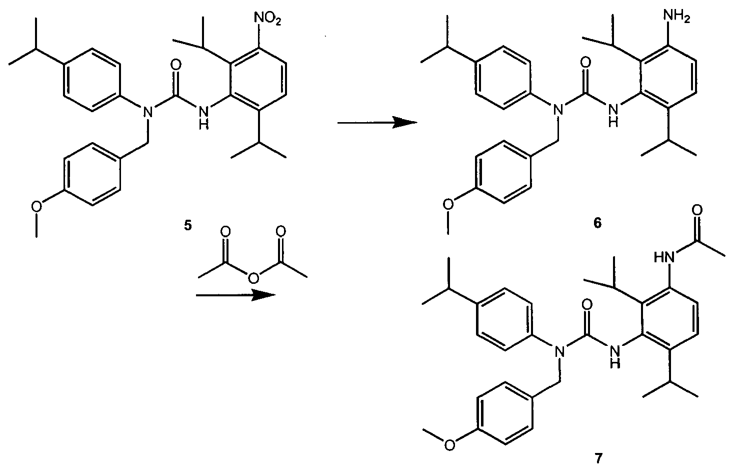 Die in Stufe 2 erhaltene Nitroverbindung **5** wird gemäß AAV-3B reduziert und das erhaltene Rohprodukt per HPLC gereinigt. Man erhält 18 mg der Verbindung **6.** 10.0 mg der Verbindung **6** werden in Acetonitril (20 ml) gelöst und mit 10 eq. Essigsäureanhydrid und 2 eq. TEA 30 min bei RT gerührt. Nach Aufreinigung mittels HPLC werden 9 mg der Verbindung 7 erhalten.

### Beispiel 13: Synthese von 84

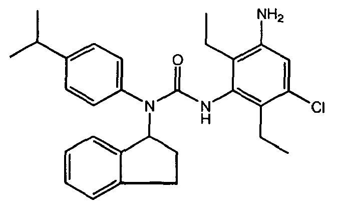

Stufe 1: 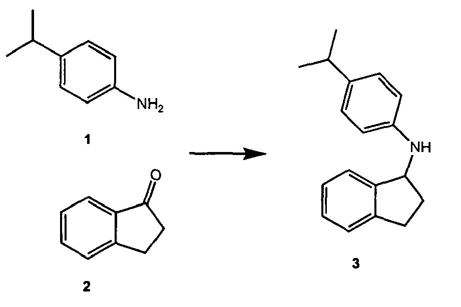 1.46 ml (18 mmol) 4-Isopropylanilin **(1)** wird gemäß AAV-1A mit 1-Indanon **(2)** umgesetzt. Man erhält 1.1 g (4.3 mmol; 72%) der Verbindung **3** nach HPLC-Reinigung.

Stufe 2: 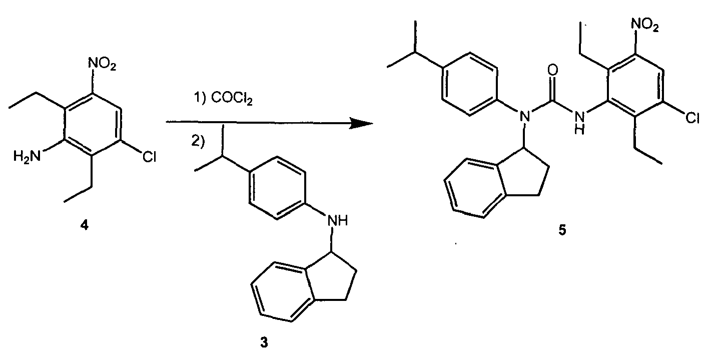 Man setzt 180 mg des Nitroanilins **4** mit 180 mg (0.76 mmol; 1 eq.) Amin **3** nach AAV-2A um. Das erhaltene Rohprodukt wird ohne Reinigung für den nächsten Reaktionsschritt eingesetzt.

Stufe 3: 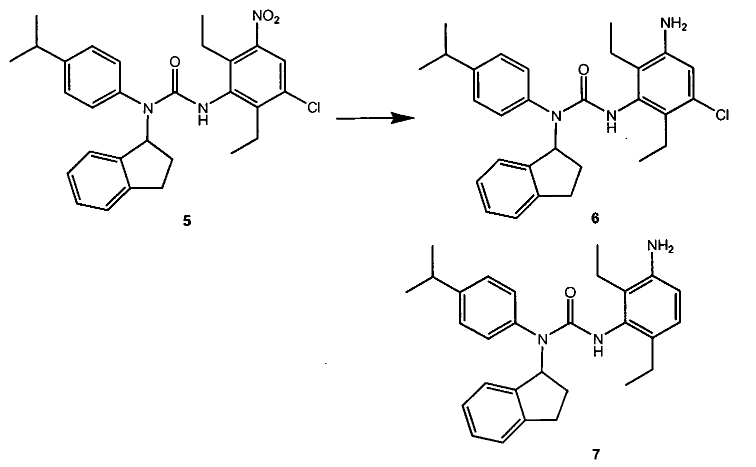 Die in Stufe 2 erhaltene Nitroverbindung **5** wird gemäß AAV-3A reduziert und das erhaltene Rohprodukt per HPLC gereinigt. Man erhält 19 mg der Verbindung 6 sowie 5.0 mg der durch reduktive Dechlorierung entstandenen Verbindung **7.**

### Beispiel 14: Synthese von 110

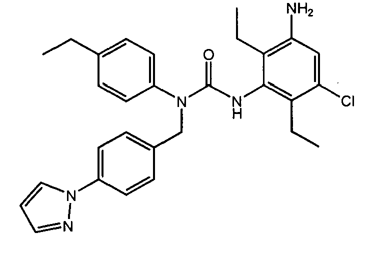

Stufe 1: 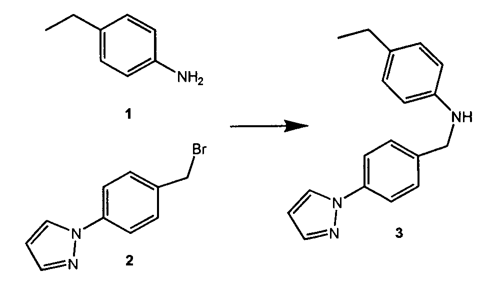 150 µl (1.2 mmol) 4-Isopropylanilin **(1)** wird gemäß AAV-1C mit 1-(4-Brommethyl-phenyl)-1H-pyrazol **(2)** umgesetzt. Man erhält 180 mg (0.78 mmol; 64%) der Verbindung **3,** die ohne weiter Reinigung weiter umgesetzt wird.

Stufe 2: 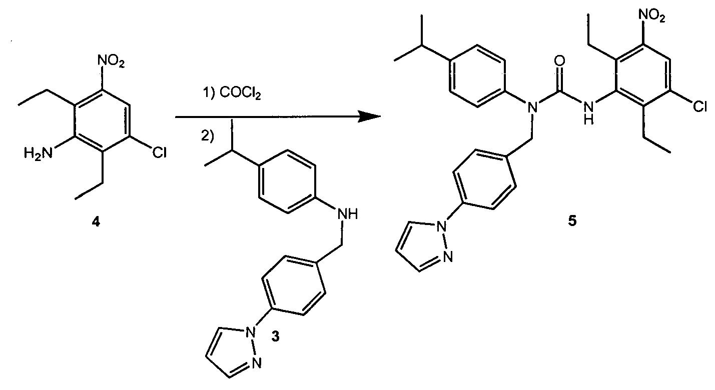 Man setzt 60 mg des Nitroanilins **4** mit 75 mg (0.26 mmol; 1 eq.) Amin **3** nach AAV-2A um. Das erhaltene Rohprodukt wird ohne Reinigung für den nächsten Reaktionsschritt eingesetzt.

Stufe 3: 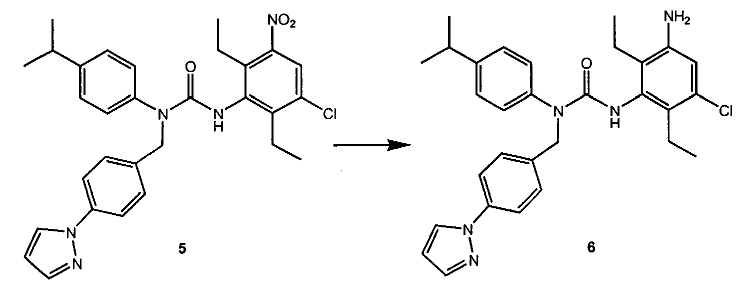 Die in Stufe 2 erhaltene Nitroverbindung **5** wird gemäß AAV-3B reduziert und das erhaltene Rohprodukt per HPLC gereinigt. Man erhält 12 mg der Verbindung **6.**

### Beispiel 15: Synthese von 116

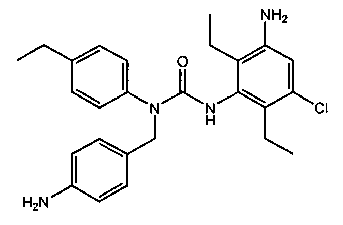

Stufe 1: 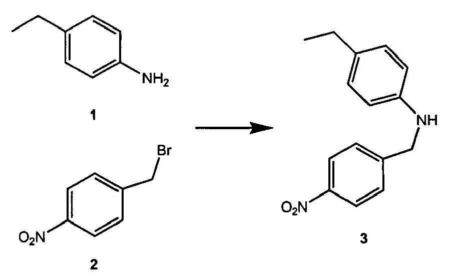 150 µl (1.2 mmol) 4-Isopropylanilin **(1)** wird gemäß AAV-1C mit 4-Nitrobenzylbromid **(2)** umgesetzt. Man erhält 260 mg (1.02 mmol; 85%) der Verbindung **3,** die ohne weitere Reinigung weiter umgesetzt wird.

Stufe 2: 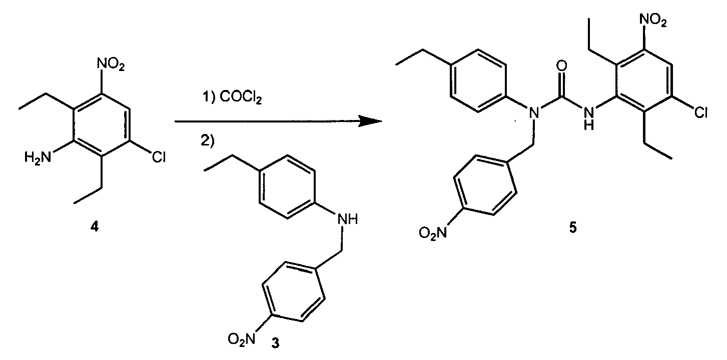 Man setzt 60 mg des Nitroanilins **4** mit 70 mg (0.26 mmol; 1 eq.) Amin **3** nach AAV-2A um. Das erhaltene Rohprodukt wird ohne Reinigung für den nächsten Reaktionsschritt eingesetzt.

Stufe 3: 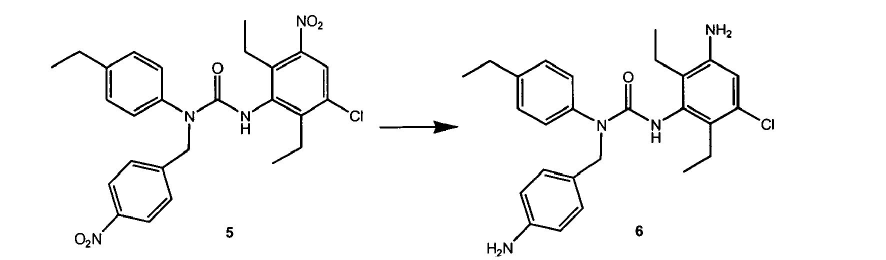 Die in Stufe 2 erhaltene Nitroverbindung **5** wird gemäß AAV-3A reduziert und das erhaltene Rohprodukt per HPLC gereinigt. Man erhält 6 mg der Verbindung **6.**

### Beispiel 16: Synthese von 120

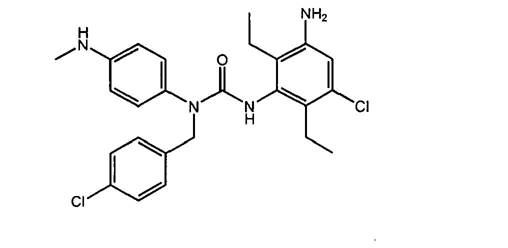

Stufe 1: 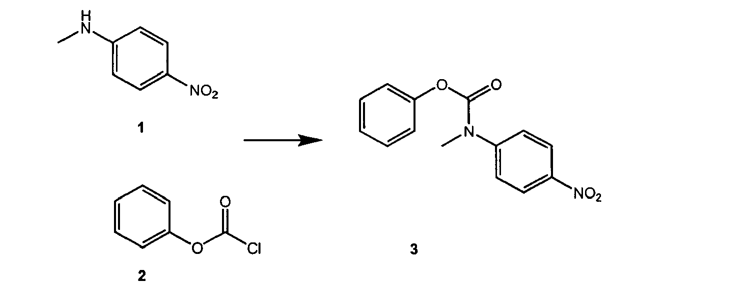 107 mg N-Methyl-4-nitroanilin **(1)** werden in 10 ml THF gelöst und mit 186 mg (1.1 eq.) Phenylchloroformiat **(2)** in der Gegenwart von 187 mg (1 eq.) Kaliumcarbonat versetzt und 16 h bei RT gerührt. Das Lösungsmittel wird im Vakuum entfernt und der Rückstand in Essigester aufgenommen und zweimal mit Wasser extrahiert. Man erhält 250 mg an Rohprodukt, welches ohne weitere Aufreinigung umgesetzt wird.

Stufe 2: 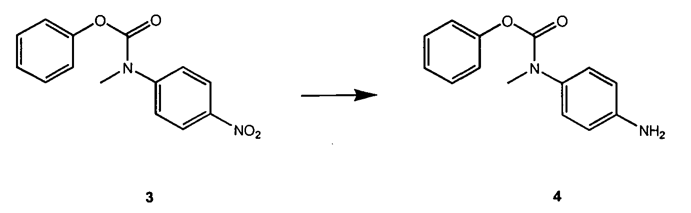 Die in Stufe 1 erhaltene Nitroverbindung **3** wird gemäß AAV-3B reduziert und das erhaltene Rohprodukt **4** (180 mg) ohne weitere Aufreinigung umgesetzt.

Stufe 3: 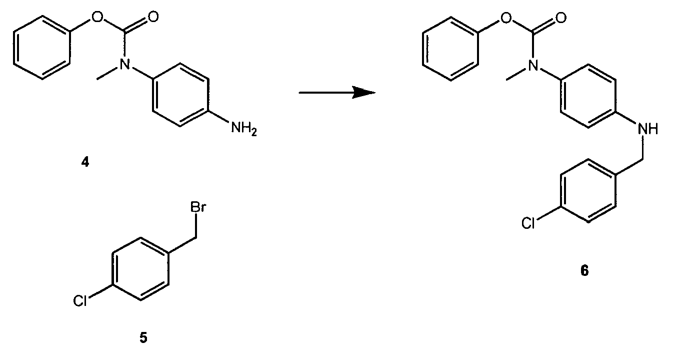 180 mg (0.744 mmol) Amin **4** wird gemäß AAV-1C mit 4-Chlorbenzylbromid **(5)** umgesetzt. Man erhält 300 mg der Verbindung **6** als Rohprodukt, das ohne weiter Reinigung weiter umgesetzt wird.

Stufe 4: 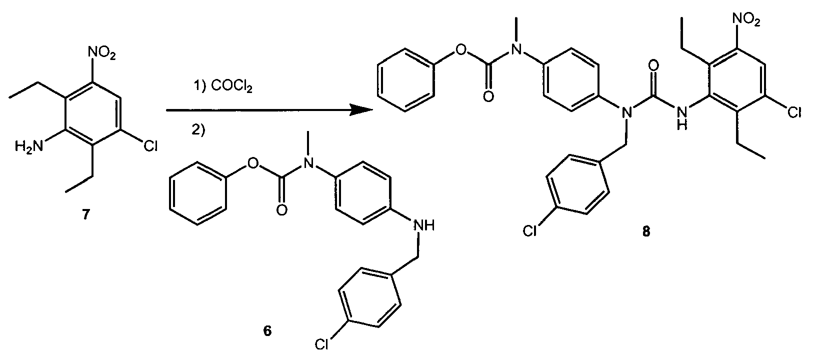 Man setzt 63 mg des Nitroanilins **7** mit 120 mg (0.33 mmol; 1.1 eq.) Amin **6** nach AAV-2A um. Das erhaltene Rohprodukt **8** wird ohne Reinigung für den nächsten Reaktionsschritt eingesetzt.

Stufe 5: 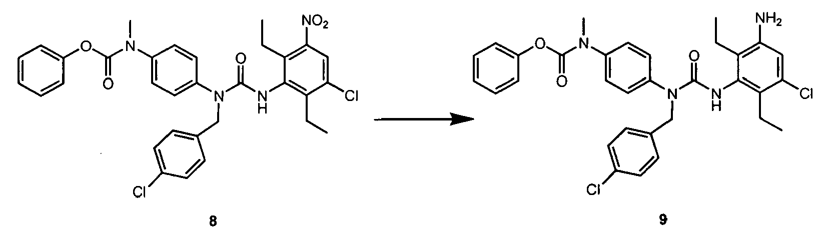 Die in Stufe 2 erhaltene Nitroverbindung **8** wird gemäß AAV-3A reduziert und das erhaltene Rohprodukt **9** weiter umgesetzt.

Stufe 6: 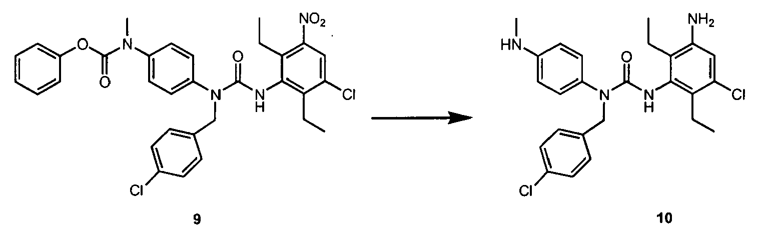 Das Rohprodukt des Harnstoffs **9** wird in einer Mischung aus 5 ml THF, 3 ml DMSO und 2,5 ml 2 M NaOH gelöst und unter starkem Rühren über Nacht zu der Verbindung **10** umgesetzt. Nach Aufreinigung mittels HPLC erhält man 3 mg der Verbindung **10.**

### Beispiel 17: Synthese von 31

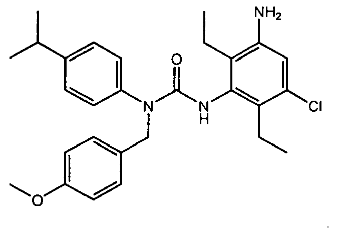

Stufe 1: 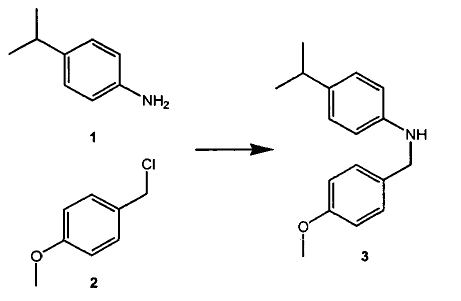 1.46 ml (18 mmol) 4-Isopropylanilin **(1)** wird gemäß AAV-1C mit 4-Methoxybenzylchlorid **(2)** umgesetzt. Man erhält 1.1g(4.3 mmol; 72%) der Verbindung 3 nach HPLC-Reinigung.

Stufe 2: 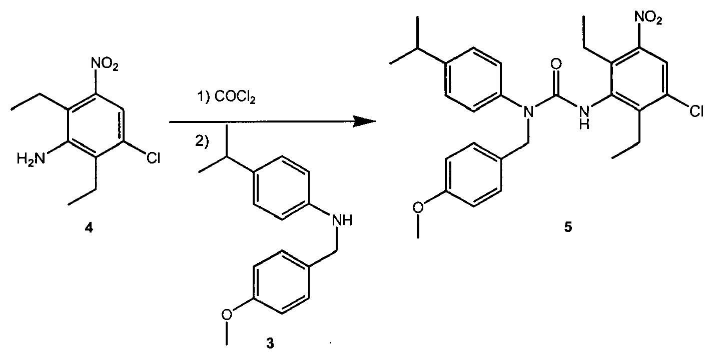 Man setzt 53 mg des Nitroanilins **4** mit 67 mg (0.26 mmol; 1.5 eq.) Amin **3** nach AAV-2A um. Das erhaltene Rohprodukt wird ohne Reinigung für den nächsten Reaktionsschritt eingesetzt.

Stufe 3: 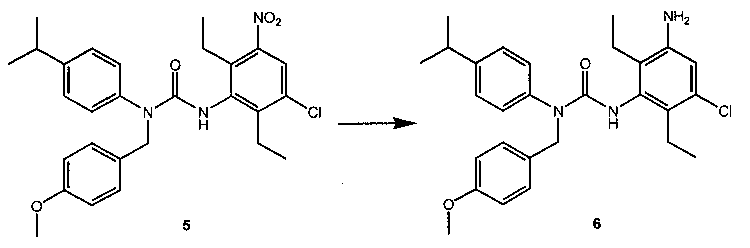 Die in Stufe 2 erhaltene Nitroverbindung **5** wird gemäß AAV-3B reduziert und das erhaltene Rohprodukt per HPLC gereinigt. Man erhält 13 mg der Verbindung **6.**

### Beispiel 18: Synthese von 80

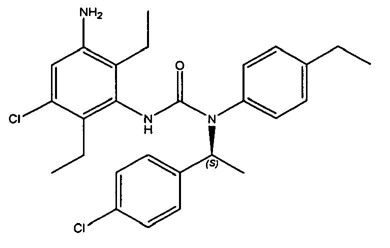

Stufe 1: 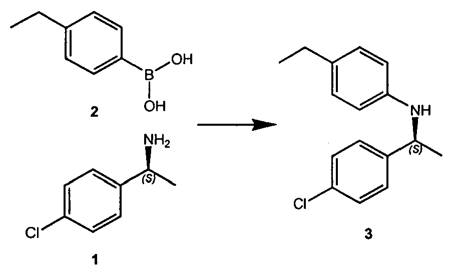 841 µl (6.0 mmol) (S)-4-Chlor-alpha-Methylbenzylamin **(1)** wird gemäß AAV-1B mit 1.35 g (9.0 mmol; 1.5 eq.) 4-Ethylphenylboronsäure **(2)** umgesetzt. Nach flashchromatographischer Reinigung (Hexan / Ethylacetat 12:1) erhält man 450 mg (1.73 mmol; 29%) des gewünschten Amins **3.**

Stufe 2: 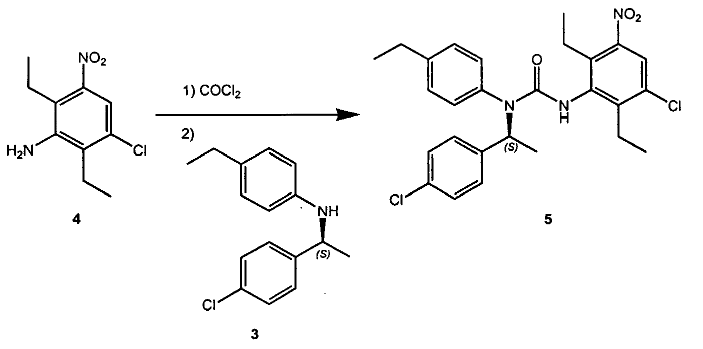 Man setzt 131.7 mg (0.576 mmol) 3-Chlor-2,6-diethyl-5-nitro-phenylamin **(4)** nach AAV-2A mit 485 µl (0.922 mmol; 1.6 eq.) einer 20%igen Lösung von Phosgen in Toluol und mit 224.4 mg (0.864 mmol; 1.5 eq.) (*S*)-[1-(4-Chlorphenyl)-ethyl]-(4-ethyl-phenyl)-amin **(3)** um. Das erhaltene Rohprodukt wird per HPLC gereinigt. Man erhält 276 mg (0.537 mmol; 93%) der Verbindung **5.**

Stufe 3: 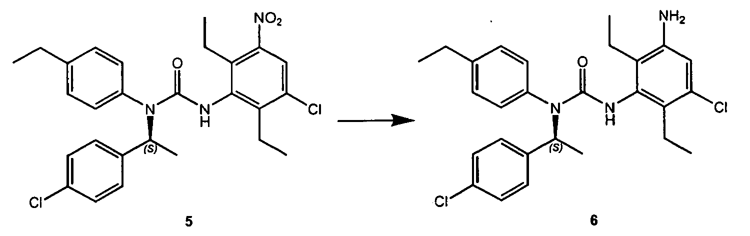 Die in Stufe 2 erhaltene Nitroverbindung **5** wird gemäß AAV-3B mit 9 eq. Zinndichlorid-Dihydrat reduziert und das erhaltene Rohprodukt per HPLC gereinigt. Man erhält 190 mg der gewünschten Verbindung **6.**

### Beispiel 19: Synthese von 102

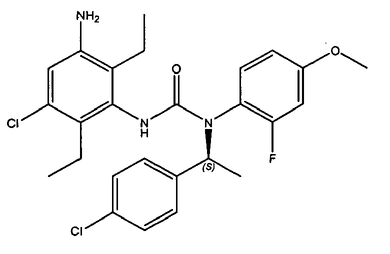

Stufe 1: 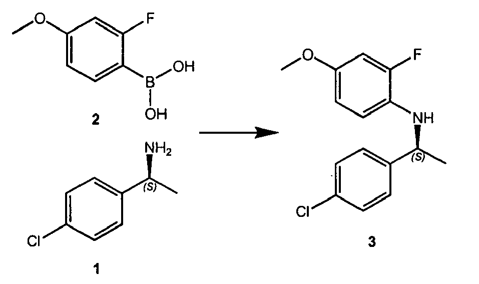 701 µl (5.0 mmol) (S)-4-Chlor-alpha-Methylbenzylamin **(1)** wird gemäß AAV-1B mit 757 µl (6.5 mmol; 1.3 eq.) Lutidin, mit 1.18 g (6.5 mmol; 1.3 eq.) Kupfer-II-acetat und mit 1.7 g (10.0 mmol; 2.0 eq.) 2-Fluor-4-Methoxybenzyl-boronsäure **(2)** umgesetzt. Nach flashchromatographischer Reinigung (Hexan / Ethylacetat 10:1) erhält man 23.9 mg (0.086 mmol) des gewünschten Amins **3.**

Stufe 2: 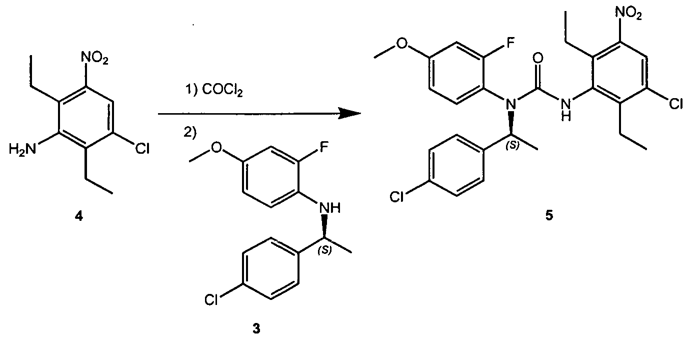 Man setzt 42.5 mg (0.186 mmol; 2 eq.) 3-Chlor-2,6-diethyl-5-nitro-phenylamin **(4)** nach AAV-2A mit 24.6 mg (0.232 mmol; 2.5 eq.) Natriumcarbonat, mit 122 µl (0.232 mmol; 2.5 eq.) einer 20%igen Lösung von Phosgen in Toluol und mit 26.0 mg (0.093 mmol; 1.0 eq.) (*S*)-[1-(4-Chlorphenyl)-ethyl]-(2-fluor-4-methoxy-phenyl)-amin **(3)** um. Das erhaltene Rohprodukt wird per HPLC gereinigt. Man erhält 12 mg (0.023 mmol; 26%) der Verbindung **5.**

Stufe 3: 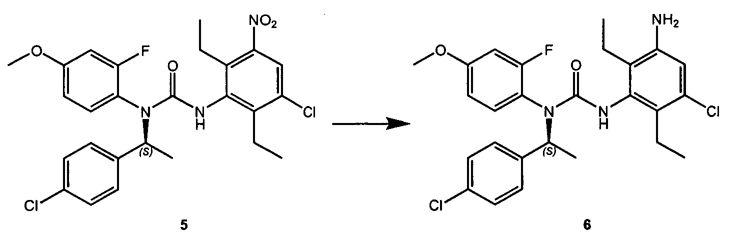 Die in Stufe 2 erhaltene Nitroverbindung **5** wird gemäß AAV-3B mit 9 eq. Zinndichlorid-Dihydrat reduziert und das erhaltene Rohprodukt per HPLC gereinigt. Man erhält 8 mg der gewünschten Verbindung **6.**

### Beispiel 20: Synthese von 36

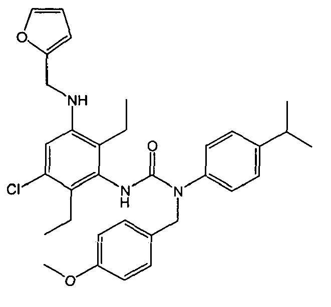

Stufe 1: 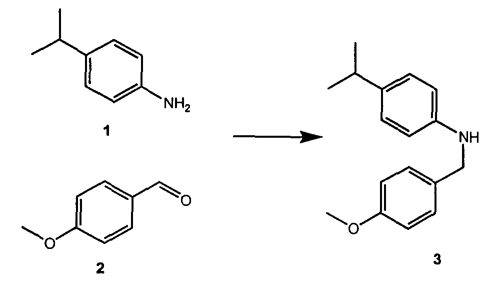 2.02 ml (14.8 mmol) 4-Isopropylanilin **(1)** wird gemäß AAV-1A mit 4-Methoxy-benzaldehyd **(2)** umgesetzt. Man erhält 3.44 g (13.5 mmol; 91%) der Verbindung 3 als Rohprodukt, das ohne Reinigung für den nächsten Reaktionsschritt eingesetzt wird.

Stufe 2: 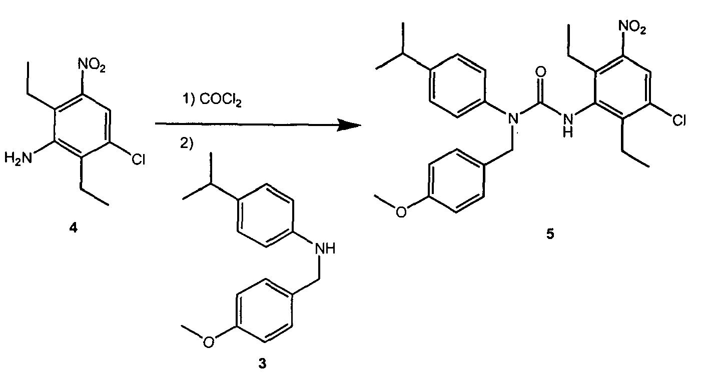 Man setzt 724 mg (3.17 mmol) 3-Chlor-2,6-diethyl-5-nitro-phenylamin **(4)** nach AAV-2A mit 1.05 g (4.12 mmol; 1.3 eq.) (4-Isopropyl-phenyl)-(4-methoxy-benzyl)-amin **(3)** um. Nach flashchromatographischer Reinigung (Hexan / Ethylacetat 12:1) erhält man 78 mg (0.15 mmol) der Verbindung **5.**

Stufe 3: 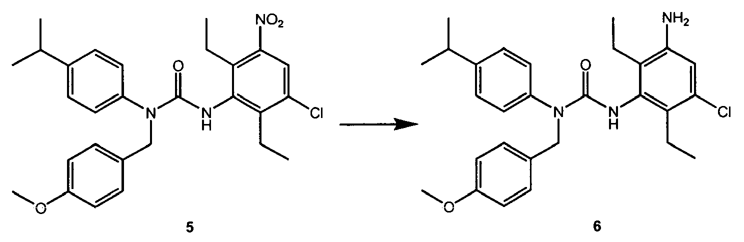 Die in Stufe 2 erhaltene Nitroverbindung **5** wird gemäß AAV-3A reduziert. Man erhält 34.4 mg (0.072 mmol; 47%) der Verbindung **6** als Rohprodukt, das ohne Reinigung für den nächsten Reaktionsschritt eingesetzt wird.

Stufe 4: 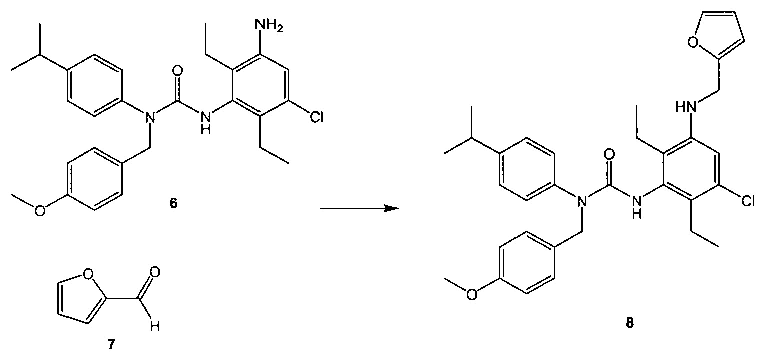 34.4 mg (0.072 mmol) 3-(3-Amino-5-chlor-2,6-diethyl-phenyl)-1-(4-isopropyl-phenyl)-1-(4-methoxy-benzyl)-harnstoff **(6)** wird gemäß AAV-1A mit 9.0 µl (0.108 mmol, 1.5 eq.) 2-Furaldehyd **(7),** mit 32.1 µl (0.108 mmol; 1.5 eq.) Titantetraisopropylat und mit 4.1 mg (0.108 mmol; 1.5 eq.) Natriumborhydrid umgesetzt und das erhaltene Rohprodukt per HPLC gereinigt. Man erhält 14 mg der gewünschten Verbindung **8.**

### Beispiel 21: Synthese von 37

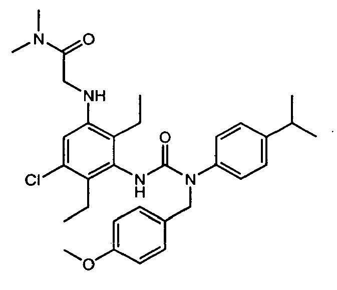

Stufe 1: wie bei Beispiel 19, Stufe 1

Stufe 2: wie bei Beispiel 19, Stufe 2
**4:** 1.06 g (4.63 mmol)
**3:** 1.54 g (6.02 mmol; 1.3 eq.)
Ausbeute: 410 mg (0.8 mmol; 17%)

Stufe 3: wie bei Beispiel 19, Stufe 3
Ausbeute: 220 mg (0.46 mmol; 57%)

Stufe 4: 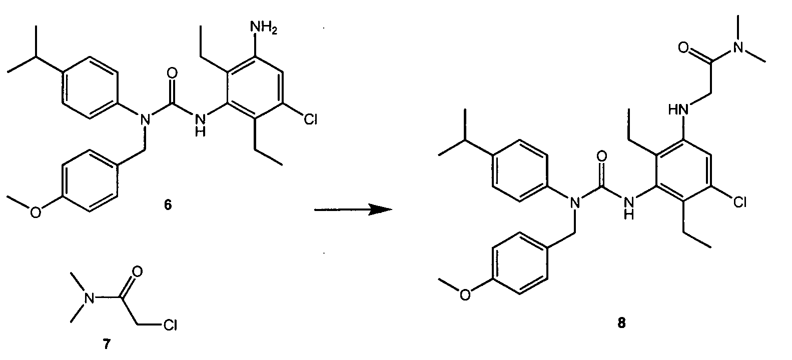 10.0 mg (0.021 mmol) 3-(3-Amino-5-chlor-2,6-diethyl-phenyl)-1-(4-isopropyl-phenyl)-1-(4-methoxy-benzyl)-harnstoff **(6)** werden in DMF gelöst. Man gibt 11.1 mg (0.105 mmol, 5.0 eq.) Natriumcarbonat zu und versetzt die erhaltene Lösung mit 10.8 µl (0.105 mmol, 5.0 eq.) 2-Chlor-N,N-dimethyl-acetamid **(7).** Man erhitzt auf 40°C und rührt für 4 Tage. Das erhaltene Rohprodukt wird per HPLC gereinigt. Man erhält 5 mg der gewünschten Verbindung **8.**

### Beispiel 22: Synthese von 2,6-Diethyl-3-nitro-phenylamin (2)

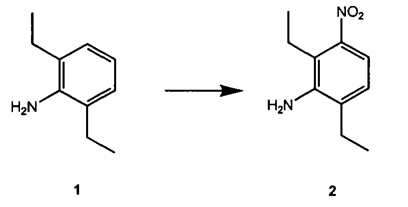

4.42 ml (26.8 mmol) 2,6-Diethyl-phenylamin **(1)** wird gemäß Beispiel 3 umgesetzt. Flashchromatographische Trennung liefert 4.41 g (22.7 mmol; 85%) der Verbindung **2.**

### Beispiel 23: Synthese von 142

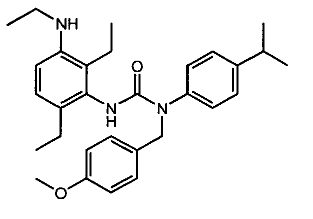

Stufe 1: wie bei Beispiel 19, Stufe 1
**1:** 2.02 ml (14.8 mmol)
**2:** 1.79 ml (14.8 mmol)
Ausbeute: 3.39 g (13.3 mmol; 90%)

Stufe 2: 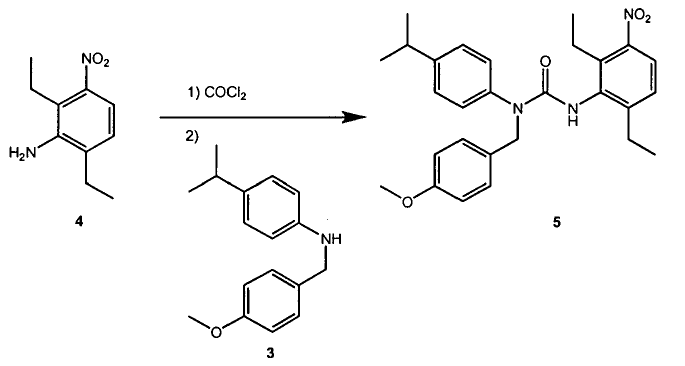 Man setzt 587 mg (3.02 mmol) 2,6-Diethyl-5-nitro-phenylamin **(4)** nach AAV-2A mit 2.54 ml (4.83 mmol; 1.6 eq.) einer 20%igen Lösung von Phosgen in Toluol und mit 1.0 g (3.92 mmol; 1.3 eq.) (4-Isopropyl-phenyl)-(4-methoxy-benzyl)-amin **(3)** um. Nach flashchromatographi-scher Reinigung (Hexan / Ethylacetat 12:1) erhält man 1.16 g (2.43 mmol; 80.5%) der Verbindung **5.**

Stufe 3: 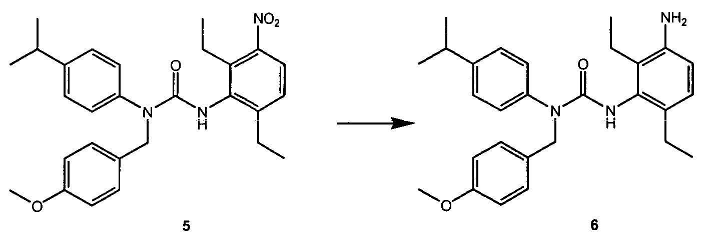 Die in Stufe 2 erhaltene Nitroverbindung **5** wird gemäß AAV-3A reduziert und das erhaltene Rohprodukt per HPLC gereinigt. Man erhält 800 mg des gewünschten Amins **6**.

Stufe 4: 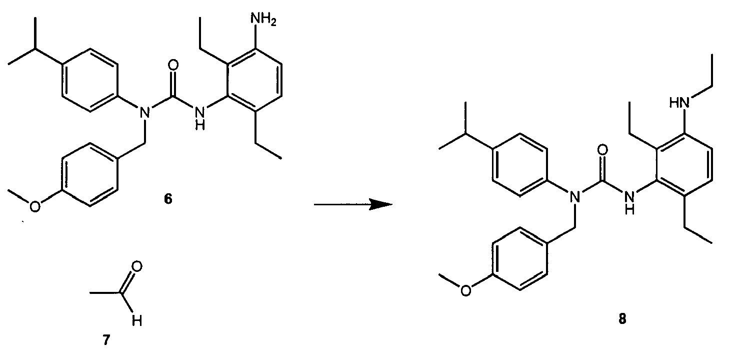 44.6 mg (0.1 mmol) 3-(3-Amino-2,6-diethyl-phenyl)-1-(4-isopropyl-phenyl)-1-(4-methoxybenzyl)-harnstoff **(6)** wird gemäß AAV-1A mit 2.8 µl (0.05 mmol, 0.5 eq.) Acetaldehyd (7), aber ohne Titantetraisopropylat, umgesetzt und das erhaltene Rohprodukt per HPLC gereinigt. Man erhält 21 mg ( mmol) der gewünschten Verbindung **8.**

### Beispiel 24: Synthese von 109

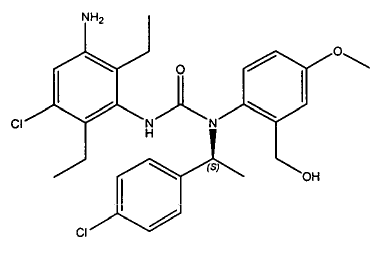

Stufe 1: 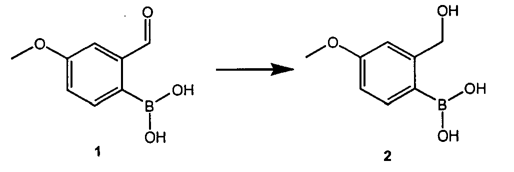 50 mg (0.278 mmol) 4-Methoxy-2-formylphenylboronsäure **(1)** wird in Methanol gelöst. Man gibt 32 mg (0.834 mmol; 3 eq.) Natriumborhydrid vorsichtig zu. Nach zweistündigem Rühren bei Raumtemperatur wird das Lösungsmittel am Rotationsverdampfer entfernt. Nach Zugabe von ca. 50 ml gesättigter NaCl-Lösung und ca. 50 ml Ethylacetat extrahiert man die wässrige Phase dreimal mit Ethylacetat. Die vereinigten organischen Phasen werden über Magnesiumsulfat getrocknet und das Lösungsmittel im Vakuum entfernt. Das erhaltene Rohprodukt wird ohne Reinigung für den nächsten Reaktionsschritt eingesetzt.

Stufe 2: 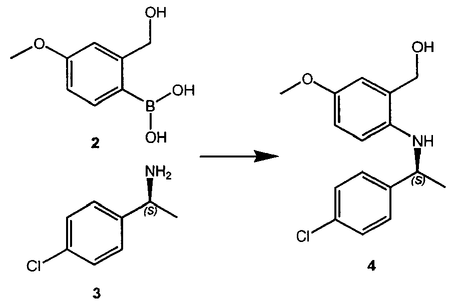 292 µl (2.085 mmol; 5eq.) (S)-4-Chlor-alpha-Methylbenzylamin **(3)** wird gemäß AAV-1B mit 76 mg (0.417 mmol; 1.0 eq.) 2-Hydroxymethyl-4-Methoxybenzyl-boronsäure **(2)** umgesetzt. Nach flashchromatographischer Reinigung (Hexan / Ethylacetat 10:1) erhält man 58.6 mg (0.2 mmol; 48%) des gewünschten Amins **4.**

Stufe 3: 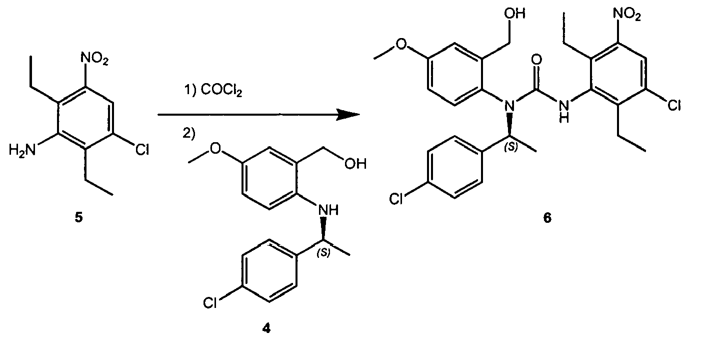 Man setzt 55.1 mg (0.24 mmol; 1.2 eq.) 3-Chlor-2,6-diethyl-5-nitro-phenylamin **(5)** nach AAV-2A mit 58.6 mg (0.2 mmol; 1.0 eq.) (*S*)-[1-(4-Chlorphenyl)-ethyl]-(2-hydroxymethyl-4-methoxy-phenyl)-amin **(4)** um. Das erhaltene Rohprodukt wird per HPLC gereinigt. Man erhält 82.8 mg (0.152 mmol; 76%) der Verbindung **6.**

Stufe 4: 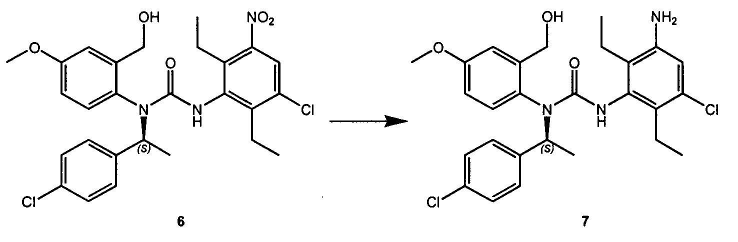 Die in Stufe 3 erhaltene Nitroverbindung 6 wird gemäß AAV-3C reduziert und das erhaltene Rohprodukt per HPLC gereinigt. Man erhält 65 mg der gewünschten Verbindung 7.

### Beispiel 25: Lagerstabilität verschiedener Verbindungen

Die Verbindungen **11** und **13** wurden synthetisiert und einer Reinigung per HPLC unterzogen (Laufmittel MeCN/H₂O mit wechselndem Volumenanteil, 0.05% TFA).

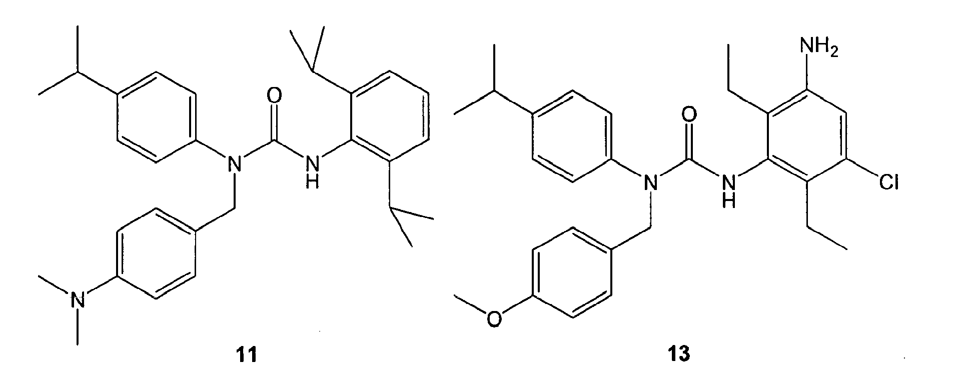

Die Synthese der Verbindung **13** ist im Beispielteil beschrieben.
**11** wird folgendermaßen synthetisiert: 50 mg (0.186 mmol) (4-Isopropylphenyl)-(4-dimethylaminobenzyl)amin wird in ca. 2 ml Acetonitril gelöst. Man gibt 60 µl (0.280 mmol; 1.5 eq.) 2,6-Diisopropylphenylisocyanat sowie 51 mg (0.373 mmol; 2.0 eq.) Kaliumcarbonat zu und rührt für einen Tag bei Raumtemperatur. Zur Aufarbeitung wird die Reaktionslösung im Vakuum eingeengt, mit 20 ml Wasser versetzt und zweimal mit EE extrahiert. Die vereinigten organischen Phasen werden über Magnesiumsulfat getrocknet und das Lösungsmittel im Vakuum entfernt. Reinigung per HPLC liefert 38 mg (0.081 mmol; 44%) der gewünschten Verbindung **11.**

Man lyophilisiert die Fraktionen, die die gewünschte Verbindung enthalten, und isoliert so 1-40 mg der jeweiligen Verbindung **11** bzw. **13** als Feststoff. Aufgrund der basischen Aminogruppen in den Molekülen fallen sie als TFA-Salze an. Zur Evaluierung der Lagerstabilität werden 0.5-10 mg des Feststoffes bei Raumtemperatur unter Lichteinfluß gelagert.

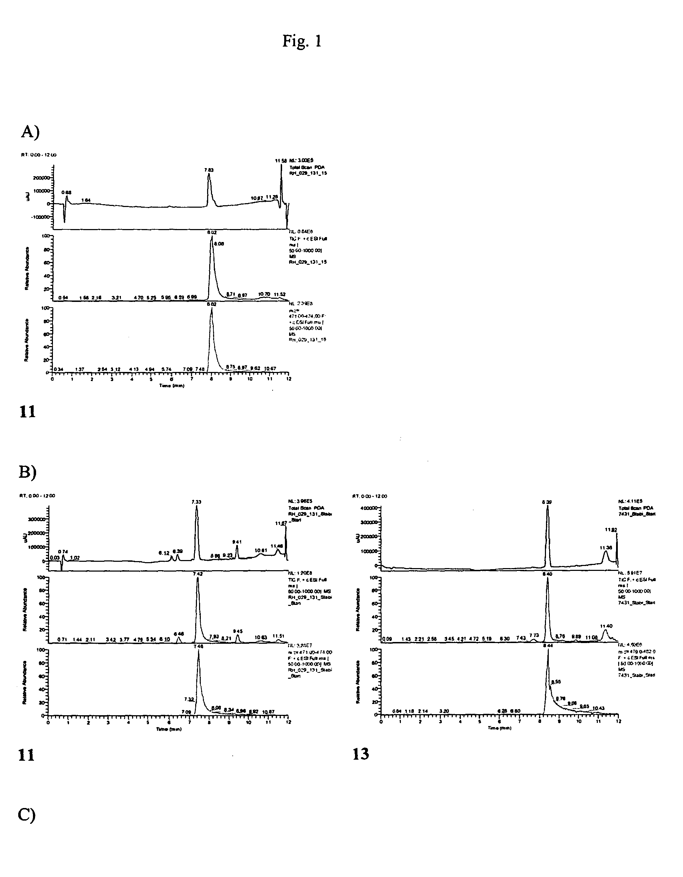

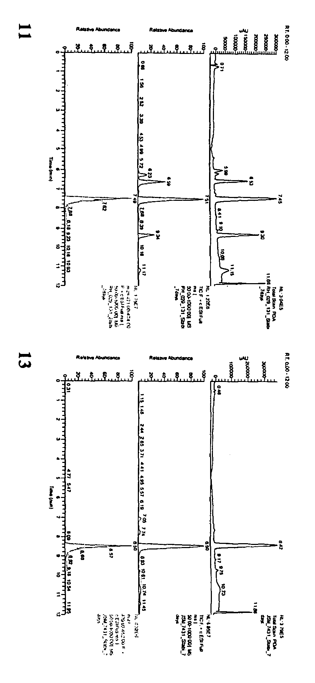

Fig. 1 zeigt analytische RP-HPLC-Chromatogramme von **11** und **13** unter verschiedenen Lagerungsbedingungen sowie mögliche Route bei der Zersetzung von **11.** Der obere Teil der Darstellkung zeigt die UV-Detektion, der mittlere Teil die MS-Detektion und der untere Teil die MS-Detektion der jeweiligen Zielmasse. Die Bedingungen waren dabei wie folgt:
- Fig. 1 A):: nach der Reinigung per HPLC (Lösung in MeCN/H₂O/TFA),
- Fig. 1 B): nach dem Lyophilisieren (0 Tage Lagerung als Feststoff), und
- Fig. 1 C): nach 7 Tagen Lagerung als Feststoff.

Es zeigt sich, dass die Verbindung gemäß des Standes der Technik **(11;** für diese wurde z.B. eine Bindungsaffinität zum C5a-Rezeptor von 100 nM beschrieben; EP1308438) unter den beschriebenen Bedingungen nicht stabil ist. Es findet eine Zersetzung in 2 Verbindungen der Masse 338 und 604 statt, vermutlich durch Benzyl-Übertragung (siehe Reaktionsgleichung unten). Damit ist die Verbindung - zumindestens als TFA-Salz - nicht geeignet, um als Medikament eingesetzt zu werden.

Die Verbindung gemäß der vorliegenden Erfindung **(13)** zeigte dagegen keinerlei Lagerinstabilität, was einen deutlichen Vorteil gegenüber Verbindungen des Standes der Technik darstellt.

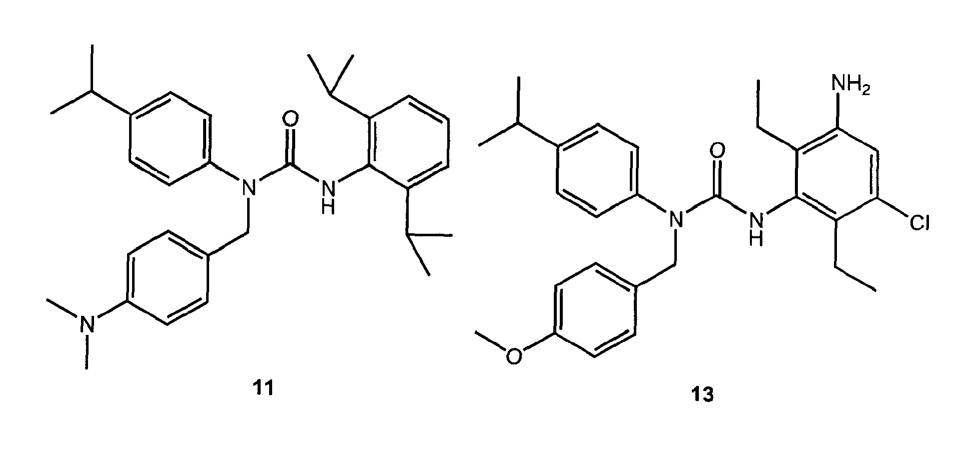

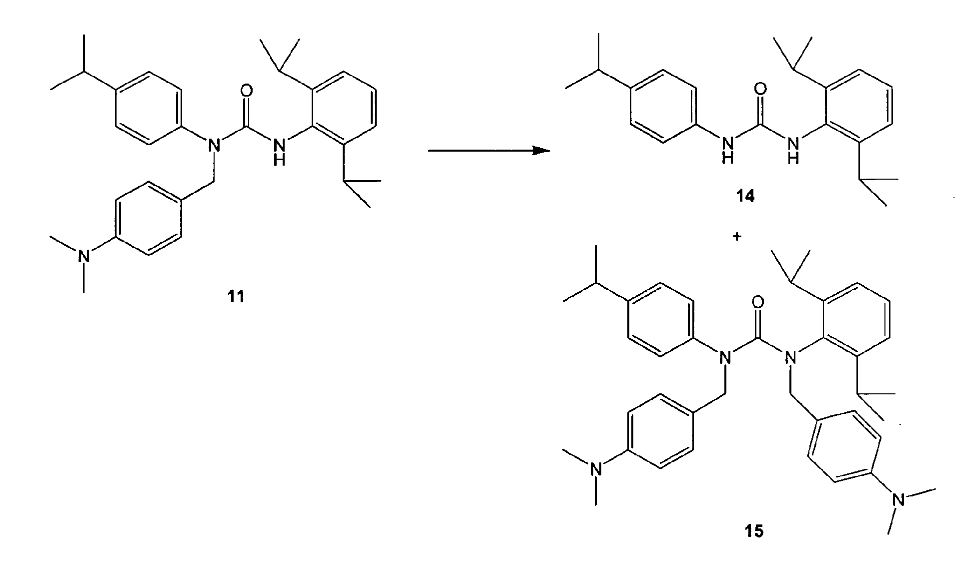

### Beispiel 26: Bestimmung des IC₅₀ Wertes mit humanem Vollblut

Der Vollblutassay wird nach der Anweisung aus dem Phagoburst-Kit (Orpegen Pharma) durchgeführt. Zur Gerinungshemmung werden 50 µg/ml Refludan verwendet (Mollnes et al. 2002 Blood 100: 1869).

Frisch genommenes, antikoaguliertes Blut wird auf Eis gekühlt und 100 µl davon werden zu 10 µl Substanzverdünnung mit maximal 1.3% DMSO gegeben. Das Gemisch wird für 10 min bei 37°C inkubiert. Anschliessend werden die Proben wieder auf Eis gestellt und 20 µl opsonierte *E*. *coli* Suspension (10⁹ Zellen/ml) werden zugegeben. Anschließend erfolgt eine 10 minütige Inkubation bei 37°C. Zu den Zellen werden 20 µl Substrat gegeben und es schließt sich ein weiterer, 10minütiger Inkubationsschritt bei 37°C an. Anschliessend werden die Zellen mit 2 ml Lysispuffer versetzt und 20 min bei Raumtemperatur inkubiert. Durch eine Zentrifugation bei 250xg für 5 min werden die zellulären Bestandteile abgetrennt, der Überstand wird verworfen und das Pellet wird mit 3 ml Waschlösung versetzt. Anschließend werden die Zellen wieder zentrifugiert und anschließend das Pellet in 200 µl DNA-Färbelösung aufgenommen. Mit Hilfe eines FACS-Gerätes (FACSCalibur, Becton-Dickinson) werden die Neutrophilen nach den im Manual angegebenen Parametern analysiert. Tabelle 5 zeigt IC₅₀, die aus humanem Vollblut bestimmt wurden. Es wird deutlich, dass die erfindungsgemäßen Verbindungen aufgrund ihrer Aktivität als Therapeutika für den Menschen geeignet sind.

**Tabelle 5: IC₅₀ Werte der Inhibition des E. coli induzierten oxidativen Burst in humanem Vollblut**

| Molekülbezeichnung | IC₅₀ [µM] |
|---|---|
| **20** | 3.84 |
| **102** | 3.2 |
| **103** | 0.8 |
| **106** | 1.6 |
| **120** | 1.9 |
| **122** | 3.5 |
| **132** | 0.62 |

### Beispiel 27: Bestimmung des IC₅₀ Wertes für die Bindung der Verbindungen an den C5a Rezeptor

Die Bindungsstudien werden als IC₅₀-Bestimmungen mit radioaktiv markiertem C5a (125I) als Tracer durchgeführt. Dafür werden HEK293 Zellen, die mit dem humanen C5aR transfiziert wurden, auf eine Konzentration von 5x106 1/ml eingestellt. In eine Mikrotiterplatte werden 30 µl dieser Zellsuspension gegeben. Anschließend erfolgt die Zugabe des C5a-Rezeptor-Antagonisten und es folgt ein 10minütiger Präinkubationsschritt bei RT. Dann werden 10 µl markiertes C5a zugegeben (15.000 cpm) zugegeben. Der Ansatz wird für 30 min auf Eis inkubiert. Die Zellen werden dann über eine Filterplatte (Millipore MHVB 4510) abgetrennt und mit 2x je 100 µl HAG-CM (siehe Beispiel 32) gewaschen. Die Zellen werden getrocknet und nachdem die Filterplatte auf eine entsprechende Adapterplatte (Canberra 6005178) gebracht wurde, werden 50 µl/well Microscint O zugegeben und die Platte wird im Scintillationszähler vermessen.
Tabelle 6 zeigt IC₅₀ Werte für die Bindung an den C5a Rezeptor. Es wird deutlich, dass die erfindungsgemäßen Verbindungen Inhibitoren der Bindung von C5a an den C5a-Rezeptor sind.

**Tabelle 6: IC_{5O}-Werte für die Inhibition der Bindung von C5a an den C5a-Rezeptor**

| Molekülbezeichnung | IC₅₀ [nM] |
|---|---|
| C5a | 4.7 |
| **20** | 118 |
| **88** | 44 |
| **103** | 58.6 |
| **120** | 86.5 |
| **132** | 43.4 |

### Beispiel 28: Bestimmung des IC₅₀ Wertes in einem Enzymfreisetzungsassay

Die Durchführung des Assays ist bei Köhl (Köhl 1997 The Anaphylatoxins. In: Dodds, A.W., Sim, R.B. (Eds.), Complement: A Practical Approach. Oxford: 135) beschrieben. Basophile Leukämie-Zellen aus Ratten (RBL), die den humanen C5a-Rezeptor (CD88) exprimieren, werden in DMEM mit 10% fötalem Kälberserum, 100 U/ml Penicillin, 100 µg/ml Streptomycin und 2 mM Glutamin (alle Medienbestandteile Biochrome, Berlin) bis zur Konfluenz bei 37°C und 10% CO₂ angezogen. Alle folgenden Angaben beziehen sich auf eine Kulturflasche mit 75 cm² Fläche. Verbrauchtes Medium wird abgegossen. Zellen werden mit 10 ml PBS (Dulbecco's PBS, Biochrome) gewaschen und anschließend mit 3 ml Cell Dissociation Solution (CDS, Sigma) überschichtet. Zellen werden bei RT 1 min inkubiert. Anschließend wird die CDS entfernt und die Zellen werden weitere 10-15 min bei 37°C zum Ablösen inkubiert. Im Assay werden 20 µl Lösung der zu testenden Verbindung verwendet. Die Assaylösung darf nicht mehr als 2.8% DMSO enthalten. In Verdünnungsreihen wird in 1/3 oder 1/2 Schritten verdünnt. Zu den 20 µl Lösungen der Verbindungen werden 75 µl folgendermaßen behandelter RBL-Zellen gegeben: Nach der Ablösephase werden die Zellen heftig abgeklopft und in 10 ml auf 37°C temperierten HAG-CM aufgenommen (20 mM HEPES; 125 mM NaCl, 5mM KCl, 1 mM CaCl₂, 1mM MgCl₂, 0.5 mM Glucose, 0.25% BSA. HEPES-Herstellung: 2.3 g/l HEPES-Salz + 2.66 g/l HEPES Säure). Zellen werden gezählt und zentrifugiert (200g, 10 min). Das Zellpellet wird mit vorgewärmten HAG-CM aufgenommen, und die Zelldichte auf 2x10⁶ Zellen/ml eingestellt. Die Zellen werden bei 37°C für 5 min inkubiert. Zu den Zellen kommen pro ml Zellsuspension 2.7µl einer Cytochalasin B-Lösung (1000 µg/ml in DMSO, Sigma). Die Zellen werden weitere 3 min bei 37°C inkubiert. 75 µl Zellsuspension werden zu den 20 µl Lösung mit der zu testenden Verbindung gegeben. Damit ergibt sich ein Volumen von 95 µl pro Well. Die Zellen werden 10 min bei 37°C inkubiert. Dann werden pro Well 10 µl hrC5a (10.5 nM in HAG-CM, Sigma) gegeben. Es folgt eine Inkubation für 5 min bei 37°C. Anschließend werden die Platten auf Eis gestellt und bei 1200xg und 4°C für 3 min zentrifugiert. 75 µl des Überstands werden zu 100 µl Substrat-Lösung (2.7 mg/ml p-Nitrophenyl-N-acetyl-b-D-Glucosaminide (Sigma) in 42.5 mM Na-Acetat pH 4.5) gegeben. Die Platte wird für 1 h bei 37°C inkubiert. Pro Well werden 75 µl 0.4 M Glycin pH 10.4 gegeben. Die Platte kann anschließend bei 405 nm gemessen werden. Der IC₅₀-Wert wird durch die Lösung der 4-Parametergleichung y=((A-D)/(1+(x/C)^{B}))+D bestimmt.

Die Ergebnisse des Tests zur Bestimmung der IC₅₀-Werte sind in Tabelle 1 dargestellt.

**Tabelle 1: Daten zur antagonistischen Aktivität repräsentativer erfindungsgemäßer Verbindungen**

| Aktivitätsbereiche: | |
|---|---|
| 1C₅₀ ≤60 nM: | A |
| 60 nM < IC₅₀ ≤200 nM: | B |
| 200 nM < IC₅₀ ≤5000 nM: | C |
| 5000 nM < IC₅₀ | D |

| Struktur | Nr. | [M+H]+ berechnet | [M+H]+ gemessen | Aktivitäts-Bereich | Chemischer Name |
|---|---|---|---|---|---|
| 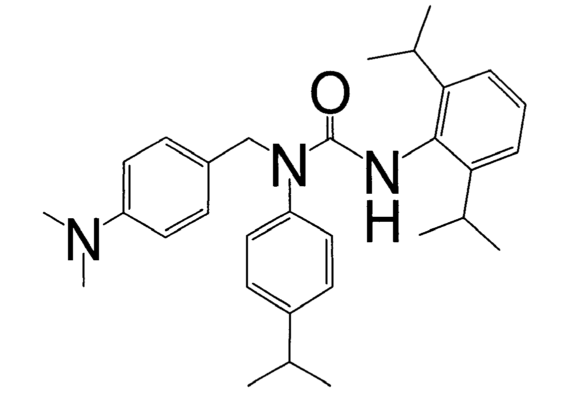 | **19** | 472.3 | 472.7 | C | 3-(2,6-Diisopropyl-phenyl)-1-(4-dimethylamino-benzyl)-1-(4-isopropyl-phenyl)-harnstoff |
| 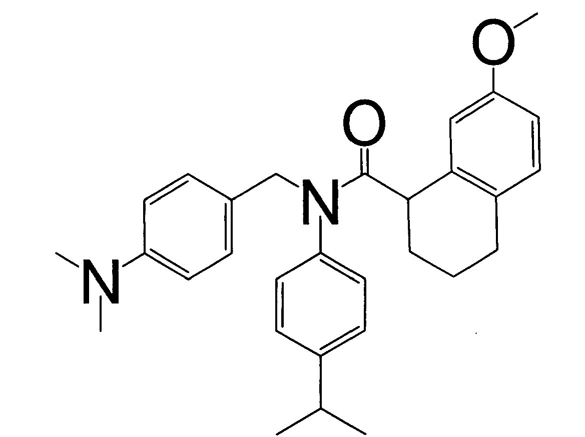 | **20** | 457.3 | 457.4 | B, 89 nM | 7-Methoxy-1,2,3,4-tetrahydro-naphthalin-1-carbonsäure (4-dimethylamino-benzyl)-(4-isopropyl-phenyl)-amid |
| 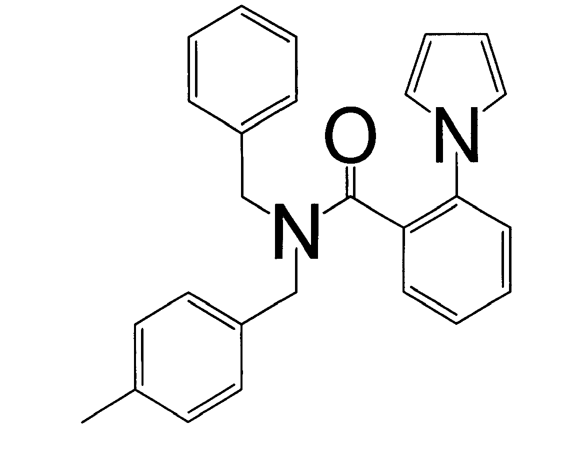 | **21** | 381.2 | 381.1 | C, 1917 nM | N-Benzyl-N-(4-methyl-benzyl)-2-pyrrol-1-yl-benzamid |
| 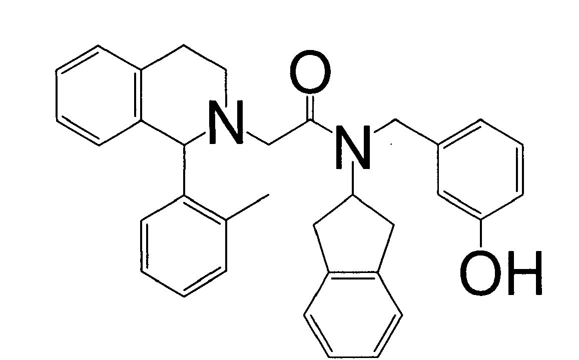 | **22** | 503.3 | 503.5 | D, 7142 nM | N-(3-Hydroxy-benzyl)-N-indan-2-yl-2-(1-o-tolyl-3,4-dihydro-1H-isochinolin-2-yl)-acetamid |
| 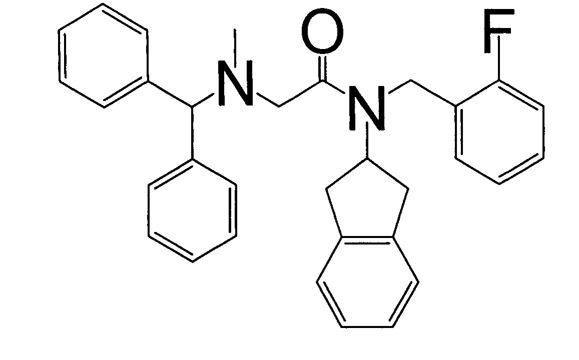 | **23** | 479.2 | 479.1 | D, > 4760 nM | 2-(Benzhydryl-methyl-amino)-N-(2-fluor-benzyl)-N-indan-2-yl-acetamid |
| 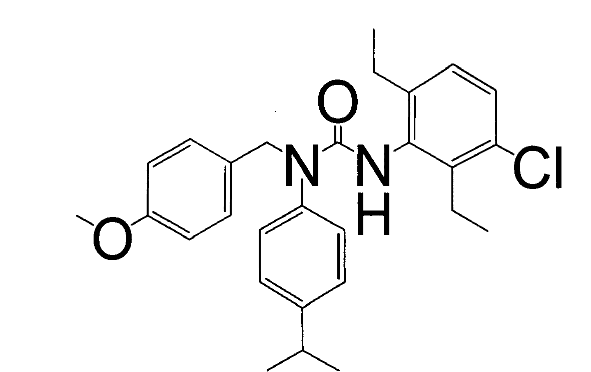 | **24** | 465.2 | 465.9 | C, 1051 nM | 3-(3-Chlor-2,6-diethyl-phenyl)-1-(4-isopropyl-phenyl)-1-(4-methoxy-benzyl)-harnstoff |
| 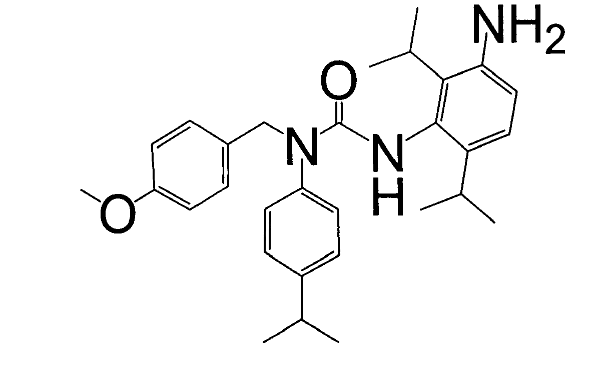 | **25** | 474.3 | 474.8 | C | 3-(3-Amino-2,6-diisopropyl-phenyl)-1-(4-isopropyl-phenyl)-1-(4-methoxy-benzyl)-harnstoff |
| 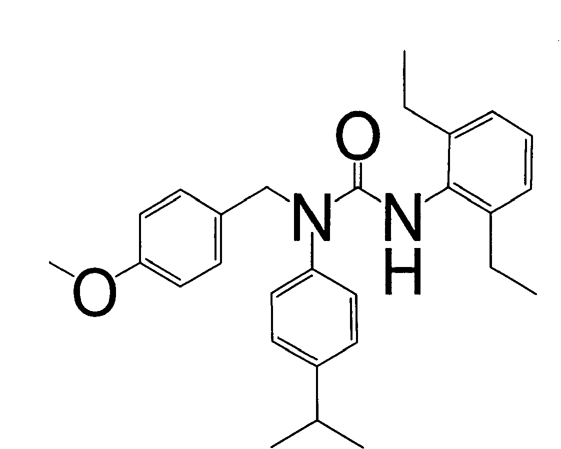 | **26** | 431.3 | 431.8 | C, 524 nM | 3-(2,6-Diethyl-phenyl)-1-(4-isopropyl-phenyl)-1-(4-methoxy-benzyl)-harnstoff |
| 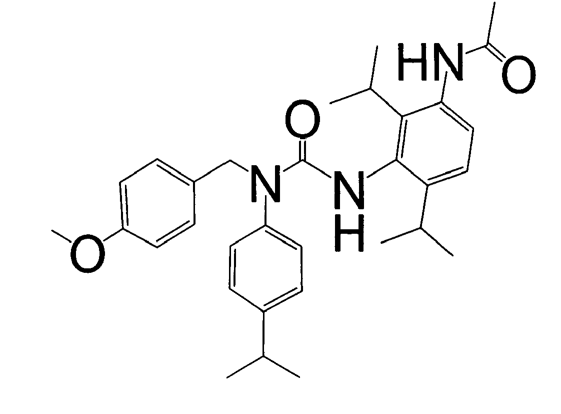 | **27** | 516.3 | 516.3 | D | N-{2,4-Diisopropyl-3-[3-(4-isopropyl-phenyl)-3-(4-methoxy-benzyl)-ureido]-phenyl}-acetamid |
| 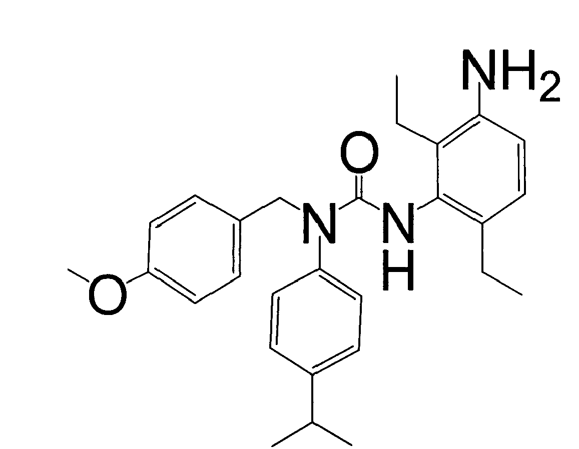 | **28** | 446.3 | 446.3 | C | 3-(3-Amino-2,6-diethyl-phenyl)-1-(4-isopropyl-phenyl)-1-(4-methoxy-benzyl)-harnstoff |
| 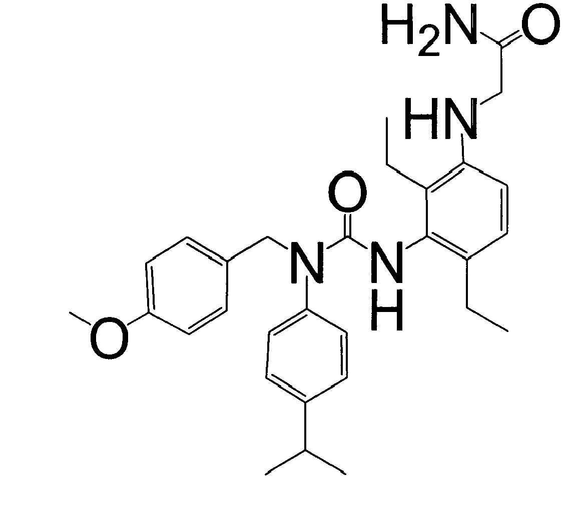 | **29** | 503.3 | 504.2 | C | 2-{2,4-Diethyl-3-[3-(4-isopropyl-phenyl)-3-(4-methoxy-benzyl)-ureido]-phenylamino}-acetamid |
| 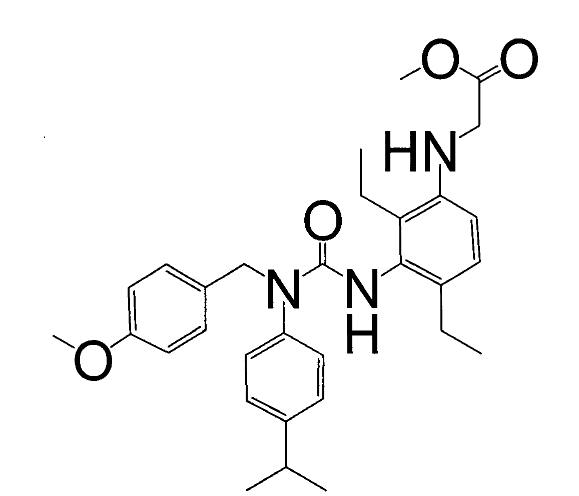 | **30** | 518.3 | 518.2 | C | {2,4-Diethyl-3-[3-(4-isopropyl-phenyl)-3-(4-methoxy-benzyl)-ureido]-phenylamino}-essigsäure methyl ester |
| 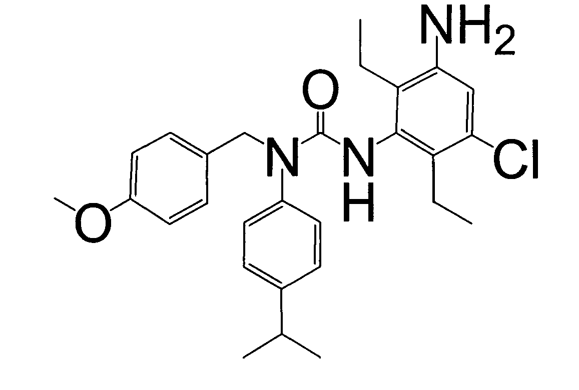 | **31** | 480.2 | 480.1 | B, 164 nM | 3-(3-Amino-5-chlor-2,6-diethyl-phenyl)-1-(4-isopropyl-phenyl)-1-(4-methoxy-benzyl)-harnstoff |
| 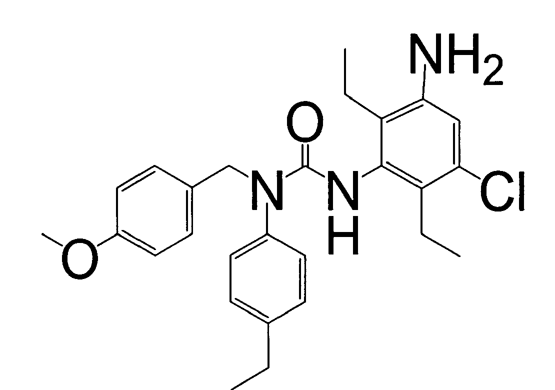 | **32** | 466.2 | 466.1 | C | 3-(3-Amino-5-chlor-2,6-diethyl-phenyl)-1-(4-ethyl-phenyl)-1-(4-methoxy-benzyl)-harnstoff |
| 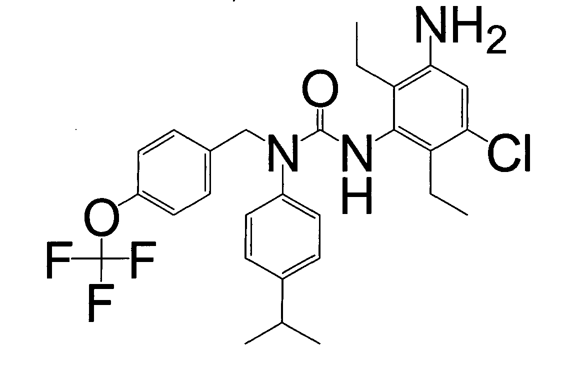 | **33** | 534.2 | 534.1 | C | 3-(3-Amino-5-chlor-2,6-diethyl-phenyl)-1-(4-isopropyl-phenyl)-1 -(4-trifluormethoxy-benzyl)-harnstoff |
| 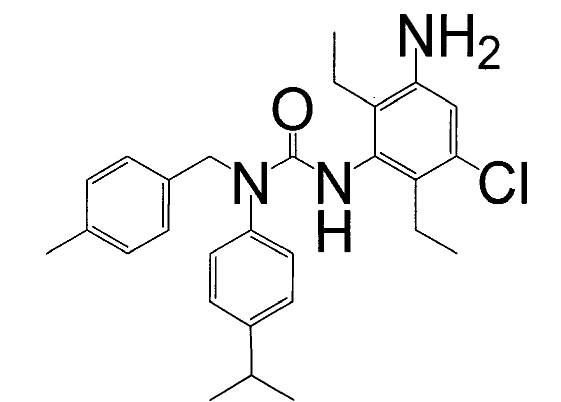 | **34** | 464.2 | 464.2 | B | 3-(3-Amino-5-chlor-2,6-diethyl-phenyl)-1-(4-isopropyl-phenyl)-1-(4-methyl-benzyl)-harnstoff |
| 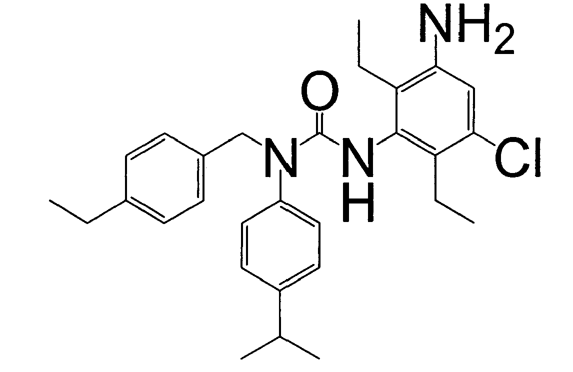 | **35** | 478.3 | 478.2 | C | 3-(3-Amino-5-chlor-2,6-diethyl-phenyl)-1-(4-ethyl-benzyl)-1 -(4-isopropyl-phenyl)-harnstoff |
| 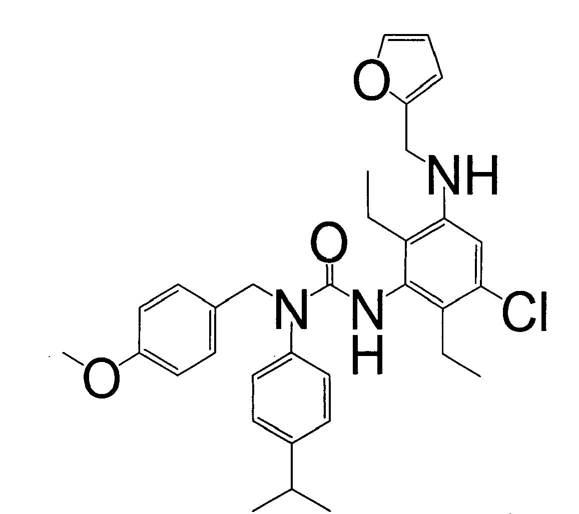 | **36** | 560.3 | 560.9 | C | 3- {3-Chlor-2,6-diethyl-5-[(furan-2-ylmethyl)-amino]-phenyl} -1-(4-isopropyl-phenyl)-1-(4-methoxy-benzyl)-harnstoff |
| 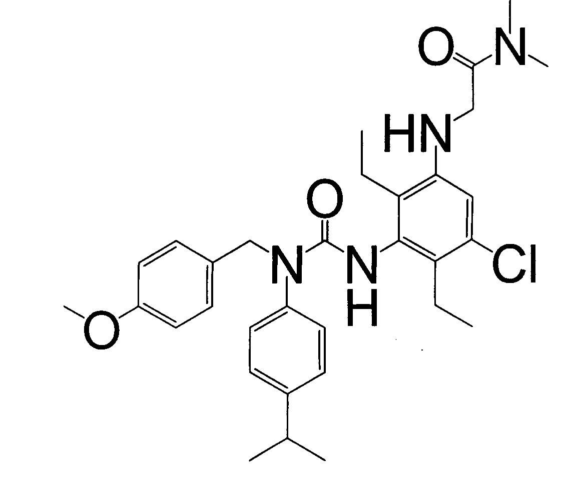 | **37** | 565.3 | 566.0 | C | 2-{5-Chlor-2,4-diethyl-3-[3-(4-isopropyl-phenyl)-3-(4-methoxy-benzyl)-ureido]-phenylamino}-N,N-dimethyl-acetamid |
| 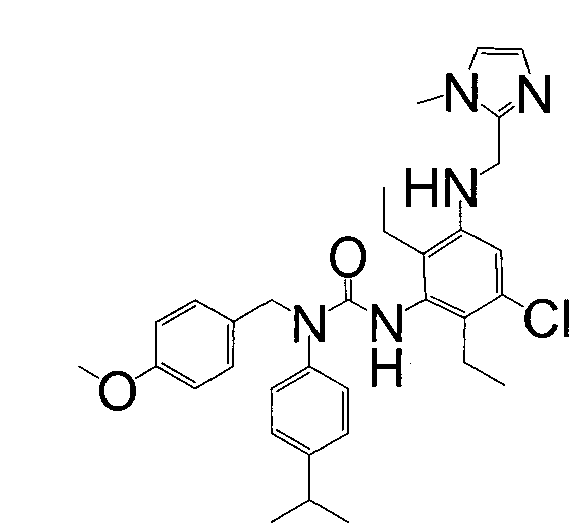 | **38** | 574.3 | 574.2 | C | 3-{3-Chlor-2,6-diethyl-5-[(1-methyl-1H-imidazol-2-ylmethyl)-amino]-phenyl} -1-(4-isopropyl-phenyl)- 1-(4-methoxy-benzyl)-harnstoff |
| 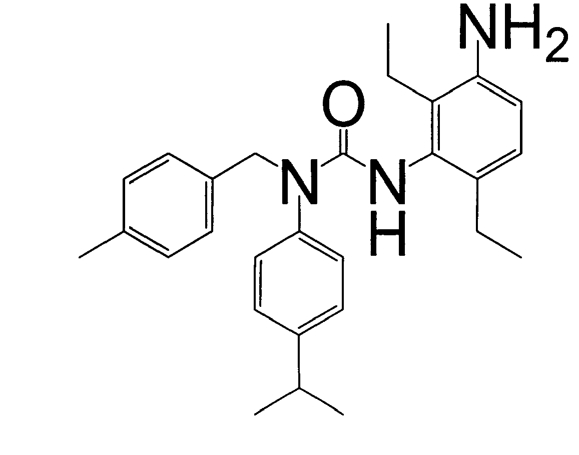 | **39** | 430.3 | 430.2 | C | 3-(3-Amino-2,6-diethyl-phenyl)-1-(4-isopropyl-phenyl)-1-(4-methyl-benzyl)-harnstoff |
| 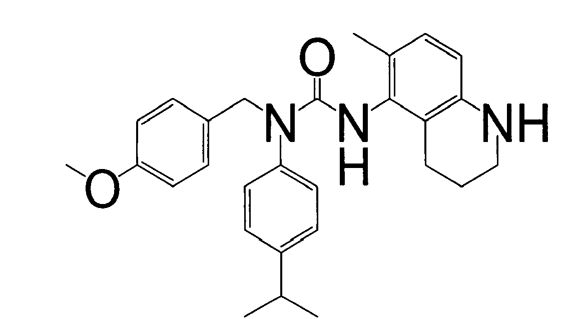 | **40** | 444.3 | 444.2 | D | 1-(4-Isopropyl-phenyl)-1-(4-methoxy-benzyl)-3-(6-methyl-1,2,3,4-tetrahydro-chinolin-5-yl)-harnstoff |
| 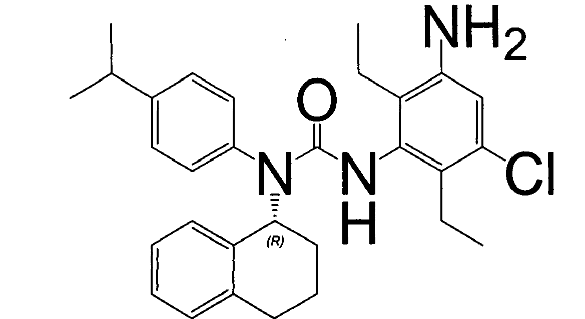 | **41** | 490.3 | 490.1 | C | (R)-3-(3-Amino-5-chlor-2,6-diethyl-phenyl)-1-(4-isopropyl-phenyl)-1-(1,2,3,4-tetrahydro-naphthalin-1-yl)-harnstoff |
| 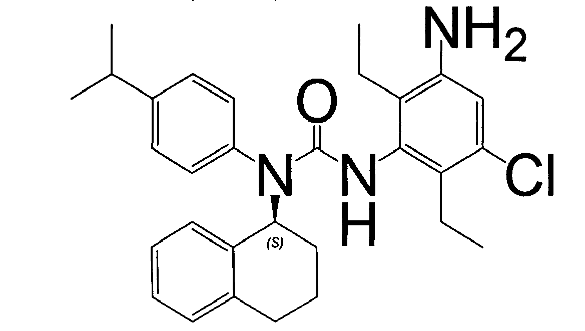 | **42** | 490.3 | 490.1 | B | (S)-3-(3-Amino-5-chlor-2,6-diethyl-phenyl)-1-(4-isopropyl-phenyl)-1-(1, 2, 3,4-tetrahydro-naphthalin-1-yl)-harnstoff |
| 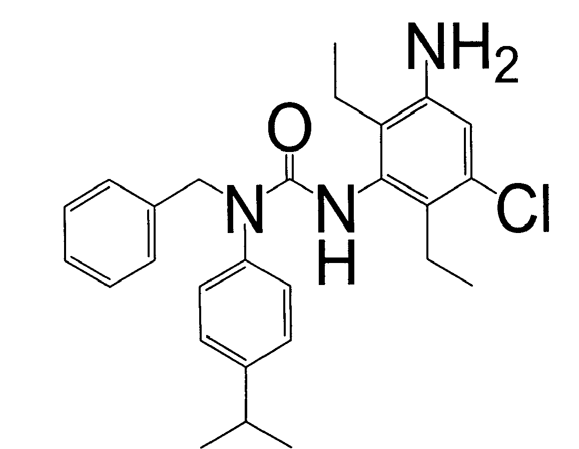 | **43** | 450.2 | 450.2 | C | 3-(3-Amino-5-chlor-2,6-diethyl-phenyl)-1-benzyl-1-(4-isopropyl-phenyl)-harnstoff |
| 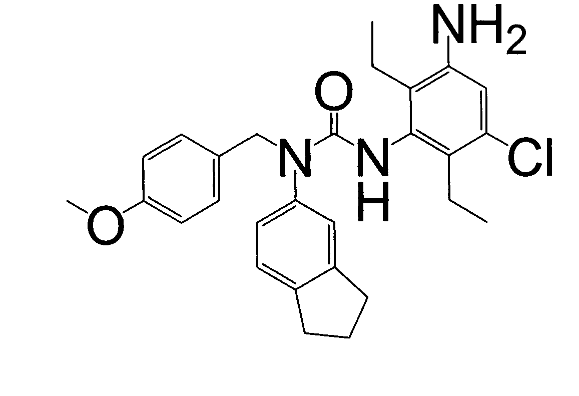 | **44** | 478.2 | 478.1 | C | 3-(3-Amino-5-chlor-2,6-diethyl-phenyl)-1-indan-5-yl-1-(4-methoxy-benzyl)-harnstoff |
| 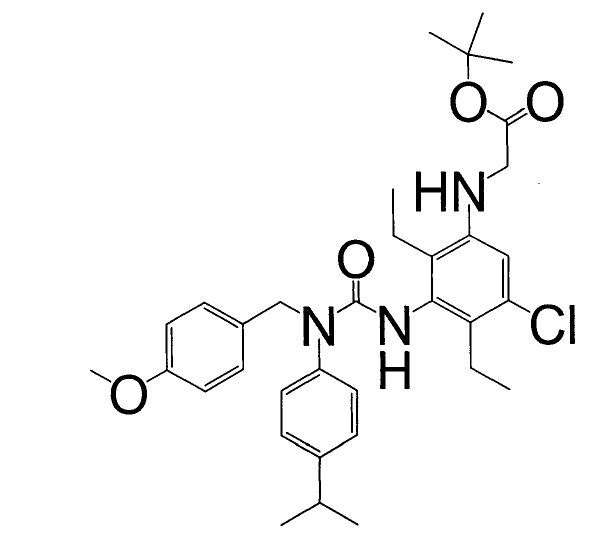 | **45** | 594.3 | 594.1 | B | {5-Chlor-2,4-diethyl-3-[3-(4-isopropyl-phenyl)-3-(4-methoxy-benzyl)-ureido]-phenylamino}-essigsäure tert-butyl ester |
| 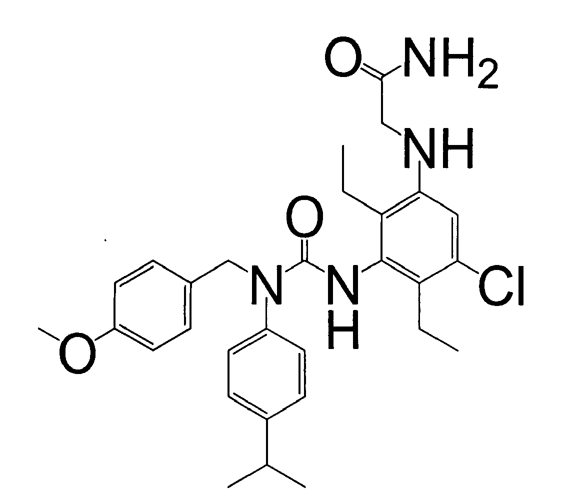 | **46** | 537.3 | 537.9 | C | 2-{5-Chlor-2,4-diethyl-3-[3-(4-isopropyl-phenyl)-3-(4-methoxy-benzyl)-ureido]-phenylamino} -acetamid |
| 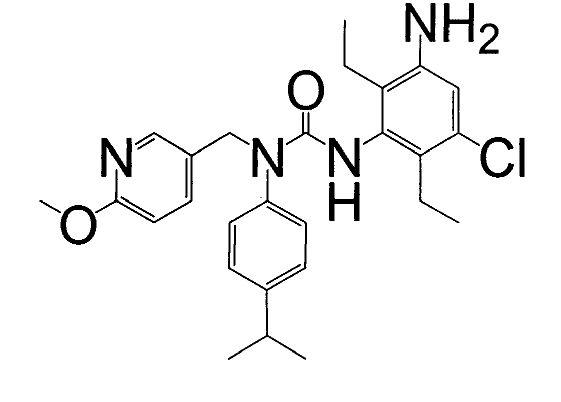 | **47** | 481.2 | 481.2 | C | 3-(3-Amino-5-chlor-2,6-diethyl-phenyl)-1-(4-isopropyl-phenyl)-1-(6-methoxy-pyridin-3-ylmethyl)-harnstoff |
| 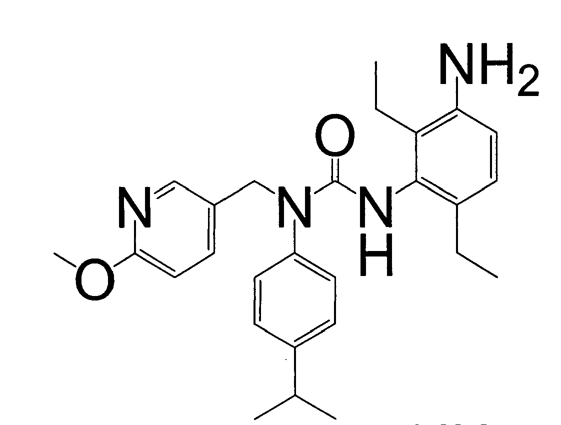 | **48** | 447.3 | 447.3 | C | 3-(3-Amino-2,6-diethyl-phenyl)-1-(4-isopropyl-phenyl)-1-(6-methoxy-pyridin-3-ylmethyl)-harnstoff |
| 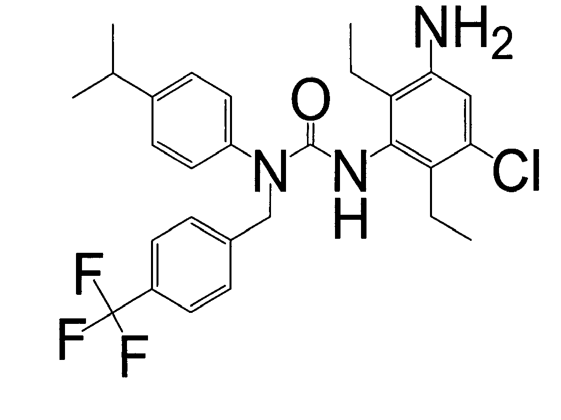 | **49** | 518.2 | 518.1 | C | 3-(3-Amino-5-chlor-2,6-diethyl-phenyl)-1-(4-isopropyl-phenyl)-1-(4-trifluormethyl-benzyl)-harnstoff |
| 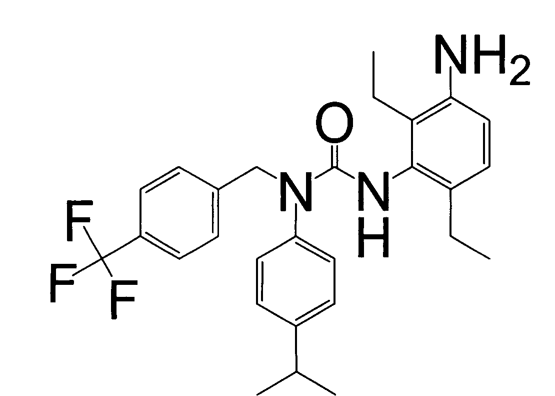 | **50** | 484.3 | 484.2 | C | 3-(3-Amino-2,6-diethyl-phenyl)-1-(4-isopropyl-phenyl)-1-(4-trifluormethyl-benzyl)-harnstoff |
| 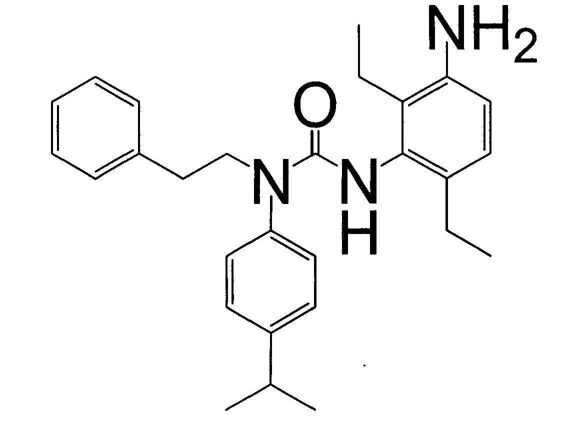 | **51** | 430.3 | 430.8 | C | 3-(3-Amino-2,6-diethyl-phenyl)-1-(4-isopropyl-phenyl)-1-phenethyl-harnstoff |
| 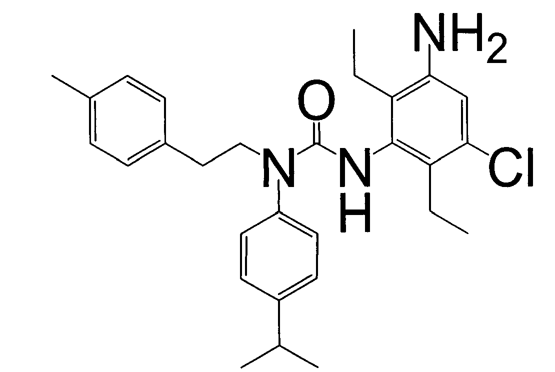 | **52** | 478.3 | 478.4 | C | 3-(3-Amino-5-chlor-2,6-diethyl-phenyl)-1-(4-isopropyl-phenyl)-1-(2-p-tolyl-ethyl)-harnstoff |
| 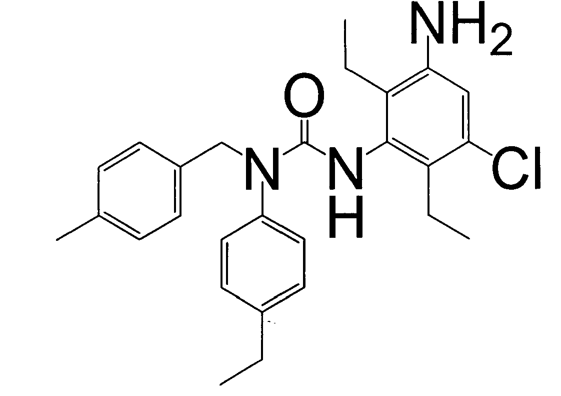 | **53** | 450.2 | 450.2 | B | 3-(3-Amino-5-chlor-2,6-diethyl-phenyl)-1-(4-ethyl-phenyl)-1-(4-methyl-benzyl)-harnstoff |
| 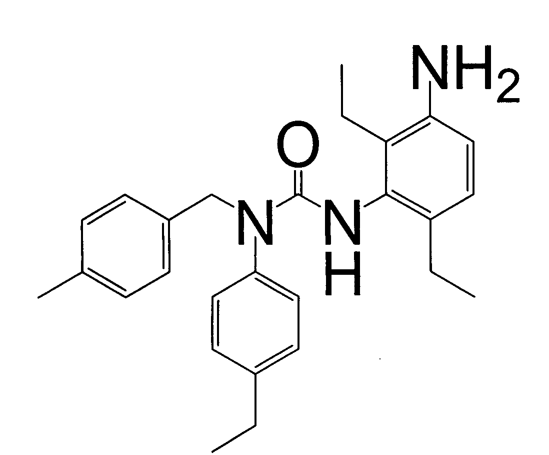 | **54** | 416.3 | 416.2 | C | 3-(3-Amino-2,6-diethyl-phenyl)-1-(4-ethyl-phenyl)-1-(4-methyl-benzyl)-harnstoff |
| 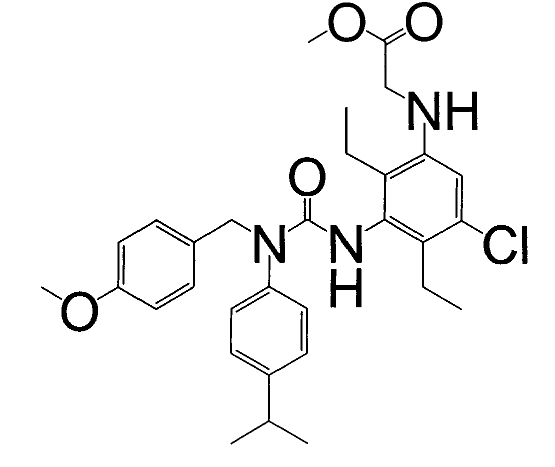 | **55** | 552.3 | 553.5 | C | {5-Chlor-2,4-diethyl-3-[3-(4-isopropyl-phenyl)-3-(4-methoxy-benzyl)-ureido]-phenylamino}-essigsäuremethylester |
| 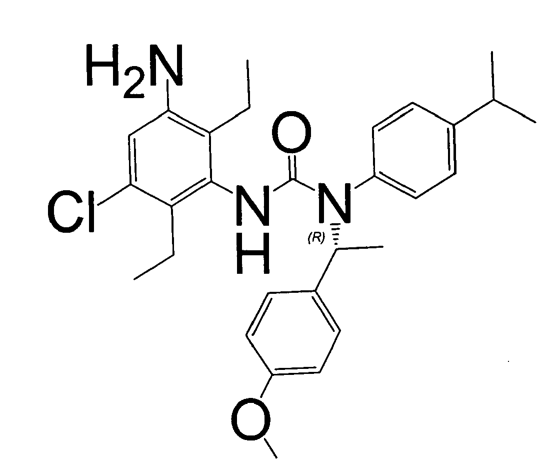 | **56** | 494.3 | 494.9 | C, 619 nM | (R)-3-(3-Amino-5-chlor-2,6-diethyl-phenyl)-1-(4-isopropyl-phenyl)- 1 -[1 -(4-methoxy-phenyl)-ethyl]-harnstoff |
| 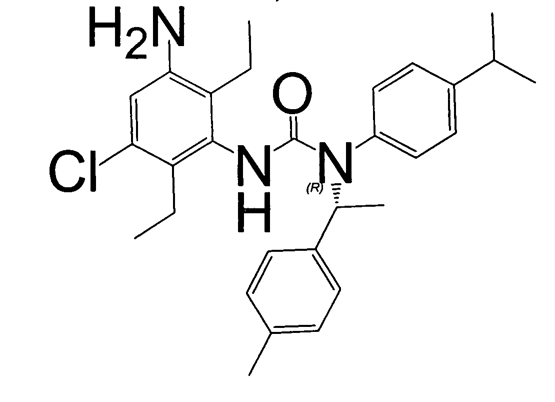 | **57** | 478.3 | 479.0 | C | (R)-3-(3-Amino-5-chlor-2,6-diethyl-phenyl)-1-(4-isopropyl-phenyl)-1-(1-p-tolyl-ethyl)-harnstoff |
| 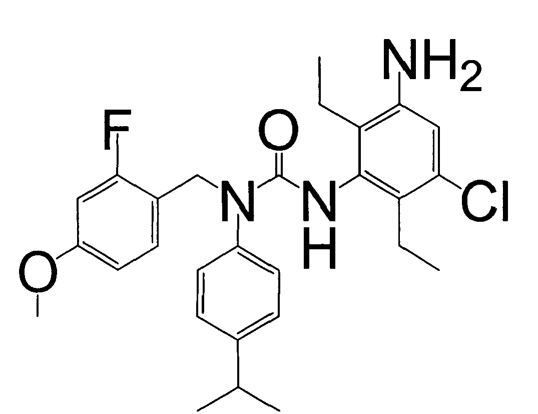 | **58** | 498.2 | 498.1 | C | 3-(3-Amino-5-chlor-2,6-diethyl-phenyl)-1-(2-fluor-4-methoxy-benzyl)-1-(4-isopropyl-phenyl)-harnstoff |
| 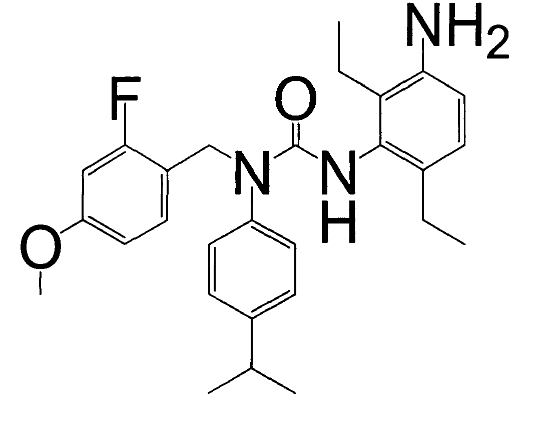 | **59** | 464.3 | 464.2 | C | 3-(3-Amino-2,6-diethyl-phenyl)-1-(2-fluor-4-methoxy-benzyl)-1-(4-isopropyl-phenyl)-harnstoff |
| 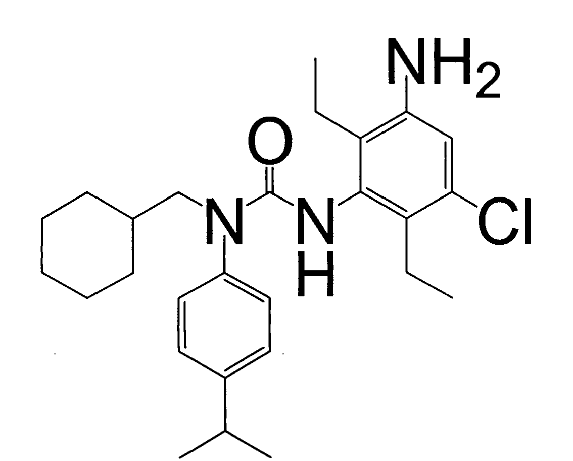 | **60** | 456.3 | 456.2 | C | 3-(3-Amino-5-chlor-2,6-diethyl-phenyl)-1-cyclohexylmethyl-1-(4-isopropyl-phenyl)-harnstoff |
| 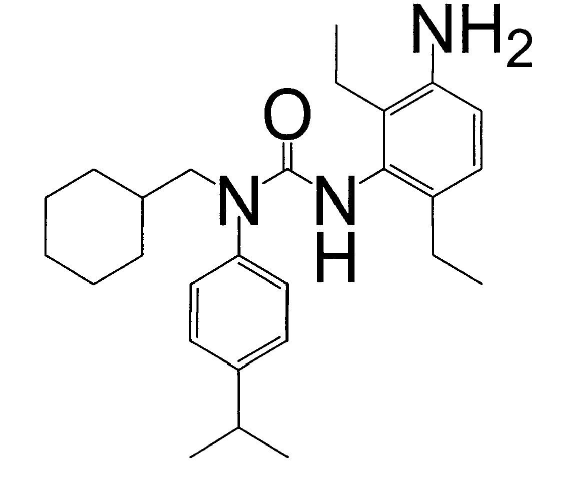 | **61** | 422.3 | 422.3 | C | 3-(3-Amino-2,6-diethyl-phenyl)-1-cyclohexylmethyl-1-(4-isopropyl-phenyl)-harnstoff |
| 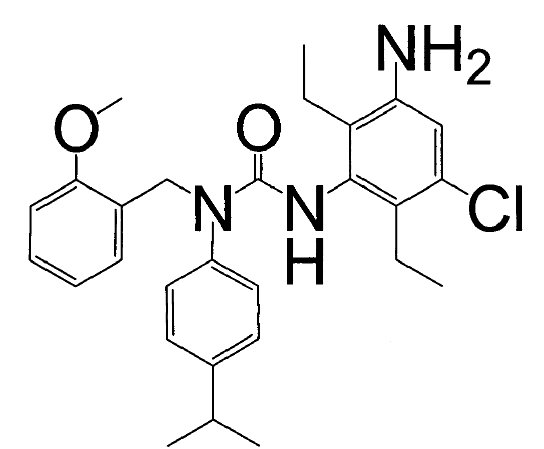 | **62** | 480.2 | 480.2 | C | 3-(3-Amino-5-chlor-2,6-diethyl-phenyl)-1-(4-isopropyl-phenyl)-1-(2-methoxy-benzyl)-harnstoff |
| 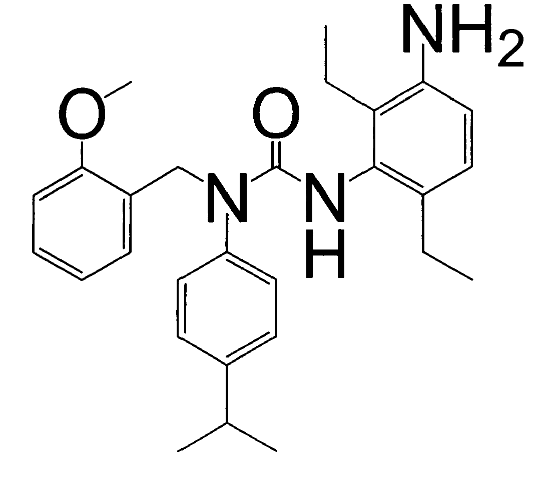 | **63** | 446.3 | 446.7 | C | 3-(3-Amino-2,6-diethyl-phenyl)-1-(4-isopropyl-phenyl)-1-(2-methoxy-benzyl)-harnstoff |
| 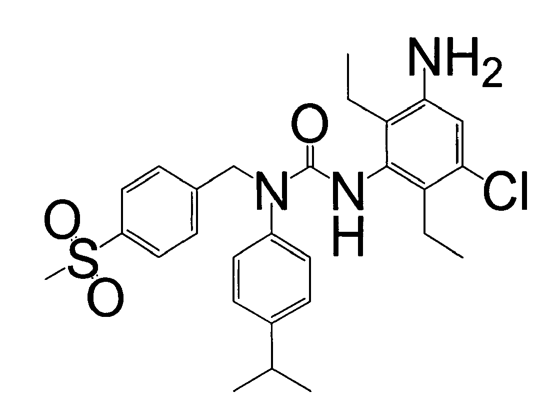 | **64** | 528.2 | 528.1 | C | 3-(3-Amino-5-chlor-2,6-diethyl-phenyl)-1-(4-i sopropyl-phenyl)-1-(4-methansulfonyl-benzyl)-harnstoff |
| 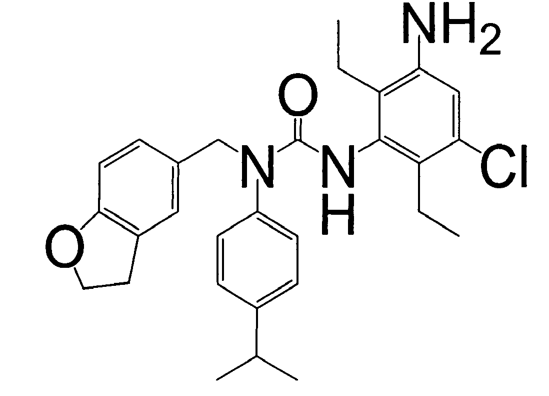 | **65** | 492.2 | 492.1 | B | 3-(3-Amino-5-chlor-2,6-diethyl-phenyl)-1-(2,3-dihydro-benzofuran-5-ylmethyl)-1-(4-isopropyl-phenyl)-harnstoff |
| 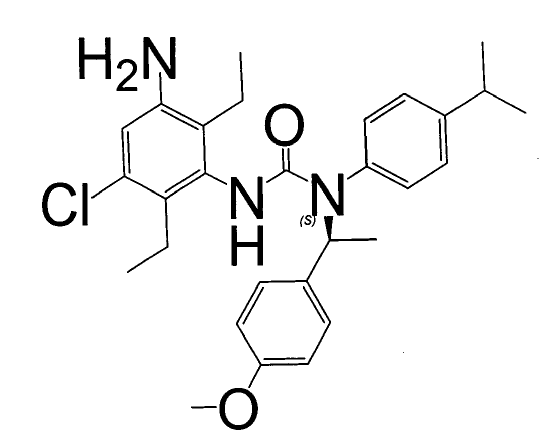 | **66** | 494.3 | 494.1 | A, 55 nM | (S)-3-(3-Amino-5-chlor-2,6-diethyl-phenyl)-1-(4-isopropyl-phenyl)-1-[1-(4-methoxy-phenyl)-ethyl]-harnstoff |
| 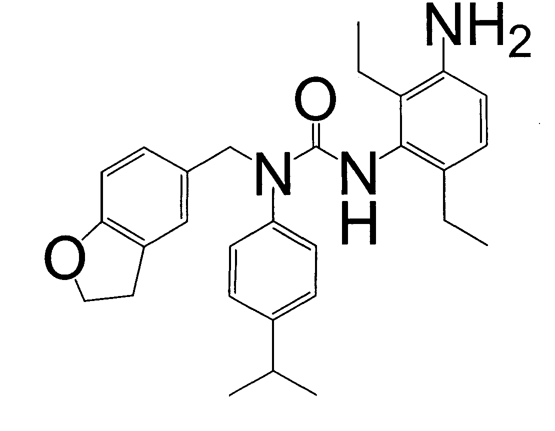 | **67** | 458.3 | 458.2 | C | 3-(3-Amino-2,6-diethyl-phenyl)-1-(2,3-dihydro-benzofuran-5-ylmethyl)-1-(4-isopropyl-phenyl)-harnstoff |
| 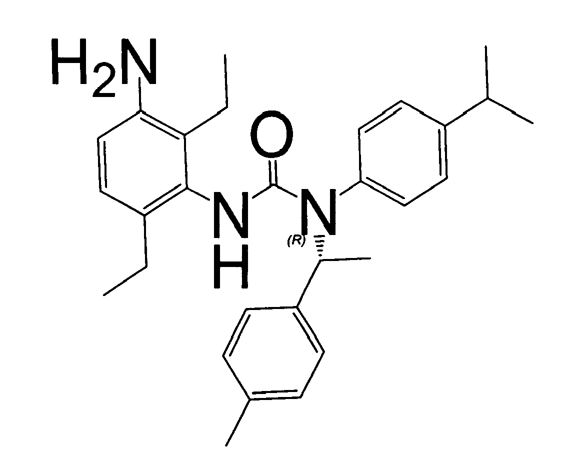 | **68** | 444.3 | 444.9 | C | (R)-3-(3-Amino-2,6-diethyl-phenyl)-1-(4-isopropyl-phenyl)-1-(1-p-tolyl-ethyl)-harnstoff |
| 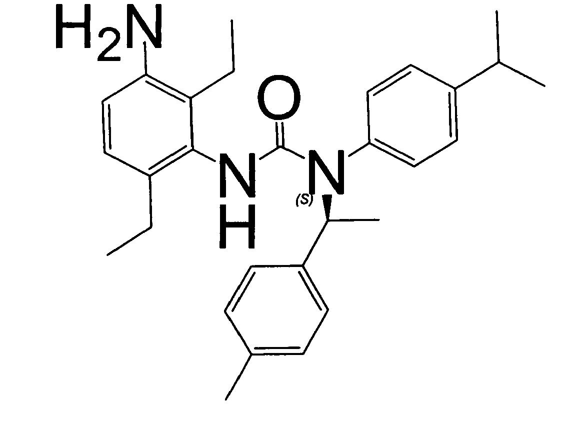 | **69** | 444.3 | 444.8 | B | (S)-3-(3-Amino-2,6-diethyl-phenyl)-1-(4-isopropyl-phenyl)-1-(1-p-tolyl-ethyl)-harnstoff |
| 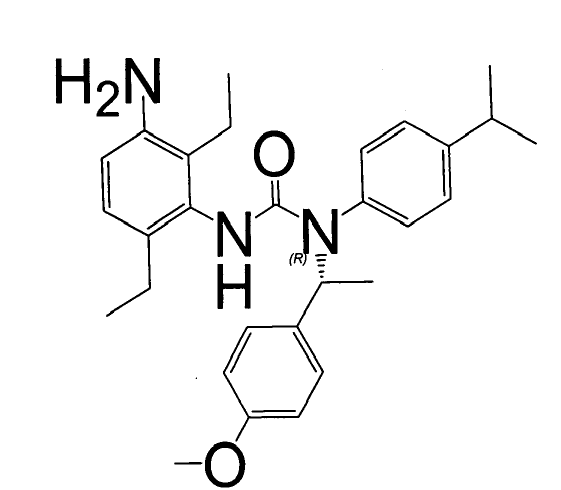 | **70** | 460.3 | 461.0 | C | (R)-3-(3-Amino-2,6-diethyl-phenyl)-1-(4-isopropyl-phenyl)-1-[1-(4-methoxy-phenyl)-ethyl]-harnstoff |
| 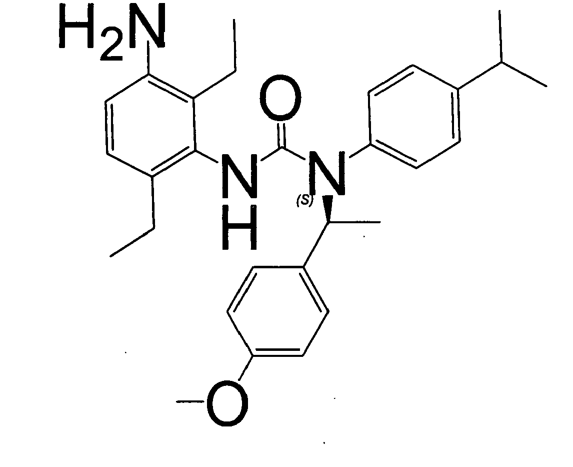 | **71** | 460.3 | 462.3 | B | (S)-3-(3-Amino-2,6-diethyl-phenyl)-1-(4-isopropyl-phenyl)-1-[1-(4-methoxy-phenyl)-ethyl]-harnstoff |
| 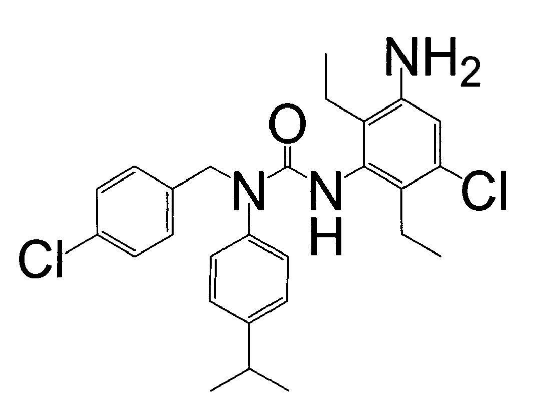 | **72** | 484.2 | 484.1 | B | 3-(3-Amino-5-chlor-2,6-diethyl-phenyl)-1-(4-chlor-benzyl)-1-(4-isopropyl-phenyl)-harnstoff |
| 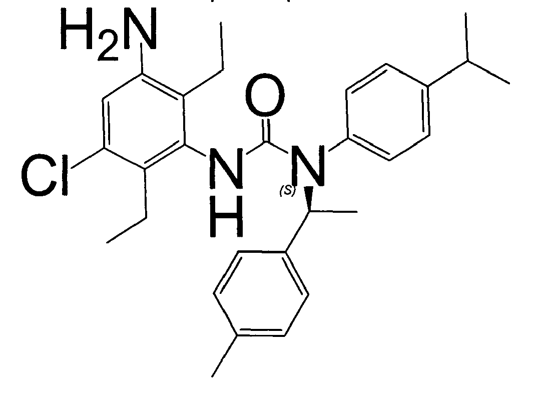 | **73** | 478.3 | 478.9 | A, 32 nM | (S)-3-(3-Amino-5-chlor-2,6-diethyl-phenyl)-1-(4-isopropyl-phenyl)-1-(1-p-tolyl-ethyl)-harnstoff |
| 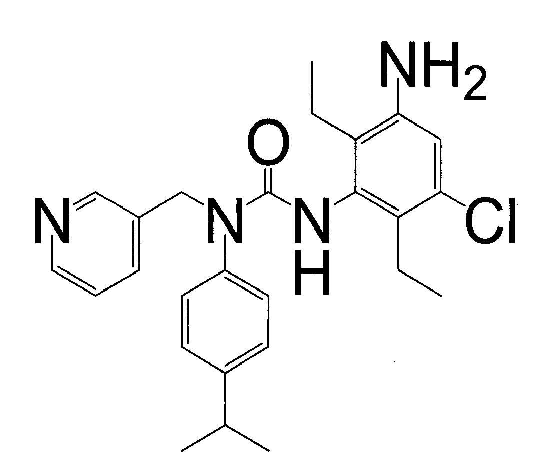 | **74** | 451.2 | 451.3 | C | 3-(3-Amino-5-chlor-2,6-diethyl-phenyl)-1-(4-isopropyl-phenyl)-1-pyridin-3-ylmethyl-harnstoff |
| 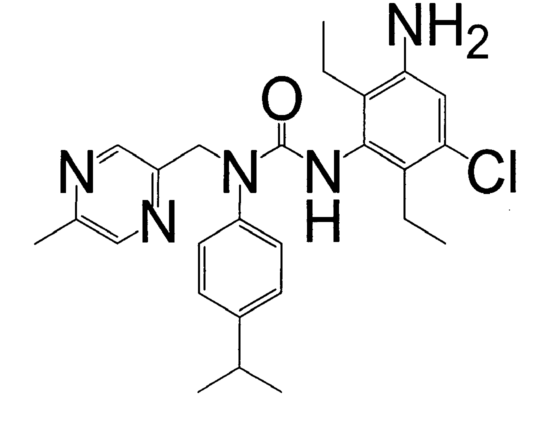 | **75** | 466.2 | 466.1 | B | 3-(3-Amino-5-chlor-2,6-diethyl-phenyl)-1-(4-isopropyl-phenyl)-1-(5-methyl-pyrazin-2-ylmethyl)-harnstoff |
| 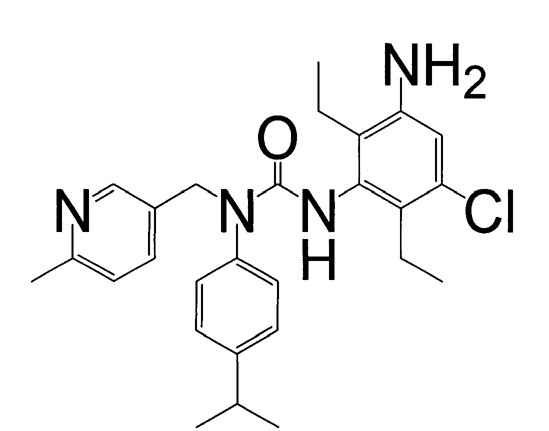 | **76** | 465.2 | 465.2 | B | 3-(3-Amino-5-chlor-2,6-diethyl-phenyl)-1-(4-isopropyl-phenyl)-1-(6-methyl-pyridin-3-ylmethyl)-harnstoff |
| 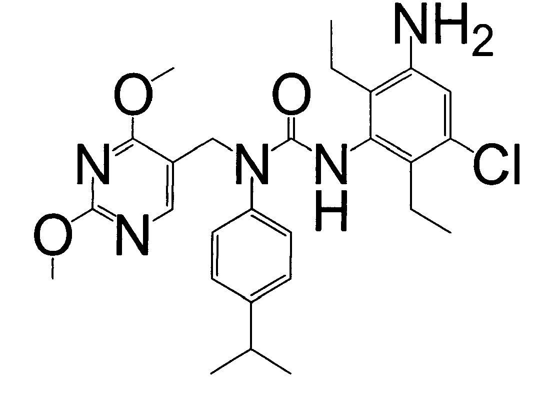 | **77** | 512.2 | 512.3 | C | 3-(3-Amino-5-chlor-2,6-diethyl-phenyl)-1-(2,4-dimethoxy-pynmidin-5-ylmethyl)-1-(4-isopropyl-phenyl)-harnstoff |
| 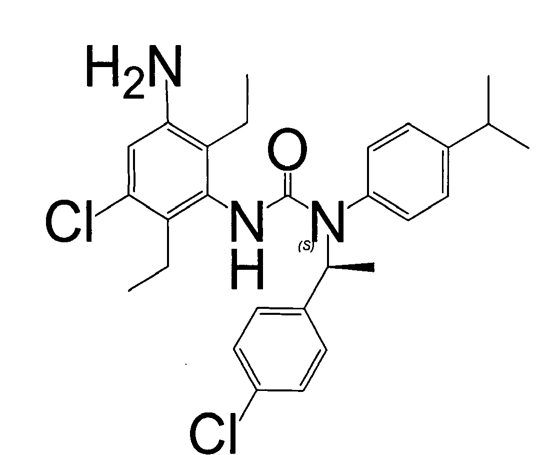 | **78** | 498.2 | 498.9 | A | (S)-3-(3-Amino-5-chlor-2,6-diethyl-phenyl)-1-[1-(4-chlor-phenyl)-ethyl]-1-(4-i sopropyl-phenyl)-harnstoff |
| 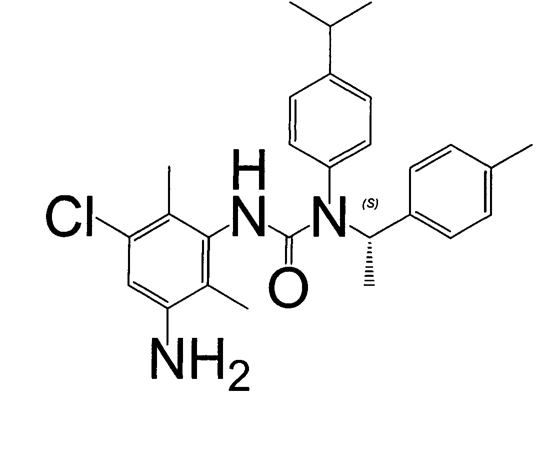 | **79** | 450.2 | 450.8 | C | (S)-3-(3-Amino-5-chlor-2,6-dimethyl-phenyl)-1-(4-isopropyl-phenyl)-1-(1-p-tolyl-ethyl)-harnstoff |
| 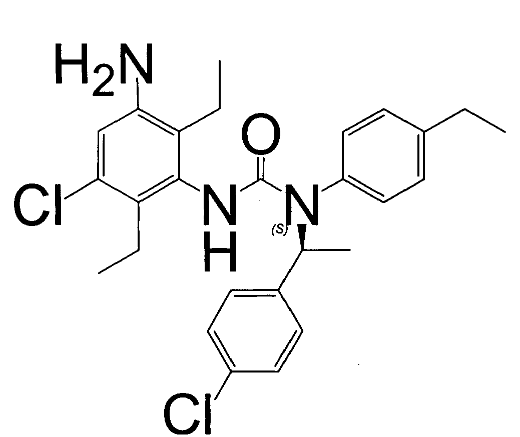 | **80** | 484.2 | 484.9 | A | (S)-3-(3-Amino-5-chlor-2,6-diethyl-phenyl)-1-[1-(4-chlor-phenyl)-ethyl]-1-(4-ethyl-phenyl)-harnstoff |
| 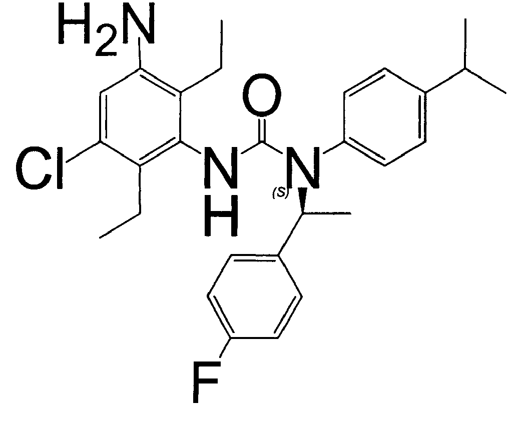 | **81** | 482.2 | 483.0 | A, 38 nM | (S)-3-(3-Amino-5-chlor-2,6-diethyl-phenyl)-1-[1-(4-fluor-phenyl)-ethyl]-1-(4-isopropyl-phenyl)-harnstoff |
| 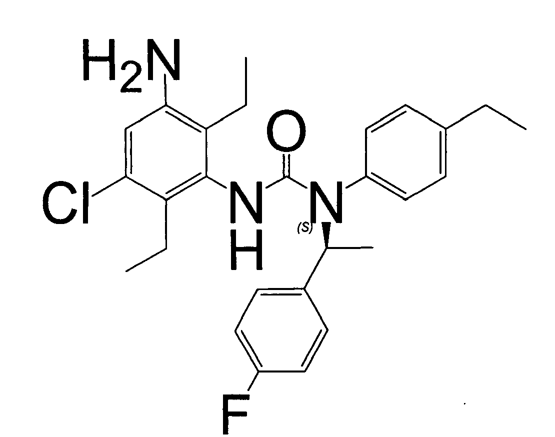 | **82** | 468.2 | 468.9 | B | (S)-3-(3-Amino-5-chlor-2,6-diethyl-phenyl)-1-(4-ethyl-phenyl)-1-[1-(4-fluor-phenyl)-ethyl]-harnstoff |
| 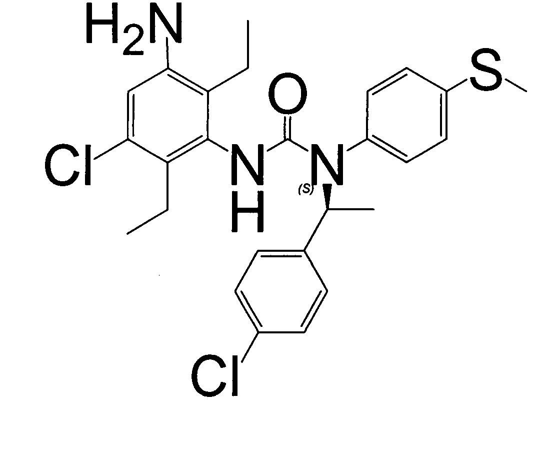 | **83** | 502.1 | 503.1 | B | (S)-3-(3-Amino-5-chlor-2,6-diethyl-phenyl)-1-[1-(4-chlor-phenyl)-ethyl]-1-(4-methylsulfanyl-phenyl)-harnstoff |
| 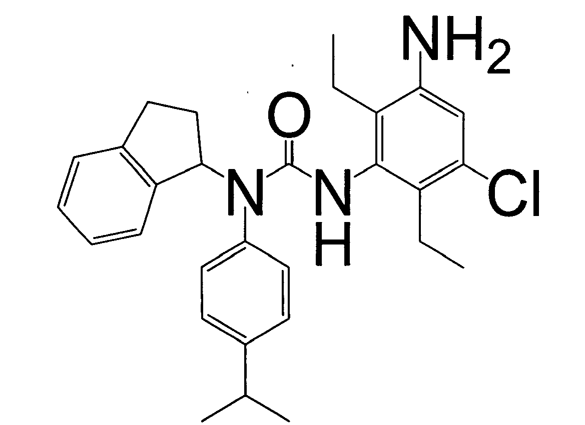 | **84** | 476.2 | 476.8 | C | 3-(3-Amino-5-chlor-2,6-diethyl-phenyl)-1-indan-1-yl-1-(4-isopropyl-phenyl)-harnstoff |
| 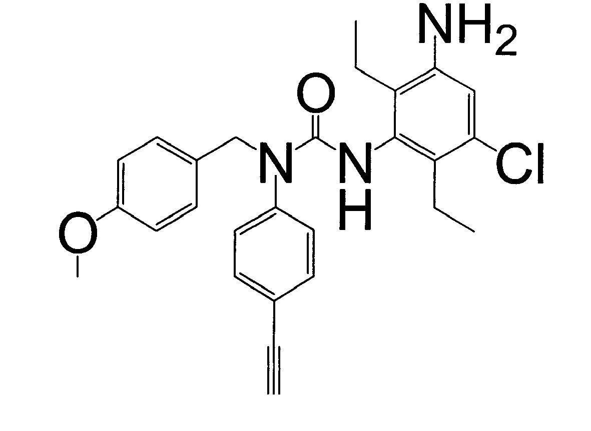 | **85** | 462.2 | 462.5 | C | 3-(3-Amino-5-chlor-2,6-diethyl-phenyl)-1-(4-ethynyl-phenyl)-1-(4-methoxy-benzyl)-harnstoff |
| 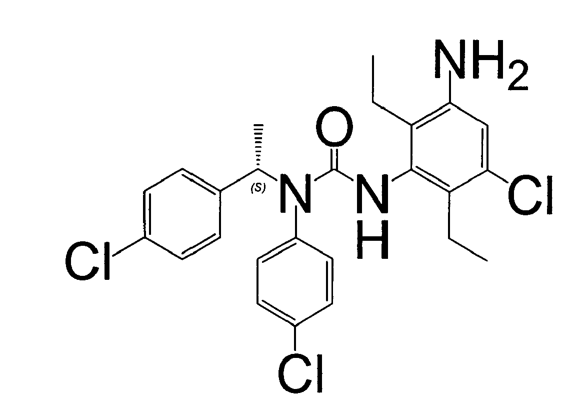 | **86** | 490.1 | 490.9 | C | (S)-3-(3-Amino-5-chlor-2,6-diethyl-phenyl)-1-(4-chlor-phenyl)-1-[1-(4-chlor-phenyl)-ethyl]-harnstoff |
| 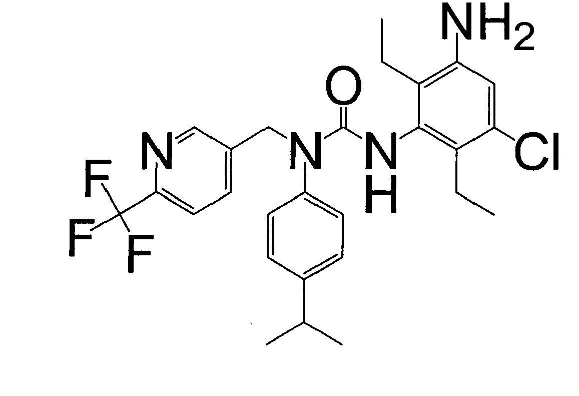 | **87** | 519.2 | 519.2 | C | 3-(3-Amino-5-chlor-2,6-diethyl-phenyl)-1-(4-i sopropyl-phenyl)-1-(6-trifluormethyl-pyridin-3-ymethyl)-harnstoff |
| 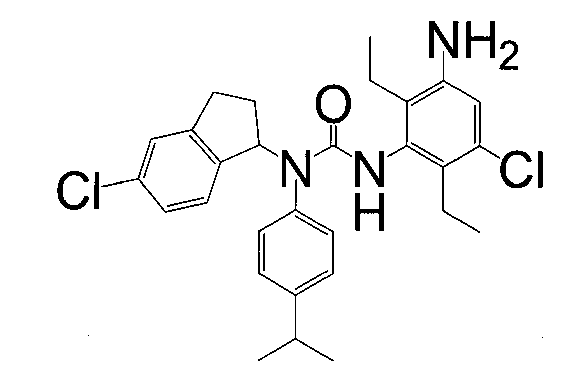 | **88** | S 10.2 | 510.0 | B | 3-(3-Amino-5-chlor-2,6-diethyl-phenyl)-1-(5-chlor-indan-1-yl)-1-(4-i sopropyl-phenyl)-harnstoff |
| 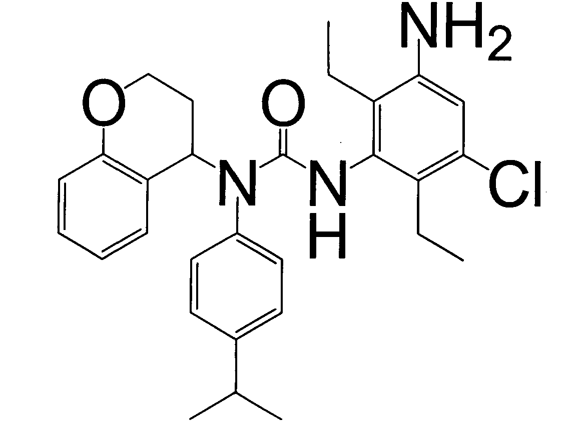 | **89** | 492.2 | 492.1 | C | 3-(3-Amino-5-chlor-2,6-diethyl-phenyl)-1-chroman-4-yl-1-(4-isopropyl-phenyl)-harnstoff |
| 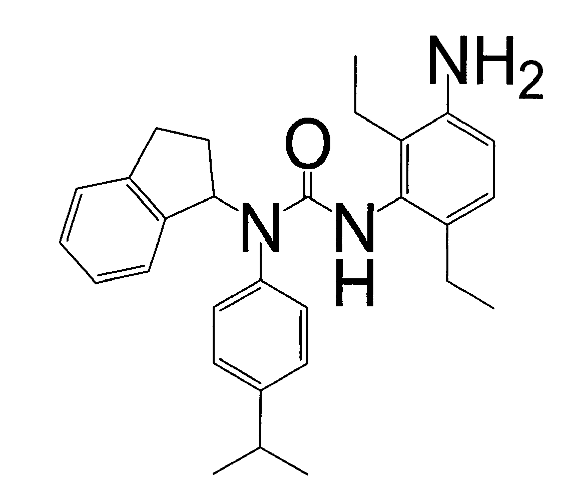 | **90** | 442.3 | 442.1 | C | 3-(3-Amino-2,6-diethyl-phenyl)-1-indan-1-yl-1-(4-isopropyl-phenyl)-harnstoff |
| 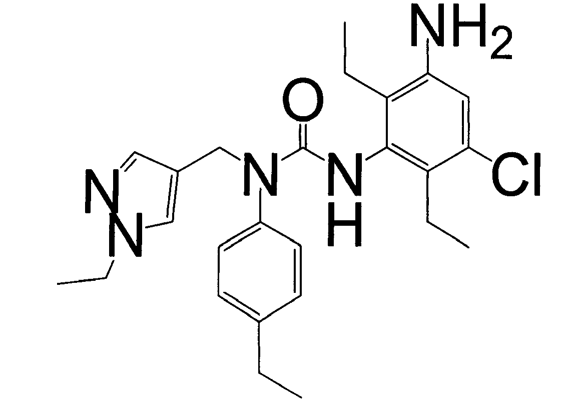 | **91** | 454.2 | 454.9 | C | 3-(3-Amino-5-chlor-2,6-diethyl-phenyl)-1-(4-ethyl-phenyl)-1-(1-ethyl-1H-pyrazol-4-ylmethyl)-harnstoff |
| 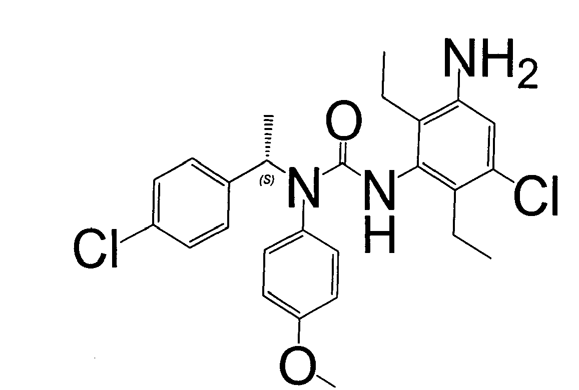 | **92** | 486.2 | 486.9 | B | (S)-3-(3-Amino-5-chlor-2,6-diethyl-phenyl)-1-[1-(4-chlor-phenyl)-ethyl]-1-(4-methoxy-phenyl)-harnstoff |
| 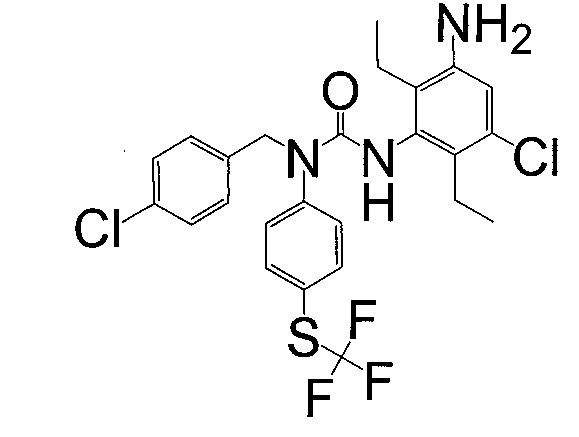 | **93** | 542.1 | 543.1 | C | 3-(3-Amino-5-chlor-2,6-diethyl-phenyl)-1-(4-chlor-b enzyl)-1-(4-trifluormethylsulfanyl-phenyl)-harnstoff |
| 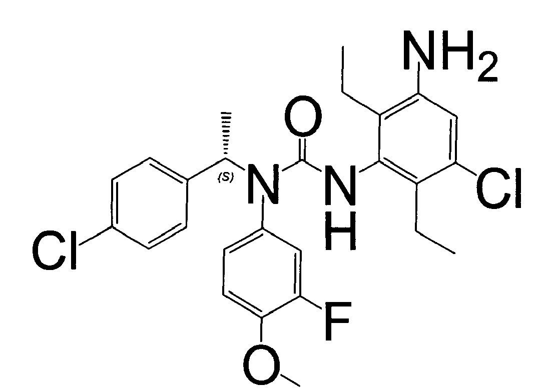 | **94** | 504.2 | 505.0 | A | (S)-3-(3-Amino-5-chlor-2,6-diethyl-phenyl)-1-[1-(4-chlor-phenyl)-ethyl]-1-(3-fluor-4-methoxy-phenyl)-harnstoff |
| 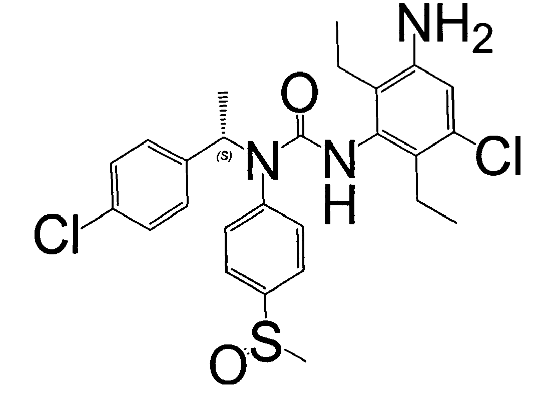 | **95** | 518.1 | 518.5 | C | (S)-3-(3-Amino-5-chlor-2,6-diethyl-phenyl)-1-[1-(4-chlor-phenyl)-ethyl]-1-(4-methansulfinyl-phenyl)-harnstoff |
| 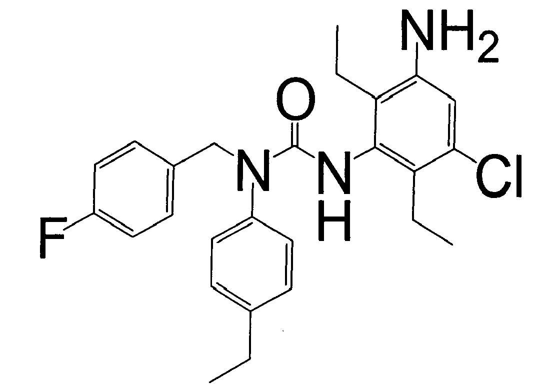 | **96** | 454.2 | 454.1 | B | 3-(3-Amino-5-chlor-2,6-diethyl-phenyl)-1-(4-ethyl-phenyl)-1-(4-fluor-benzyl)-harnstoff |
| 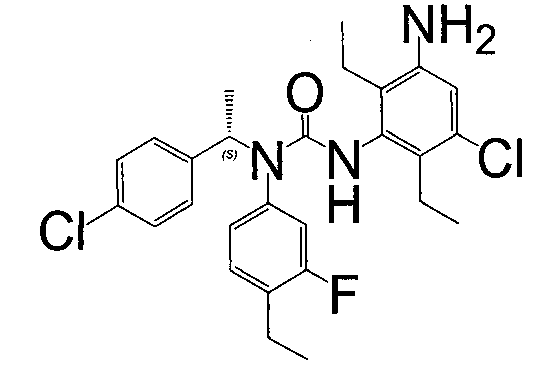 | **97** | 502.2 | 502.5 | A, 35 nM | (S)-3-(3-Amino-5-chlor-2,6-diethyl-phenyl)-1-[1-(4-chlor-phenyl)-ethyl]-1-(4-ethyl-3-fluor-phenyl)-harnstoff |
| 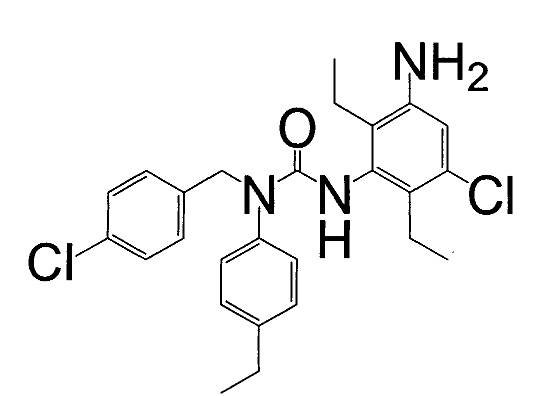 | **98** | 470.2 | 470.2 | B, 86 nM | 3-(3-Amino-5-chlor-2,6-diethyl-phenyl)-1-(4-chlor-benzyl)-1-(4-ethyl-phenyl)-harnstoff |
| 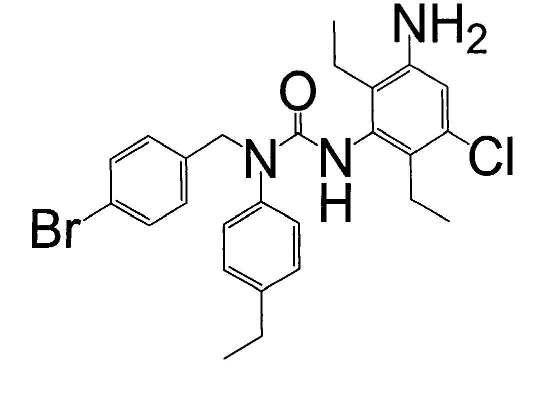 | **99** | 514.1 | 514.1 | A | 3-(3-Amino-5-chlor-2,6-diethyl-phenyl)-1-(4-brom-benzyl)-1-(4-ethyl-phenyl)-harnstoff |
| 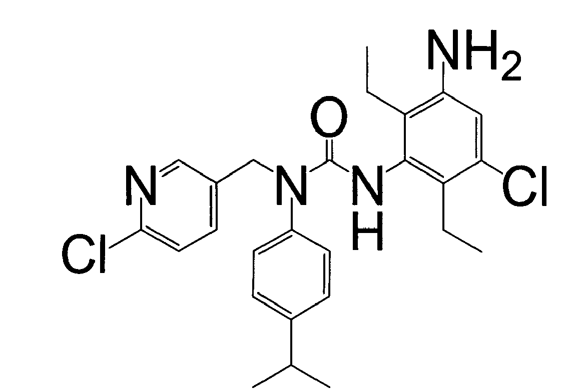 | **100** | 485.2 | 485.2 | B, 62 nM | 3-(3-Amino-5-chlor-2,6-diethyl-phenyl)-1-(6-chlor-pyridin-3-ylmethyl)-1-(4-isopropyl-phenyl)-harnstoff |
| 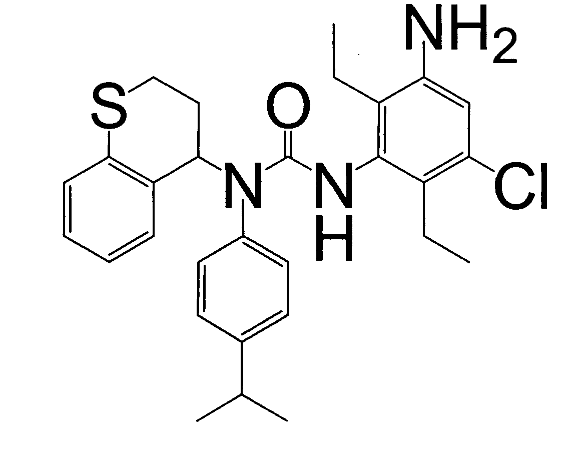 | **101** | 508.2 | 508.7 | C | 3-(3-Amino-5-chlor-2,6-diethyl-phenyl)-1-(4-isopropyl-phenyl)-1-thiochroman-4-yl-harnstoff |
| 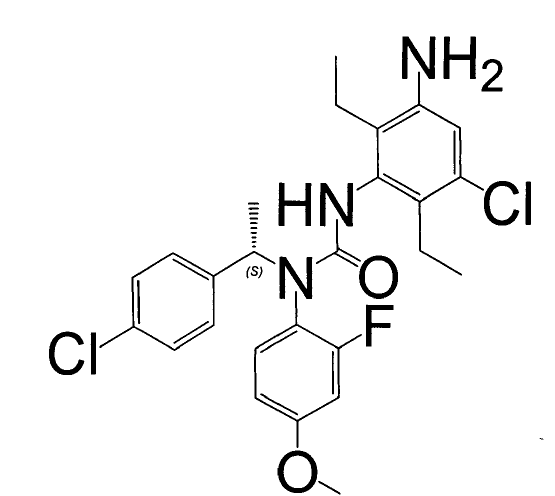 | **102** | 504.2 | 504.9 | A, 31 nM | (S)-3-(3-Amino-5-chlor-2,6-diethyl-phenyl)-1-[1-(4-chlor-phenyl)-ethyl]-1-(2-fluor-4-methoxy-phenyl)-hamstoff |
| 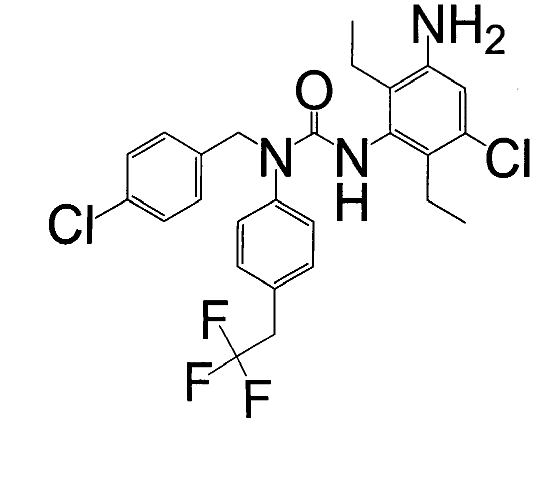 | **103** | 524.1 | 524.1 | B | 3-(3-Amino-S-chlor-2,6-diethyl-phenyl)-1-(4-chlor-benzyl)-1-[4-(2,2,2-trifluor-ethyl)-phenyl]-hamstoff |
| 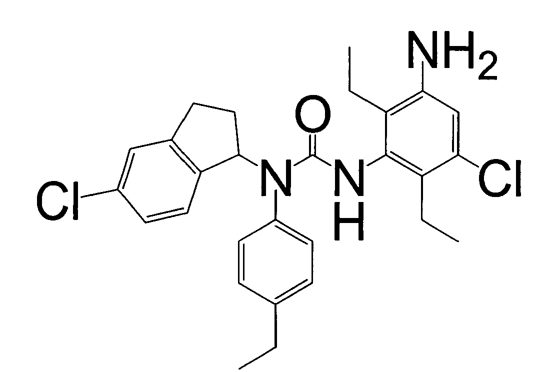 | **104** | 496.2 | 496.1 | B | 3-(3-Amino-5-chlor-2,6-diethyl-phenyl)-1-(5-chlor-indan-1-yl)-1-(4-ethyl-phenyl)-harnstoff |
| 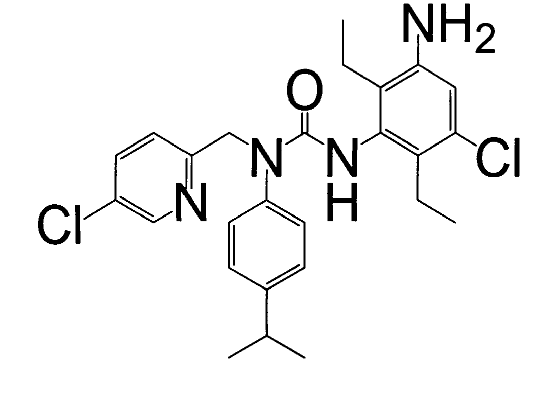 | **105** | 485.2 | 485.1 | C | 3-(3-Amino-5-chlor-2,6-diethyl-phenyl)-1-(5-chlor-pyridin-2-ylmethyl)-1-(4-isopropyl-phenyl)-harnstoff |
| 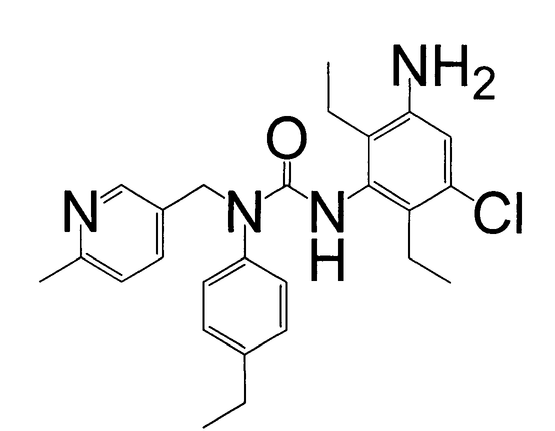 | **106** | 451.2 | 451.8 | B | 3-(3-Amino-5-chlor-2,6-diethyl-phenyl)-1-(4-ethyl-phenyl)-1-(6-methyl-pyridin-3-ylmethyl)-harnstoff |
| 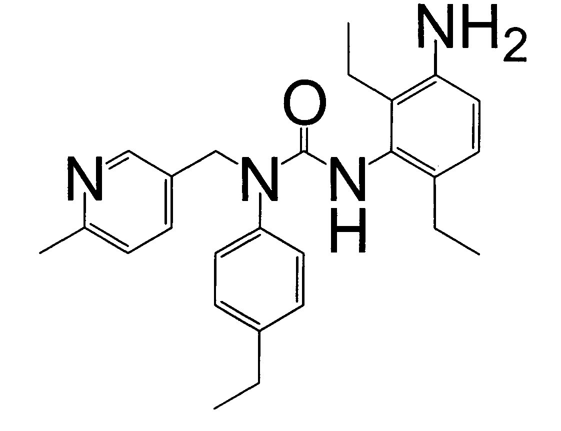 | **107** | 417.3 | 417.3 | C | 3-(3-Amino-2,6-diethyl-phenyl)-1-(4-ethyl-phenyl)-1-(6-methyl-pyridin-3-ylmethyl)-harnstoff |
| 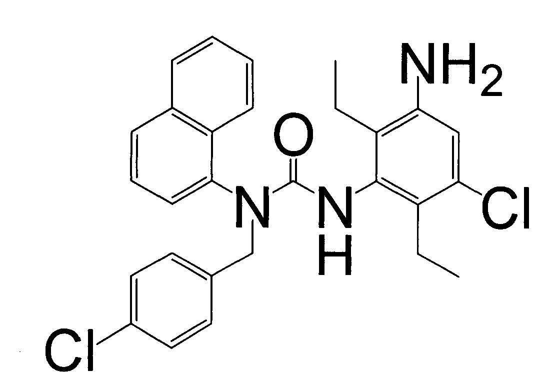 | **108** | 492.2 | 492.1 | C | 3-(3-Amino-5-chlor-2,6-diethyl-phenyl)-1-(4-chlor-benzyl)-1-naphthalin-1-yl-harnstoff |
| 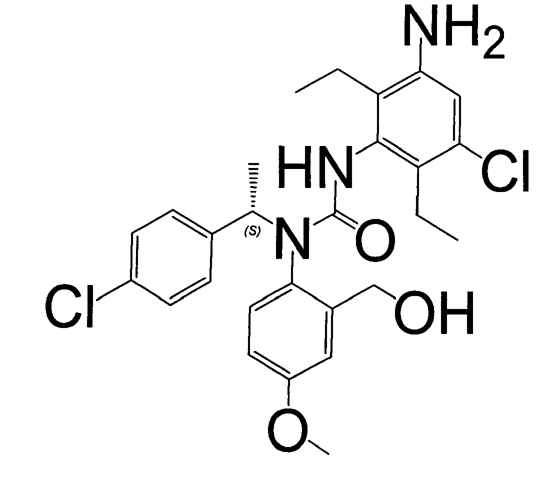 | **109** | 516.2 | 516.7 | C | (S)-3-(3-Amino-5-chlor-2,6-diethyl-phenyl)-1-[1-(4-chlor-phenyl)-ethyl]-1-(2-hydroxymethyl-4-methoxy-phenyl)-harnstoff |
| 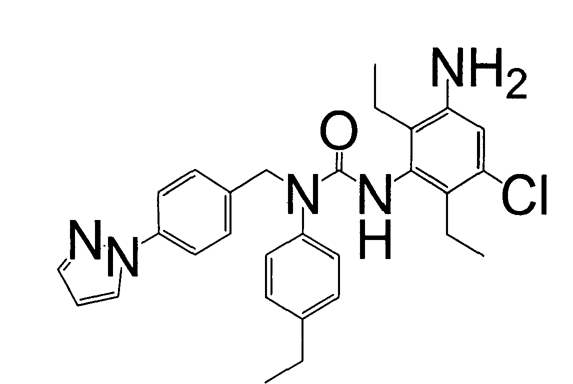 | **110** | 502.2 | 502.1 | C | 3-(3-Amino-5-chlor-2,6-diethyl-phenyl)-1-(4-ethyl-phenyl)-1-(4-pyrazol-1-yl-benzyl)-harnstoff |
| 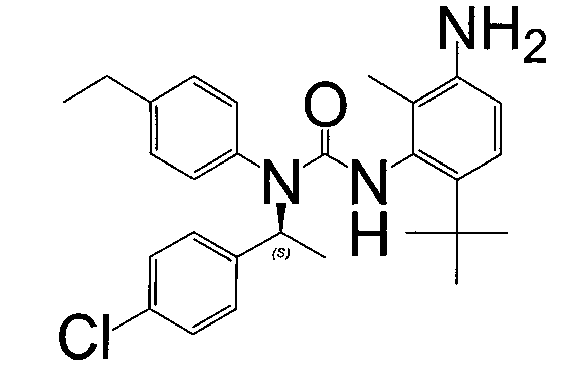 | **111** | 464.2 | 464.0 | C | (S)-3-(3-Amino-6-tert-butyl-2-methyl-phenyl)-1-[1-(4-chlor-phenyl)-ethyl]-1-(4-ethyl-phenyl)-harnstoff |
| 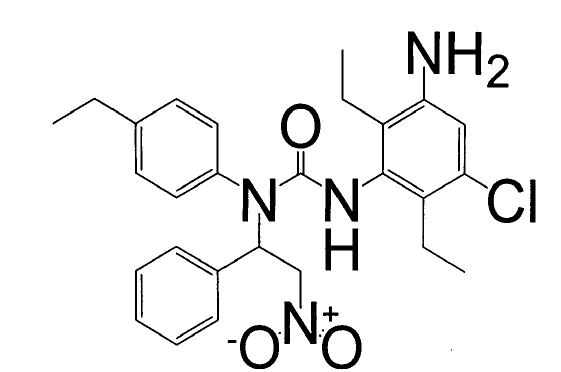 | **112** | 495.2 | 495.6 | C | 3-(3-Amino-5-chlor-2,6-diethyl-phenyl)-1-(4-ethyl-phenyl)- 1 -(2-nitro- 1 -phenyl-ethyl)-harnstoff |
| 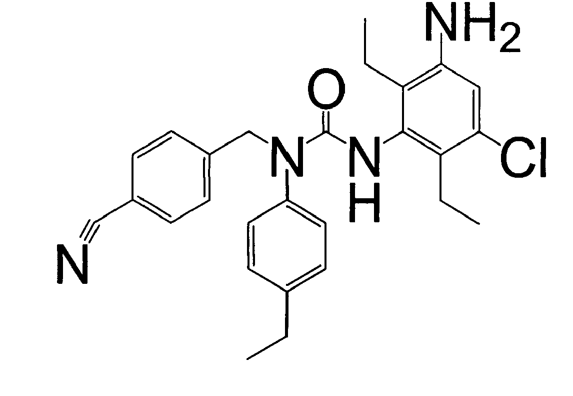 | **113** | 461.2 | 461.1 | C | 3-(3-Amino-5-chlor-2,6-diethyl-phenyl)-1-(4-cyano-benzyl)-1-(4-ethyl-phenyl)-harnstoff |
| 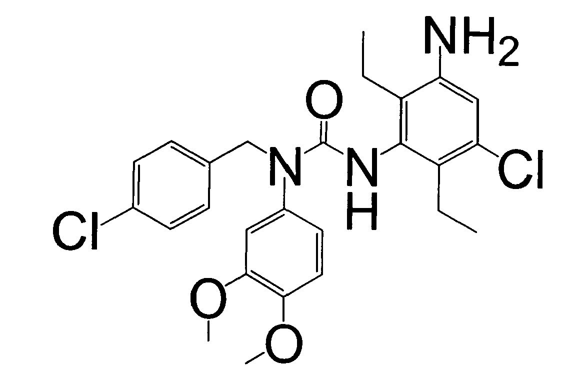 | **114** | 502.2 | 502.1 | C | 3-(3-Amino-5-chlor-2,6-diethyl-phenyl)-1-(4-chlor-benzyl)-1-(3,4-dimethoxy-phenyl)-harnstoff |
| 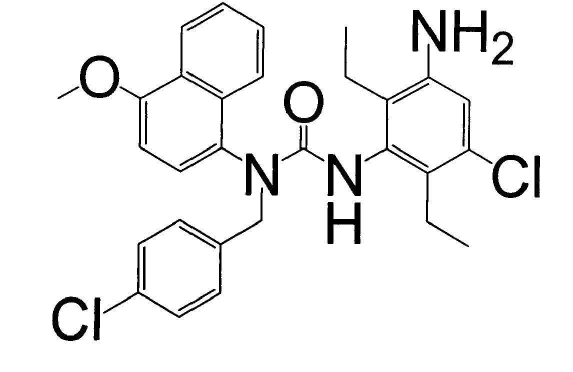 | **115** | 522.2 | 522.5 | B | 3-(3-Amino-5-chlor-2,6-diethyl-phenyl)-1-(4-chlor-benzyl)-1-(4-methoxy-naphthalin-1-yl)-harnstoff |
| 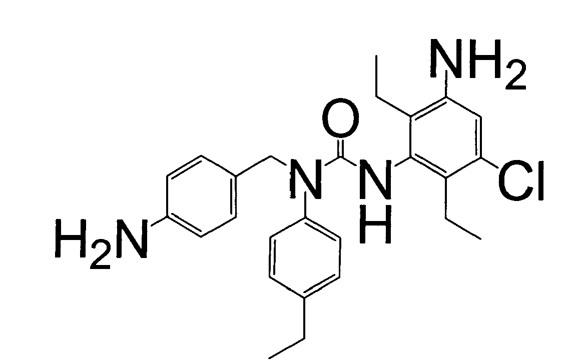 | **116** | 451.2 | 451.8 | C | 1-(4-Amino-benzyl)-3-(3-amino-5-chlor-2,6-diethyl-phenyl)-1-(4-ethyl-phenyl)-harnstoff |
| 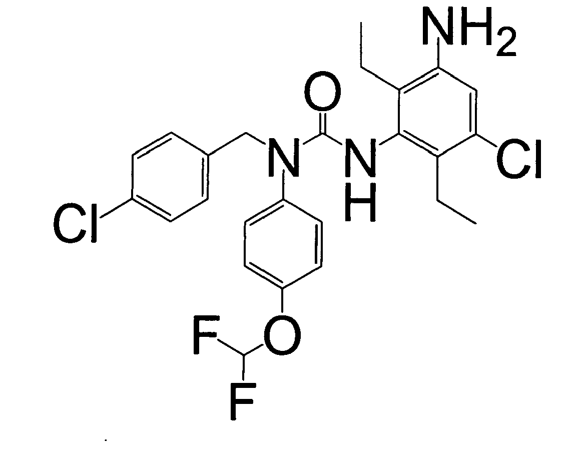 | **117** | 508.1 | 508.9 | C | 3-(3-Amino-5-chlor-2,6-diethyl-phenyl)-1-(4-chlor-benzyl)-1-(4-difluormethoxy-phenyl)-harnstoff |
| 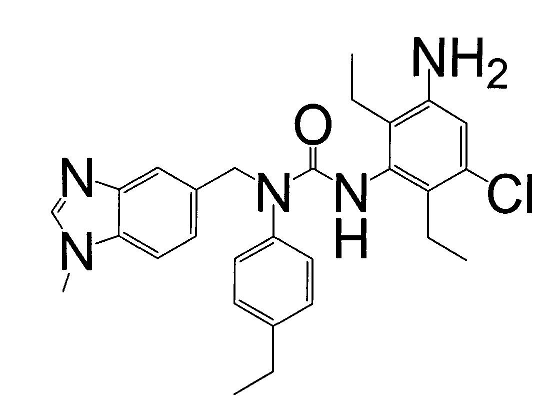 | **118** | 490.2 | 490.2 | C | 3-(3-Amino-5-chlor-2,6-diethyl-phenyl)-1-(4-ethyl-phenyl)-1-(1-methyl-1H-benzoimidazol-5-ylmethyl)-harnstoff |
| 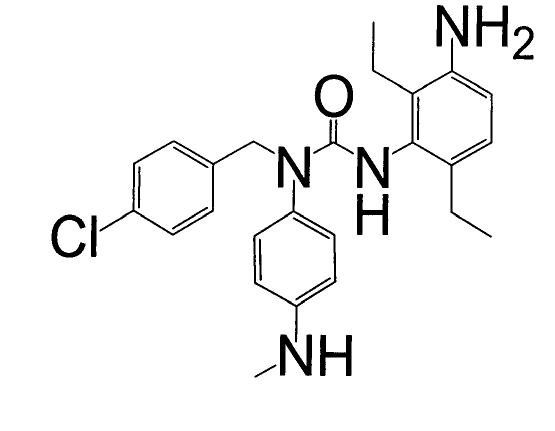 | **119** | 437.2 | 437.1 | B | 3-(3-Amino-2,6-diethyl-phenyl)-1-(4-chlor-benzyl)-1-(4-methylamino-phenyl)-harnstoff |
| 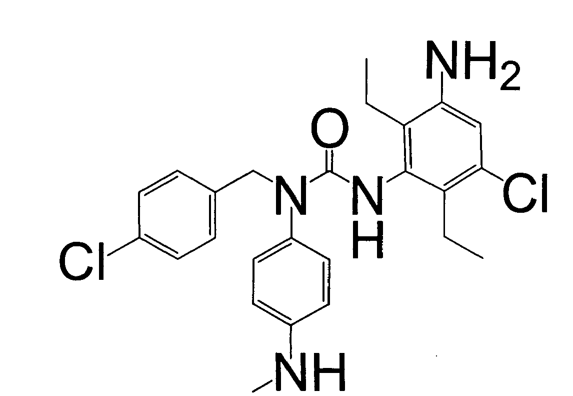 | **120** | 471.2 | 471.1 | A, 19 nM | 3-(3-Amino-5-chlor-2,6-diethyl-phenyl)-1-(4-chlor-benzyl)-1-(4-methylamino-phenyl)-harnstoff |
| 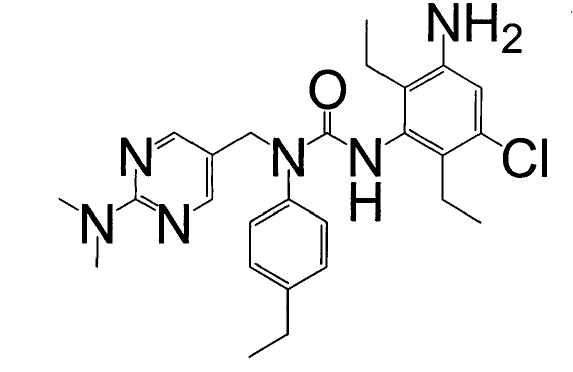 | **121** | 481.2 | 481.3 | C | 3-(3-Amino-5-chlor-2,6-diethyl-phenyl)-1-(2-dimethylamino-pyrimidin-5-ylmethyl)-1-(4-ethyl-phenyl)-harnstoff |
| 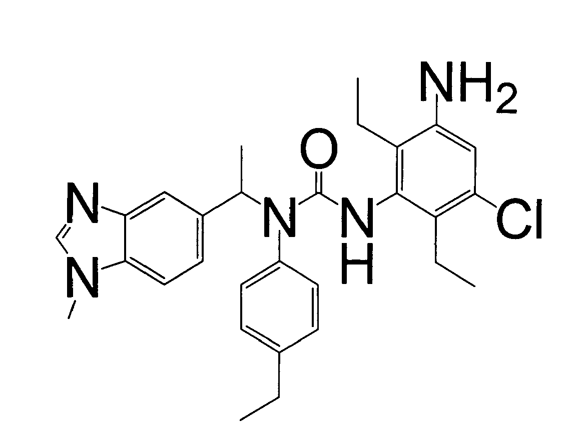 | **122** | 504.3 | 504.3 | B | 3-(3-Amino-5-chlor-2,6-diethyl-phenyl)-1-(4-ethyl-phenyl)-1-[1-(1-methyl-1H-benzoimidazol-5-yl)-ethyl]-hamstoff |
| 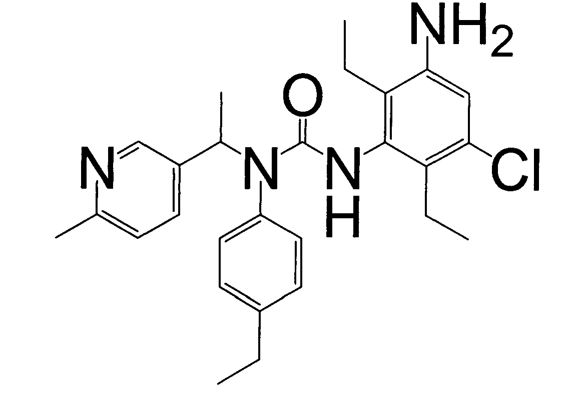 | **123** | 465.2 | 465.3 | B | 3-(3-Amino-5-chlor-2,6-diethyl-phenyl)-1-(4-ethyl-phenyl)-1-[1-(6-methyl-pyridin-3-yl)-ethyl]-harnstoff |
| 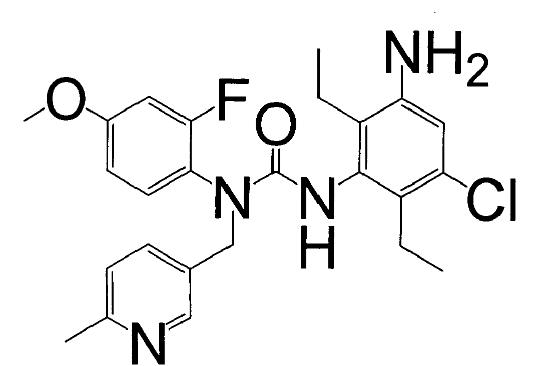 | **124** | 471.2 | 471.4 | C | 3-(3-Amino-5-chlor-2,6-diethyl-phenyl)-1-(2-fluor-4-methoxy-phenyl)-1-(6-methyl-pyridin-3-ylmethyl)-harnstoff |
| 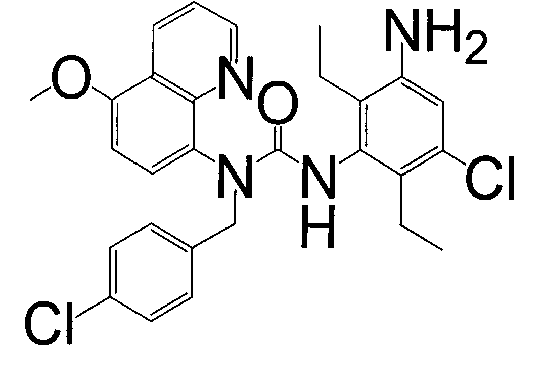 | **125** | 523.2 | 523.0 | B | 3-(3-Amino-5-chlor-2,6-diethyl-phenyl)-1-(4-chlor-benzyl)-1-(5-methoxy-chinolin-8-yl)-harnstoff |
| 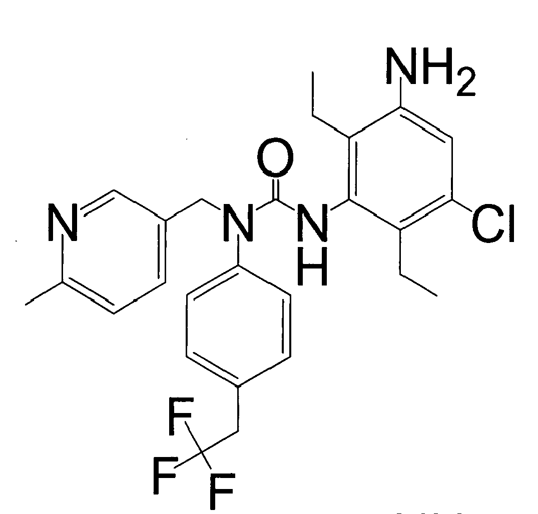 | **126** | 505.2 | 505.3 | B | 3-(3-Amino-5-chlor-2,6-diethyl-phenyl)-1-(6-methyl-pyridin-3-ylmethyl)-1-[4-(2,2,2-trifluor-ethyl)-phenyl]-harnstoff |
| 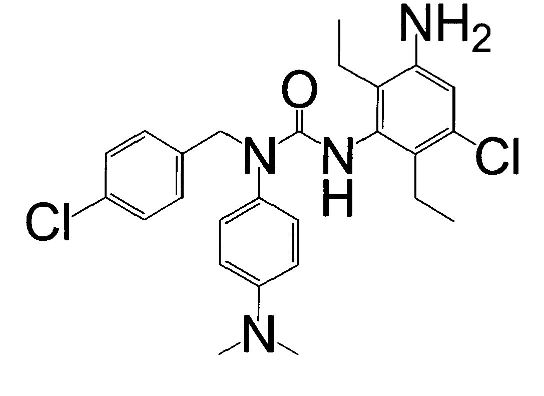 | **127** | 485.2 | 485.6 | C | 3-(3-Amino-5-chlor-2,6-diethyl-phenyl)-1-(4-chlor-benzyl)-1-(4-dimethylamino-phenyl)-harnstoff |
| 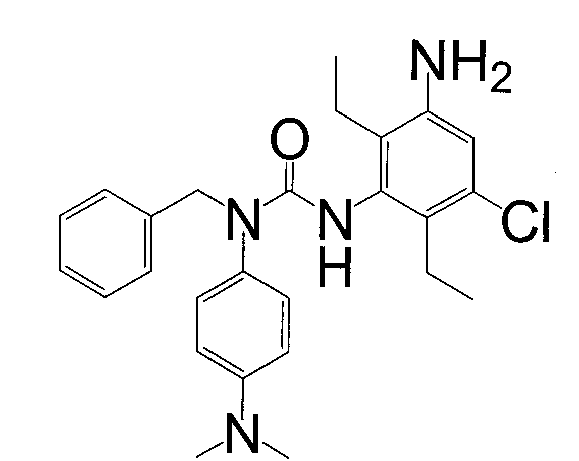 | **128** | 451.2 | 452.0 | C | 3-(3-Amino-5-chlor-2,6-diethyl-phenyl)-1-benzyl-1-(4-dimethylamino-phenyl)-harnstoff |
| 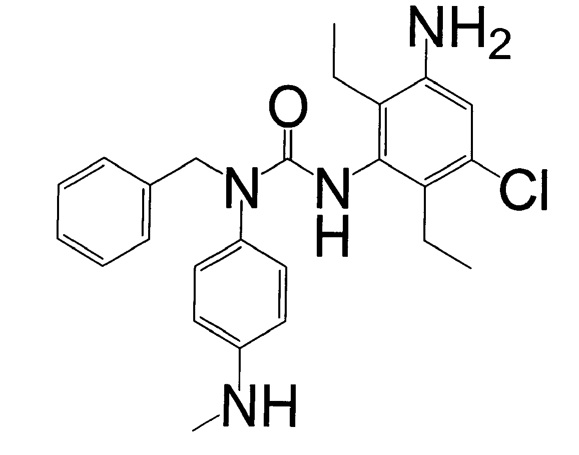 | **129** | 437.2 | 437.1 | B | 3-(3-Amino-5-chlor-2,6-diethyl-phenyl)-1-benzyl-1-(4-methylamino-phenyl)-harnstoff |
| 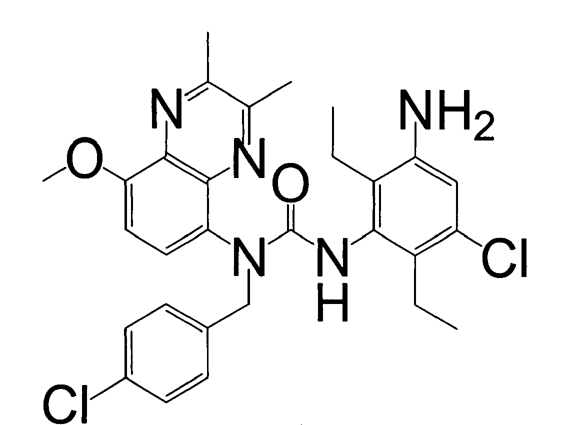 | **130** | 552.2 | 552.1 | C | 3-(3-Amino-5-chlor-2,6-diethyl-phenyl)-1-(4-chlor-benzyl)-1-(8-methoxy-2,3-dimethyl-chinoxalin-5-yl)-harnstoff |
| 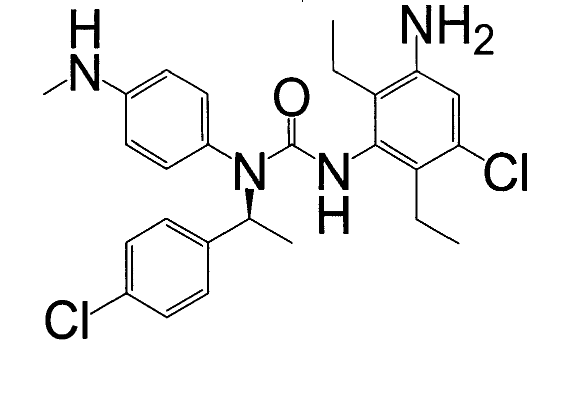 | **131** | 485.2 | 485.8 | A, 3 nM | (S)-3-(3-Amino-5-chlor-2,6-diethyl-phenyl)-1-[1-(4-chlor-phenyl)-ethyl]-1-(4-methylamino-phenyl)-harnstoff |
| 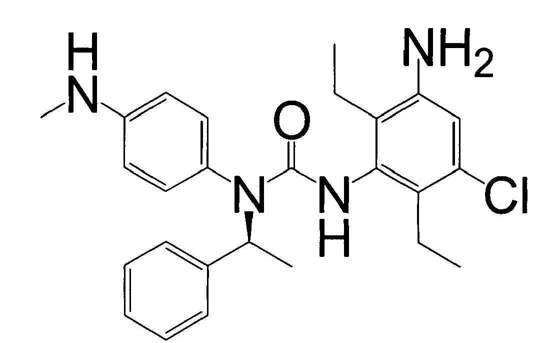 | **132** | 451.2 | 451.7 | A | (S)-3-(3-Amino-5-chlor-2,6-diethyl-phenyl)-1-(4-methylamino-phenyl)-1-(1-phenyl-ethyl)-harnstoff |
| 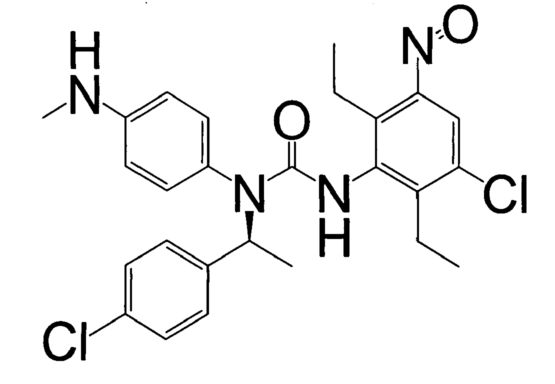 | **133** | 499.2 | 499.7 | A | (S)-3-(3-Chlor-2,6-diethyl-5-nitroso-phenyl)-1-[1-(4-chlor-phenyl)-ethyl]-1-(4-methylamino-phenyl)-harnstoff |
| 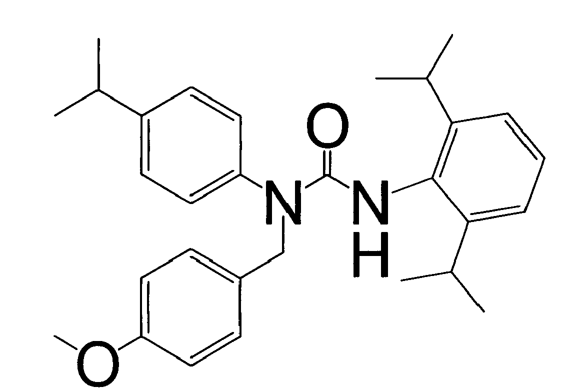 | **134** | 459.3 | 459.2 | C | 3-(2,6-Diisopropyl-phenyl)- 1 -(4-isopropyl-phenyl)-1-(4-methoxy-benzyl)-harnstoff |
| 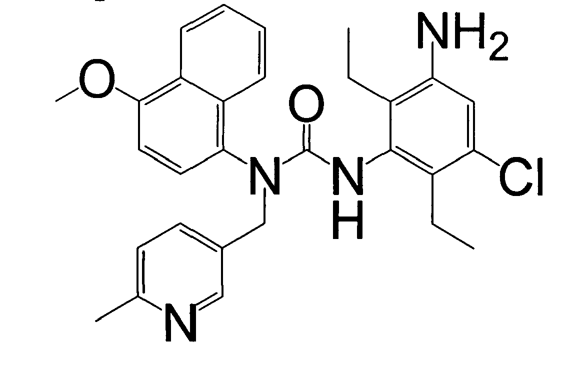 | **135** | 503.2 | 503.2 | A, 55 nM | 3-(3-Amino-5-chlor-2,6-diethyl-phenyl)-1-(4-methoxy-naphthalin-1-yl)-1-(6-methyl-pyridin-3-ylmethyl)-harnstoff |
| 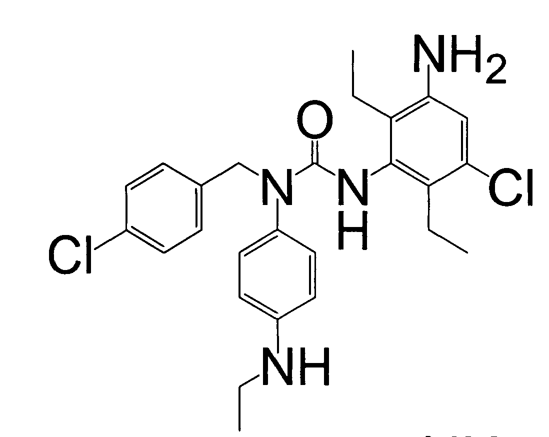 | **136** | 485.2 | 485.8 | A | 3-(3-Amino-5-chlor-2,6-diethyl-phenyl)-1-(4-chlor-benzyl)-1-(4-ethylamino-phenyl)-harnstoff |
| 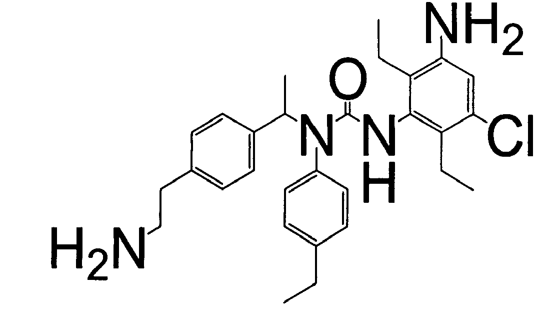 | **137** | 493.3 | 493.2 | C | 3-(3-Amino-5-chlor-2,6-diethyl-phenyl)-1-[4-(2-amino-ethyl)-benzyl]-1-(4-ethyl-phenyl)-harnstoff |
| 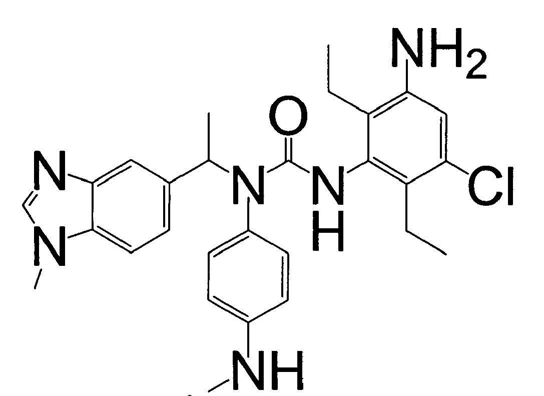 | **138** | 505.2 | 505.8 | B | 3-(3-Amino-5-chlor-2,6-diethyl-phenyl)-1-(4-methylamino-phenyl)-1-[1-(1-methyl-1H-benzoimidazol-5-yl)-ethyl]-harnstoff |
| 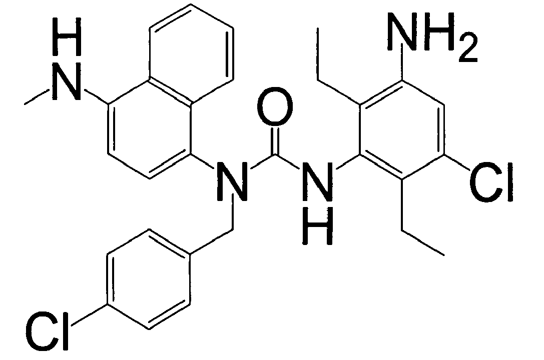 | **139** | 521.2 | 521.9 | C | 3-(3-Amino-5-chlor-2,6-diethyl-phenyl)-1-(4-chlor-benzyl)-1-(4-methylamino-naphthalin-1-yl)-harnstoff |
| 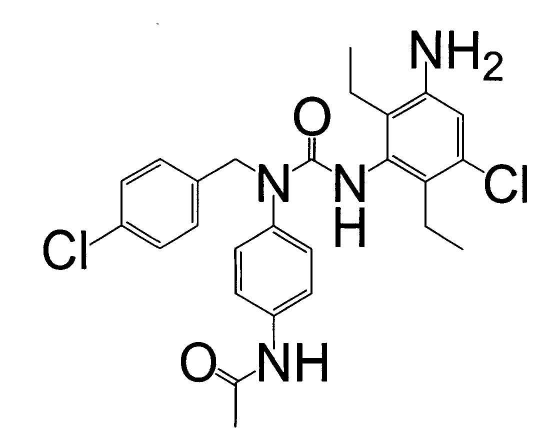 | **140** | 499.2 | 500.1 | C | N-{4-[3-(3-Amino-5-chlor-2,6-diethyl-phenyl)-1-(4-chlor-benzyl)-ureido]-phenyl}-acetamid |
| 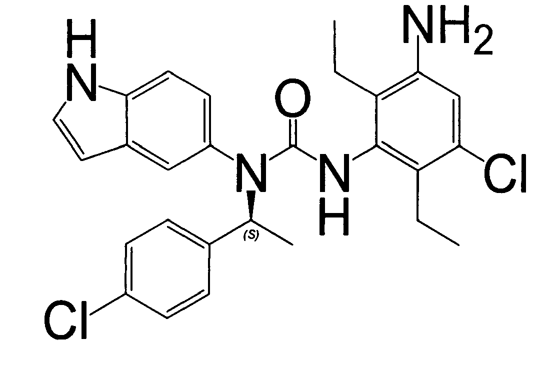 | **141** | 495.2 | 495.6 | A | (S)-3-(3-Amino-5-chlor-2,6-diethyl-phenyl)-1-[1-(4-chlor-phenyl)-ethyl]-1-(1H-indo1-5-yl)-harnstoff |
| 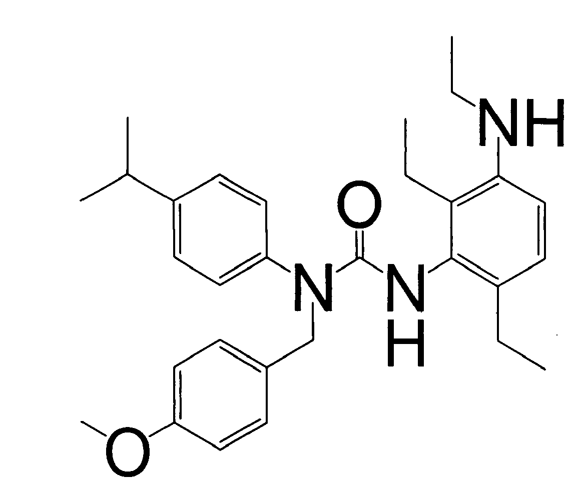 | **142** | 474.3 | 474.3 | D | 3-(2,6-Diethyl-3-ethylamino-phenyl)-1-(4-isopropyl-phenyl)-1-(4-methoxy-benzyl)-harnstoff |

### Beispiel 29: Erhöhung der antagonistischen Aktivität durch die Einführung einer NH₂₋Gruppe

Dass eine NR21R22-Gruppe zu höherer antagonistischer Aktivität führen kann, zeigt ein Vergleich der Verbindungen **24** und **31,** bei denen durch die Einführung einer NH₂-Gruppe in **24** eine Aktivitätserhöhung um den Faktor 6.4 erreicht wird (es ist jeweils der IC₅₀-Wert für die Inhibition C5a-getriggerter Enzymfreisetzung gemäß Beispiel 28 angegeben).

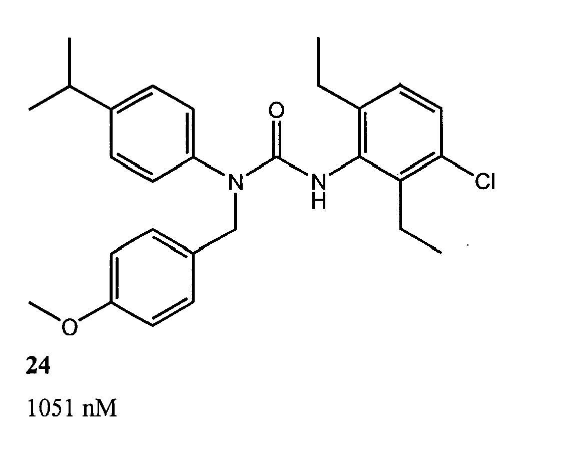

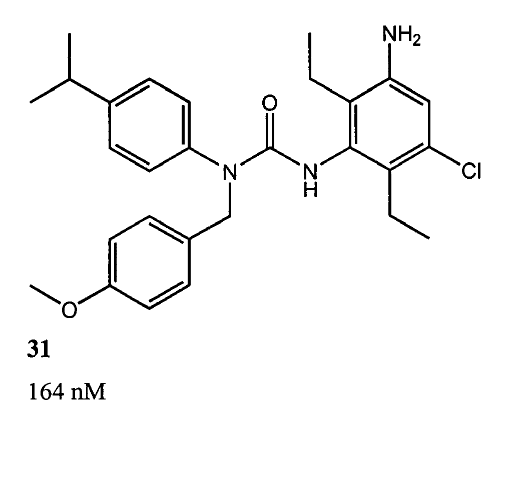

### Beispiel 30: Erhöhung der antagonistischen Aktivität durch die Einführung eines Stereozentrums

Die geeignete Wahl eines Stereozentrums kann sich positiv auf die antagonistische Aktivität eines Moleküls auswirken. Das wird beispielsweise durch die Verbindungen **66, 31** und **56** deutlich, bei denen die Verbindung mit der (S)-Konfiguration **(66)** eine um den Faktor 11 höhere antagonistische Aktivität als die entsprechende (R)-konfigurierte Verbindung **56** aufweist (es ist jeweils der IC₅₀-Wert für die Inhibition C5a-getriggerter Enzymfreisetzung gemäß Beispiel 32 angegeben).

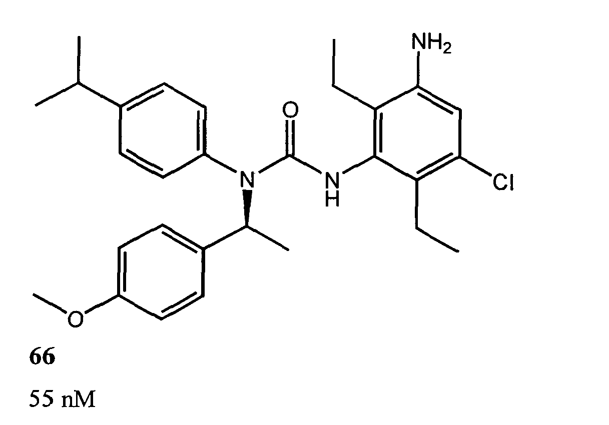

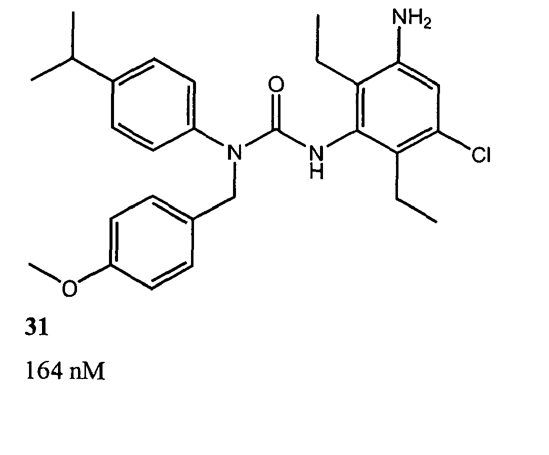

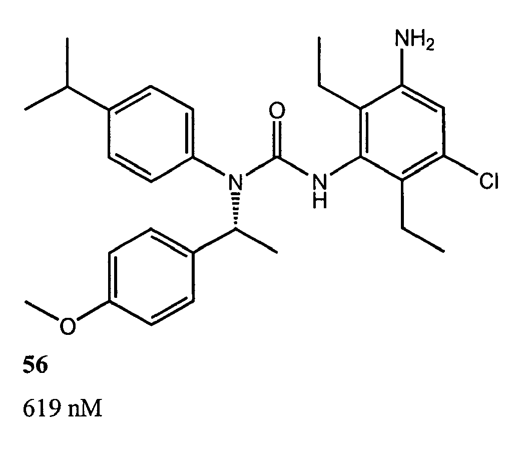

### Beispiel 31: Erhöhung der antagonistischen Aktivität durch die Einführung eines H-Donors

Die vorliegenden Erfinder haben überraschenderweise auch erkannt, dass die Einführung eines H-Donors an der Position von R3 in der Struktur (IV) zu einer Erhöhung der antagonistischen Aktivität führen kann.

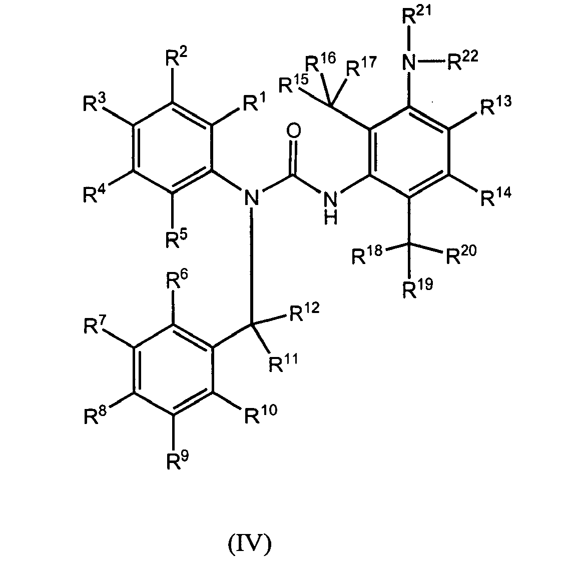

Das wird beispielsweise durch die Verbindungen **98** und **120** deutlich, bei denen durch den Austausch einer CH₂-Gruppe durch eine NH-Gruppe eine Erhöhung der antagonistischen Aktivität um den Faktor 4.5 erreicht wird (es ist jeweils der IC₅₀-Wert für die Inhibition C5a-getriggerter Enzymfreisetzung gemäß Beispiel 32 angegeben).

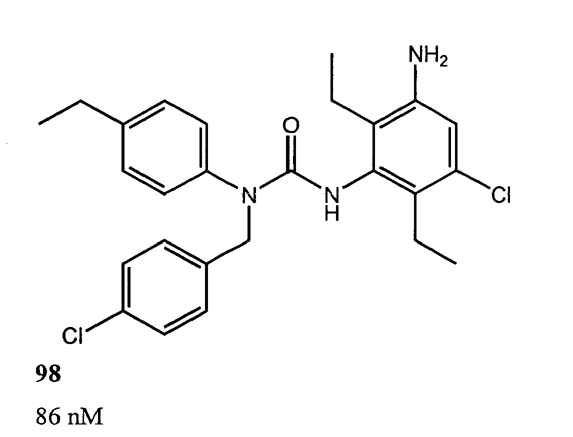

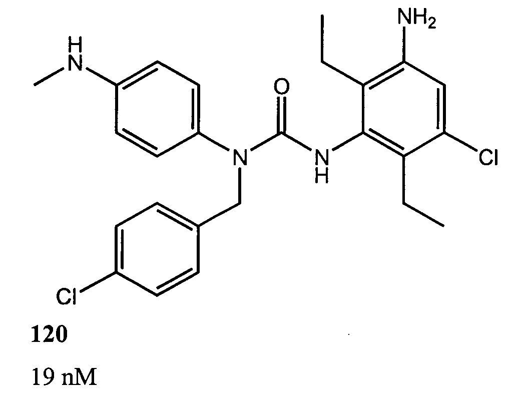

### Beispiel 32: Bestimmung des EC₅₀-Wertes in einem Enzymfreisetzungsassay

Die Bestimmung des EC₅₀-Wertes verläuft vergleichbar mit dem in Beispiel 32 beschriebenen Vorgehen. Einziger Unterschied ist, dass 30 µl der zu testenden Substanzen mit 75 µl der unter Beispiel 28 beschriebenen Zellsuspension gemischt werden. Es erfolgt keine Vorinkubation und keine Zugabe von C5a zur Stimulation der Enzymfreisetzung. Die Ergebnisse für die getesteten Verbindungen sind in Tabelle 2 wiedergegeben. Keine der getesteten Verbindungen in Tabelle 2 zeigt nennenswerte agonistische Aktivität.

**Tabelle 2: Agonismus von verschiedenen Verbindungen**

| Molekülbezeichnung | Eingesetzte Konzentration | Agonismus (% der maximalen Enzymfreisetzung durch C5a induziert) |
|---|---|---|
| 20 | 7.1 µM | 0.06 |
| **31** | 7.1 µM | 0.12 |
| **102** | 7.1 µM | 0.00 |
| **103** | 7.1 µM | 0.00 |
| **106** | 7.1 µM | 0.00 |
| **122** | 7.1 µM | 0.00 |
| **125** | 7.1 µM | 0.00 |
| C5a | 228 nM | 100 |

### Beispiel33: Bestimmung der Löslichkeit von ausgewählten C5a-Rezeptor-Antagonisten

**Zur** Bestimmung der Löslichkeit wird die zu messende Verbindung (z.B. 2 µM einer 5 mM-Lösung in DMSO) jeweils in organischem Lösungsmittel (z.B. 198 µl DMSO) und in wässriger Pufferlösung (z.B. 198 µl HEPES-Puffer) gelöst. Beide Lösungen werden per analytischer HPLC analysiert und die Peakflächen miteinander verglichen, wobei die im organischen Lösungsmittel gelöste Probe als 100%-Wert genommen wird. Die vorgestellten Verbindungen (Tabelle 3) sind in wässrigem HEPES-Puffer deutlich besser löslich als die Verbindung **20.**

**Tabelle 3: Löslichkeit von verschiedenen Verbindungen in wässerigem HEPES-Puffer**

| Molekülbezeichnung | Löslichkeit [µM] |
|---|---|
| **20** | 1 |
| **31** | 2 |
| **80** | 4 |
| **103** | 7 |
| **106** | 83 |
| **122** | 29 |
| **125** | 4 |

### Beispiel 34: Bestimmung der AB-Permeabilität in einem TC-7 basierten Assaysystem

Zu testende Verbindungen werden auf eine Konzentration von 50 µM in HBSS-MES (5 mM, pH 6.5) eingestellt (aus einer 10 mM Stammlösung in 100% DMSO). ¹⁴C-Mannitol (ca. 4 µM) wird zu der Probe gemischt. Diese Lösung wird anschließend zentrifugiert und der Überstand wird zur apikalen Seite einer TC-7 Zellkultur gegeben (Passage 15, in 24 Well Transwell Platte), so dass sich eine DMSO-Konzentration von 1% einstellt. Auf der basolatheralen Seite befindet sich HBSS-HEPES (5 mM, pH 7.4). Anschließend werden die Zellen 120 min bei 37°C inkubiert. Die Integrität der TC-7 Zellschicht wird über das zugegebene Mannitol geprüft (Pₐₚₚ <2.5 10⁻⁶ cm/s). Die Permeabilität Pₐₚₚ [cm/s] ist gleich (V_{R}XC_{R120})/(Δtx,Ax(C_{D,mid}-C_{R,mid})), wobei V_{R} das Volumen der Aufnahmekammer (engl. receiver chamber), C_{R120} die Konzentration der Testverbindung in der Aufnahmekammer nach 120 min, Δt die Inkubationszeit, A die Fläche der TC-7 Zellschicht, C_{D,mid} die midpoint Konzentration der Testsubstanz in der donor chamber und C_{R,mid} die Konzentration der Testverbindung in der receiver chamber ist.

Vorgestellte Verbindungen (Tabelle 4) sind permeabel, was mit einer gewissen Wahrscheinlichkeit auf orale Verfügbarkeit hindeutet.

**Tabelle 4: AB-Permeabilität verschiedener Verbindungen**

| Molekülbezeichnung | AB-Permeabilität (10⁻⁶ cm/s) |
|---|---|
| **102** | 1.1 |
| **106** | 22.3 |
| **122** | 3.5 |
| **125** | 2.9 |

### Beispiel 35: Effektivität der Verbindungen 131 und 80 in einem Modell der C5a-induzierten Neutropänie

Die C5a induzierte Neutropänie ist ein Modell für schockartige Erkrankungen (septischer Schock), in denen u.a. die systemische Wirkung von C5a (z.B. Blutdruckabfall und Neutropänie) eine wichtige Rolle spielt. Das durch C5a ausgelöste Anheften der Neutrophilen an die Gefäßwand führt zu einer Abnahme der Neutrophilenzahl im zirkulierenden Blut (Neutropänie). Dieser Prozess des Rekrutierens von Neutrophilen spielt aber auch bei vielen anderen Erkrankungen eine entscheidende Rolle, wie z.B. beim Reperfusionsschaden. Das Modell wurde u.a. auch von Short et al. (1999 British Journal of Pharmacology 125: 551) beschrieben.
Männliche Gerbils *(Meriones unguiculatus*) werden i.p. mit Ketamine (80 mg/kg) und Xylazine (12 mg/kg) betäubt. Die Tiere werden intubiert und ein Katheter wird in die Jugularvene eingeführt. Es schließt sich die folgende Behandlung an:
1. Die Gerbils werden vorbehandelt mit Vehikel oder erfindungsgemäßer Verbindung **80** (3 mg/kg) bzw. **131** (1 mg/kg) mittels i.v. Infusion. Eine Blutprobe wird eine Minute vorher entnommen.
2. 10 min nach der Infusion der Verbindungen werden die Gerbils mit 100 µg/kg hrC5a i.v. behandelt.
   Blutproben werden kurz vor der hrC5a-Gabe und zu verschiedenen Zeitpunkten danach genommen.
3. Blutproben (ca. 0.2 ml) werden in Lithium-Heparin Röhrchen aus der Jugularvene entnommen. Aus den Blutproben werden Diffentialblutbilder gewonnen.

### Weiße Blutkörperchen:

Weiße Blutkörperchen werden mit einem Hämatologie-Cell-Counter bestimmt.

### Differentialblutbild:

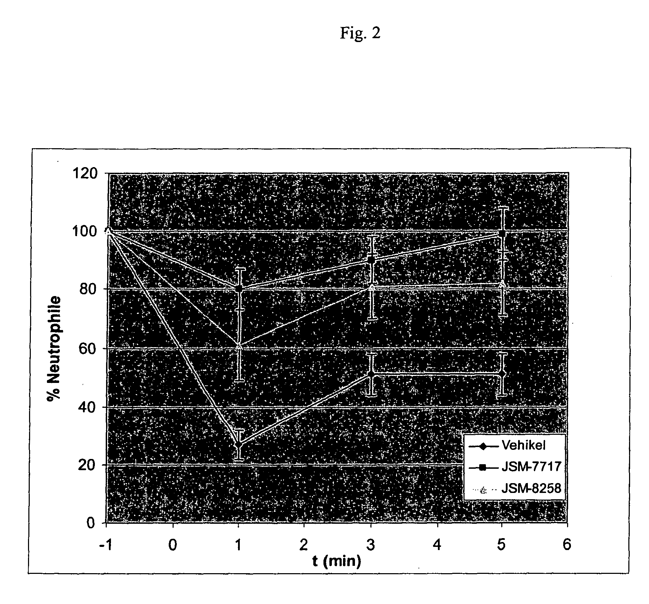

Aus den heparinisierten Blutproben werden Blut-Ausstriche hergestellt. Man dehydriert jede Probe wird vor der Färbung mit Methanol. Nach der Fixierung wird jeder Objekträger mit May Grünwald Färbung für 5 min inkubiert. Danach werden die Objektträger mit dest. Wasser gespült. Anschließend wird mit Giemsa Färbung für 2 min gefärbt und die Objektträger werden für ein weiteres Mal in destilliertem Wasser gewaschen.
Die differentielle Zellzahl wird als Summe von Neutrophilen, Eosinophilen, Easophilen, Lymphocyten and Monocyten aus 100 Zellen bestimmt. Dann wird der Prozentsatz der Neutrophilen an der Zahl aller Weißen Blutkörperchen bestimmt.
Das Ergebnis ist in Fig. 2 dargestellt und zeigt, dass die Gabe der Verbindung **131** und **80** die C5a-induzierte Neutropänie deutlich vermindert und so die gewünschte antiinflammatorische Wirkung in diesem Entzündungsmodell hat. Genauer zeigt Fig. 2 den Verlauf der Neutropänie im Gerbil nach C5a-Gabe und Vehikel bzw. Verbindungsgabe.

### Beispiel 36: Effektivität eines C5a-Rezeptor-Antagonisten allein und in Kombination mit einem BR2-Antagonisten in einem Verbrennungsmodell

Männliche Wistar Ratten werden mit Sodium Pentobarbital (Eutasil™, 60 mg/kg i.p.) betäubt. Die Tiefe der Narkose wird nach Bedarf durch weitere Gabe von Pentobarbital gesteuert. Die Tiere werden auf eine Heizmatte positioniert, die die Körpertemperatur bei 38±1°C konstant hält. Die Tiere werden 6 Stunden nach der Verbrennung getötet. Blut, Urin und Organe werden für die Analyse von Feucht/Trockengewichten, Serum- und Urinparametern entnommen.

Die Verbrennung erfolgt auf 30% der Körperfläche durch Kontakt der rasierten Areale mit 95°C warmen Wassers für 10 Sekunden. Die Kontrolltiere werden mit Wasser in Kontakt gebracht, dass auf Raumtemperatur gebracht wurde.

Die Tiere werden je nach ihrer Zuordnung zu einer Behandlungsgruppe mit einem C5a-Rezeptor-Antagonisten, mit einem Bradykinin-Rezeptor-Antagonisten, mit einer Kombination aus einem Bradykinin-Antagonisten und einem C5a-Rezeptor-Antagonisten oder einer Vehikel-Kontrolle behandelt. Die Behandlung mit einem C5a-Rezeptor-Antagonisten erfolgt 5 min vor (und 3 h nach) oder 15 min nach (und 3 h nach) der Verbrennung. Die Behandlungsgruppen mit einem Bradykinin-Rezeptor-Anagonisten und der Kombination aus Bradykinin-Rezeptor-Antagonisten und C5a-Rezeptor-Antagonisten wird nur nach der Verbrennung getestet. Der Bradykinin-Rezeptor-Antagonist wird ab 15 min nach der Verbrennung über eine Infusion bis zum Versuchsende gegeben.

Die in der vorangehenden Beschreibung, den Beispielen, den Ansprüchen und den Zeichnungen offenbarten Merkmale der Erfindung können sowohl einzeln als auch in beliebiger Kombination zur Verwirklichung der Erfindung in ihren verschiedenen Ausführungsformen wesentlich sein.

## Patentansprüche

1. Verbindung, bevorzugtererweise ein C5a-Rezeptorantagonist, mit der folgenden Struktur (IV): 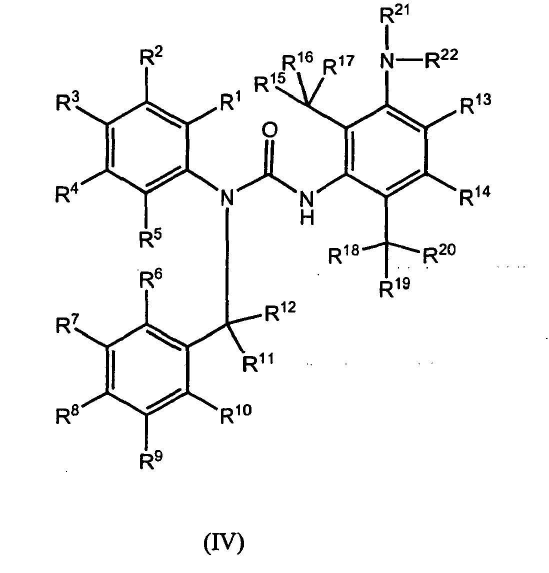 wobei R1, R2, R3, R4, R5, R6, R7, R8, R9, R10, R11, R12, R13, R14, R15, R16, R17, R18, R19, R20, R21 und R22 jeweils einzeln und unabhängig voneinander ausgewählt ist aus der Gruppe, die H, Alkyl, substituiertes Alkyl, Alkenyl, substituiertes Alkenyl, Alkynyl, substituiertes Alkynyl, Cycloalkyl, substituiertes Cycloalkyl, Heterocyclyl, substituiertes Heterocyclyl, Aryl, substituiertes Aryl, Heteroaryl, substituiertes Heteroaryl, Arylalkyl, substituiertes Arylalkyl, Heteroarylalkyl, substituiertes Heteroarylalkyl, Alkoxyl, substituiertes Alkokyl, Aryloxy, substituiertes Aryloxy, Arylalkyloxy, substituiertes Arylalkyloxy, Acyloxy, substituiertes Acyloxy, Halogen, Hydroxyl, Nitro, Cyano, Acyl, substituiertes Acyl, Mercapto, Alkylthio, substituiertes Alkylthio, Amino, substituiertes Amino, Alkylamino, substituiertes Alkylamino, Dialkylamino, substituiertes Dialkylamino, cyclisches Amino, substituiertes cyclisches Amino, Carbamoyl (-CONH₂), substituiertes Carbamoyl, Carboxyl, Carbamat, Alkoxycarbonyl, substituiertes Alkoxycarbonyl, Acylamino, substituiertes Acylamino, Sulfamoyl (-SO₂NH₂), substituiertes Sulfamoyl, Haloalkyl, Haloalkyloxy, -C(O)H, Trialkylsilyl und Azido umfaßt.

2. Verbindung nach Anspruch 1, wobei
R1, R2, R3, R4 und R5 jeweils einzeln und unabhängig voneinander ausgewählt ist aus der Gruppe, die H, Alkyl, substituiertes Alkyl, Alkynyl, Cycloalkyl, Alkoxyl, substituiertes Alkokyl, Acyloxy, Halogen, Nitro, Cyano, Acyl, Alkylthio, substituiertes Alkylthio, Amino, substituiertes Amino, Alkylamino, substituiertes Alkylamino, Dialkylamino, cyclisches Amino, Carbamoyl (-CONH₂), Acylamino, und substituiertes Acylamino umfaßt,
oder der Molekülteil 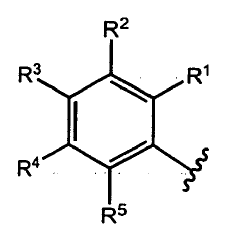 durch einen Molekülteil ersetzt ist, der ausgewählt ist aus der Gruppe, die die Molekülteile 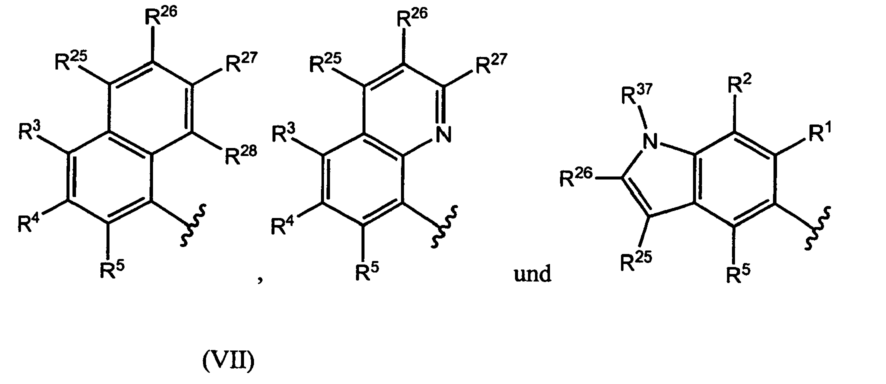 enthält,
wobei R1, R2, R3, R4 und R5 jeweils einzeln und unabhängig voneinander wie oben definiert ist,
wobei R25, R26, R27 und R28 jeweils einzeln und unabhängig voneinander ausgewählt ist aus der Gruppe, die H, Alkyl, substituiertes Alkyl, Alkenyl, substituiertes Alkenyl, Alkynyl, substituiertes Alkynyl, Cycloalkyl, substituiertes Cycloalkyl, Heterocyclyl, substituiertes Heterocyclyl, Aryl, substituiertes Aryl, Heteroaryl, substituiertes Heteroaryl, Arylalkyl, substituiertes Arylalkyl, Heteroarylalkyl, substituiertes Heteroarylalkyl, Alkoxyl, substituiertes Alkokyl, Aryloxy, substituiertes Aryloxy, Arylalkyloxy, substituiertes Arylalkyloxy, Acyloxy, substituiertes Acyloxy, Halogen, Hydroxyl, Nitro, Cyano, Acyl, substituiertes Acyl, Mercapto, Alkylthio, substituiertes Alkylthio, Amino, substituiertes Amino, Alkylamino, substituiertes Alkylamino, Dialkylamino, substituiertes Dialkylamino, cyclisches Amino, substituiertes cyclisches Amino, Carbamoyl (-CONH₂), substituiertes Carbamoyl, Carboxyl, Carbamat, Alkoxycarbonyl, substituiertes Alkoxycarbonyl, Acylamino, substituiertes Acylamino, Sulfamoyl (-SO₂NH₂), substituiertes Sulfamoyl, Haloalkyl, Haloalkyloxy, -C(O)H, Trialkylsilyl und Azido umfasst,
und R37 ausgewählt ist aus der Gruppe, die H, Alkyl, substituiertes Alkyl umfasst.

3. Verbindung nach Anspruch 1,
wobei
R1, R2, R4 und R5 jeweils einzeln und unabhängig voneinander ausgewählt ist aus der Gruppe, die H, Alkyl, Alkoxyl und Halogen umfaßt,
R3 ausgewählt ist aus der Gruppe, die H, Alkyl, substituiertes Alkyl, Alkynyl, Cycloalkyl, Alkoxyl, Acyl, Alkylthio, substituiertes Alkylthio, Alkylamino und substituiertes Alkylamino, umfaßt,
oder der Molekülteil 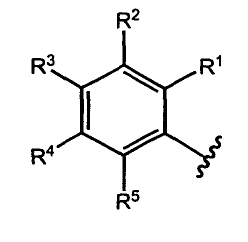 durch einen Molekülteil ersetzt ist, der ausgewählt ist aus der Gruppe, die die Molekülteile 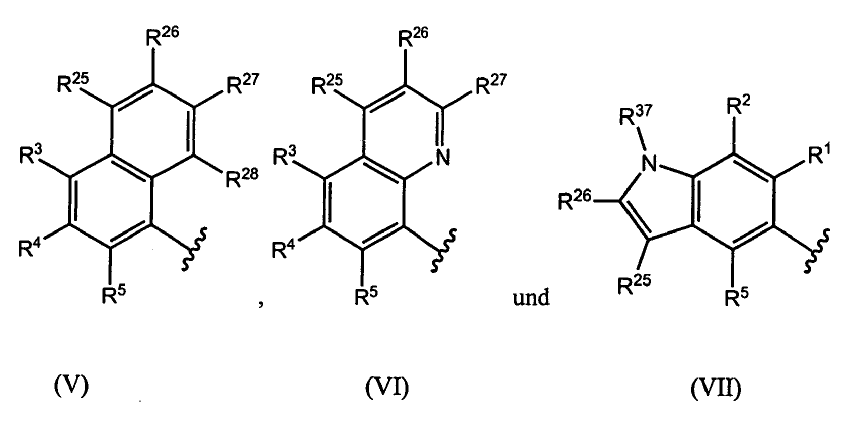 enthält,
wobei R1, R2, R4, R5 und R3 jeweils einzeln und unabhängig voneinander wie oben definiert ist,
wobei R25, R26, R27 und R28 jeweils einzeln und unabhängig voneinander ausgewählt ist aus der Gruppe, die H, Alkyl, Alkoxyl und Halogen umfasst,
und R37 ausgewählt ist aus der Gruppe, die H, Alkyl und substituiertes Alkyl umfasst.

4. Verbindung nach Anspruch 1, wobei
R1, R2, R4 und R5 jeweils einzeln und unabhängig voneinander ausgewählt ist aus der Gruppe, die H, Me, OMe, F, Cl und Br umfaßt,
R3 ausgewählt ist aus der Gruppe, die Et-, iPr-, CF₃CH₂-, Cyclopropyl, HCC-, MeO-, MeS-, CF₃S-, MeNH-, und CF₃NH- umfaßt,
oder der Molekülteil 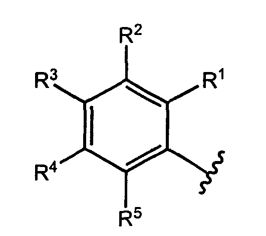 durch einen Molekülteil ersetzt ist, der ausgewählt ist aus der Gruppe, die die Molekülteile 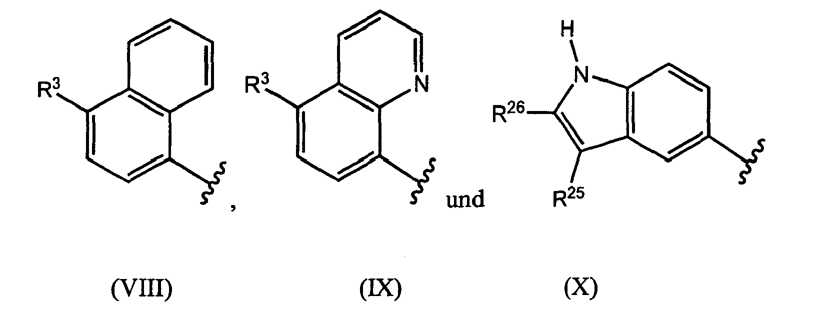 enthält,
wobei R3 wie oben definiert ist,
wobei R25 und R26 jeweils einzeln und unabhängig voneinander ausgewählt ist aus der Gruppe, die H, Me, OMe, F, Cl, Br und CF₃ umfasst.

5. Verbindung nach Anspruch 1, wobei
R6, R7, R8, R9 und R10 jeweils einzeln und unabhängig voneinander ausgewählt ist aus der Gruppe, die H, Alkyl, substituiertes Alkyl, Heterocyclyl, substituiertes Heterocyclyl, Aryl, substituiertes Aryl, Heteroaryl, substituiertes Heteroaryl, Alkoxyl, substituiertes Alkokyl, Acyloxy, Halogen, Nitro, Cyano, Acyl, Alkylthio, substituiertes Alkylthio, Amino, substituiertes Amino, Alkylamino, substituiertes Alkylamino, Dialkylamino, substituiertes Dialkylamino, cyclisches Amino, Carbamoyl (-CONH₂) und Acylamino umfaßt,
oder der Molekülteil 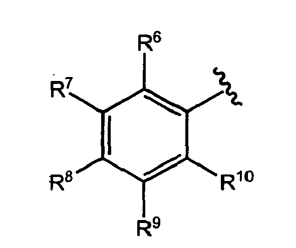 durch den folgenden Molekülteil ersetzt ist 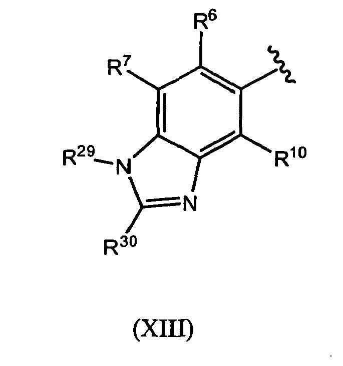 wobei R6, R7 und R10 jeweils einzeln und unabhängig voneinander wie oben definiert ist,
wobei R29 und R30 jeweils einzeln und unabhängig ausgewählt ist aus der Gruppe, die H, Alkyl, substituiertes Alkyl, Heterocyclyl, substituiertes Heterocyclyl, Aryl, substituiertes Aryl, Heteroaryl, substituiertes Heteroaryl, Alkoxyl, substituiertes Alkokyl, Acyloxy, Halogen, Nitro, Cyano, Acyl, Alkylthio, substituiertes Alkylthio, Amino, substituiertes Amino, Alkylamino, substituiertes Alkylamino, Dialkylamino, substituiertes Dialkylamino, cyclisches Amino, Carbamoyl (-CONH₂), und Acylamino umfaßt.

6. Verbindung nach Anspruch 5, wobei
R6, R7, R9 und R10 jeweils einzeln und unabhängig voneinander ausgewählt ist aus der Gruppe, die H, Alkyl, substituiertes Alkyl, Heterocyclyl, substituiertes Heterocyclyl, Aryl, Heteroaryl, Alkoxyl, substituiertes Alkokyl, Halogen, Alkylthio, substituiertes Alkylthio, Amino, substituiertes Aminound cyclisches Amino umfaßt,
R8 ausgewählt ist aus der Gruppe, die H, Alkyl, substituiertes Alkyl, Heterocyclyl, substituiertes Heterocyclyl, Aryl, Heteroaryl, substituiertes Heteroaryl, Alkoxyl, substituiertes Alkokyl, Halogen, Alkylthio, substituiertes Alkylthio, Amino, substituiertes Amino, Alkylamino, substituiertes Alkylamino, Dialkylamino, substituiertes Dialkylamino und cyclisches Amino umfaßt,
oder der Molekülteil 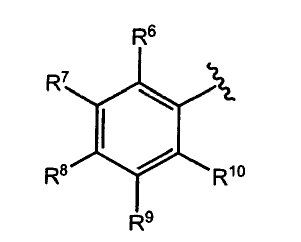 durch den folgenden Molekülteil ersetzt ist 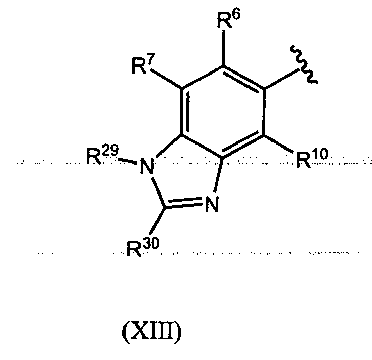 wobei R6, R7 und R10 jeweils einzeln und unabhängig voneinander wie oben definiert ist,
wobei R29 und R30 jeweils einzeln und unabhängig voneinander ausgewählt ist aus der Gruppe, die H, Alkyl, substituiertes Alkyl, Heterocyclyl, substituiertes Heterocyclyl, Aryl, Heteroaryl, Alkoxyl, substituiertes Alkokyl, Halogen, Alkylthio, substituiertes Alkylthio, Amino, substituiertes Amino und cyclisches Amino umfaßt.

7. Verbindung nach Anspruch 5, wobei
R6, R7, R9 und R10 jeweils einzeln und unabhängig voneinander ausgewählt ist aus der Gruppe, die H-, Me-, -CF₃, -OMe, -OCF₃, -F, -Cl, -Br und -SCF₃ umfaßt,
R8 ausgewählt ist aus der Gruppe, die H-, Me-, -CF₃, -OMe, -F, -Cl, -Br, -SMe, -NMe₂ und - NHMe umfaßt,
oder der Molekülteil 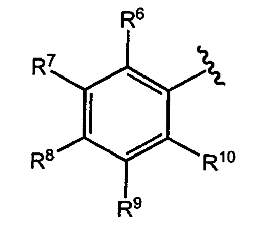 durch den folgenden Molekülteil ersetzt ist 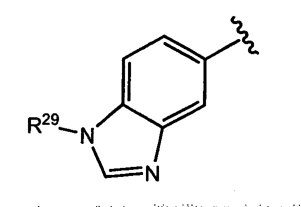 wobei R29 ausgewählt ist aus der Gruppe, die H-, Me-, -CH₂F, CHF₂, -CF₃ und -CF₃ umfaßt.

8. Verbindung nach Anspruch 1, wobei
R11 und R12 jeweils einzeln und unabhängig voneinander ausgewählt ist aus der Gruppe, die H, Alkyl, substituiertes Alkyl und Halogen umfaßt,
oder
R11 und R12 zusammen einen Cycloalkylring bilden,
oder der Molekülteil 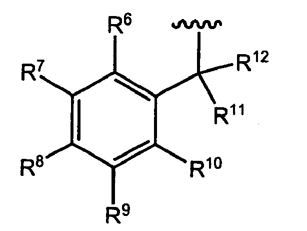 durch den Molekülteil 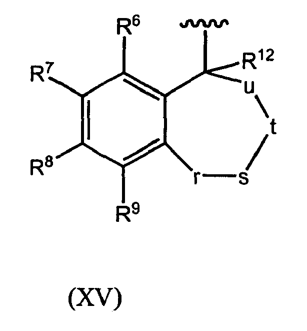 ersetzt ist,
wobei R 12 ausgewählt ist aus der Gruppe, die H, Alkyl, substituiertes Alkyl, und Halogen umfaßt,
wobei r, s, t und u jeweils einzeln und unabhängig ausgewählt-ist aus der Gruppe, die -CH₂-, -O-, -NAlkyl- und -NH- umfasst, oder wobei r, s, t, und u jeweils einzeln und unabhängig für eine chemische Bindung steht.

9. Verbindung nach Anspruch 8, wobei
R1 1 und R12 jeweils einzeln und unabhängig ausgewählt-ist aus der Gruppe, die -H, -Me, -Et, -CF₃, und -F umfaßt,
oder der Molekülteil 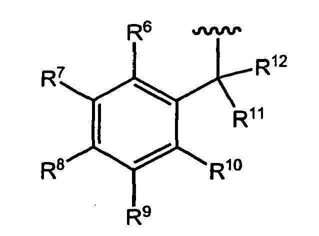 durch den Molekülteil 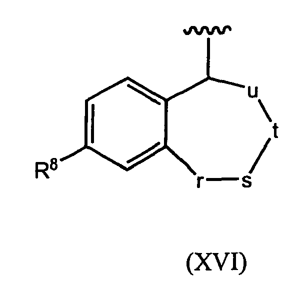 ersetzt ist, wobei r, s, t und u jeweils einzeln und unabhängig ausgewählt ist aus der Gruppe, die -CH₂- und -O- umfasst oder wobei r, s, t, und u jeweils einzeln und unabhängig für eine chemische Bindung steht.

10. Verbindung nach Anspruch 1, wobei
der Molekülteil 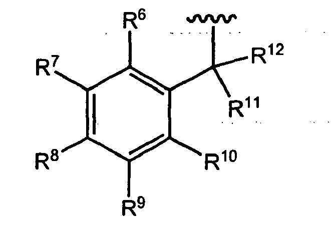 durch den Molekülteil 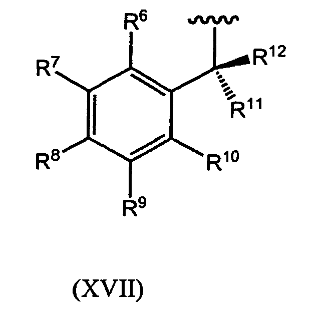 ersetzt ist,
wobei R11 H ist,
wobei R12 ausgewählt ist aus der Gruppe, die H, Alkyl, substituiertes Alkyl und Halogen umfaßt.

11. Verbindung nach Anspruch 10,
wobei R11 H ist, und
wobei R12 ausgewählt ist aus der Gruppe, die -H, -Me, -Et, -CF₃ und -F umfaßt.

12. Verbindung nach Anspruch 1, wobei
R13 und R14 jeweils einzeln und unabhängig voneinander ausgewählt ist aus der Gruppe, die H, Alkyl, substituiertes Alkyl, Alkoxyl, substituiertes Alkokyl, Halogen, Cyano, Alkylthio, Amino und substituiertes Amino umfaßt.

13. Verbindung nach Anspruch 12, wobei R13 und R14 jeweils einzeln und unabhängig voneinander ausgewählt ist aus der Gruppe, die -H, -Me, -CF₃, -OMe, -F, -Cl und -Br umfaßt.

14. Verbindung nach Anspruch 1, wobei
R15, R16, R17, R18, R19 und R20 jeweils einzeln und unabhängig voneinander ausgewählt ist aus der Gruppe, die H, Alkyl, substituiertes Alkyl, Alkoxyl, substituiertes Alkokyl, Acyloxy, Halogen, Alkylthio, substituiertes Alkylthio, Amino, substituiertes Amino und Carbamoyl (-CONH₂) umfaßt,
oder
zwei oder drei der Reste aus der Gruppe umfassend R15, R16 und R17 und/oder aus der Gruppe umfassend R18, R19, und R20 zusammen Alkynyl, substituiertes Alkynyl, Cycloalkyl, substituiertes Cycloalkyl, Heterocyclyl, Aryl, Heteroaryl oder Keto bilden.

15. Verbindung nach Anspruch 14, wobei
R15, R16, R17, R18, R19 und R20 jeweils einzeln und unabhängig voneinander ausgewählt ist aus der Gruppe, die -H, -Me, -CF₃, und -F umfaßt.

16. Verbindung nach Anspruch 1, wobei
R21 und R22 jeweils einzeln und unabhängig voneinander ausgewählt ist aus der Gruppe, die H, Alkyl, substituiertes Alkyl, Acyl, substituiertes Acyl, Alkylthio und substituiertes Alkylthio umfaßt,
oder der Molekülteil 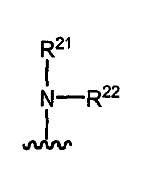 ersetzt ist durch einen Rest der ausgewählt ist aus der Gruppe, die Nitro, Nitroso (NO) und Azido umfaßt.

17. Verbindung nach Anspruch 16, wobei
R21 und R22 jeweils einzeln und unabhängig voneinander ausgewählt ist aus der Gruppe, die -H, -Me und -CF₃ umfaßt,
oder der Molekülteil 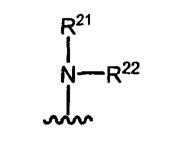 ersetzt ist durch Nitroso (NO).

18. Verbindung, wobei die Verbindung eine der folgenden Strukturen aufweist 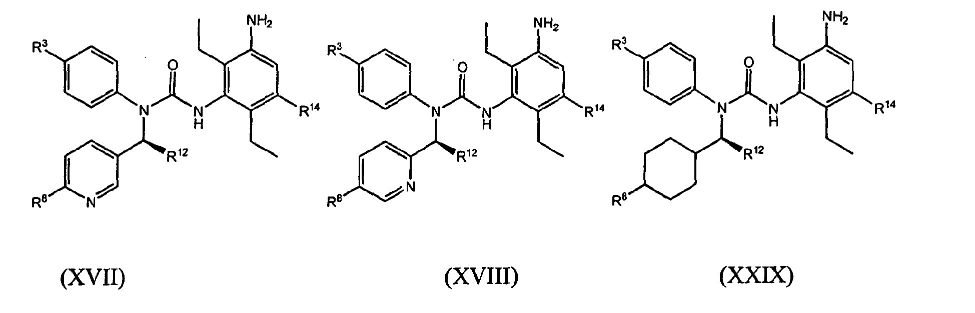 und wobei
R3 ausgewählt ist aus der Gruppe, die Et-, iPr-, CF₃CH₂-, Cyclopropyl, HCC-, MeO-, MeS-, CF₃S-, MeNH- und CF₃NH- umfaßt,
R8 ausgewählt ist aus der Gruppe, die H-, Me-, -CF₃, -OMe, -F, -Cl, -Br, -SMe, -NMe₂ und - NHMe umfaßt,
R12 ausgewählt ist aus der Gruppe, die H- und Me- umfasst, und
R14 ausgewählt ist aus der Gruppe, die H- und -Cl umfaßt.

19. Verbindung nach Anspruch 1, wobei die Verbindung die folgende Struktur aufweist 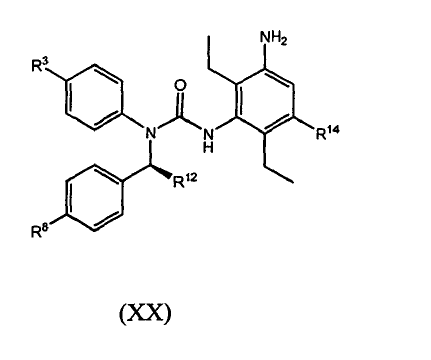 und wobei
R3 ausgewählt ist aus der Gruppe, die Et-, iPr-, CF₃CH₂-, Cyclopropyl, HCC-, MeO-, MeS-, CF₃S-, MeNH- und CF₃NH- umfaßt,
R8 ausgewählt ist aus der Gruppe, die H-, Me-, -CF₃, -OMe, -F, -Cl, -Br, -SMe, -NMe₂, und - NHMe umfaßt,
R12 ausgewählt ist aus der Gruppe, die H- und Me- umfasst, und
R14 ausgewählt ist aus der Gruppe, die H-und -Cl umfaßt,

20. Verbindung nach Anspruch 4, wobei die Verbindung eine der folgenden Strukturen aufweist: 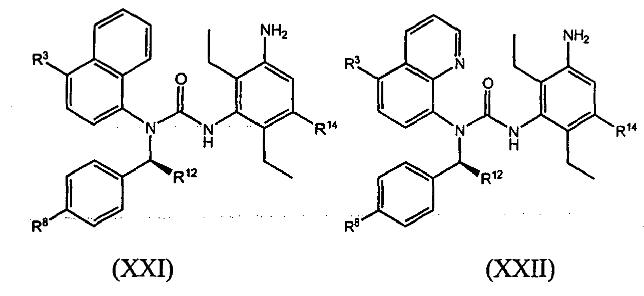 oder 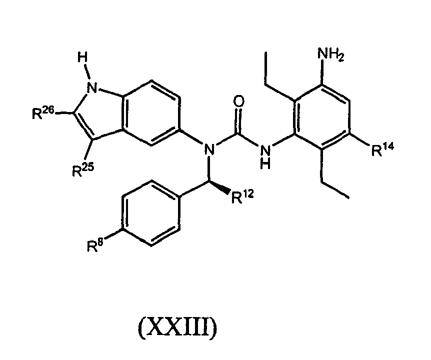 und wobei
R3, R25 und R26 jeweils einzeln und unabhängig voneinander ausgewählt ist aus der Gruppe, die H-, Et-, iPr-, CF₃CH₂-, Cyclopropyl, HCC-, MeO-, MeS-, CF₃S-, MeNH-, CF₃NH- und umfaßt,
R8 ausgewählt ist aus der Gruppe, die H-, Me-, -CF₃, -OMe, -F, -Cl, -Br, -SMe, -NMe₂ und - NHMe umfaßt,
R12 ausgewählt ist aus der Gruppe, die H- und Me- umfasst, und
R14 ausgewählt ist aus der Gruppe, die H-und -Cl umfaßt.

21. Verbindung nach Anspruch 7 oder 9, wobei die Verbindung eine der folgenden Strukturen aufweist: 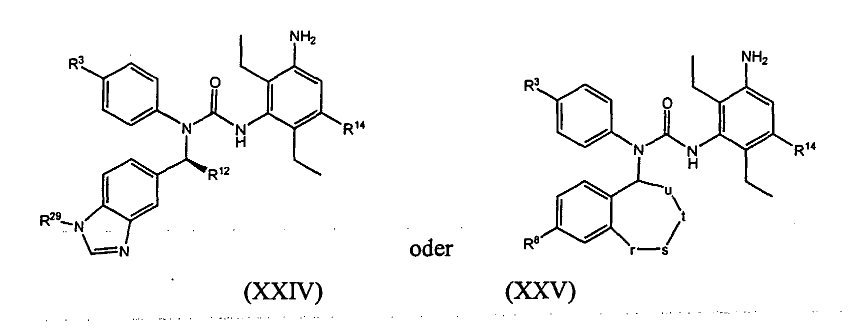 und wobei R3 ausgewählt ist aus der Gruppe, die Et-, iPr-, CF₃CH₂-, Cyclopropyl, HCC-, MeO-, MeS-, CF₃S-, MeNH-und CF₃NH- umfaßt,
R8 ausgewählt ist aus der Gruppe, die H-, Me-, -CF₃, -OMe, -F, -Cl, -Br, -SMe, -NMe₂ und - NHMe umfaßt,
R29 ausgewählt ist aus der Gruppe, die H-, Me-, -CH₂F, CHF₂, -CF₃ und -CF₃ umfaßt.
R14 ausgewählt ist aus der Gruppe, die H- und -Cl umfaßt,
R12 ausgewählt ist aus der Gruppe, die H- und Me- umfasst, und
r, s, t und u jeweils einzeln und unabhängig ausgewählt ist aus der Gruppe, die -CH₂- und -O-umfasst oder wobei r, s, t und u jeweils einzeln und unabhängig für eine chemische Bindung steht.

22. Verbindung nach einem der Ansprüche 1 bis 21, wobei die Verbindung ausgewählt ist aus der Gruppe umfassend
3-(3-Amino-2,6-diisopropyl-phenyl)-1-(4-isopropyl-phenyl)-1-(4-methoxy-benzyl)-harnstoff
3-(3-Amino-2,6-diethyl-phenyl)-1-(4-isopropyl-phenyl)-1-(4-methoxy-benzyl)-harnstoff
2- {2,4-Diethyl-3-[3-(4-isopropyl-phenyl)-3-(4-methoxy-benzyl)-ureido]-phenylamino} - acetamid
{2,4-Diethyl-3-[3-(4-isopropyl-phenyl)-3-(4-methoxy-benzyl)-ureido]-phenylamino}-essigsäuremethylester
3-(3-Amino-5-chlor-2,6-diethyl-phenyl)-1-(4-isopropyl-phenyl)-1-(4-methoxy-benzyl)-harnstoff
3-(3-Amino-5-chlor-2,6-diethyl-phenyl)-1-(4-ethyl-phenyl)-1-(4-methoxy-benzyl)-harnstoff
3-(3-Amino-5-chlor-2,6-diethyl-phenyl)-1-(4-isopropyl-phenyl)-1-(4-trifluormethoxy-benzyl)-harnstoff
3-(3-Amino-5-chlor-2,6-diethyl-phenyl)-1-(4-isopropyl-phenyl)-1-(4-methyl-benzyl)-harnstoff
3-(3-Ammo-5-chlor-2,6-diethyl-phenyl)-1-(4-ethyl-benzyl)-1-(4-isopropyl-phenyl)-harnstoff
3-{3-Chlor-2,6-diethyl-5-[(furan-2-ylmethyl)-amino]-phenyl}-1-(4-isopropyl-phenyl)-1-(4-methoxy-benzyl)-harnstoff
2- {5-Chlor-2,4-diethyl-3-[3-(4-isopropyl-phenyl)-3-(4-methoxy-benzyl)-ureido]-phenylamino}-N,N-dimethyl-acetamid
3- {3-Chlor-2,6-diethyl-5-[(1-methyl-1H-imidazol-2-ylmethyl)-amino]-phenyl}-1-(4-isopropyl-phenyl)-1 -(4-methoxy-benzyl)-hamstoff
3-(3-Amino-2,6-diethyl-phenyl)-1-(4-isopropyl-phenyl)-1-(4-methyl-benzyl)-harnstoff
(R)-3-(3-Amino-5-chlor-2,6-diethyl-phenyl)-1-(4-isopropyl-phenyl)-1-(1,2,3,4-tetrahydro-naphthalen-1-yl)-harnstoff
(*S*)-3-(3-Amino-5-chlor-2,6-diethyl-phenyl)-1-(4-isopropyl-phenyl)-1-(1,2,3,4-tetrahydro-naphthalen-1-yl)-harnstoff
3-(3-Amino-5-chlor-2,6-diethyl-phenyl)-1-benzyl-1-(4-isopropyl-phenyl)-harnstoff
3-(3-Amino-5-chlor-2,6-diethyl-phenyl)-1-indan-5-yl-1-(4-methoxy-benzyl)-harnstoff
2- {5-Chlor-2,4-diethyl-3-[3-(4-isopropyl-phenyl)-3-(4-methoxy-benzyl)-ureido]-phenylamino}-acetamid
3-(3-Amino-5-chlor-2,6-diethyl-phenyl)-1-(4-isopropyl-phenyl)-1-(6-methoxy-pyridin-3-ylmethyl)-harnstoff
3-(3-Amino-2, 6-diethyl-phenyl)-1-(4-isopropyl-phenyl)-1-(6-methoxy-pyridin-3-ylmethyl)-harnstoff
3-(3-Amino-5-chlor-2,6-diethyl-phenyl)- 1-(4-isopropyl-phenyl)- 1 -(4-trifluormethyl-benzyl)-harnstoff
3-(3-Amino-2,6-diethyl-phenyl)-1-(4-isopropyl-phenyl)-1-(4-trifluormethyl-benzyl)-harnstoff
3-(3-Amino-2,6-diethyl-phenyl)-1-(4-isopropyl-phenyl)-1-phenethyl-harnstoff
3-(3-Amino-5-chlor-2,6-diethyl-phenyl)-1-(4-isopropyl-phenyl)-1-(2-p-tolyl-ethyl)-harnstoff
3-(3-Amino-5-chlor-2, 6-diethyl-phenyl)-1-(4-ethyl-phenyl)-1-(4-methyl-benzyl)-harnstoff
3-(3-Amino-2,6-diethyl-phenyl)-1-(4-ethyl-phenyl)-1-(4-methyl-benzyl)-harnstoff
{5-Chlor-2,4-diethyl-3-[3-(4-isopropyl-phenyl)-3-(4-methoxy-benzyl)-ureido]-phenylamino}-essigsäuremethylester
(R)-3 -(3-Amino-5-chlor-2,6-diethyl-phenyl)-1-(4-isopropyl-phenyl)-1-[1-(4-methoxyphenyl)-ethyl]-harnstoff
(R)-3-(3-Amino-5-chlor-2,6-diethyl-phenyl)-1-(4-isopropyl-phenyl)-1-(1-p-tolyl-ethyl)-harnstoff
3-(3-Amino-5-chlor-2, 6-diethyl-phenyl)-1-(2-fluor-4-methoxy-benzyl)-1-(4-isopropylphenyl)-harnstoff
3-(3-Amino-2,6-diethyl-phenyl)-1-(2-fluor-4-methoxy-benzyl)-1-(4-isopropyl-phenyl)-hamstoff
3-(3-Amino-5-chlor-2,6-diethyl-phenyl)-1-cyclohexylmethyl-1-(4-isopropyl-phenyl)-harnstoff
3-(3-Amino-2,6-diethyl-phenyl)-1-cyclohexylmethyl-1-(4-isopropyl-phenyl)-harnstoff
3-(3-Amino-5-chlor-2,6-diethyl-phenyl)-1-(4-isopropyl-phenyl)-1-(2-methoxy-benzyl)-hamstoff
3-(3-Amino-2,6-diethyl-phenyl)-1-(4-isopropyl-phenyl)-1-(2-methoxy-benzyl)-harnstoff
3-(3-Amino-5-chlor-2,6-diethyl-phenyl)-1-(4-isopropyl-phenyl)-1-(4-methanesulfonyl-benzyl)-harnstoff
3-(3-Amino-5-chlor-2,6-diethyl-phenyl)-1-(2,3-dihydro-benzofuran-5-ylmethyl)-1-(4-isopropyl-phenyl)-harnstoff
(S)-3-(3-Amino-5-chlor-2,6-diethyl-phenyl)-1-(4-isopropyl-phenyl)-1-[1-(4-methoxyphenyl)-ethyl]-harnstoff
3-(3-Amino-2,6-diethyl-phenyl)-1-(2,3-dihydro-benzofuran-5-ylmethyl)-1-(4-isopropylphenyl)-harnstoff
(R)-3-(3-Amino-2,6-diethyl-phenyl)-1-(4-isopropyl-phenyl)-1-(1-p-tolyl-ethyl)-harnstoff
(*S*)-3-(3-Amino-2,6-diethyl-phenyl)-1-(4-isopropyl-phenyl)-1-(1-p-tolyl-ethyl)-harnstoff
(R)-3-(3-Amino-2,6-diethyl-phenyl)-1-(4-isopropyl-phenyl)-1- [1-(4-methoxy-phenyl)-ethyl]-harnstoff
(*S*)-3-(3-Amino-2,6-diethyl-phenyl)-1-(4-isopropyl-phenyl)-1-[1-(4-methoxy-phenyl)-ethyl]-harnstoff
3-(3-Amino-5-chlor-2,6-diethyl-phenyl)-1-(4-chlor-benzyl)-1-(4-isopropyl-phenyl)-harnstoff
(*S*)-3-(3-Amino-5-chlor-2,6-diethyl-phenyl)-1-(4-isopropyl-phenyl)-1-(1-p-tolyl-ethyl)-harnstoff
3-(3-Amino-5-chlor-2,6-diethyl-phenyl)-1-(4-isopropyl-phenyl)-1-pyridin-3-ylmethyl-harnstoff
3-(3-Amino-5-chlor-2,6-diethyl-phenyl)-1-(4-isopropyl-phenyl)-1-(5-methyl-pyrazin-2-ylmethyl)-harnstoff
3-(3-Amino-5-chlor-2,6-diethyl-phenyl)-1-(4-isopropyl-phenyl)-1-(6-methyl-pyridin-3-ylmethyl)-harnstoff
3-(3-Amino-5-chlor-2,6-diethyl-phenyl)-1-(2,4-dimethoxy-pyrimidin-5-ylmethyl)-1-(4-isopropyl-phenyl)-harnstoff
(S)-3-(3-Amino-5-chlor-2,6-diethyl-phenyl)-1-[1-(4-chl.or-phenyl)-ethyl]-1-(4-isopropyl-phenyl)-harnstoff
(S)-3-(3-Ammo-5-chlor-2,6-dimethyl-phcnyl)-1-(4-isopropyl-phenyl)-1-(1-p-tolyl-ethyl)-harnstoff
(S)-3-(3-Amino-5-chlor-2,6-diethyl-phenyl)-1-[1-(4-chlor-phenyl)-ethyl]-1-(4-ethyl-phenyl)-harnstoff
(S)-3-(3-Amino-5-chlor-2,6-diethyl-pheny1)-1-[1-(4-fluor-phenyl)-ethyl]-1-(4-isopropylphenyl)-harnstoff
(S)-3-(3-Amino-5-chlor-2,6-diethyl-phenyl)-1-(4-ethyl-phenyl)-1-[1-(4-fluor-phenyl )-ethyl]-harnstoff
(S)-3-(3-Amino-5-chlor-2,6-diethyl-phenyl)-1-[1-(4-chlor-phenyl)-ethyl]-1-(4-methylsulfanyl-phenyl)-harnstoff
3-(3-Amino-5-chlor-2,6-diethyl-phenyl)-1-indan- 1-yl-1-(4-isopropyl-phenyl)-harnstoff
3-(3-Amino-5-chlor-2,6-diethyl-phenyl)-1-(4-ethynyl-phenyl)-1-(4-methoxy-benzyl)-harnstoff
(S)-3-(3-Amino-5-chlor-2,6-diethyl-phenyl)-1-(4-chlor-phenyl)-1-[1-(4-chlor-phenyl)-ethyl]-harnstoff
3-(3-Amino-5-chlor-2,6-diethyl-phenyl)-1-(4-isopropyl-phenyl)-1-(6-trifluormethyl-pyridin-3-ylmethyl)-harnstoff
3-(3-Amino-5-chlor-2,6-diethyl-phenyl)-1-(5-chlor-indan-1-yl)-1-(4-isopropyl-phenyl)-harnstoff
3-(3-Amino-5-chlor-2,6-diethyl-phenyl)-1-chroman-4-yl-1-(4-isopropyl-phenyl)-hamstoff
3-(3-Amino-2, 6-diethyl-phenyl)-1-indan-1-yl-1-(4-isopropyl-phenyl)-harnstoff
3-(3-Amino-5-chlor-2,6-diethyl-phenyl)-1-(4-ethyl-phenyl)-1-(1-ethyl-1H-pyrazol-4-ylmethyl)-harnstoff
(S)-3-(3-Amino-5-chlor-2,6-diethyl-phenyl)-1-[1-(4-chlor-phenyl)-ethyl]-1-(4-methoxyphenyl)-harnstoff
3-(3-Amino-5-chlor-2,6-diethyl-phenyl)-1-(4-chlor-benzyl)-1-(4-trifluormethylsulfanyl-phenyl)-harnstoff
(S)-3-(3-Amino-5-chlor-2,6-diethyl-phenyl)-1-[1-(4-chlor-phenyl)-ethyl]-1-(3-fluor-4-methoxy-phenyl)-harnstoff
(S)-3-(3-Amino-5-chlor-2,6-diethyl-phenyl)-1-[1-(4-chlor-phenyl)-ethyl]-1-(4-methanesulfinyl-phenyl)-harnstoff
3-(3-Amino-5-chlor-2,6-diethyl-phenyl)-1-(4-ethyl-phenyl)-1-(4-fluor-benzyl)-harnstoff
(S)-3-(3-Amino-5-chlor-2,6-diethyl-phenyl)-1-[1-(4-chlor-phenyl)-ethyl]-1-(4-ethyl-3-fluorphenyl)-harnstoff
3-(3-Amino-5-chlor-2,6-diethyl-phenyl)-1-(4-chlor-benzyl)-1-(4-ethyl-phenyl)-harnstoff
3-(3-Amino-5-chlor-2,6-diethyl-phenyl)-1-(4-bromo-benzyl)-1-(4-ethyl-phenyl)-harnstoff
3-(3-Amino-5-chlor-2,6-diethyl-phenyl)-1-(6-chlor-pyridin-3-ylmethyl)-1-(4-isopropylphenyl)-harnstoff
3-(3-Amino-5-chlor-2,6-diethyl-phenyl)-1-(4-isopropyl-phenyl)-1-thiochroman-4-yl-harnstoff
(S)-3-(3-Amino-5-chlor-2,6-diethyl-phenyl)-1-[1-(4-chlor-phenyl)-ethyl]-1-(2-fluor-4-methoxy-phenyl)-harnstoff
3-(3-Amino-5-chlor-2,6-diethyl-phenyl)-1-(4-chlor-benzyl)-1-[4-(2,2,2-trifluor-ethyl)-phenyl]-harnstoff
3-(3-Amino-5-chlor-2,6-diethyl-phenyl)-1-(5-chlor-indan-1-yl)-1-(4-ethyl-phenyl)-harnstoff
3-(3-Amino-5-chlor-2,6-diethyl-phenyl)-1-(5-chlor-pyridin-2-ylmethyl)-1-(4-isopropylphenyl)-harnstoff
3-(3-Amino-5-chlor-2,6-diethyl-phenyl)-1-(4-ethyl-phenyl)-1-(6-methyl-pyridin-3-ylmethyl)-harnstoff
3-(3-Amino-2,6-diethyl-phenyl)-1-(4-ethyl-phenyl)-1-(6-methyl-pyridin-3-ylmethyl)-harnstoff
3-(3-Amino-5-chlor-2,6-diethyl-phenyl)-1-(4-chlor-benzyl)-1-naphthalen-1-yl-harnstoff
(S)-3-(3-Amino-5-chlor-2,6-diethyl-phenyl)-1-[1-(4-chlor-phenyl)-ethyl]-1-(2-hydroxymethyl-4-methoxy-phenyl)-harnstoff
3-(3-Amino-5-chlor-2,6-diethyl-phenyl)-1-(4-ethyl-phenyl)-1-(4-pyrazol-1-yl-benzyl)-harnstoff
(S)-3-(3-Amino-6-tert-butyl-2-methyl-phenyl)-1-[1-(4-chlor-phenyl)-ethyl)-1-(4-ethylphenyl)-harnstoff
3-(3-Amino-5-chlor-2,6-diethyl-phenyl)-1-(4-ethyl-phenyl)-1-(2-nitro-1-phenyl-ethyl)-harnstoff
3-(3-Amino-5-chlor-2,6-diethyl-phenyl)-1-(4-cyano-benzyl)-1-(4-ethyl-phenyl)-harnstoff
3-(3-Amino-5-chlor-2,6-diethyl-phenyl)-1-(4-chlor-benzyl)-1-(3,4-dimethoxy-phenyl)-harnstoff
3-(3-Amino-5-chlor-2,6-diethyl-phenyl)-1-(4-chlor-benzyl)-1-(4-methoxy-naphthalen-1-yl)-harnstoff
1-(4-Amino-benzyl)-3-(3-amino-5-chlor-2,6-diethyl-phenyl)-1-(4-ethyl-phenyl)-harnstoff
3-(3-Amino-5-chlor-2,6-diethyl-phenyl)-1-(4-chlor-benzyl)-1-(4-difluormethoxy-phenyl)-harnstoff
3-(3-Amino-5-chlor-2,6-diethyl-phenyl)-1-(4-ethyl-phenyl)-1-(1-methyl-1H-benzoimidazol-5-ylmethyl)-harnstoff
3-(3-Amino-2,6-diethyl-phenyl)-1-(4-chlor-benzyl)-1-(4-methylamino-phenyl)-harnstoff
3-(3-Amino-5-chlor-2,6-diethyl-phenyl)-1-(4-chlor-benzyl)-1-(4-methylamino-phenyl)-harnstoff
3-(3-Amino-5-chlor-2,6-diethyl-phenyl)-1-(2-dimethylamino-pyrimidin-5-ylmethyl)-1-(4-ethyl-phenyl)-harnstoff
3-(3-Amino-5-chlor-2,6-diethyl-phenyl)-1-(4-ethyl-phenyl)-1-[1-(1-methyl-1H-benzoimidazol-5-yl)-ethyl]-harnstoff
3-(3-Amino-5-chlor-2,6-diethyl-phenyl)-1-(4-ethyl-phenyl)-1-[1-(6-methyl-pyridin-3-yl)-ethyl]-harnstoff
3-(3-Amino-5-chlor-2,6-diethyl-phenyl)-1-(2-fluor-4-methoxy-phenyl)-1-(6-methyl-pyridin-3-ylmethyl)-harnstoff
3-(3-Amino-5-chlor-2,6-diethyl-phenyl)-1-(4-chlor-benzyl)-1-(5-methoxy-chinolin-8-yl)-harnstoff
3-(3-Amino-5-chlor-2,6-diethyl-phenyl)-1-(6-methyl-pyridin-3-ylmethyl)-1-[4-(2,2,2-trifluorethyl)-phenyl]-harnstoff
3-(3-Amino-5-chlor-2,6-diethyl-phenyl)-1-(4-chlor-benzyl)-1-(4-dimethylamino-phenyl)-harnstoff
3-(3-Amino-5-chlor-2,6-diethyl-phenyl)-1-benzyl-1-(4-dimethylamino-phenyl)-harnstoff
3-(3-Amino-5-chlor-2,6-diethyl-phenyl)-1-benzyl-1-(4-methylamino-phenyl)-harnstoff
3-(3-Amino-5-chlor-2,6-diethyl-phenyl)-1-(4-chlor-benzyl)-1-(8-methoxy-2,3-dimethyl-chinoxalin-5-yl)-harnstoff
3-(3-Amino-5-chlor-2,6-diethyl-phenyl)-1-[1-(4-chlor-phenyl)-ethyl]-1-(4-methylamino-phenyl)-harnstoff
3-(3-Amino-5-chlor-2,6-diethyl-phenyl)-1-(4-methylamino-phenyl)-1-(1 1-phenyl-ethyl)-harnstoff
3-(3-Chlor-2,6-diethyl-5-nitroso-phenyl)-1-[1-(4-chlor-phenyl)-ethyl]-1-(4-methylamino-phenyl)-harnstoff
3-(3-Amino-5-chlor-2,6-diethyl-phenyl)-1-(4-methoxy-naphthalen-1-yl)-1-(6-methyl-pyridin-3-ylmethyl)-harnstoff
3-(3-Amino-5-chlor-2,6-diethyl-phenyl)-1-(4-chlor-benzyl)-1-(4-ethylamino-phenyl)-harnstoff
3-(3-Amino-5-chlor-2,6-diethyl-phenyl)-1-[4-(2-amino-ethyl)-benzyl]-1-(4-ethyl-phenyl)-harnstoff
3-(3-Amino-5-chlor-2,6-diethyl-phenyl)-1-(4-methylamino-phenyl)-1 -[1-(1-methyl-1H-benzoimidazol-5-yl)-ethyl]-harnstoff
3-(3-Amino-5-chlor-2, 6-diethyl-phenyl)-1-(4-chlor-benzyl)-1-(4-methylamino-naphthalen-1-yl)-harnstoff
N-{4-[3-(3-Amino-5-chlor-2,6-diethyl-phenyl)-1-(4-chlor-benzyl)-ureido]-phenyl}-acetamid
3-(3-Amino-5-chlor-2,6-diethyl-phenyl)-1-[1-(4-chlor-phenyl)-ethyl]-1-(H-indol-5-yl)-harnstoff

23. Verbindung nach Anspruch 22, wobei die Verbindung ausgewählt ist aus der Gruppe umfassen
3-(3-Amino-2,6-diisopropyl-phenyl)-1-(4-isopropyl-phenyl)-1-(4-methoxy-benzyl)-harnstoff
3-(3-Amino-2,6-diethyl-phenyl)-1-(4-isopropyl-phenyl)-1-(4-methoxy-benzyl)-harnstoff
3-(3-Amino-5-chlor-2,6-diethyl-phenyl)-1-(4-isopropyl-phenyl)-1-(4-methoxy-benzyl)-harnstoff
3-(3-Amino-5-chlor-2,6-diethyl-phenyl)-1 -(4-ethyl-phenyl)-1 -(4-methoxy-benzyl)-hamstoff
3-(3-Amino-5-chlor-2,6-diethyl-phenyl)-1-(4-isopropyl-phenyl)-1-(4-methyl-benzyl)-harnstoff
3-(3-Amino-5-chlor-2,6-diethyl-phenyl)-1-(4-ethyl-benzyl)-1-(4-isopropyl-phenyl)-harnstoff
3-(3-Amino-2,6-diethyl-phenyl)-1-(4-isopropyl-phenyl)-1-(4-methyl-benzyl)-harnstoff
(R)-3-(3-Amino-5-chlor-2,6-diethyl-phenyl)-1-(4-isopropyl-phenyl)-1-(1,2,3,4-tetrahydro-naphthalen-1-yl)-harnstoff
(S)-3-(3-Amino-5-chlor-2,6-diethyl-phenyl)-1-(4-isopropyl-phenyl)-1-(1,2,3,4-tetrahydro-naphthalen-1-yl)-harnstoff
3-(3-Amino-5-chlor-2,6-diethyl-phenyl)-1-benzyl-1-(4-isopropyl-phenyl)-harnstoff
3-(3-Amino-5-chlor-2,6-diethyl-phenyl)-1-(4-isopropyl-phenyl)-1-(6-methoxy-pyridin-3-ylmethyl)-harnstoff
3-(3-Amino-5-chlor-2,6-diethyl-phenyl)-1-(4-isopropyl-phenyl)-1-(4-trifluormethyl-benzyl)-harnstoff
3-(3-Amino-2,6-diethyl-phenyl)-1-(4-isopropyl-phenyl)-1-(4-trifluormethyl-benzyl)-harnstoff
3-(3-Amino-5-chlor-2,6-diethyl-phenyl)-1-(4-isopropyl-phenyl)-1-(2-p-tolyl-ethyl)-harnstoff
3-(3-Amino-5-chlor-2,6-diethyl-phenyl)-1-(4-ethyl-phenyl)-1-(4-methyl-benzyl)-harnstoff
3-(3-Amino-2,6-diethyl-phenyl)-1-(4-ethyl-phenyl)-1-(4-methyl-benzyl)-harnstoff
(R)-3-(3-Amino-5-chlor-2, 6-diethyl-phenyl)-1-(4-isopropyl-phenyl)-1-[1-(4-methoxyphenyl)-ethyl]-harnstoff
(R)-3-(3 -Amino-5-chlor-2, 6-diethyl-phenyl)-1-(4-isopropyl-phenyl)-1-(1-p-tolyl-ethyl)-harnstoff
3-(3-Amino-5-chlor-2,6-diethyl-phenyl)-1-(2-fluor-4-methoxy-benzyl)-1-(4-isopropylphenyl)-harnstoff
3-(3-Amino-2,6-diethyl-phenyl)-1-(2-fluor-4-methoxy-benzyl)-1-(4-isopropyl-phenyl)-harnstoff
3-(3-Amino-2,6-di ethyl-phenyl)-1-cyc lohexylmethyl-1-(4-i sopropyl-phenyl)-harnstoff
3-(3 -Amino-5-chlor-2,6-diethyl-phenyl)-1-(4-isopropyl-phenyl)-1-(2-methoxy-benzyl)-harnstoff
3-(3-Amino-5-chlor-2,6-diethyl-phenyl)-1-(2,3-dihydro-benzokran-5-ylmethyl)-1-(4-isopropyl-phenyl)-harnstoff
(S)-3-(3-Amino-5-chlor-2,6-diethyl-phenyl)-1-(4-isopropyl-phenyl)-1-[1-(4-methoxyphenyl)-ethyl]-harnstoff
3-(3-Amino-2,6-diethyl-phenyl)-1-(2, 3-dihydro-benzofuran-5-ylmethyl)-1-(4-isopropylphenyl)-harnstoff
(S)-3-(3-Amino-2,6-diethyl-phenyl)-1-(4-isopropyl-phenyl)-1-(1-p-tolyl-ethyl)-hamstoff
(S)-3-(3-Amino-2,6-diethyl-phenyl)-1-(4-isopropyl-phenyl)-1-[1-(4-methoxy-phenyl)-ethyl]-harnstoff
3-(3-Amino-5-chlor-2,6-diethyl-phenyl)-1-(4-chlor-benzyl)-1-(4-isopropyl-phenyl)-harnstoff
(S)-3-(3-Amino-5-chlor-2,6-diethyl-phenyl)-1-(4-isopropyl-phenyl)-1-(1-p-tolyl-ethyl)-harnstoff
3-(3-Amino-5-chlor-2,6-diethyl-phenyl)-1-(4-isopropyl-phenyl)-1-pyridin-3-ylmethyl-harnstoff
3-(3-Amino-5-chlor-2,6-diethyl-phenyl)-1-(4-isopropyl-phenyl)-1-(5-methyl-pyrazin-2-ylmethyl)-harnstoff
3-(3-Amino-5-chlor-2,6-diethyl-phenyl)-1-(4-isopropyl-phenyl)-1-(6-methyl-pyridin-3-ylmethyl)-harnstoff
3-(3-Amino-5-chlor-2,6-diethyl-phenyl)-1-(2,4-dimethoxy-pyrimidin-5-ylmethyl)-1-(4-isopropyl-phenyl)-harnstoff
(S)-3-(3-Amino-5-chlor-2,6-diethyl-phenyl)-1-[1-(4-chlor-phenyl)-ethyl]-1-(4-isopropylphenyl)-harnstoff
(S)-3-(3-Amino-5-chlor-2,6-dimethyl-phenyl)-1-(4-isopropyl-phenyl)-1-(1-p-tolyl-ethyl)-harnstoff
(S)-3-(3-Amino-S-chlor-2,6-diethyl-phenyl)-1-[1-(4-chlor-phenyl)-ethyl]-1-(4-ethyl-phenyl)-harnstoff
(S)-3-(3-Amino-5-chlor-2,6-diethyl-phenyl)-1-[1-(4-fluor-phenyl)-ethyl]-1-(4-isopropylphenyl)-harnstoff
(S)-3-(3-Amino-5-chlor-2,6-diethyl-phenyl)-1-(4-ethyl-phenyl)-1-[1-(4-fluor-phenyl)-ethyl]-harnstoff
(S)-3-(3-Amino-5-chlor-2,6-diethyl-phenyl)-1-[1-(4-chlor-phenyl)-ethyl]-1-(4-methylsulfanyl-phenyl)-harnstoff
3-(3-Amino-5-chlor-2,6-diethyl-phenyl)-1-indan-1-yl-1-(4-isopropyl-phenyl)-harnstoff
3-(3-Amino-5-chlor-2,6-diethyl-phenyl)-1-(4-ethynyl-phenyl)-1-(4-methoxy-benzyl)-harnstoff
(S)-3-(3-Amino-5-chlor-2,6-diethyl-phenyl)-1-(4-chlor-phenyl)-1-[1-(4-chlor-phenyl)-ethyl]-harnstoff
3-(3-Amino-5-chlor-2,6-diethyl-phenyl)-1-(4-isopropyl-phenyl)-1-(6-trifluormethyl-pyridin-3-ylmethyl)-harnstoff
3-(3-Amino-5-chlor-2,6-diethyl-phenyl)-1-(5-chlor-indan-1-yl)-1-(4-isopropyl-phenyl)-harnstoff
3-(3-Amino-5-chlor-2,6-diethyl-phenyl)-1-chroman-4-yl-1-(4-isopropyl-phenyl)-harnstoff
3-(3-Amino-2,6-diethyl-phenyl)-1-indan-1-yl-1-(4-isopropyl-phenyl)-harnstoff
3-(3-Amino-5-chlor-2,6-diethyl-phenyl)-1-(4-ethyl-phenyl)-1-(1-ethyl-1H-pyrazol-4-ylmethyl)-harnstoff
(S)-3-(3-Amino-5-chlor-2,6-diethyl-phenyl)-1-[1-(4-chlor-phenyl)-ethyl]-1-(4-methoxyphenyl)-harnstoff
3-(3-Amino-5-chlor-2,6-diethyl-phenyl)-1-(4-chlor-benzyl)-1-(4-trifluormethylsulfanyl-phenyl)-harnstoff
(S)-3-(3-Amino-S-chlor-2,6-diethyl-phenyl)-1-[1-(4-chlor-phenyl)-ethyl]-1-(3-fluor-4-methoxy-phenyl)-harnstoff
(S)-3-(3-Amino-5-chlor-2,6-diethyl-phenyl)-1-[1-(4-chlor-phenyl)-ethyl]-1-(4-methanesulfinyl-phenyl)-harnstoff
3-(3-Amino-5-chlor-2, 6-diethyl-phenyl)-1-(4-ethyl-phenyl)-1-(4-fluor-benzyl)-harnstoff
(S)-3-(3-Amino-5-chlor-2,6-diethyl-phenyl)-1-[1-(4-chlor-phenyl)-ethyl]-1-(4-ethyl-3-fluorphenyl)-harnstoff
3-(3-Amino-5-chlor-2,6-diethyl-phenyl)-1-(4-chlor-benzyl)-1-(4-ethyl-phenyl)-harnstoff
3-(3-Amino-5-chlor-2,6-diethyl-phenyl)-1-(4-bromo-benzyl)-1-(4-ethyl-phenyl)-harnstoff
3-(3-Amino-5-chlor-2,6-diethyl-phenyl)-1-(6-chlor-pyridin-3-ylmethyl)-1-(4-isopropylphenyl)-harnstoff
(S)-3-(3-Amino-5-chlor-2,6-diethyl-phenyl)-1-[1-(4-chlor-phenyl)-ethyl]-1-(2-fluor-4-methoxy-phenyl)-harnstoff
3-(3-Amino-5-chlor-2,6-diethyl-phenyl)-1-(4-chlor-benzyl)-1-[4-(2,2,2-trifluor-ethyl)-phenyl]-harnstoff
3-(3-Amino-5-chlor-2,6-diethyl-phenyl)-1-(5-chlor-indan-1-yl)-1-(4-ethyl-phenyl)-harnstoff
3-(3-Amino-5-chlor-2,6-diethyl-phenyl)-1-(5-chlor-pyridin-2-ylmethyl)-1-(4-isopropylphenyl)-harnstoff
3-(3-Amino-5-chlor-2,6-diethyl-phenyl)-1-(4-ethyl-phenyl)-1-(6-methyl-pyridin-3-ylmethyl)-harnstoff
3-(3-Amino-2,6-diethyl-phenyl)-1-(4-ethyl-phenyl)-1-(6-methyl-pyridin-3-ylmethyl)-harnstoff
3-(3-Amino-5-chlor-2,6-diethyl-phenyl)-1-(4-chlor-benzyl)-1-naphthalen-1-yl-hamstoff
(S)-3-(3-Amino-6-tert-butyl-2-methyl-phenyl)-1-[1-(4-chlor-phenyl)-ethyl]-1-(4-ethylphenyl)-harnstoff
3-(3-Amino-5-chlor-2,6-diethyl-phenyl)-1-(4-cyano-benzyl)-1-(4-ethyl-phenyl)-harnstoff
3-(3-Amino-5-chlor-2,6-diethyl-phenyl)- 1 -(4-chlor-benzyl)- 1 -(3,4-dimethoxy-phenyl)-harnstoff
3-(3-Amino-5-chlor-2,6-diethyl-phenyl)-1-(4-chlor-b enzyl)-1-(4-methoxy-naphthalen-1-yl)-harnstoff
3-(3-Amino-5-chlor-2, 6-diethyl-phenyl)-1-(4-chlor-benzyl)-1-(4-difluormethoxy-phenyl)-harnstoff
3-(3-Amino-5-chlor-2, 6-diethyl-phenyl)-1-(4-ethyl-phenyl)-1-(1-methyl-1 H-benzoimidazol-5-ylmethyl)-harnstoff
3-(3-Amino-2,6-diethyl-phenyl)-1-(4-chlor-benzyl)-1-(4-methylamino-phenyl)-harnstoff
3-(3-Amino-5-chlor-2,6-diethyl-phenyl)-1-(4-chlor-benzyl)-1-(4-methylamino-phenyl)-harnstoff
3-(3-Amino-5-chlor-2,6-diethyl-phenyl)-1-(4-ethyl-phenyl)-1-[1-(1-methyl-1H-benzoimidazol-5-yl)-ethyl]-harnstoff
3-(3-Amino-5-chlor-2,6-diethyl-phenyl)-1-(4-ethyl-phenyl)-1-[1-(6-methyl-pyridin-3-yl)-ethyl]-harnstoff
3-(3-Amino-5-chlor-2,6-diethyl-phenyl)-1-(2-fluor-4-methoxy-phenyl)-1-(6-methyl-pyridin-3-ylmethyl)-harnstoff
3-(3-Amino-5-chlor-2,6-diethyl-phenyl)-1-(4-chlor-benzyl)-1-(5-methoxy-chinolin-8-yl)-harnstoff
3-(3-Amino-5-chlor-2,6-diethyl-phenyl)-1-(6-methyl-pyridin-3-ylmethyl)-1-[4-(2,2,2-trifluorethyl)-phenyl]-harnstoff
3-(3-Amino-5-chlor-2,6-diethyl-phenyl)-1-(4-chlor-benzyl)-1-(4-dimethylamino-phenyl)-harnstoff
3-(3-Amino-5-chlor-2,6-diethyl-phenyl)-1-benzyl-1-(4-methylamino-phenyl)-harnstoff
3-(3-Amino-5-chlor-2,6-diethyl-phenyl)-1-[1-(4-chlor-phenyl)-ethyl]-1-(4-methylamino-phenyl)-harnstoff
3-(3-Amino-5-chlor-2,6-diethyl-phenyl)-1-(4-methylamino-phenyl)-1-(1-phenyl-ethyl)-harnstoff
3-(3-Chlor-2,6-diethyl-5-nitroso-phenyl)-1-[1-(4-chlor-phenyl)-ethyl]-1-(4-methylamino-phenyl)-harnstoff
3-(3-Amino-5-chlor-2,6-diethyl-phenyl)-1-(4-methoxy-naphthalen-1-yl)-1-(6-methyl-pyridin-3-ylmethyl)-harnstoff
3-(3-Amino-5-chlor-2,6-diethyl-phenyl)-1-(4-chlor-benzyl)-1-(4-ethylamino-phenyl)-harnstoff
3-(3-Amino-5-chlor-2,6-diethyl-phenyl)-1-[4-(2-amino-ethyl)-benzyl]-1-(4-ethyl-phenyl)-harnstoff
3-(3-Amino-5-chlor-2,6-diethyl-phenyl)-1-(4-methylamino-phenyl)-1-[1-(1-methyl-1H-benzoimidazol-5-yl)-ethyl]-harnstoff
N- {4-[3-(3-Amino-5-chlor-2,6-diethyl-phenyl)-1-(4-chlor-benzyl)-ureido]-phenyl}-acetamid
3-(3-Amino-5-chlor-2,6-diethyl-phenyl)-1-[1-(4-chlor-phenyl)-ethyl]- 1-(1H-indol-5-yl)-harnstoff

24. Pharmazeutische Zusammensetzung umfassend mindestens eine Verbindung gemäß einem der vorangehenden Ansprüchen und zusätzlich ein pharmazeutisch akzeptables Trägermittel.

25. Verwendung mindestens einer Verbindung nach einem der vorangehenden Ansprüche zur Herstellung eines Medikamentes.

26. Verwendung nach Anspruch 25, **dadurch gekennzeichnet, dass** das Medikament für die Prävention und/oder Behandlung einer Erkrankung verwendet wird, bei der das Komplementsystem aktiviert ist und/oder bei der die Inhibierung des Komplementsystems eine Linderung der Symptome hervorruft.

27. Verwendung nach Anspruch 25, **dadurch gekennzeichnet, dass** das Medikament für die Prävention und/oder Behandlung einer Erkrankung verwendet wird, bei der die Inhibierung der Aktivierung des C5a Rezeptors allein und/oder in Kombination mit anderen Therapeutika eine Linderung der Symptome hervorruft.

28. Verwendung nach Anspruch 25, 26 oder 27, **dadurch gekennzeichnet, dass** die Erkrankung und/oder die zu behandelnden Symptome ausgewählt sind aus der Gruppe umfassend Autoimmunerkrankungen, akute und chronische inflammatorische Erkrankungen, Traumata, lokale Entzündungen, Schock und Verbrennungen.

29. Verwendung nach einem der Ansprüche 25, 26 oder 27, **dadurch gekennzeichnet, dass** die Erkrankungen schwere Verbrennungen sind.

30. Verwendung nach Anspruch 25, 26 oder 27**dadurch gekennzeichnet, dass** die Erkrankung durch Verbrennungen verursachte Folgeschäden sind, wobei die Folgeschäden Organversagen, Schock, SIRS (severe/systemic inflammatory response syndrome), Sepsis, Ödembildung, Komplikationen bei der operativen Entfernung von angegriffenen Hautarealen und Vemarbung von Haut oder Organen umfassen.

31. Verwendung nach einem der Ansprüche 25 bis 28, **dadurch gekennzeichnet, dass** die Erkrankung ausgewählt ist aus der Gruppe umfassend septischer Schock, hämorrhagischer Schock, SIRS (systemic/severe inflammatory response syndrom), MOF (Multiorganversagen), akute respiratorische Insuffizienz (ARDS), Gehirnschlag, Herzinfarkt, Reperfusionsschaden und akute Verletzungen des zentralen Nervensystems.

32. Verwendung nach Anspruch 31, **dadurch gekennzeichnet**, das der Reperfusionsschaden an einem oder mehreren Organen, Organsystemen oder Körperteilen auftritt, wobei diese ausgewählt sind aus der Gruppe umfassend Leber, Niere, Darm, Lunge, Herz, Milz, Harnblase, Magen, Muskeln, Haut, Extremitäten, Gehirn und Bauchspeicheldrüse.

33. Verwendung nach Anspruch 31 oder 32, **dadurch gekennzeichnet, dass** akute Folgen und/oder die chronischen Folgen eines Reperfusionsschaden behandelt werden, wobei bevorzugterweise akute Folgen das akute Organversagen oder die Bildung nekrotischer Bereiche sind, und bevorzugterweise chronische Folgen Veränderungen wie die dilatative Kardiomyopathie oder Fibrosen, bevorzugtererweise Narbenbildung nach einem Trauma, Narbenbildung beim Herzinfarkt oder bei der Transplantation sind, wobei die Folgen auch bevorzugterweise, eine eingeschränkte Organfunktion sind.

34. Verwendung nach Anspruch 33 **dadurch gekennzeichnet, dass** der Reperfusionsschaden nach einem Myokardinfarkt auftritt.

35. Verwendung nach Anspruch 33 **dadurch gekennzeichnet, dass** der Reperfusionsschaden an der Niere auftritt.

36. Verwendung nach Anspruch 33, **dadurch gekennzeichnet, dass** der Reperfusionsschaden nach oder während einer Aneurismaoperation auftritt.

37. Verwendung nach einem der Ansprüche 25 bis 28, **dadurch gekennzeichnet, dass** die Erkrankung ausgewählt ist aus der Gruppe umfassend Asthma, Myokarditis, entzündliche Darmerkrankungen (IBD: inflammatory bowel disease; Morbus Crohn und Colitis ulcerosa), entzündliche Erkrankungen des Auges, Glomerulonephritis, entzündliche Gefäßerkrankungen, lokale Manifestationen systemischer Erkrankungen.

38. Verwendung nach Anspruch 37, **dadurch gekennzeichnet, dass** die entzündlichen Erkrankungen des Auges aus der Gruppe ausgewählt ist, die Uveitis, altersabhängige Makulardegenration, diabetische Retinopathie, diabetisches makulares Ödem, okularen Pemphigoid, Keratoconjunctivitis, Stevens-Johnson Syndrom und Graves Ophthalmophatie umfasst.

39. Verwendung nach Anspruch 37, **dadurch gekennzeichnet, dass** die entzündliche Erkrankung des Auges die altersabhängige Makulardegeneration ist.

40. Verwendung nach Anspruch 37, **dadurch gekennzeichnet, dass** die Erkrankung eine lokale Manifestation systemischer Erkrankungen ist, wobei die systemische Erkrankung aus der Gruppe ausgewählt ist, die Rheuma, SLE und Typ I und Typ II Diabetes umfasst.

41. Verwendung nach einem der Ansprüche 37 oder 40, **dadurch gekennzeichnet, dass** die Manifestation ausgewählt ist aus der Gruppe die Manifestationen am Auge, am oder im Gehirn, an den Gefäßen, am Herzen, an der Lunge, an den Nieren, an der Leber, des gastrointestinalen Traktes, der Milz, der Haut, am Knochensystem, am lymphatischen System und im Blut ausgewählt ist.

42. Verwendung nach Anspruch 28, **dadurch gekennzeichnet, dass** die chronisch inflammatorische Erkrankung eine Autoimmunkrankheit ist, wobei die Autoimmunkrankheit bevorzugterweise aus der Gruppe ausgewählt ist umfassend Alopecia areata, autoimmun-hämolytische Anämie (AIHA) vom Kältetyp (Kälteagglutininkrankheit), autoimmun-hämolytische Anämie (AIHA) vom Wärmetyp, pernizöse Anämie (Morbus Biermer, Addison Anämie), Antiphospholipid-Syndrom (APS), Arteriitis temporalis, Atherosklerose, Autoimmun-Adrenalitis (autoimmune Nebennierenrinden-Atrophie, Morbus Addison), chronisches Erschöpfungssyndrom (CFIDS), chronisch-inflammatorische, demyelinisierende Polyneuropathie, Churg-Strauss Syndrom, Cogan-Syndrom, Colitis ulcerosa, CREST-Syndrom, Diabetes mellitus Typ I, Dermatitis Herpetiformis During, Dermatomyositis, Fibromyalgie, chronisch autoimmune Gastritis, Goodpasture Syndrom (Anti-GBM vermittelte Glomerulonephritis), Guillain-Barré-Syndrom (GBS; Polyradikuloneuritis), Hashimoto Thyroiditis, autoimmune Hepatitis, idiopathische pulmonale Fibrose, Immun-thrombozytopenische Purpura (Morbus Werlhof), autoimmune Infertilität, autoimmune Innenohrschwerhörigkeit (AIED), juvenile rheumatoide Arthritis, autoimmune Kardiomyopathie, Lambert-Eaton Syndrom, Lichen selerosis, Lupus erythematodes insbesondere die diskoide Form, Lyme-Arthritis (Lyme-Krankheit), Mischkollagenose, Morbus Basedow (Graves Disease), Morbus Behçet, Morbus Bechterew (Spondylitis ankylosans), Morbus Ménière, Morbus Reiter, Multiple Sklerose (MS, Encephalomyelitis), Myasthenia gravis (Myasthenie), sympathische Ophtalmie, vernarbendes Pemphigoid, bullöses Pemphigoid, Pemphigus vulgaris, Polyarteriitis nodosa, Polychondritis (Panchondritis), Polyglanduläres Autoimmun-(PGA)-Syndrom, Polymyalgia rheumatica, Polymoysitis, Primäre biliäre Zirrhose (primäre Autoimmun-Cholangitis), Psoriasis, rheumatisches Fieber, rheumatoide Arthritis, Sarkoidose (Morbus Boeck, Besnier-Boeck-Schaumann Krankheit), Sjörgensen-Syndrom, Sklerodermie, Sprue/Zöliakie, Stiff-Man-Syndrom (Moersch-Woltmann-Syndrom), systemischer Lupus erythematodes, Takayasu Arteriitis (Aortenbogen-Syndrom), transiente Gluten-Intoleranz, Urticaria, autoimmune Uveitis, Vaskulitiden und Vitiligo (Weißfleckkrankheit).

43. Verwendung nach Anspruch 37, **dadurch gekennzeichnet, dass** die entzündliche Gefäßerkrankung aus der Gruppe ausgewählt ist, die Vaskulitis, Vascular Leakage und Artherosklerose umfasst.

44. Verwendung nach Anspruch 43, **dadurch gekennzeichnet, dass** die Vaskulitis ausgewählt ist aus der Gruppe umfassend primäre Vaskulitis und sekundäre Vaskulitis.

45. Verwendung nach Anspruch 44, **dadurch gekennzeichnet, dass** die primäre Vaskulitis eine solche ist, die aus der Gruppe von Vaskulitiden ausgewählt ist, die Morbus Wegener, das Churg-Strauss-Syndrom und mikroskopische Polyangiitis umfasst.

46. Verwendung nach Anspruch 44, **dadurch gekennzeichnet, dass** die sekundäre Vaskulitits eine solche ist, die aus der Gruppe von Vaskulitiden ausgewählt ist, die durch Medikamente induzierte Vaskulitiden und durch andere Erkrankungen hervorgerufene Vaskultiden umfasst.

47. Verwendung nach Anspruch 46, **dadurch gekennzeichnet, dass** die anderen Erkrankungen ausgewählt sind aus der Gruppe umfassend AIDS, Hepatitis B, Hepatitis C und Cytomegalie-Virus-Infektion.

48. Verwendung nach Anspruch 42, **dadurch gekennzeichnet, dass** die Urticaria aus der Gruppe ausgewählt ist, die spontane und physikalische Urticaria sowie besondere Formen der Urticaria umfasst.

49. Verwendung nach Anspruch 48, **dadurch gekennzeichnet, dass** die physikalische Urticaria aus der Gruppe ausgewählt wird, die Urticaria factitia, Kälteurticaria, Hitzeurticaria, Druckurticaria und Lichturticaria umfasst.

50. Verwendung nach Anspruch 48, **dadurch gekennzeichnet, dass** die spontane Urticaria aus der Gruppe ausgewählt wird, die akute Urticaria und chronische Urticaria umfasst.

51. Verwendung nach Anspruch 48, **dadurch gekennzeichnet, dass** die spontane Urticaria **dadurch gekennzeichnet ist, dass** bei den betroffenen Patienten Autoantikörper gegen IgE oder den IgE-Rezeptor nachzuweisen sind.

52. Verwendung nach Anspruch 48, **dadurch gekennzeichnet, dass** die besonderen Formen der Urticaria cholinergische Urticaria, adrenergische Urticaria, Kontakturticaria und Urticaria, die durch Wasser ausgelöst wird (aquagenic urticaria), sind.

53. Verwendung nach einem der Ansprüche 25 bis 27 **dadurch gekennzeichnet, dass** das Medikament zur Prävention und/oder Unterstützung chirurgischer Eingriffe verwendet wird.

54. Verwendung nach Anspruch 53, **dadurch gekennzeichnet, dass** das Medikament bzw. die Verbindung zur Unterstützung und/oder zur Prävention und/oder Nachsorge eines chirurgischen Eingriffs verwendet werden, wobei der chirurgische Eingriff ausgewählt ist aus der Gruppe, die CABG, PCTA, PTA, MidCAB, OPCAB, Thrombolyse, Organtransplantation, Aneurysmaoperationen und Gefäßverschluss (clamping) umfasst, bevorzugterweise ist ein Aspekt dabei, die bei extrakorporaler Zirkulation (z.B. Herzlungen-Maschine) möglicherweise folgenden neurokognitiven Dysfunktionen zu vermindern bzw. zu verhindern.

55. Verwendung nach einem der Ansprüche 25 bis 27, **dadurch gekennzeichnet, dass** das Medikament als Unterstützung für die thrombolytische Behandlung verwendet wird.

56. Verwendung nach einem der Ansprüche 25 bis 27 , **dadurch gekennzeichnet, dass** das Medikament im Rahmen einer Dialyse-Behandlung, gegebenenfalls vor, während oder danach, verwendet wird.

57. Verwendung nach einem der Ansprüche 25 bis 27 , **dadurch gekennzeichnet, dass** das Medikament zur Vorbeugung von Schädigungen eines transplantierten und/oder zu transplantierenden Organs verwendet wird.

58. Verwendung nach einem der Ansprüche 25 bis 27, **dadurch gekennzeichnet, dass** das Medikament zur Konservierung oder Unterstützung der Konservierung von Organen, die für die Transplantation bestimmt sind, verwendet werden kann.

59. Verwendung nach einem der Ansprüche 25 bis 27, **dadurch gekennzeichnet, dass** das Medikament zur Vorbeugung oder Behandlung von Abstoßungsreaktionen eines transplantierten Organs verwendet wird.

60. Verwendung nach einem der Ansprüche 57 bis 59 **dadurch gekennzeichnet, dass** das transplantierte bzw. zu transplantierende Organ ausgewählt ist aus der Gruppe, die Nieren, Leber, Lunge, Herz, Haut, Hornhaut, Bauchspeicheldrüse und Darm umfasst.

61. Verwendung nach einem der Ansprüche 57 bis 60, **dadurch gekennzeichnet, dass** es sich bei dem Organ um eine Eigenspende, bevorzugterweise um eine Eigenspende von Haut zur Behandlung bei Brandverletzten oder um eine Eigenspende von Blut handelt.

62. Verwendung nach einem der Ansprüche 25 bis 27 **dadurch gekennzeichnet, dass** das Medikament zur Vorbeugung von fibrotischen Ereignissen eingesetzt wird, bevorzugtererweise zur Behandlung zur Verhinderung oder Verminderung der Bildung von Narbengewebe.

63. Verwendung nach Anspruch 62 wobei das fibrotische Ereignis in einem oder mehreren der folgenden Organe auftreten kann: Leber, Lunge, Nieren, Haut, Herz und anderen Organen.

64. Verwendung nach einem der Ansprüche 25 bis 27, **dadurch gekennzeichnet, dass** das Medikament zur Vorbeugung oder Behandlung der IgA-Nephropathie verwendet wird.

65. Verwendung einer Verbindung nach einem der Ansprüche 1 bis 23 zur kosmetischen Behandlung eines menschlichen oder tierischen Körpers.

66. Verwendung nach Anspruch 37, **dadurch gekennzeichnet, dass** die entzündliche Darmerkrankung ausgewählt aus der Gruppe umfassend Morbus Crohn oder Ulcerative colitis ist.

67. Verwendung nach einem der Ansprüche 25 bis 27, **dadurch gekennzeichnet, dass** die Erkrankung durch intrazelluläre Parasiten oder Viren hervorgerufen wird.

68. Verwendung nach Anspruch 67, **dadurch gekennzeichnet, dass** die intrazellulären Parasiten aus der Gruppe ausgewählt sind umfassend Leischmanien, Rickettsien, Chlamydien, Coxiella, Plasmodien, Brucella, Mycobakterien, Listerien, Toxoplasmen und Trypanosomen.

69. Verwendung nach Anspruch 27, **dadurch gekennzeichnet, dass** die Prävention und/oder Behandlung mit einem Medikament wie in einem der Ansprüche 25 bis 68 definiert in Kombination mit einem oder mehreren antiinflammatorischen und/oder einem oder mehreren immunsupressiven Therapeutika erfolgt.

70. Verwendung nach Anspruch 27, **dadurch gekennzeichnet, dass** die Prävention und/oder Behandlung mit einem Medikament wie in einem der Ansprüche 25 bis 68 definiert in Kombination mit einem oder mehreren immunsuppressiven Therapeutika erfolgt.

71. Verwendung nach Anspruch 70, **dadurch gekennzeichnet, dass** die Kombinationen besteht aus einem Medikament wie in einem der Ansprüche 25 bis 68 definiert und einem Medikament ausgewählt aus der Gruppe umfassend Calcineurin-Inhibitoren oder einem Medikament umfassend eine oder mehrere Substanzen ausgewählt aus der Gruppe umfassend Cyclosporin A, Methotrexate, Azathioprin, FK506 (Tacrolimus), Rapamycin, Leflunomid, Mycophenolatmofetil, Brequinar, Mizoribin, Thalidomid oder Deoxyspergualin.

72. Verwendung nach Anspruch 27, **dadurch gekennzeichnet, dass** die Prävention und/oder Behandlung mit einem Medikament wie in einem der Ansprüche 25 bis 68 definiert in Kombination mit einem oder mehreren Antihistaminika erfolgt.

73. Verwendung nach Anspruch 72, **dadurch gekennzeichnet, dass** das Antihistaminikum ausgewählt ist aus der Gruppe, die Meclozin, Clemastin, Dimetinden, Bamipin, Ketotifen, Cetirizin, Lovecetirizin, Loratidin, Desloratidin, Azelastin, Mizolastin, Levocabastin, Terfenadin, Fexofenadin und Ebastin umfasst.

74. Verwendung nach Anspruch 27, **dadurch gekennzeichnet, dass** die Prävention und/oder Behandlung mit einem erfindungsgemäßen Medikament in Kombination mit einem oder mehreren Glukokortikoiden erfolgt.

75. Verwendung nach Anspruch 74, **dadurch gekennzeichnet, dass** das Glukokortikoid ausgewählt ist aus der Gruppe, die Betamethason, Effervescent, Budesonide, Kortison, Dexamethason Elixir, Hydrokortison, Methylprednisolon, Prednisolon, Prednison und Triamcinolone umfasst.

76. Verwendung nach Anspruch 27, **dadurch gekennzeichnet, dass** die Prävention und/oder Behandlung mit einem Medikament wie in einem der Ansprüche 25 bis 68 definiert in Kombination mit einem oder mehreren Antibiotika erfolgt.

77. Verwendung nach Anspruch 76, **dadurch gekennzeichnet, dass** das Antibiotikum ausgewählt ist aus der Gruppe, die Amynoglykoside, β-Lactam-Antibiotika, Glycopeptidantibiotika, Gyrasehemmer, Lincosamide, Makrolidantibiotika, Nitroimidazolderivate, Polypeptidantibiotika, Sulfonamide, Trimethoprim und Tetracycline umfasst.

78. Verwendung nach Anspruch 27, **dadurch gekennzeichnet, dass** die Prävention und/oder Behandlung mit einem Medikament wie in einem der Ansprüche 25 bis 68 definiert in Kombination mit einem oder mehreren antiinflammatorischen Biologika erfolgt.

79. Verwendung nach Anspruch 78, **dadurch gekennzeichnet, dass** das antiinflammatorische Therapeutikum ausgewählt ist aus der Gruppe, die IL-10, Erlizumab, TolerMab, Rituximab, Gomiliximab, Basiliximab, Daclizumab, HuMax-TAC, Visilizumab, HuMaxCD4, Clenoliximab, MAX 16H5, TNX 100, Toralizumab, Alemtuzumab, CY 1788, Galiximab, Pexelizumab, Eculizumab, ETI 104, FG 3019, Bertilimumab, 249417 (anti-Faktor IX), Abciximab, YM 337, Omalizumab, Talizumab, Fontolizumab, J695 (anti-IL12), HuMax IL-15, Mepolizumab, Elsilimomab, HuDREG, Adalimumab, Infliximab, Certolizumab, Afelimomab, CytoFab, AME 527, Vapaliximab, Avastin, Vitaxin, Belimumab, MLN 1202, Volociximab, F200 (anti-α5β1), Efalizumab, m60.11 (anti-CD11b), Etanercept, Onerecept, Natalizumab und Siplizumab umfasst.

80. Verwendung nach Anspruch 27, **dadurch gekennzeichnet, dass** die Prävention und/oder Behandlung mit einem Medikament wie in einem der Ansprüche 25 bis 68 definiert in Kombination mit der photodynamischen Therapie mit Visodyne erfolgt.

81. Verwendung nach Anspruch 27, **dadurch gekennzeichnet, dass** die Erkrankung AMD ist und die Prävention und/oder Behandlung der AMD mit einem Medikament wie in einem der Ansprüche 25 bis 68 definiert in Kombination mit einem Medikament erfolgt, das ausgewählt ist aus der Gruppe umfassend Visodyne, VEGF-Inhibitoren und α5β 1-Inhibitoren.

82. Verwendung nach Anspruch 27, **dadurch gekennzeichnet, dass** die Prävention und/oder Behandlung mit einem Medikament wie in einem der Ansprüche 25 bis 68 definiert in Kombination mit einem Medikament ausgewählt aus der Gruppe umfassend Acetylsalicylsäure, Ibuprofen, Diclofenac und Naproxen erfolgt.

83. Verwendung nach Anspruch 27, **dadurch gekennzeichnet, dass** die Prävention und/oder Behandlung mit einem Medikament wie in einem der Ansprüche 25 bis 68 definiert in Kombination mit einem Medikament erfolgt, das aus der Gruppe ausgewählt ist, die Antagonisten des Bradykininrezeptors 1 und Antagonisten des Bradykininrezeptors 2 umfasst.

84. Verwendung nach Anspruch 83, **dadurch gekennzeichnet, dass** die Prävention und/oder Behandlung der Behandlung von akuten inflammatorischen Erkrankungen dient, wobei die Erkrankung ausgewählt ist aus der Gruppe umfassend Sepsis, schwere Verbrennungen, Reperfusionsschaden, Myokardinfarkt, Organabstoßung und hämorrhagischer Schock.

85. Verwendung nach Anspruch 84, **dadurch gekennzeichnet, dass** die Prävention und/oder Behandlung der Behandlung von chronischen Autoimmunerkrankungen und/oder der Behandlung von Infektionserkrankungen dient.

86. Kombination aus einem Antagonisten des Bradykininrezeptors 2 und einem C5a-Rezeptor-Antagonisten zur Therapie schwerer Verbrennungen.
